# EUROPEAN PATENT SPECIFICATION

(11) **EP 3 909 946 B1**
(45) Date of publication and mention of the grant of the patent: **17.12.2025**
(21) Application number: 21169952.5
(22) Date of filing: 22.04.2021
(51) Int. Cl.: C07D 307/77, C07D 333/50, C07D 405/12, C07D 409/12, C07D 493/10, C07D 495/10

(54) **ORGANIC ELECTROLUMINESCENCE DEVICE AND POLYCYCLIC COMPOUND FOR ORGANIC ELECTROLUMINESCENCE DEVICE**
ORGANISCHE ELEKTROLUMINESZENZVORRICHTUNG UND POLYCYCLISCHE VERBINDUNGEN FÜR ORGANISCHE ELEKTROLUMINESZENZVORRICHTUNG
DISPOSITIF ÉLECTROLUMINESCENT ORGANIQUE ET COMPOSÉS POLYCYCLIQUES POUR DISPOSITIF ÉLECTROLUMINESCENT ORGANIQUE

(30) Priority: 11.05.2020 KR 20200056177
(43) Date of publication of application: 17.11.2021
(60) Divisional application: 23201879.6
(73) Proprietor: Samsung Display Co., Ltd., Yongin-si, Gyeonggi-Do 17113 (KR)
(72) Inventor: UNO, Takuya, Yokohama (JP)
(74) Representative: Russell, Tim

(56) References cited:
- EP-A1- 3 466 926
- EP-A2- 3 670 510
- WO-A1-2018/016786
- WO-A1-2018/169261
- WO-A1-2019/124903
- WO-A1-2019/160335
- WO-A1-2019/190101
- WO-A1-2020/009398
- CN-A- 108 658 932
- CN-A- 108 947 902
- KR-A- 20190 130 341
- KR-A- 20190 133 617
- US-A1- 2016 351 816
- US-A1- 2017 092 869
- US-A1- 2017 125 869
- US-A1- 2017 237 013
- US-A1- 2018 186 764
- US-A1- 2019 229 271
- US-A1- 2020 203 620

## Description

### BACKGROUND

### 1. Field

One or more aspects of embodiments of the present disclosure relate to an organic electroluminescence device and a polycyclic compound for an organic electroluminescence device.

### 2. Description of Related Art

Organic electroluminescence displays are being actively developed as image displays. An organic electroluminescence display differs from a liquid crystal display, and is a so-called self-luminescent display, in which holes and electrons respectively injected from a first electrode and a second electrode recombine in an emission layer, and a light-emitting material comprising an organic compound in the emission layer emits light to attain display.

In the application of organic electroluminescence devices (or organic electroluminescence display(s)) to a display device, a decreased driving voltage, and increased emission efficiency and/or lifespan of the organic electroluminescence devices are desired, and materials for an organic electroluminescence device stably attaining these characteristics are desired.

US2018186764A1 relates to an organic compound represented by Chemical Formula 1, an organic optoelectronic diode, and a display device.

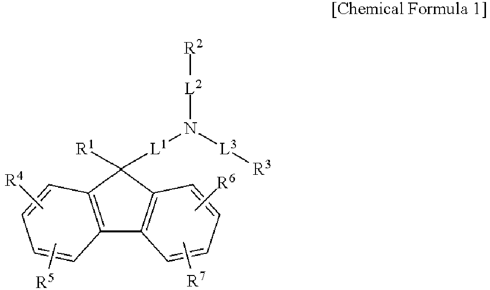

WO2020009398A1 relates to an organic electroluminescent compound represented by formula 1 and an organic electroluminescent device comprising the same.

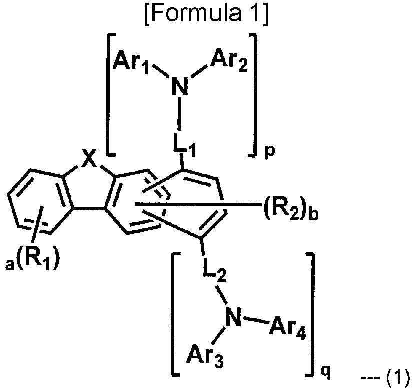

EP3466926A1 provides an organic electronic element and an electronic device therefor, the organic electronic element having a compound of Formula (1) below.

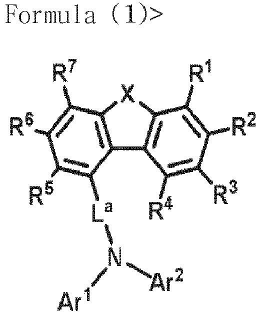

WO2019190101A1 provides a mixture reported to be capable of improving luminous efficiency, stability, and lifespan of an element, comprising a compound of Chemical Formula 1 and a compound of Chemical Formula 2.

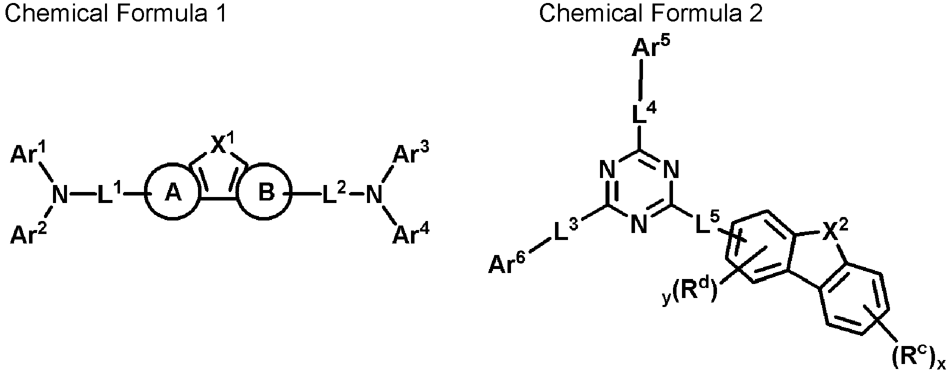

WO2019124903A1 relates to an organic light emitting device comprising a compound represented by formula 1 as a light emitting auxiliary layer material.

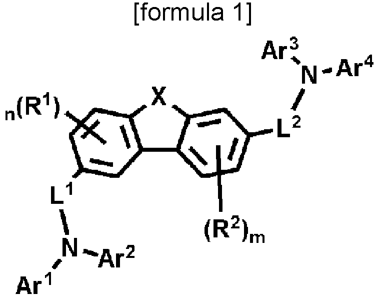

WO2018169261A1 provides a compound represented by the following formula (1) reported to be capable of enabling high luminous efficiency, a low driving voltage, and an improved lifespan of an element.

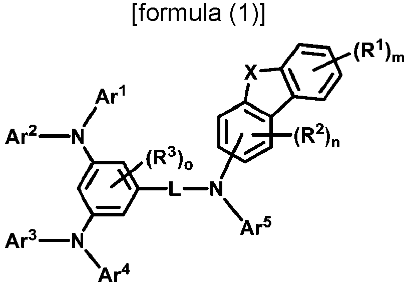

WO2018016786A1 provides a compound represented by the following formula (1) reported to be capable of implementing high luminous efficiency, a low driving voltage and improved lifespan of a device.

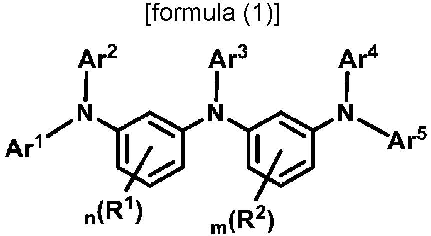

US2017237013A1 provides a compound represented by Formula (1) below for an electron blocking layer (EBL) which is reported to be capable of improving the light emitting efficiency, stability and life span of a device.

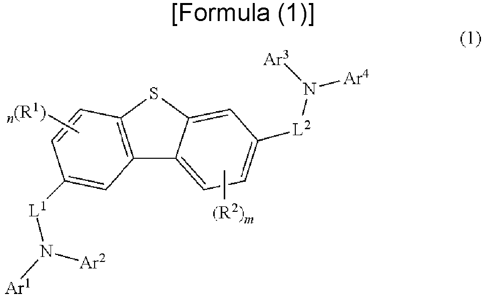

US2017125689A1 provides a compound of Formula 1 and an organic electric element including a first electrode, a second electrode, and an organic material layer between the first electrode and the second electrode and comprising the compound.

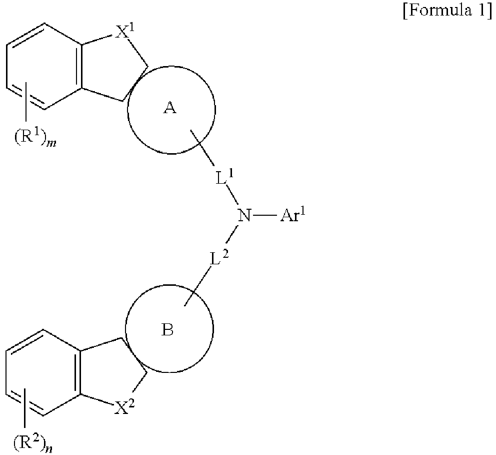

WO2019160335A1 provides an organic electric device comprising a first electrode, a second electrode, and an organic material layer formed between the first electrode and the second electrode, wherein the organic material layer includes a light emitting layer, and the light emitting layer is a phosphorescent light emitting layer. The organic electric device comprises a first host compound represented by Formula 1 and a second host compound represented by Formula 2 below.

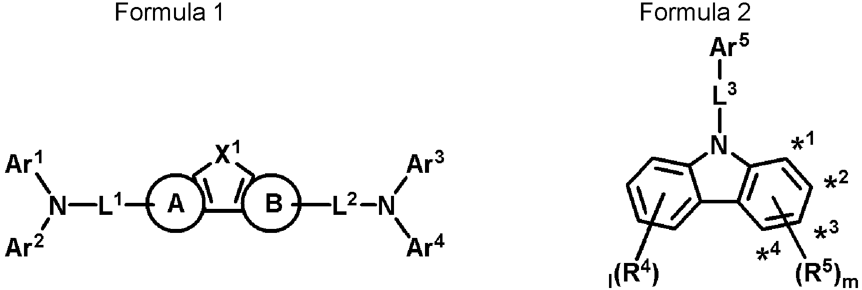

US2017092869A1 provides a compound of Formula 1 and an organic electric element including a first electrode, a second electrode, and an organic material layer between the first electrode and the second electrode and comprising the compound. 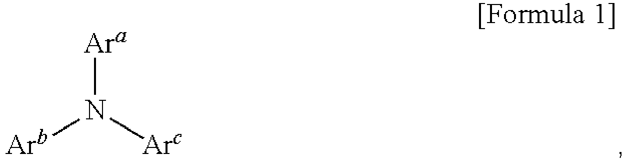 wherein,
Ar^{a} is Formula 2 below, Ar^{b} is Formula 3 below and Ar^{c} is selected from the group consisting of a C6-C60 aryl group; a fluorenyl group; a C2-C60 heterocyclic group containing at least one heteroatom selected from the group consisting of O, N, S, Si, and P; and a C1-C60 alkyl group,

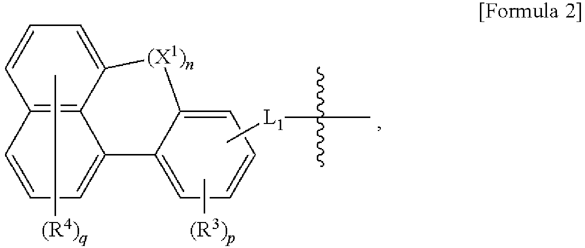

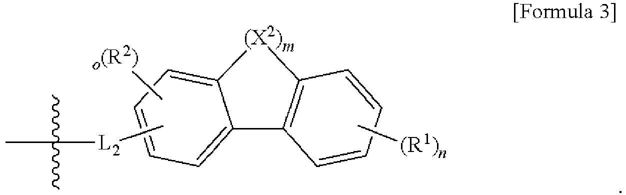

CN108658932A provides an aromatic amine compound and an organic electroluminescence device comprising the same. The aromatic amine compound contains 9,9'-spirobifluorene and dibenzothiophene, which has a structure represented by the formula (I): 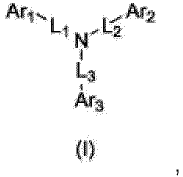 wherein Ar1 is selected from C25-C60 substituted or unsubstituted 9,9'-spirobifluorene, Ar2 is selected from C16-C30 substituted or unsubstituted dibenzothiophene, and Ar3 is selected from C6-C60 substituted or unsubstituted.

CN108947902A provides an organic electroluminescent material and an organic electroluminescent device thereof. The organic electroluminescent material is a mixture of compounds (I) and (II): 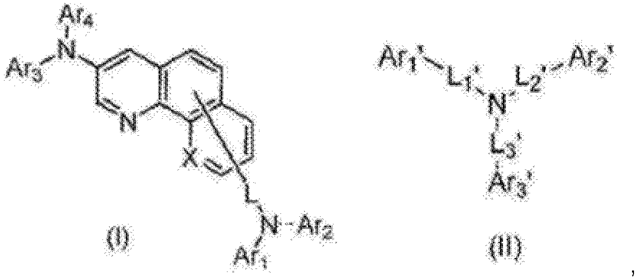 wherein Ar1, Ar2, Ar3, Ar4 are independently selected from a C6-C60 substituted or unsubstituted aryl group, a C3-C60 substituted or unsubstituted heteroaryl group, or Ar1 and Ar2 are bonded to each other to form a nitrogen-containing group. a five-membered heterocyclic ring, or Ar3 and Ar4 are bonded to each other to form a nitrogen-containing five-membered heterocyclic ring; Ar1' is selected from a substituted or unsubstituted 9,9'-spirobifluorenyl group of C25-C60; and Ar2' is selected from the group consisting of C16-C30. a substituted or unsubstituted dibenzofuranyl group, a C16-C30 substituted or unsubstituted dibenzothiophenyl group; Ar3' is selected from a C6-C60 substituted or unsubstituted aryl group, a C3-C60 substituted or unsubstituted heteroaryl.

KR20190133617A provides a compound of chemical formula 1 and an organic light emitting device comprising the same. 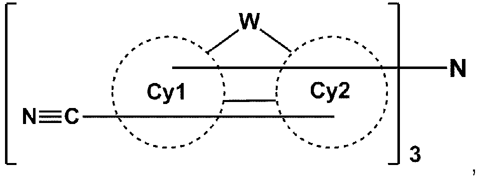 wherein
Cy1 and Cy2 are the same as or different from each other, and each independently substituted or unsubstituted benzene; or substituted or unsubstituted naphthalene, at least one of Cy1 and Cy2 is substituted or unsubstituted naphthalene, W is O, S, N (R1), C (R2) (R3) or Si (R4) (R5),
R1 is hydrogen; heavy hydrogen; halogen group; cyano group; nitro group; hydroxyl group; carbonyl group; ester group; imide group; amino group; silyl groups; boron group; substituted or unsubstituted alkyl group; a substituted or unsubstituted cycloalkyl group; substituted or unsubstituted aryl group; or a substituted or unsubstituted heterocyclic group,
R2 to R5 are the same as or different from each other, and each independently hydrogen; heavy hydrogen; halogen group; cyano group; nitro group; hydroxyl group; carbonyl group; ester group; imide group; amino group; silyl groups; boron group; substituted or unsubstituted alkyl group; a substituted or unsubstituted cycloalkyl group; substituted or unsubstituted aryl group; or a substituted or unsubstituted heterocyclic group, or combine with an adjacent group to form a substituted or unsubstituted ring.

KR20190130341A provides an organic electric device comprising a first electrode, a second electrode, and an organic material layer formed between the first electrode and the second electrode, wherein the organic material layer includes a light emitting layer, and the light emitting layer is represented by Formula 1:

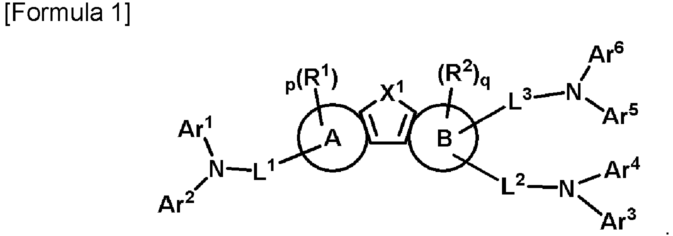

US2019229271A1 provides an organic electric element comprising a first electrode, a second electrode, and an organic material layer formed between the first electrode and the second electrode, wherein the organic material layer comprises an emitting layer and the emitting layer comprises a first host compound represented by Formula (1) and a second host compound represented by Formula (2):

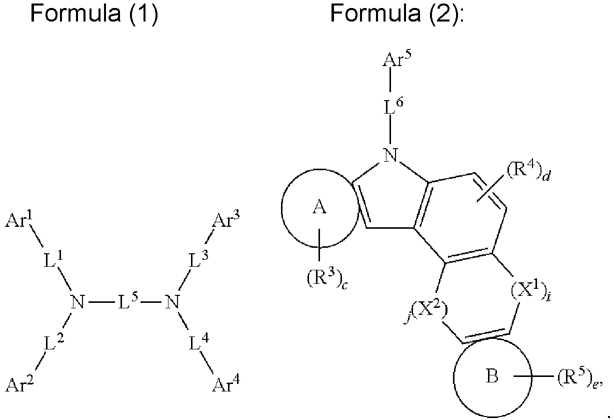

US2020203620A1 provides a compound represented by the Chemical Formula 1:and an organic light-emitting device comprising the same. The compound may be applied as a hole transport material to an organic light emitting device.

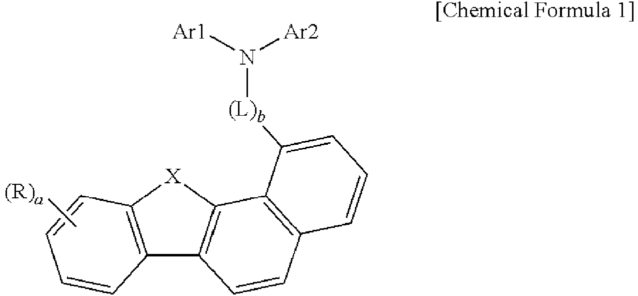

EP3670510A2 provides compounds of Formulae 1 and 3; organic electric elements comprising an organic material layer between 2 electrodes, wherein the organic material layer comprises either a mixture of compounds of Formulae 1 and 2 or a compound of Formula 3; and electronic devices comprising the organic electric elements.

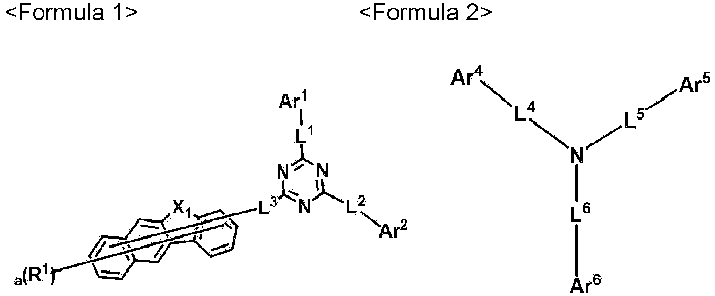

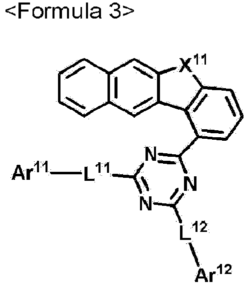

US20160351816A1 relates to an organic light-emitting device including a first electrode, a second electrode, and an organic layer between the first electrode and the second electrode and including an emission layer; wherein the organic layer includes a host first compound represented by Formula 1 and a fluorescent dopant second compound represented by Formula 2:

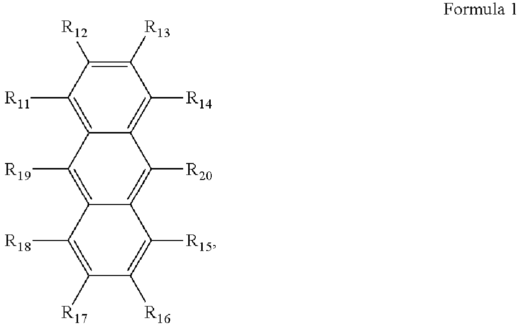

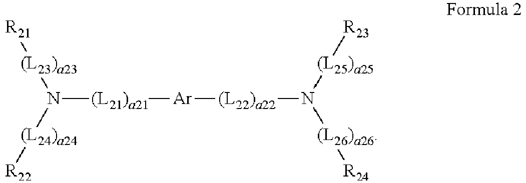

### SUMMARY

The invention is defined by the claims.

One or more aspects of embodiments of the present disclosure are directed toward an organic electroluminescence device and a polycyclic compound for an organic electroluminescence device, and for example, an organic electroluminescence device with high efficiency and a polycyclic compound comprised in the hole transport region of the organic electroluminescence device.

One or more aspects of embodiments of the present disclosure are directed to an organic electroluminescence device comprising: a first electrode; a hole transport region provided on the first electrode; an emission layer provided on the hole transport region; an electron transport region provided on the emission layer; and a second electrode provided on the electron transport region, wherein the hole transport region comprises a polycyclic compound as described herein.

One or more aspects of embodiments of the present disclosure are directed to a polycyclic compound represented by Formula 1:

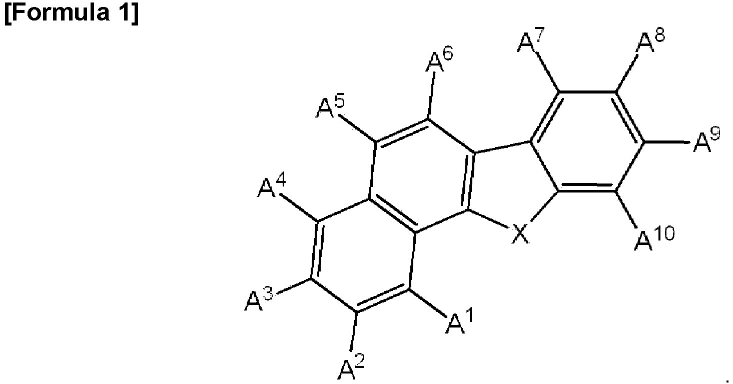

In Formula 1,
X may be O or S,
one of A¹ to A¹⁰is a substituted amine group, wherein at least one substituent of the substituted amine group comprises a cyclic group comprising between 6 and 30 ring forming carbon atoms or a polycyclic group comprising between 6 and 30 ring forming carbon atoms, and the remainder are each independently a hydrogen atom, a deuterium atom, a halogen atom, a substituted or unsubstituted alkyl group comprising 1 to 20 carbon atoms, or a substituted or unsubstituted aryl group comprising 6 to 30 ring forming carbon atoms.

In the disclosure, the substituted amine group is represented by Formula 2-1:

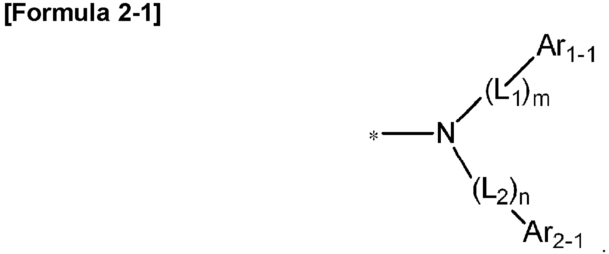

In Formula 2-1, L₁ and L₂ areeach independently a bond, a substituted or unsubstituted arylene group comprising 6 to 30 ring forming carbon atoms or a substituted or unsubstituted heteroarylene group comprising 2 to 30 ring forming carbon atoms, "m" and "n" are each independently an integer of 0 to 2, Ar₁₋₁ and Ar₂₋₁ are each independently a substituted or unsubstituted aryl group comprising 10 to 40 ring forming carbon atoms or represented by Formula 3-1 or Formula 3-2, where at least one of Ar₁₋₁ and Ar₂₋₁ is represented by Formula 3-1 or Formula 3-2. In a case where X is O, and one of A², A³, A⁸ and A¹⁰ is represented by Formula 2-1, Ar₁₋₁ and Ar₂₋₁ are each independently represented by Formula 3-1 or Formula 3-2:

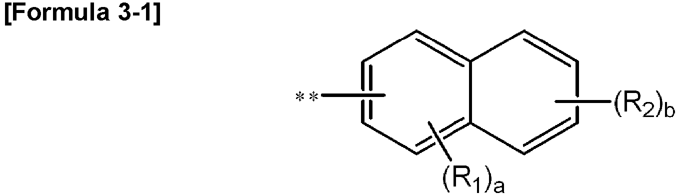

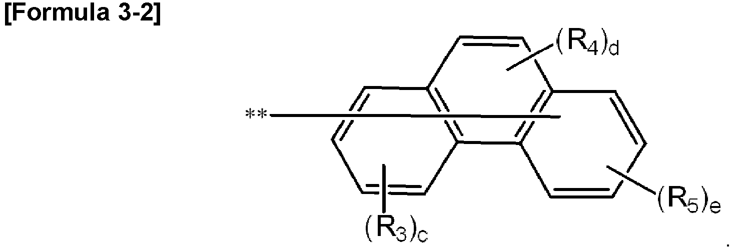

In Formula 3-1 and Formula 3-2, R₁ to R₅ are each independently a hydrogen atom, a deuterium atom, a halogen atom, a substituted or unsubstituted alkyl group comprising 1 to 20 carbon atoms, a substituted or unsubstituted aryl group comprising 6 to 30 ring forming carbon atoms, or a substituted or unsubstituted heteroaryl group comprising 2 to 30 ring forming carbon atoms, "a" is an integer of 0 to 3, "b", "c", and "e"are each independently an integer of 0 to 4, and "d" is an integer of 0 to 2, where c+d+e is an integer of 9 or less.

In the invention, the substituted amine group is represented by Formula 2-2:

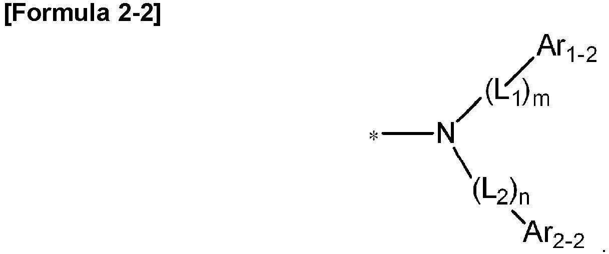

In Formula 2-2, L₁ and L₂ are each independently a bond, a substituted or unsubstituted arylene group comprising 6 to 30 ring forming carbon atoms, or a substituted or unsubstituted heteroarylene group comprising 2 to 30 ring forming carbon atoms, "m" and "n" are each independently an integer of 0 to 2, and Ar₁₋₂ and Ar₂₋₂ are each independently a substituted or unsubstituted aryl group comprising 10 to 40 ring forming carbon atoms or represented by Formula 3-3 or Formula 3-4, where at least one of Ar₁₋₂ and Ar₂₋₂ is represented by Formula 3-3:

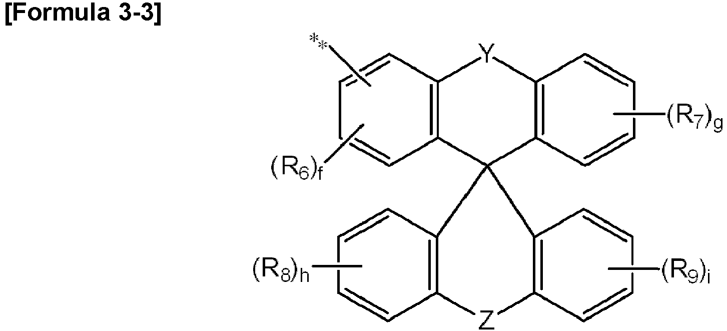

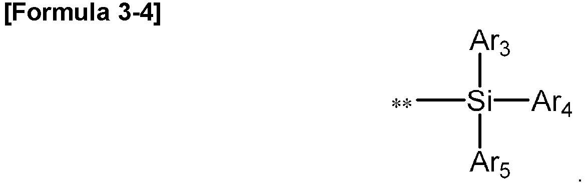

In Formula 3-3 and Formula 3-4, Y and Z are each independently a bond, O, or S, and at least one of Y and Z are O or S (e.g., Y and Z are not simultaneously a bond), R₆ to R₉ are each independently a hydrogen atom, a deuterium atom, a halogen atom, a substituted or unsubstituted alkyl group comprising 1 to 20 carbon atoms, a substituted or unsubstituted aryl group comprising 6 to 30 ring forming carbon atoms, or a substituted or unsubstituted heteroaryl group comprising 2 to 30 ring forming carbon atoms, "f" is an integer of 0 to 3, "g" to "i" are each independently an integer of 0 to 4, and Ar₃ to Ar₅ are each independently a substituted or unsubstituted aryl group comprising 6 to 30 ring forming carbon atoms.

In the disclosure, the substituted amine group is represented by Formula 2-3:

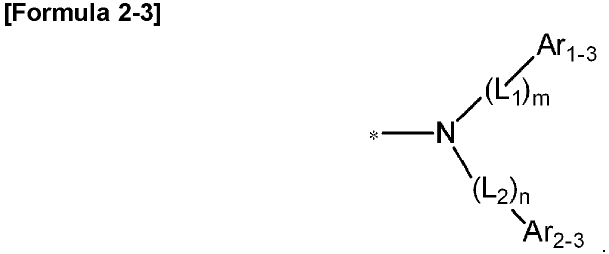

In Formula 2-3, L₁ and L₂ are each independently a bond, a substituted or unsubstituted arylene group comprising 6 to 30 ring forming carbon atoms or a substituted or unsubstituted heteroarylene group comprising 2 to 30 ring forming carbon atoms, "m" and "n" are each independently an integer of 0 to 2, and Ar₁₋₃ and Ar₂₋₃ are each independently a substituted or unsubstituted aryl group comprising 10 to 40 ring forming carbon atoms or represented by Formula 3-5 or Formula 3-6, where at least one of Ar₁₋₃ and Ar₂₋₃ is represented by Formula 3-5 or Formula 3-6:

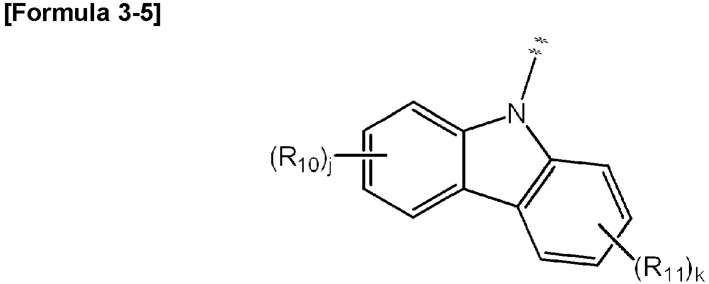

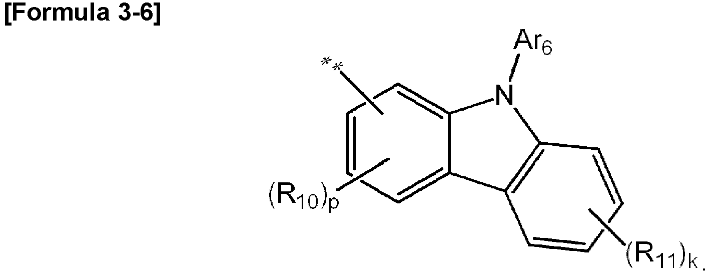

In Formula 3-5 and Formula 3-6, R₁₀ and R₁₁ are each independently a hydrogen atom, a deuterium atom, a halogen atom, a substituted or unsubstituted alkyl group comprising 1 to 20 carbon atoms, a substituted or unsubstituted aryl group comprising 6 to 30 ring forming carbon atoms, or a substituted or unsubstituted heteroaryl group comprising 2 to 30 carbon atoms; Ar₆ is a substituted or unsubstituted aryl group comprising 6 to 30 ring forming carbon atoms, "j" and "k" are each independently an integer of 0 to 4, and "p" is an integer of 0 to 3.

In the disclosure, Formula 2-1 is represented by Formula 4-1 or Formula 4-2:

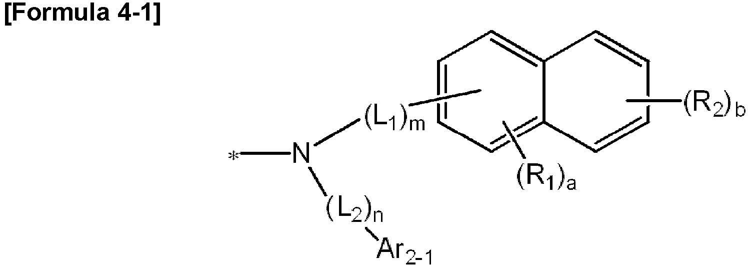

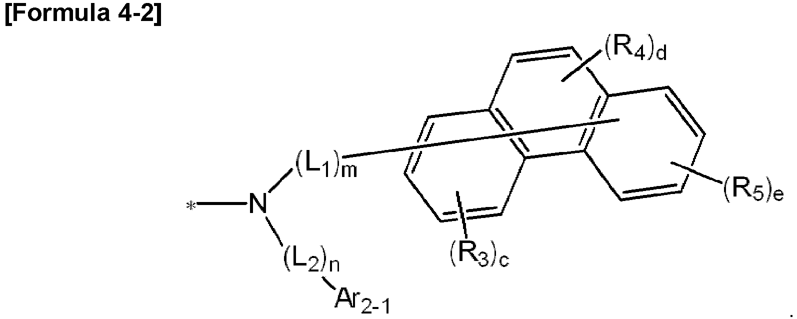

In Formula 4-1 and Formula 4-2, Ar₂₋₁ is a substituted or unsubstituted aryl group comprising 10 to 40 ring forming carbon atoms , L₁, L₂, "m" and "n" are each independently the same as defined in Formula 2-1, and R₁ to R₅, and "a" to "d" are each independently the same as defined in Formula 3-1 and Formula 3-2.

In an embodiment, Formula 2-2 is represented by Formula 5-1. In the disclosure, Formula 2-2 is represented by Formula 5-1 or Formula 5-2:

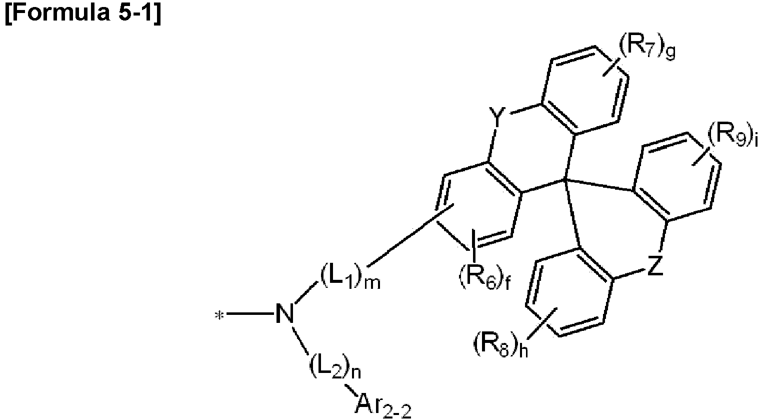

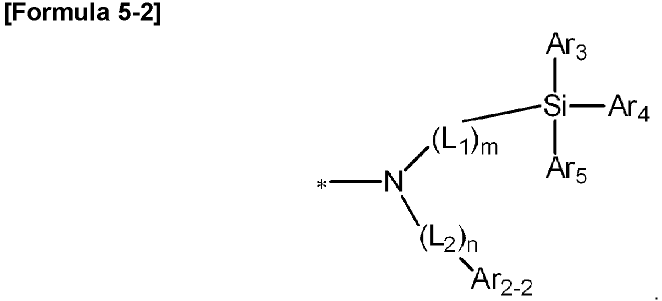

In Formula 5-1 and Formula 5-2, Ar₂₋₂ is a substituted or unsubstituted aryl group comprising 10 to 40 ring forming carbon atoms; L₁, L₂, "m" and "n" are each independently the same as defined in Formula 2-2; and Y, Z, R₆ to R₉, "f" to "i", and Ar₃ to Ar₅ are each independently the same as defined in Formula 3-3 and Formula 3-4.

In the disclosure, Formula 2-3 is represented by Formula 6-1 or Formula 6-2:

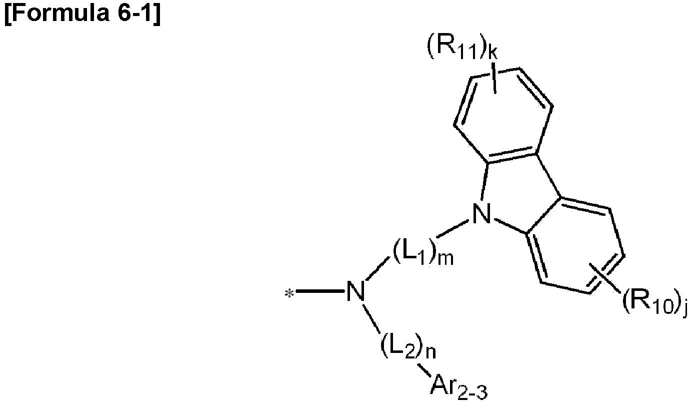

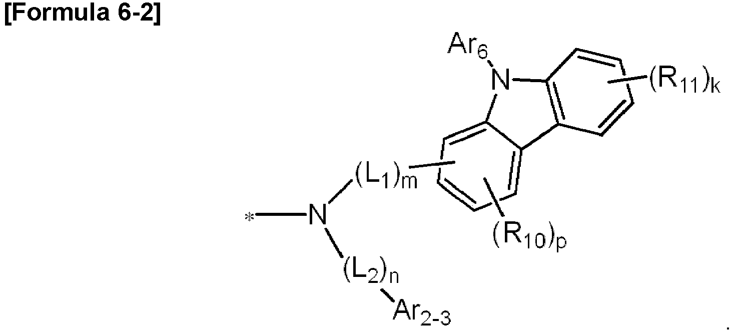

In Formula 6-1 and Formula 6-2, Ar₂₋₃ is a substituted or unsubstituted aryl group comprising 10 to 40 ring forming carbon atoms; L₁, L₂, "m" and "n" are each independently the same as defined in Formula 2-3; and R₁₀ and R₁₁, Ar₆, "j", "k", and "p" are each independently the same as defined in Formula 3-5 and Formula 3-6.

In an embodiment, any one of A¹ to A⁶ in Formula 1 is the substituted amine group.

In an embodiment, any one of A⁷ or A¹⁰ in Formula 1 is the substituted amine group.

In an embodiment, any one of A⁷ to A¹⁰ in Formula 1 is the substituted amine group.

In the disclosure, any one among A¹ to A⁶ in Formula 1 may be represented by Formula 2-1.

In the disclosure, A⁷ or A¹⁰ in Formula 1 may be represented by Formula 2-1.

In an embodiment, any one among A¹ to A⁶ in Formula 1 may be represented by Formula 2-2.

In an embodiment, any one among A⁷ to A¹⁰ in Formula 1 may be represented by Formula 2-2.

In the disclosure, any one among A¹ to A⁶ in Formula 1 may be represented by Formula 2-3.

In the disclosure, any one among A⁷ to A¹⁰ in Formula 1 may be represented by Formula 2-3.

In an embodiment, the hole transport region may comprise a hole injection layer disposed on the first electrode, and a hole transport layer disposed on the hole injection layer, where the hole transport layer comprises the polycyclic compound represented by Formula 1.

In an embodiment, L₁ and L₂ are each independently a bond, a substituted or unsubstituted phenylene group, or a substituted or unsubstituted naphthyl group.

In an embodiment, L₂ may is a bond or a substituted or unsubstituted phenyl group, "n" is 1, and Ar₂₋₁ to Ar₂₋₃ , when present, are each independently a substituted or unsubstituted phenyl group, a substituted or unsubstituted biphenyl group, a substituted or unsubstituted terphenyl group, a substituted or unsubstituted phenanthrene group, a substituted or unsubstituted naphthyl group, or a substituted or unsubstituted fluorenyl group, and L₂ and any one of Ar₂₋₁ to Ar₂₋₃ , when present are not both phenyl groups. For example, where a case in which L₂ and any one among Ar₂₋₁ to Ar₂₋₃ (e.g., L₂ and Ar₂₋₁, L₂ and Ar₂₋₂, or L₂ and Ar₂₋₃) are simultaneously phenyl groups (e.g., at the same time) may be excluded.

The polycyclic compound represented by Formula 1 may be at least one selected from the compounds represented in Compound Group 3. In the disclosure, the polycyclic compound represented by Formula 1 may be at least one selected from the compounds represented in Compound Group 1 to Compound Group 6.
One or more example embodiments of the present disclosure provide a polycyclic compound represented by Formula 1.

### BRIEF DESCRIPTION OF THE DRAWINGS

The accompanying drawings are comprised to provide a further understanding of the present disclosure, and are incorporated in and constitute a part of this specification. The drawings illustrate example embodiments of the present disclosure and, together with the description, serve to explain principles of the present disclosure. In the drawings:
FIG. 1 is a cross-sectional view schematically illustrating an organic electroluminescence device according to an embodiment of the present disclosure;
FIG. 2 is a cross-sectional view schematically illustrating an organic electroluminescence device according to an embodiment of the present disclosure;
FIG. 3 is a cross-sectional view schematically illustrating an organic electroluminescence device according to an embodiment of the present disclosure; and
FIG. 4 is a cross-sectional view schematically illustrating an organic electroluminescence device according to an embodiment of the present disclosure.

### DETAILED DESCRIPTION

The present disclosure may have various modifications and may be embodied in different forms, and example embodiments will be explained in more detail with reference to the accompanying drawings. The present disclosure may, however, be embodied in different forms and should not be construed as being limited to the embodiments set forth herein. Rather, all modifications, and substituents that are within the technical scope of the present disclosure should be comprised in the present disclosure.

It will be understood that when an element (or region, layer, part, etc.) is referred to as being "on", "connected to" or "coupled to" another element, it can be directly on, connected or coupled to the other element, or one or more intervening elements may be present. When an element is referred to as being "directly on," "directly connected to," or "directly coupled to" another element, there are no intervening elements present.

Like reference numerals refer to like elements throughout, and duplicative descriptions thereof may not be provided. In the drawings, thicknesses, ratios, and dimensions of constituent elements may be exaggerated for effective explanation of technical contents.

As used herein, expressions such as "at least one of," "one of," and "selected from," when preceding a list of elements, modify the entire list of elements and do not modify the individual elements of the list. The term "and/or" comprises any and all combinations of one or more of the associated listed items. Further, the use of "may" when describing embodiments of the present disclosure refers to "one or more embodiments of the present disclosure".

It will be understood that, although the terms first, second, etc. may be used herein to describe various elements, these elements should not be limited by these terms. These terms are only used to distinguish one element from another element. Thus, a first element could be alternatively termed a second element without departing from the teachings of the present disclosure. Similarly, a second element could be alternatively termed a first element. As used herein, the singular forms are intended to comprise the plural forms as well, unless the context clearly indicates otherwise.

In addition, the terms "below", "beneath", "on" and "above" are used for explaining spatial relationships between elements shown in the drawings. The terms are relative concepts, and are selected and to be interpreted based on the orientations shown in the drawing.

Unless otherwise defined, all terms (comprising technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and should not be interpreted in an idealized or overly formal sense unless expressly so defined herein.

Hereinafter, the organic electroluminescence device according to an embodiment of the present disclosure will be explained with reference to the attached drawings.

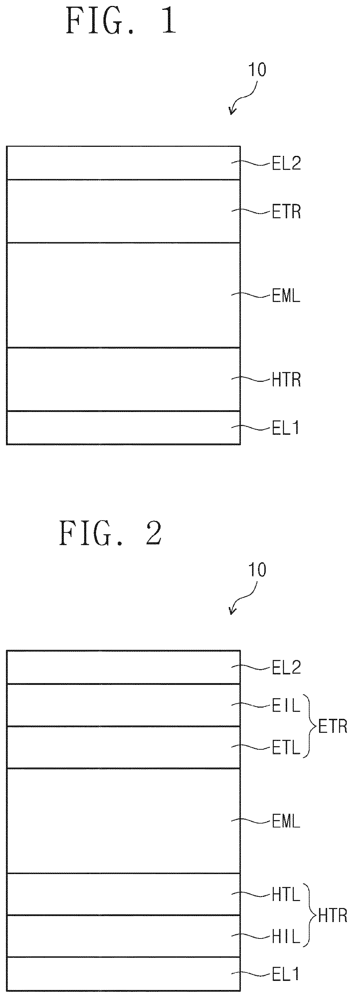

FIGS. 1 to 4 are cross-sectional views schematically showing organic electroluminescence devices according to example embodiments of the present disclosure. Referring to FIGS. 1 to 4, in an organic electroluminescence device 10 according to an embodiment, a first electrode EL1 and a second electrode EL2 are oppositely disposed, and between the first electrode EL1 and the second electrode EL2, an emission layer EML may be disposed.

In some embodiments, the organic electroluminescence device 10 of an embodiment further comprises a plurality of functional layers between the first electrode EL1 and the second electrode EL2 in addition to the emission layer EML. The plurality of functional layers may comprise a hole transport region HTR and an electron transport region ETR. For example, the organic electroluminescence device 10 of an embodiment may comprise a first electrode EL1, a hole transport region HTR, an emission layer EML, an electron transport region ETR, and a second electrode, stacked one by one. In some embodiments, the organic electroluminescence device 10 of an embodiment may comprise a capping layer CPL disposed on the second electrode EL2.

The organic electroluminescence device 10 of an embodiment comprises a polycyclic compound of an embodiment, which will be explained later, in the emission layer EML disposed between the first electrode EL1 and the second electrode EL2. However, embodiments of the present disclosure are not limited thereto, and the organic electroluminescence device 10 of an embodiment may comprise a polycyclic compound according to an embodiment in the hole transport region HTR or the electron transport region ETR (which are among the plurality of functional layers disposed between the first electrode EL1 and the second electrode EL2), or in the capping layer CPL disposed on the second electrode EL2 in addition to the emission layer EML.

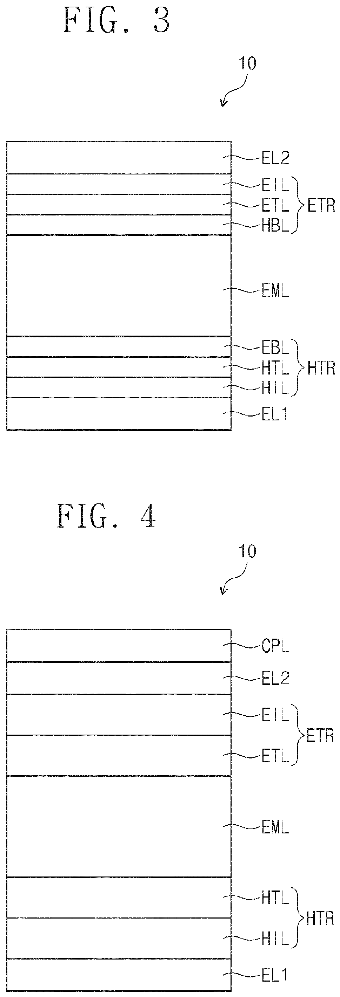

Compared with FIG. 1, FIG. 2 shows the cross-sectional view of an organic electroluminescence device 10 of an embodiment, wherein the hole transport region HTR comprises a hole injection layer HIL and a hole transport layer HTL, and the electron transport region ETR comprises an electron injection layer EIL and an electron transport layer ETL. Compared with FIG. 1, FIG. 3 shows the cross-sectional view of an organic electroluminescence device 10 of an embodiment, wherein the hole transport region HTR comprises the hole injection layer HIL, the hole transport layer HTL, and an electron blocking layer EBL, and the electron transport region ETR comprises the electron injection layer EIL, the electron transport layer ETL, and a hole blocking layer HBL. Compared with FIG. 2, FIG. 4 shows the cross-sectional view of an organic electroluminescence device 10 of an embodiment that comprises a capping layer CPL disposed on the second electrode EL2.

The first electrode EL1 has conductivity (e.g., may be conductive). The first electrode EL1 may be formed using a metal alloy or a conductive compound. The first electrode EL1 may be a pixel electrode or an anode. The first electrode EL1 may be a transmissive electrode, a transflective electrode, or a reflective electrode. When the first electrode EL1 is a transmissive electrode, the first electrode EL1 may be formed using a transparent metal oxide (such as indium tin oxide (ITO), indium zinc oxide (IZO), zinc oxide (ZnO), and/or indium tin zinc oxide (ITZO)). When the first electrode EL1 is a transflective electrode or a reflective electrode, the first electrode EL1 may comprise silver (Ag), magnesium (Mg), copper (Cu), aluminum (Al), platinum (Pt), palladium (Pd), gold (Au), nickel (Ni), neodymium (Nd), iridium (Ir), chromium (Cr), lithium (Li), calcium (Ca), LiF/Ca, LiF/Al, molybdenum (Mo), titanium (Ti), a compound thereof, or a mixture thereof (for example, a mixture of Ag and Mg). In some embodiments, the first electrode EL1 may have a structure comprising a plurality of layers, comprising a reflective layer or a transflective layer formed using the above materials, and a transmissive conductive layer formed using ITO, IZO, ZnO, and/or ITZO. For example, the first electrode EL1 may comprise a three-layer structure of ITO/Ag/ITO. However, embodiments of the present disclosure are not limited thereto. The thickness of the first electrode EL1 may be about 1,000 Å to about 10,000 Å, for example, about 1,000 Å to about 3,000 Å.

The hole transport region HTR is provided on the first electrode EL1. The hole transport region HTR may comprise at least one of a hole injection layer HIL, a hole transport layer HTL, a hole buffer layer, or an electron blocking layer EBL.

The hole transport region HTR may have a single layer formed using a single material, a single layer formed using a plurality of different materials, or a multilayer structure comprising a plurality of layers formed using a plurality of different materials.

For example, the hole transport region HTR may have the structure of a single layer of a hole injection layer HIL or a hole transport layer HTL, or may have the structure of a single layer formed using a hole injection material and a hole transport material. In some embodiments, the hole transport region HTR may have the structure of a single layer formed using a plurality of different materials, or a structure stacked from the first electrode EL1 of hole injection layer HIL/hole transport layer HTL, hole injection layer HIL/hole transport layer HTL/hole buffer layer, hole injection layer HIL/hole buffer layer, hole transport layer HTL/hole buffer layer, or hole injection layer HIL/hole transport layer HTL/electron blocking layer EBL, without limitation.

The hole transport region HTR of the organic electroluminescence device 10 of an embodiment comprises the polycyclic compound according to an embodiment of the present disclosure.

In the description, the term "substituted or unsubstituted" refers to a state of being unsubstituted, or substituted with at least one substituent selected from the group consisting of a deuterium atom, a halogen atom, a cyano group, a nitro group, an amino group, a silyl group, an oxy group, a thio group, a sulfinyl group, a sulfonyl group, a carbonyl group, a boron group, a phosphine oxide group, a phosphine sulfide group, an alkyl group, an alkenyl group, an alkoxy group, a hydrocarbon ring group, an aryl group, a cyclic group, and a polycyclic group. A "cyclic group" comprises a substituted or unsubstituted cyclic hydrocarbon group as well as a substituted or unsubstituted heterocyclic group. A "polycyclic group" comprises a substituted or unsubstituted polycyclic hydrocarbon group as well as a substituted or unsubstituted heteropolycyclic group. In addition, each of the exemplified substituents may be further substituted or unsubstituted. Further, a biphenyl group may be interpreted as a named aryl group, or as a phenyl group substituted with a phenyl group.

In the description, non-limiting examples of the halogen atom may comprise a fluorine atom, a chlorine atom, a bromine atom, and/or an iodine atom.

In the description, the term "alkyl group" may refer to a linear, branched or cyclic alkyl group. The carbon number of the alkyl group may be 1 to 50, 1 to 30, 1 to 20, 1 to 10, or 1 to 6. Examples of the alkyl group may comprise methyl, ethyl, n-propyl, isopropyl, n-butyl, s-butyl, t-butyl, i-butyl, 2-ethylbutyl, 3,3-dimethylbutyl, n-pentyl, i-pentyl, neopentyl, t-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butylhexyl, cyclohexyl, 4-methylcyclohexyl, 4-t-butylcyclohexyl, n-heptyl, 1-methylheptyl, 2,2-dimethylheptyl, 2-ethylheptyl, 2-butylheptyl, n-octyl, t-octyl, 2-ethyloctyl, 2-butyloctyl, 2-hexyloctyl, 3,7-dimethyloctyl, cyclooctyl, n-nonyl, n-decyl, adamantyl, 2-ethyldecyl, 2-butyldecyl, 2-hexyldecyl, 2-octyldecyl, n-undecyl, n-dodecyl, 2-ethyldodecyl, 2-butyldodecyl, 2-hexyldocecyl, 2-octyldodecyl, n-tridecyl, n-tetradecyl, n-pentadecyl, n-hexadecyl, 2-ethylhexadecyl, 2-butylhexadecyl, 2-hexylhexadecyl, 2-octylhexadecyl, n-heptadecyl, n-octadecyl, n-nonadecyl, n-eicosyl, 2-ethyleicosyl, 2-butyleicosyl, 2-hexyleicosyl, 2-octyleicosyl, n-henicosyl, n-docosyl, n-tricosyl, n-tetracosyl, n-pentacosyl, n-hexacosyl, n-heptacosyl, n-octacosyl, n-nonacosyl, n-triacontyl, etc., without limitation.

In the description, the term "alkenyl group" may refer to a hydrocarbon group comprising one or more carbon-carbon double bonds in the middle or at the terminal end of an alkyl group of 2 or more carbon atoms. The alkenyl group may comprise a linear chain or a branched chain. The carbon number of the alkenyl group is not specifically limited, but may be 2 to 30, 2 to 20, or 2 to 10. Examples of the alkenyl group comprise a vinyl group, a 1-butenyl group, a 1-pentenyl group, a 1,3-butadienyl aryl group, a styrenyl group, a styrylvinyl group, etc., without limitation.

In the description, the term "alkynyl group" may refer to a hydrocarbon group comprising one or more carbon-carbon triple bonds in the middle or at the terminal end of an alkyl group of 2 or more carbon atoms. The alkynyl group may comprise a linear chain or a branched chain. The carbon number of the alkynyl group is not specifically limited, but may be 2 to 30, 2 to 20, or 2 to 10. Examples of the alkynyl group comprise an ethynyl group, a propynyl group, etc., without limitation.

In the description, the term "hydrocarbon ring group" may refer to an optional functional group or substituent derived from an aliphatic hydrocarbon ring, or an optional functional group or substituent derived from an aromatic hydrocarbon ring. The carbon number for forming a ring of the hydrocarbon ring group may be 5 to 60, 5 to 30, or 5 to 20.

In the description, the term "aryl group" may refer to an optional functional group or substituent derived from an aromatic hydrocarbon ring. The aryl group may be a monocyclic aryl group or a polycyclic aryl group. The carbon number for forming a ring of the aryl group may be 6 to 30, 6 to 20, or 6 to 15. Examples of the aryl group may comprise phenyl, naphthyl, fluorenyl, anthracenyl, phenanthryl, biphenyl, terphenyl, quaterphenyl, quinquephenyl, sexiphenyl, triphenylenyl, pyrenyl, benzofluoranthenyl, chrysenyl, etc., without limitation.

In the description, the fluorenyl group may be substituted, and two substituents (e.g., at the 9H position) may be combined with each other to form a spiro structure. Examples of a substituted fluorenyl group are as follows. However, embodiments of the present disclosure are not limited thereto:

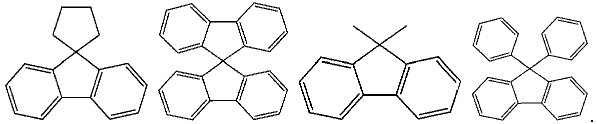

In the description, the term "heterocyclic group" may refer to an optional functional group or substituent derived from a ring comprising one or more among B, O, **N,** P, Si and S as heteroatoms. The heterocyclic group may comprise an aliphatic heterocyclic group and an aromatic heterocyclic group. The aromatic heterocyclic group may be a heteroaryl group. The aliphatic heterocyclic group and the aromatic heterocyclic group may be monocycles or polycycles.

In the description, the heterocyclic group may comprise one or more among B, O, N, P, Si and S as heteroatoms. When the heterocyclic group comprises two or more heteroatoms, the two or more heteroatoms may be the same or different. The heterocyclic group may be a monocyclic heterocyclic group or a polycyclic heterocyclic group, and has a concept comprising a heteroaryl group. The carbon number for forming a ring of the heteroaryl group may be 2 to 30, 2 to 20, or 2 to 10.

In the description, the aliphatic heterocyclic group may comprise one or more among B, O, N, P, Si and S as heteroatoms. The carbon number for forming a ring of the aliphatic heterocyclic group may be 2 to 30, 2 to 20, or 2 to 10. Examples of the aliphatic heterocyclic group comprise an oxirane group, a thiirane group, a pyrrolidine group, a piperidine group, a tetrahydrofuran group, a tetrahydrothiophene group, a thiane group, a tetrahydropyran group, a 1,4-dioxane group, etc., without limitation.

In the description, the heteroaryl group may comprise one or more among B, O, N, P, Si and S as heteroatoms. When the heteroaryl group comprises two or more heteroatoms, the two or more heteroatoms may be the same or different. The heteroaryl group may be a monocyclic heteroaryl group or polycyclic heteroaryl group. The carbon number for forming a ring of the heteroaryl group may be 2 to 30, 2 to 20, or 2 to 10. Examples of the heteroaryl group may comprise thiophene, furan, pyrrole, imidazole, triazole, pyridine, bipyridine, pyrimidine, triazine, triazole, acridyl, pyridazine, pyrazinyl, quinoline, quinazoline, quinoxaline, phenoxazine, phthalazine, pyrido pyrimidine, pyrido pyrazine, pyrazino pyrazine, isoquinoline, indole, carbazole, N-arylcarbazole, N-heteroarylcarbazole, N-alkylcarbazole, benzoxazole, benzimidazole, benzothiazole, benzocarbazole, benzothiophene, dibenzothiophene, thienothiophene, benzofuran, phenanthroline, thiazole, isooxazole, oxazole, oxadiazole, thiadiazole, phenothiazine, dibenzosilole, dibenzofuran, etc., without limitation.

In the description, the carbon number of the amine group is not specifically limited, but may be 1 to 30. The amine group may comprise an alkyl amine group, an aryl amine group, or a heteroaryl amine group. Examples of the amine group comprise a methylamine group, a dimethylamine group, a phenylamine group, a diphenylamine group, a naphthylamine group, a 9-methyl-anthracenylamine group, a triphenylamine group, etc., without limitation.

In the description, the explanation on the aryl group is applied to an arylene group except that the arylene group is a divalent group.

In the description, the explanation on the heteroaryl group is applied to a heteroarylene group except that the arylene group is a divalent group.

In the description, " 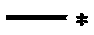 " and " 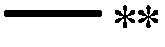 " indicate positions of connection (e.g., to other groups, moieties, etc.).

The polycyclic compound according to an embodiment of the present disclosure is represented by Formula 1:

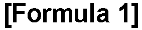

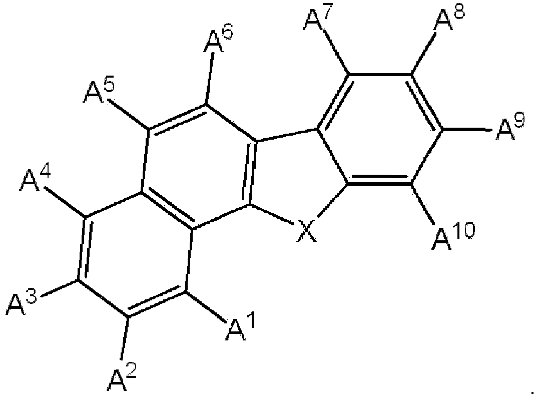

In Formula 1, X isO or S.

In Formula 1, one of A¹ to A¹⁰is a substituted amine group, wherein at least one substituent of the substituted amine group comprises a cyclic group comprising between 6 and 30 ring forming carbon atoms or a polycyclic group comprising between 6 and 30 ring forming carbon atoms, and the remainder may each independently be a hydrogen atom, a deuterium atom, a halogen atom, a substituted or unsubstituted alkyl group comprising 1 to 20 carbon atoms, or a substituted or unsubstituted aryl group comprising 6 to 30 ring forming carbon atoms.

In the disclosure, the substituted amine group is represented by Formula 2-1:

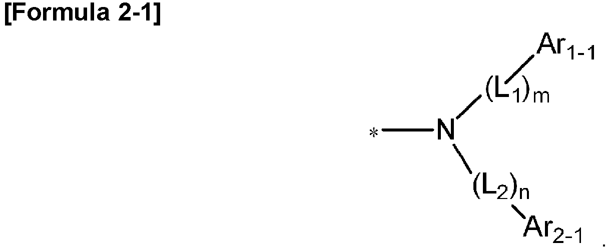

In Formula 2-1, L₁ and L₂ are each independently a bond, a substituted or unsubstituted arylene group comprising 6 to 30 ring forming carbon atoms, or a substituted or unsubstituted heteroarylene group comprising 2 to 30 ring forming carbon atoms.

In Formula 2-1, "m" and "n" are each independently an integer of 0 to 2, where when "m" is 2 or more, a plurality of L₁ groups may be the same or different from each other, and when "n" is 2 or more, a plurality of L₂ groups may be the same or different from each other.

In Formula 2-1, Ar₁₋₁ and Ar₂₋₁ each independently a substituted or unsubstituted aryl group comprising 10 to 40 ring forming carbon atoms, or may be represented by Formula 3-1 or Formula 3-2, where at least one among Ar₁₋₁ and Ar₂₋₁ is represented by Formula 3-1 or Formula 3-2.

When X is O in Formula 1, and any one among A², A³, A⁸ and A¹⁰ is represented by Formula 2-1, Ar₁₋₁ and Ar₂₋₁ are each independently represented by Formula 3-1 or Formula 3-2.

In Formula 2-1, " 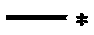 " indicates a position connected with Formula 1.

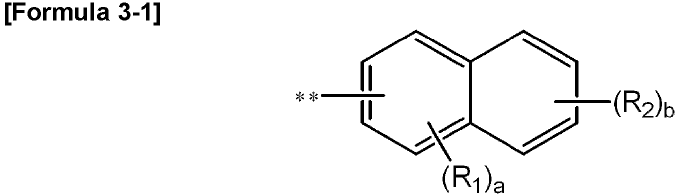

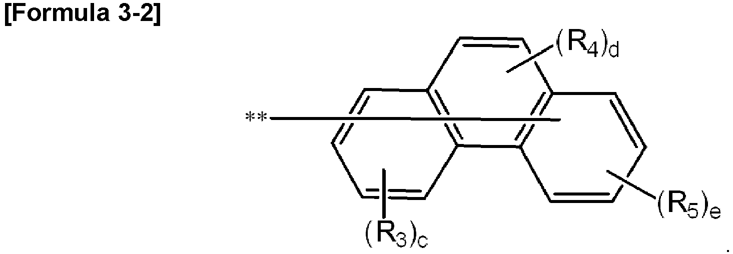

In Formula 3-1 and Formula 3-2, R₁ to R₅ are each independently a hydrogen atom, a deuterium atom, a halogen atom, a substituted or unsubstituted alkyl group comprising 1 to 20 carbon atoms, a substituted or unsubstituted aryl group comprising 6 to 30 ring forming carbon atoms, or a substituted or unsubstituted heteroaryl group comprising 2 to 30 ring forming carbon atoms.

In Formula 3-1, "a" is an integer of 0 to 3, and when "a" is 2 or more, a plurality of R₁ groups may be the same or different from each other.

In Formula 3-1, "b" is an integer of 0 to 4, and when "b" is 2 or more, a plurality of R₂ groups may be the same or different from each other.

In Formula 3-2, "c" and "e" are each independently an integer of 0 to 4, and when "c" is 2 or more, a plurality of R₃ groups may be the same or different from each other, and when "e" is 2 or more, a plurality of R₅ groups may be the same or different from each other.

In Formula 3-2, "d" is an integer of 0 to 2, and when "d" is 2, a plurality of R₄ groups may be the same or different from each other.

In Formula 3-2, c+d+e is an integer of 9 or less.

In Formula 3-1 and Formula 3-2, " 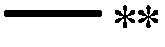 " indicates a position connected with Formula 2-1.

L₁ and L₂ of Formula 2-1 may each independently be a bond, a substituted or unsubstituted phenylene group, or a substituted or unsubstituted naphthyl group.

L₂ of Formula 2-1 may be a bond or a substituted or unsubstituted phenyl group, and when "n" is 1, each Ar₂₋₁ may independently be a substituted or unsubstituted phenyl group, a substituted or unsubstituted biphenyl group, a substituted or unsubstituted terphenyl group, a substituted or unsubstituted naphthyl group, or a substituted or unsubstituted fluorenyl group. However, a case in which L₂ and Ar₂₋₁ are phenyl groups at the same time is excluded.

In the disclosure, any one among A¹ to A⁶ in Formula 1 may be represented by Formula 2-1.

In the disclosure, A⁷ or A¹⁰ in Formula 1 may be represented by Formula 2-1.

In the invention, the substituted amine group is represented by Formula 2-2:

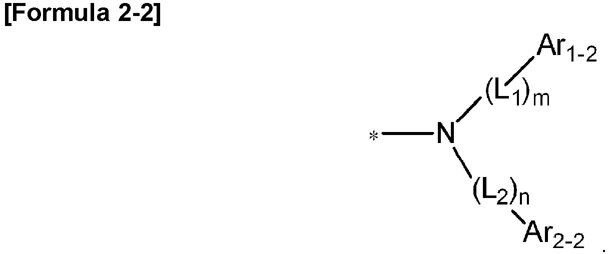

In Formula 2-2, L₁ and L₂ are each independently a bond, a substituted or unsubstituted arylene group comprising 6 to 30 ring forming carbon atoms, or a substituted or unsubstituted heteroarylene group comprising 2 to 30 ring forming carbon atoms.

In Formula 2-2, "m" and "n" are each independently an integer of 0 to 2, and when "m" is 2 or more, a plurality of L₁ groups may be the same or different from each other, and when "n" is 2 or more, a plurality of L₂ groups may be the same or different from each other.

In Formula 2-2, Ar₁₋₂ and Ar₂₋₂ each independently a substituted or unsubstituted aryl group comprising 10 to 40 ring forming carbon atoms, or may be represented by Formula 3-3 or Formula 3-4, where at least one of Ar₁₋₂ and Ar₂₋₂ is represented by Formula 3-3.

In Formula 2-2, " 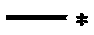 " indicates a position connected with Formula 1.

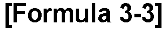

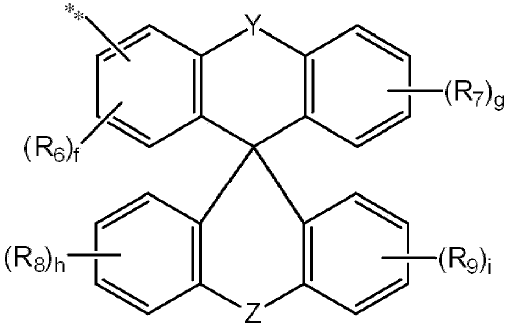

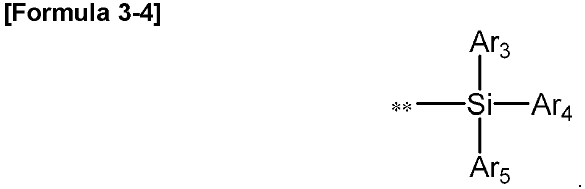

In Formula 3-3, Y and Z may each independently be a bond, O, or S, and at least one of Y and Z is O or S.

In Formula 3-3, R₆ to R₉ are each independently a hydrogen atom, a deuterium atom, a halogen atom, a substituted or unsubstituted alkyl group comprising 1 to 20 carbon atoms, a substituted or unsubstituted aryl group comprising 6 to 30 ring forming carbon atoms, or a substituted or unsubstituted heteroaryl group comprising 2 to 30 ring forming carbon atoms.

In Formula 3-3, "f" is an integer of 0 to 3, and when "f" is 2 or more, a plurality of R₆ groups may be the same or different from each other.

In Formula 3-3, "g" to "i" are each independently an integer of 0 to 4, and when "g" is 2 or more, a plurality of R₇ groups may be the same or different from each other, when "h" is 2 or more, a plurality of R₈ groups may be the same or different from each other, and when "i" is 2 or more, a plurality of R₉ groups may be the same or different from each other.

In Formula 3-4, Ar₃ to Ar₅ are each independently a substituted or unsubstituted aryl group comprising 6 to 30 ring forming carbon atoms.

In Formula 3-3 and Formula 3-4, " 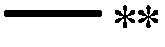 " indicates a position connected with Formula 2-2.

In an embodiment, L₁ and L₂ in Formula 2-2 may be a bond, a substituted or unsubstituted phenylene group, or a substituted or unsubstituted naphthyl group.

In an embodiment, L₂ in Formula 2-2 is a bond or a substituted or unsubstituted phenyl group, and when "n" is 1, each Ar₂₋₂ may independently be a substituted or unsubstituted phenyl group, a substituted or unsubstituted biphenyl group, a substituted or unsubstituted terphenyl group, a substituted or unsubstituted naphthyl group, or a substituted or unsubstituted fluorenyl group. However, a case in which L₂ and Ar₂₋₂ are phenyl groups at the same time is excluded.

In an embodiment, any one among A¹ to A⁶ in Formula 1 may be represented by Formula 2-2.

In an embodiment, any one among A⁷ to A¹⁰ in Formula 1 may be represented by Formula 2-2.

In the disclosure, the substituted amine group is represented by Formula 2-3:

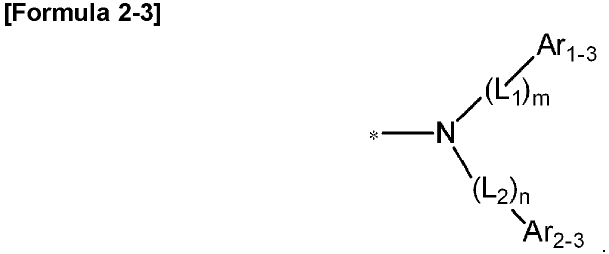

In Formula 2-3, L₁ and L₂ are each independently a bond, a substituted or unsubstituted arylene group comprising 6 to 30 ring forming carbon atoms, or a substituted or unsubstituted heteroarylene group comprising 2 to 30 ring forming carbon atoms.

In Formula 2-3, "m" and "n" are each independently an integer of 0 to 2, and when "m" is 2 or more, a plurality of L₁ groups may be the same or different from each other, and when "n" is 2 or more, a plurality of L₂ may be the same or different from each other.

In Formula 2-3, Ar₁₋₃ and Ar₂₋₃ are each independently a substituted or unsubstituted aryl group comprising 10 to 40 ring forming carbon atoms, or may be represented by Formula 3-5 or Formula 3-6 below, where at least one of Ar₁₋₃ and Ar₂₋₃ is represented by Formula 3-5 or Formula 3-6.

In Formula 2-3, " 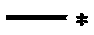 " indicates a position connected with Formula 1.

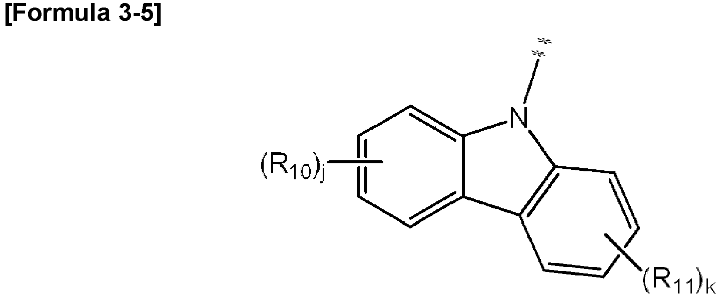

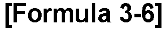

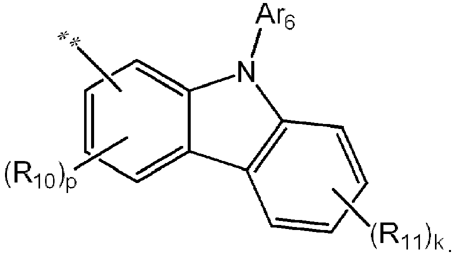

In Formula 3-5 and Formula 3-6, R₁₀ and R₁₁ are each independently a hydrogen atom, a deuterium atom, a halogen atom, a substituted or unsubstituted alkyl group comprising 1 to 20 carbon atoms, a substituted or unsubstituted aryl group comprising 6 to 30 ring forming carbon atoms, or a substituted or unsubstituted heteroaryl group comprising 2 to 30 carbon atoms.

In Formula 3-5 and Formula 3-6, "j" and "k" are each independently an integer of 0 to 4, and when "j" is 2 or more, a plurality of R₁₀ groups may be the same or different from each other, and when "k" is 2 or more, a plurality of R₁ groups may be the same or different from each other.

In Formula 3-6, "p" is an integer of 0 to 3, and when "p" is 2 or more, a plurality of R₁₀ groups may be the same or different from each other.

In Formula 3-6, Ar₆ is a substituted or unsubstituted aryl group comprising 6 to 30 ring forming carbon atoms.

In Formula 3-5 and Formula 3-6, " 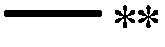 " indicates a position connected with Formula 2-3.

L₁ and L₂ in Formula 2-3 may each independently be a bond, a substituted or unsubstituted phenylene group, or a substituted or unsubstituted naphthyl group.

In an aspect of the disclosure, L₂ of Formula 2-3 is a bond or a substituted or unsubstituted phenyl group, and when "n" is 1, each Ar₂₋₃ may independently be a substituted or unsubstituted phenyl group, a substituted or unsubstituted biphenyl group, a substituted or unsubstituted terphenyl group, a substituted or unsubstituted naphthyl group, or a substituted or unsubstituted fluorenyl group. However, a case in which L₂ and Ar₂₋₃ are phenyl groups at the same time is excluded.

In the disclosure, any one among A¹ to A⁶ in Formula 1 may be represented by Formula 2-3.

In the disclosure, any one among A⁷ to A¹⁰ in Formula 1 may be represented by Formula 2-3.

In the polycyclic compound of the present disclosure, the polycyclic compound represented by Formula 1 makes a direct linkage (e.g., is directly linked) with the amine group represented by Formula 2-1 to Formula 2-3 without a linker therebetween.

In the disclosure, Formula 2-1 is represented by Formula 4-1 or Formula 4-2:

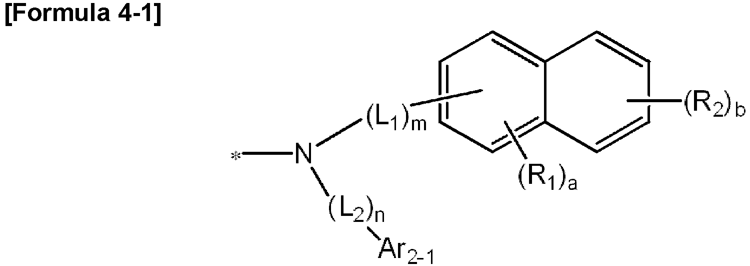

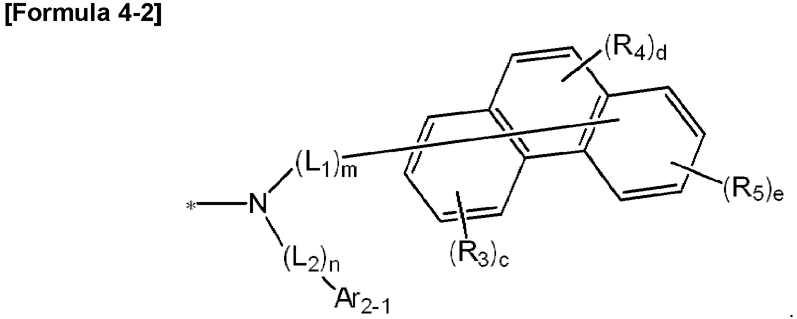

In Formula 4-1 and Formula 4-2, Ar₂₋₁ is a substituted or unsubstituted aryl group comprising 10 to 40 ring forming carbon atoms.

In Formula 4-1 and Formula 4-2, L₁, L₂, "m" and "n" are the same as defined in Formula 2-1, and R₁ to R₅, and "a" to "e" are the same as defined in Formula 3-1 and Formula 3-2.

In Formula 4-1 and Formula 4-2, " 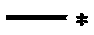 " indicates a position connected with Formula 1.

In an embodiment, Formula 2-2 is represented by Formula 5-1. In the disclosure, Formula 2-2 is represented by Formula 5-1 or Formula 5-2:

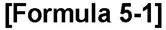

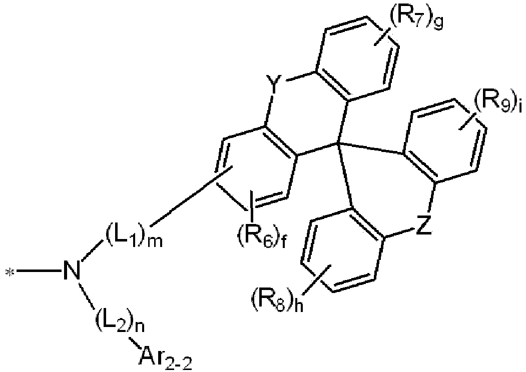

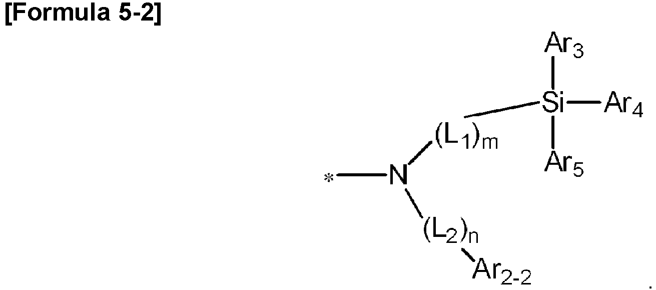

In Formula 5-1 and Formula 5-2, Ar₂₋₂ is a substituted or unsubstituted aryl group comprising 10 to 40 ring forming carbon atoms.

In Formula 5-1 and Formula 5-2, L₁, L₂, "m" and "n" are each independently the same as defined in Formula 2-2, and Y, Z, R₆ to R₉, "f" to "i", and Ar₃ to Ar₅ are each independently the same as defined in Formula 3-3 and Formula 3-4.

In Formula 5-1 and Formula 5-2, " 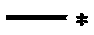 " indicates a position connected with Formula 1.

In the disclosure, Formula 2-3 is represented by Formula 6-1 or Formula 6-2:

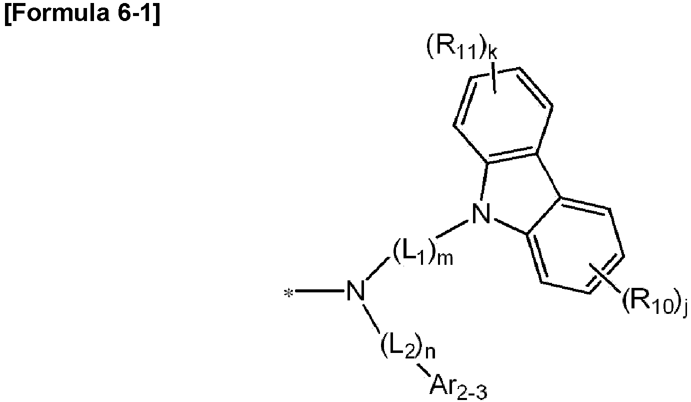

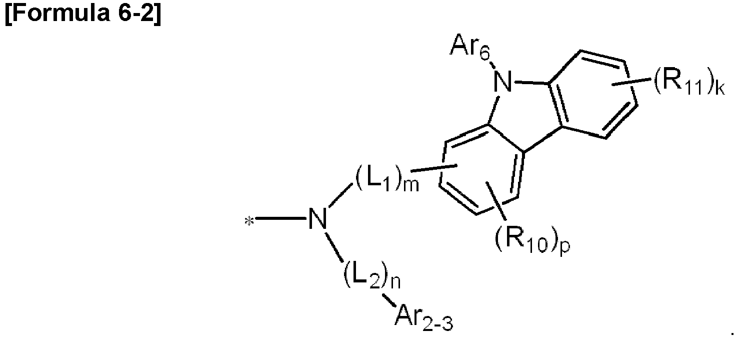

In Formula 6-1 and Formula 6-2, Ar₂₋₃ is a substituted or unsubstituted aryl group comprising 10 to 40 ring forming carbon atoms.

In Formula 6-1 and Formula 6-2, L₁, L₂, "m" and "n" are each independently the same as defined in Formula 2-3, and R₁₀ and R₁₁, Ar₆, "j", "k", and "p" are each independently the same as defined in Formula 3-5 and Formula 3-6.

In Formula 6-1 and Formula 6-2, " 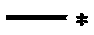 " indicates a position connected with Formula 1.

The polycyclic compound represented by Formula 1 according to an embodiment of the present invention may be at least one selected from the compounds represented in Compound Group 3 and the polycyclic compound represented by Formula 1 according to the disclosure may be at least one selected from the compounds represented in Compound Group 1 to Compound Group 6:

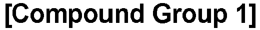

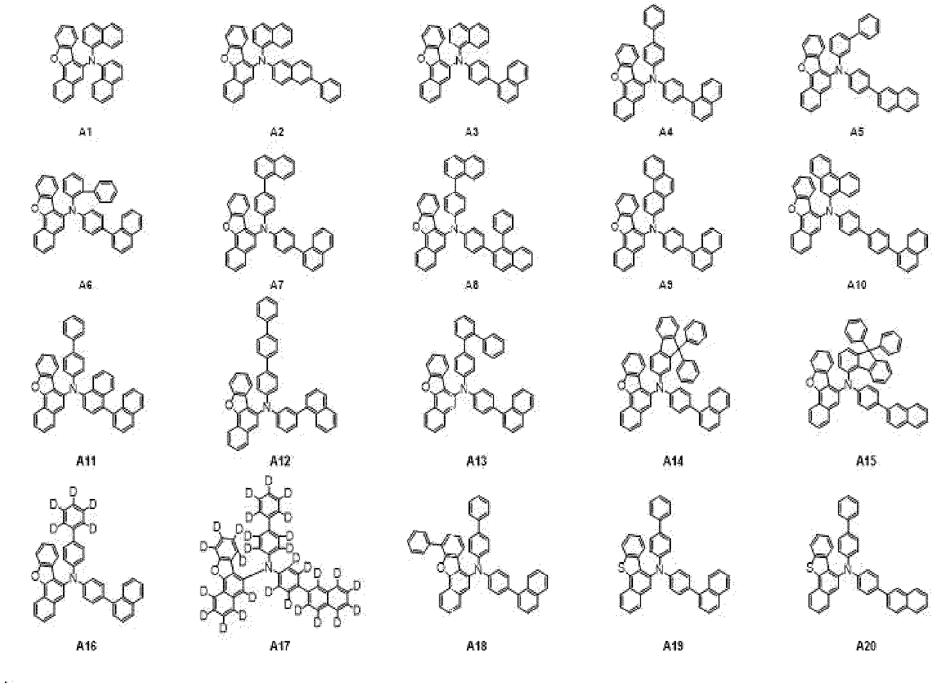

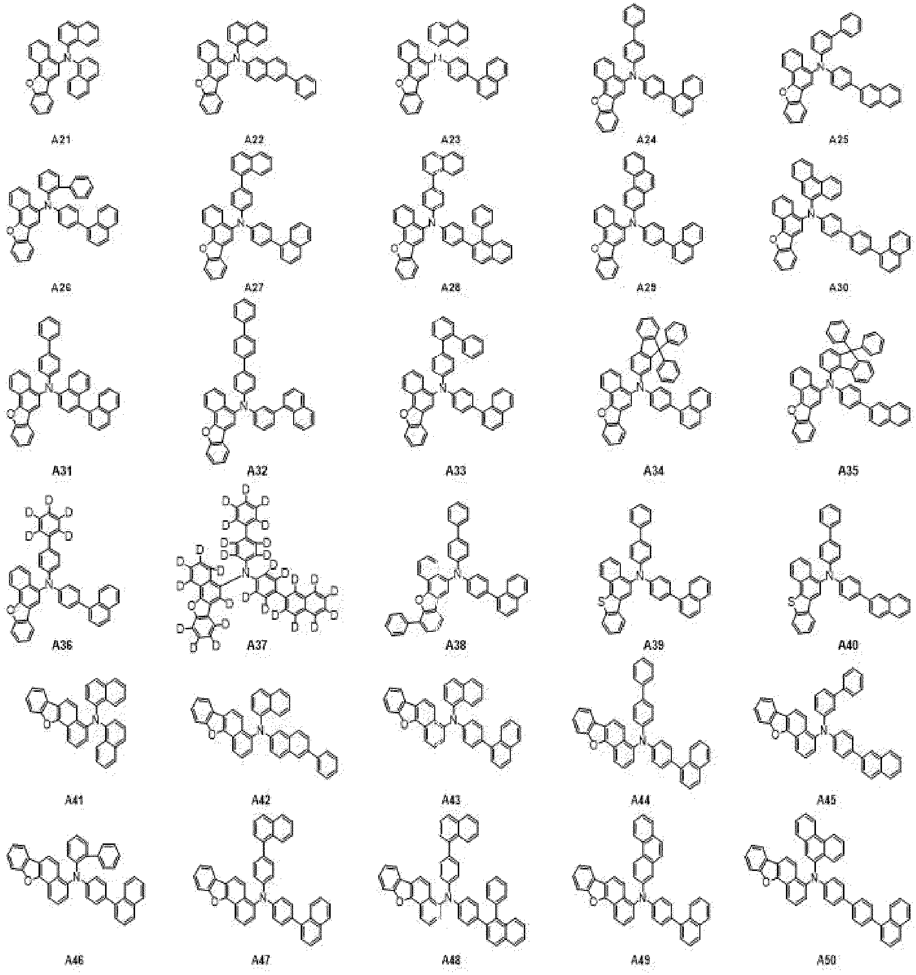

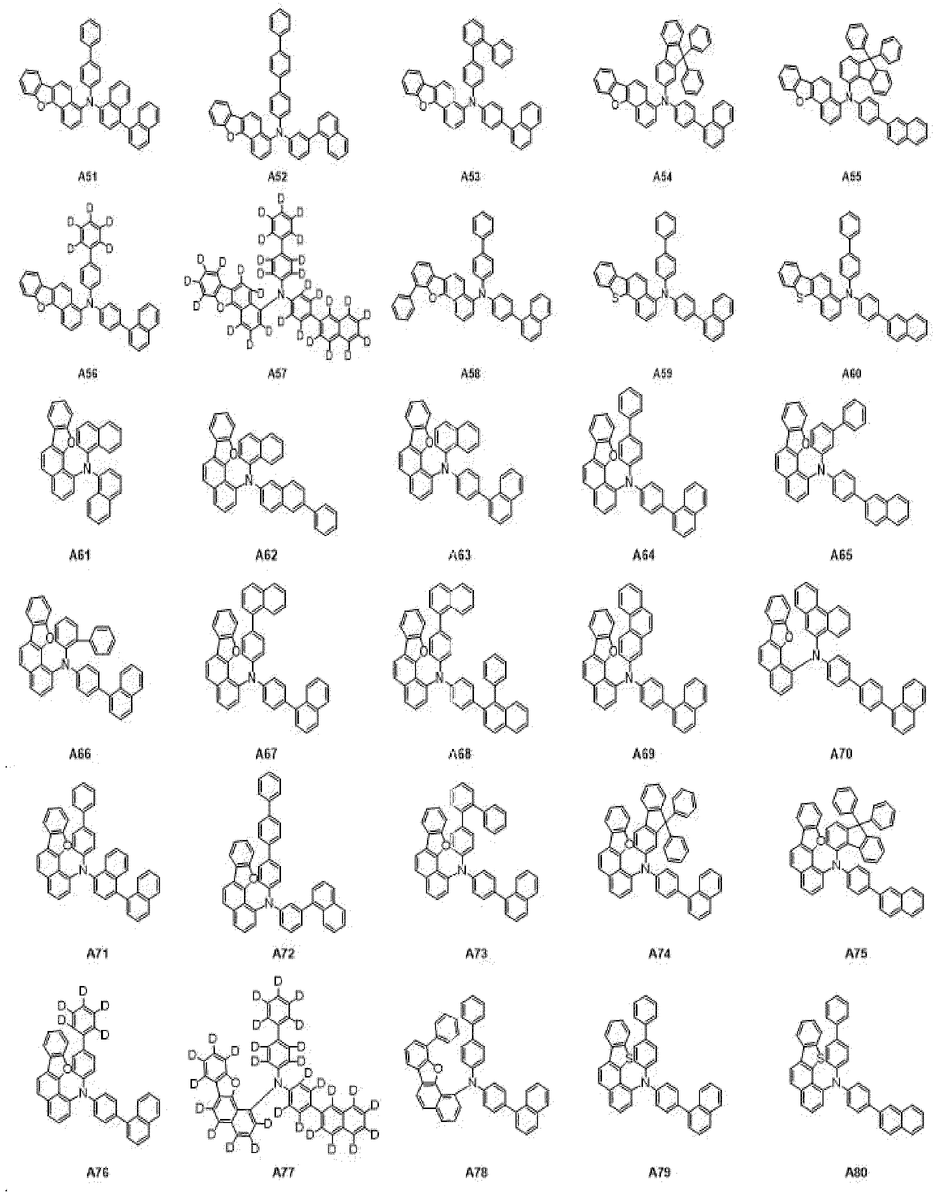

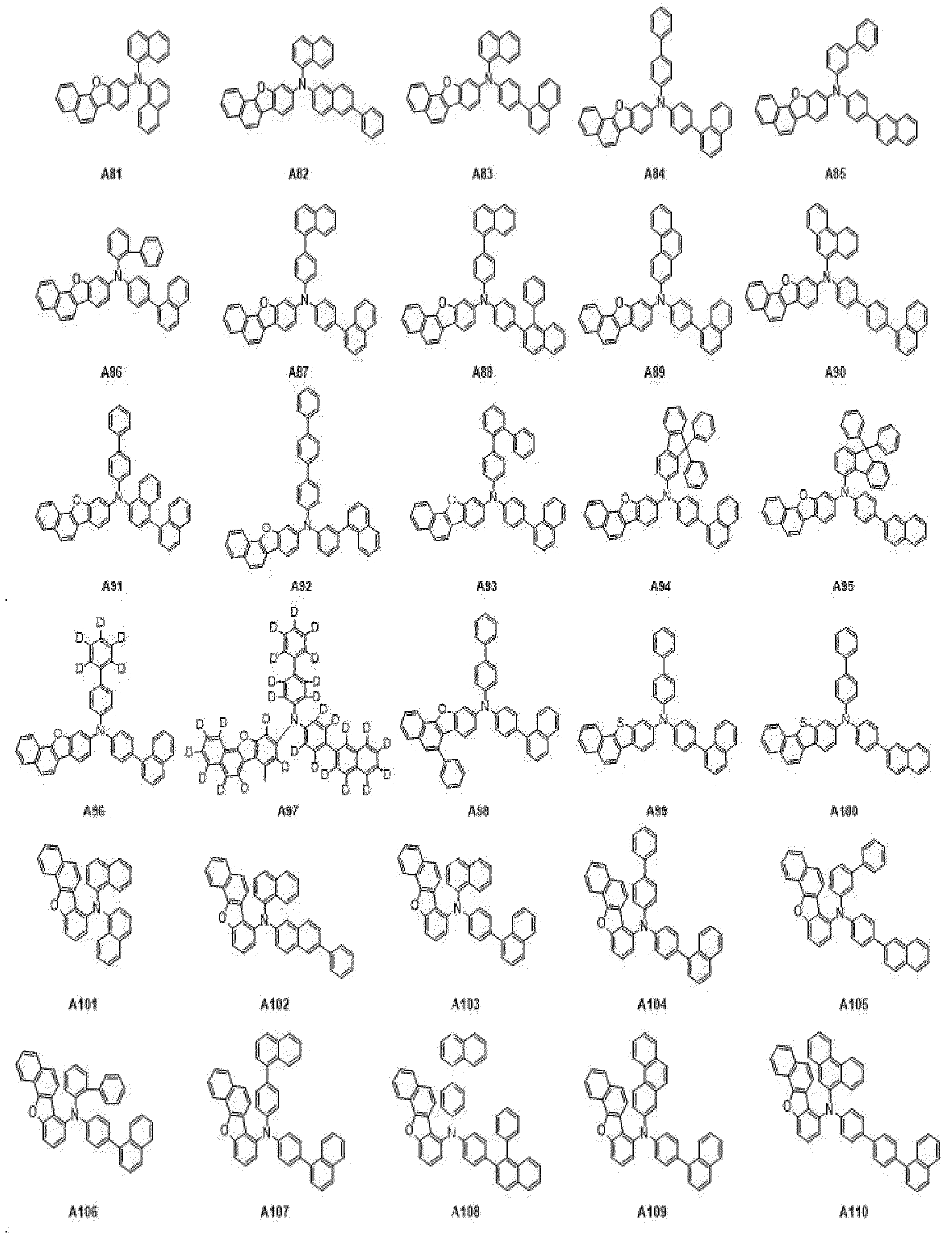

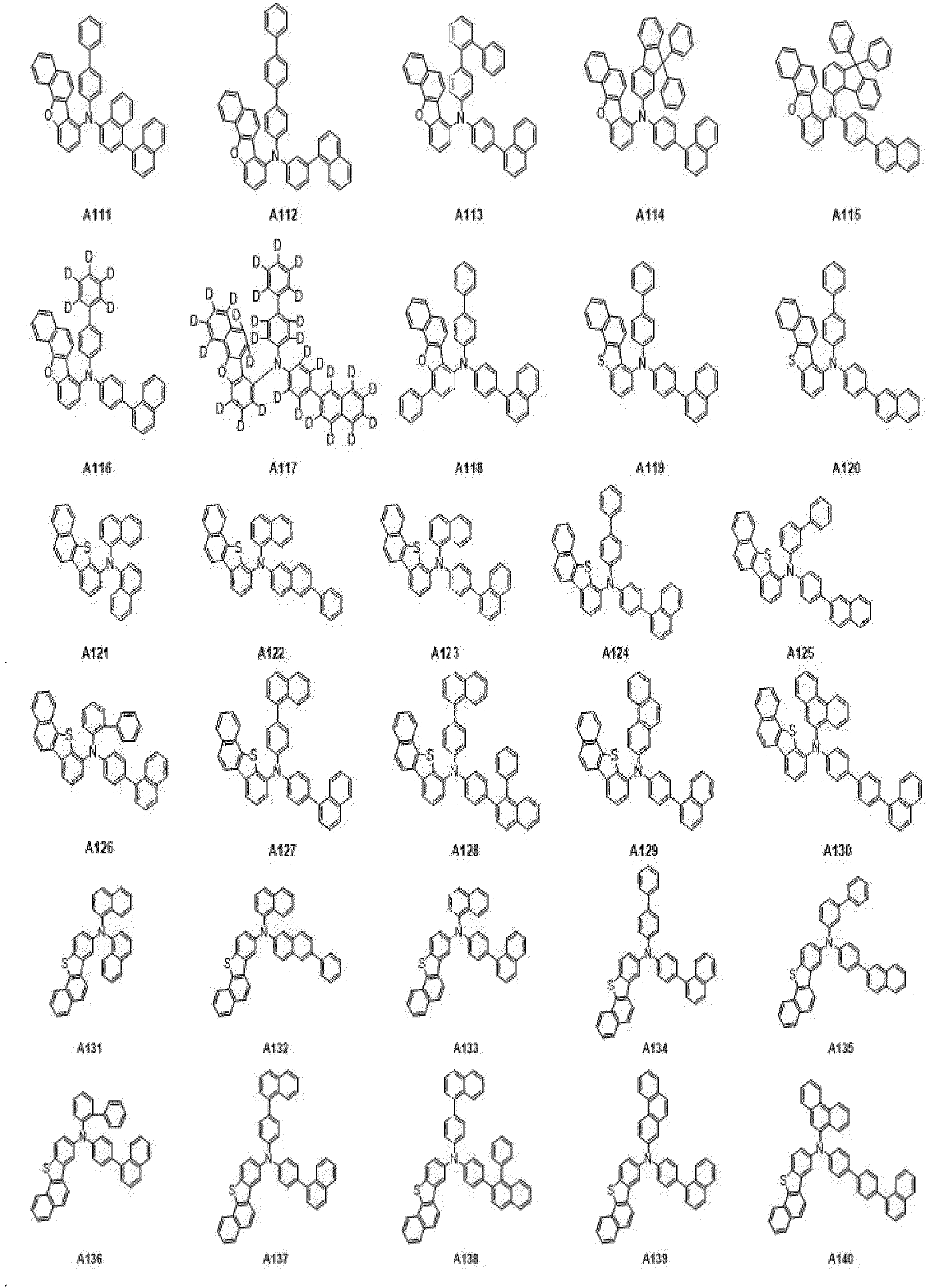

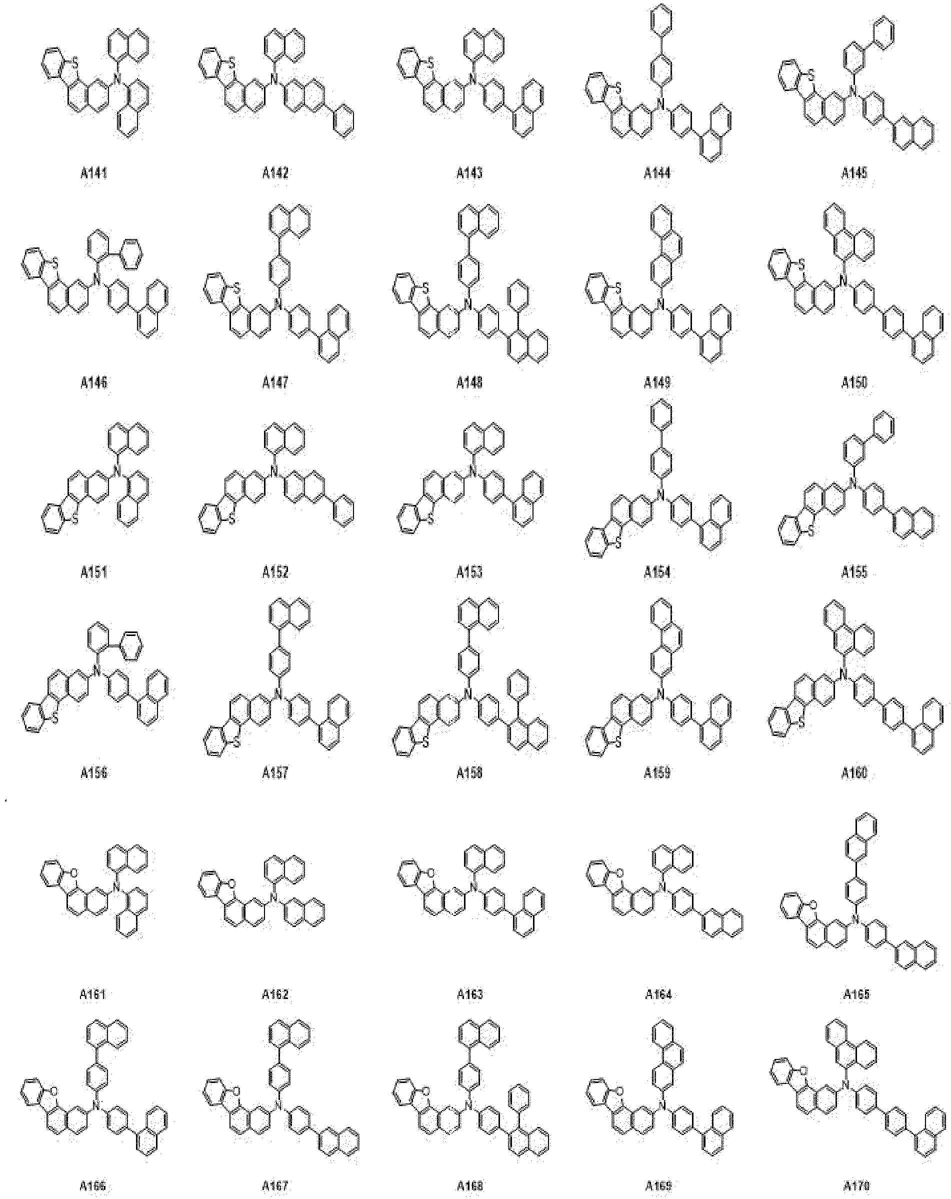

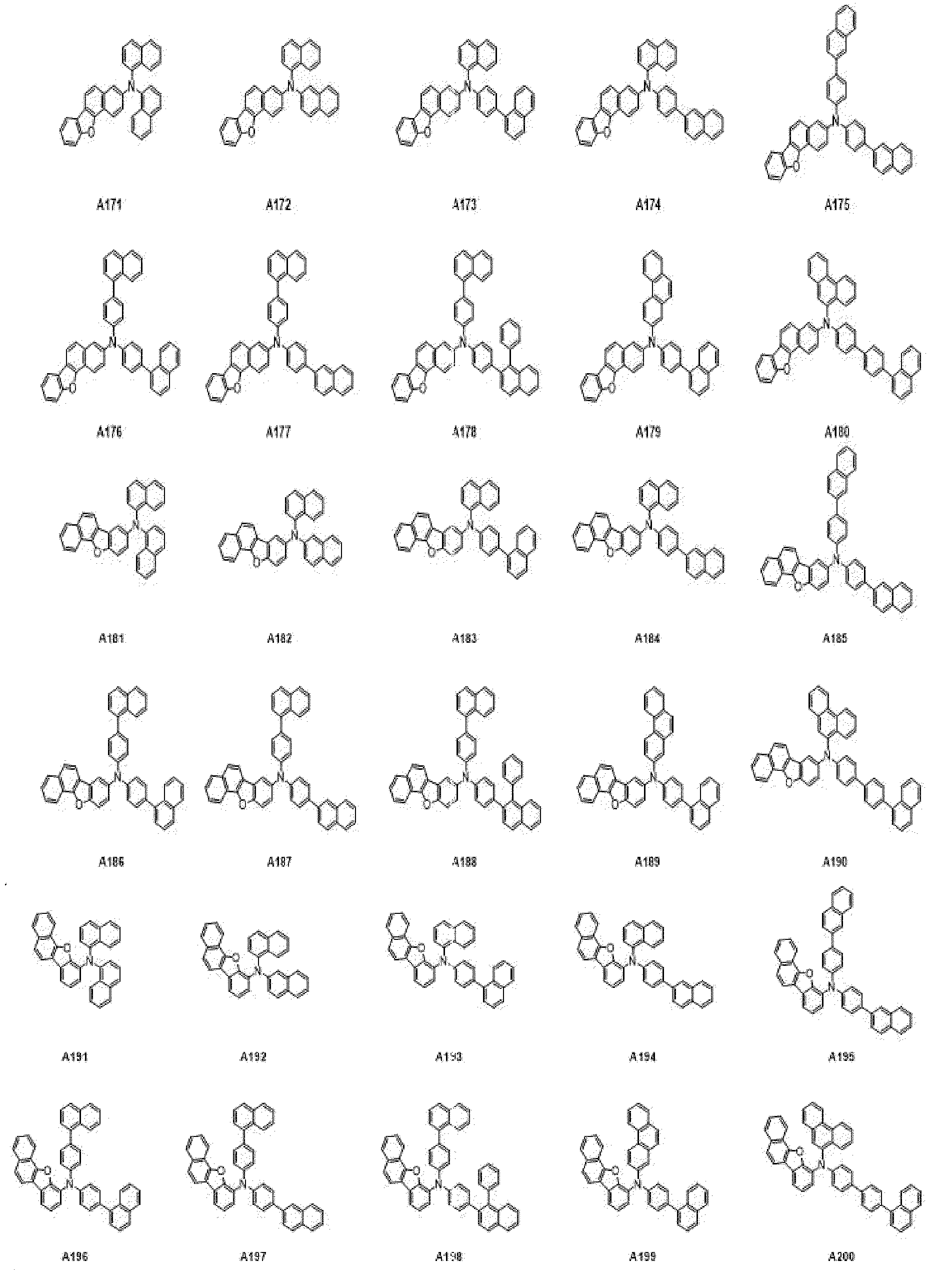

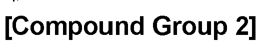

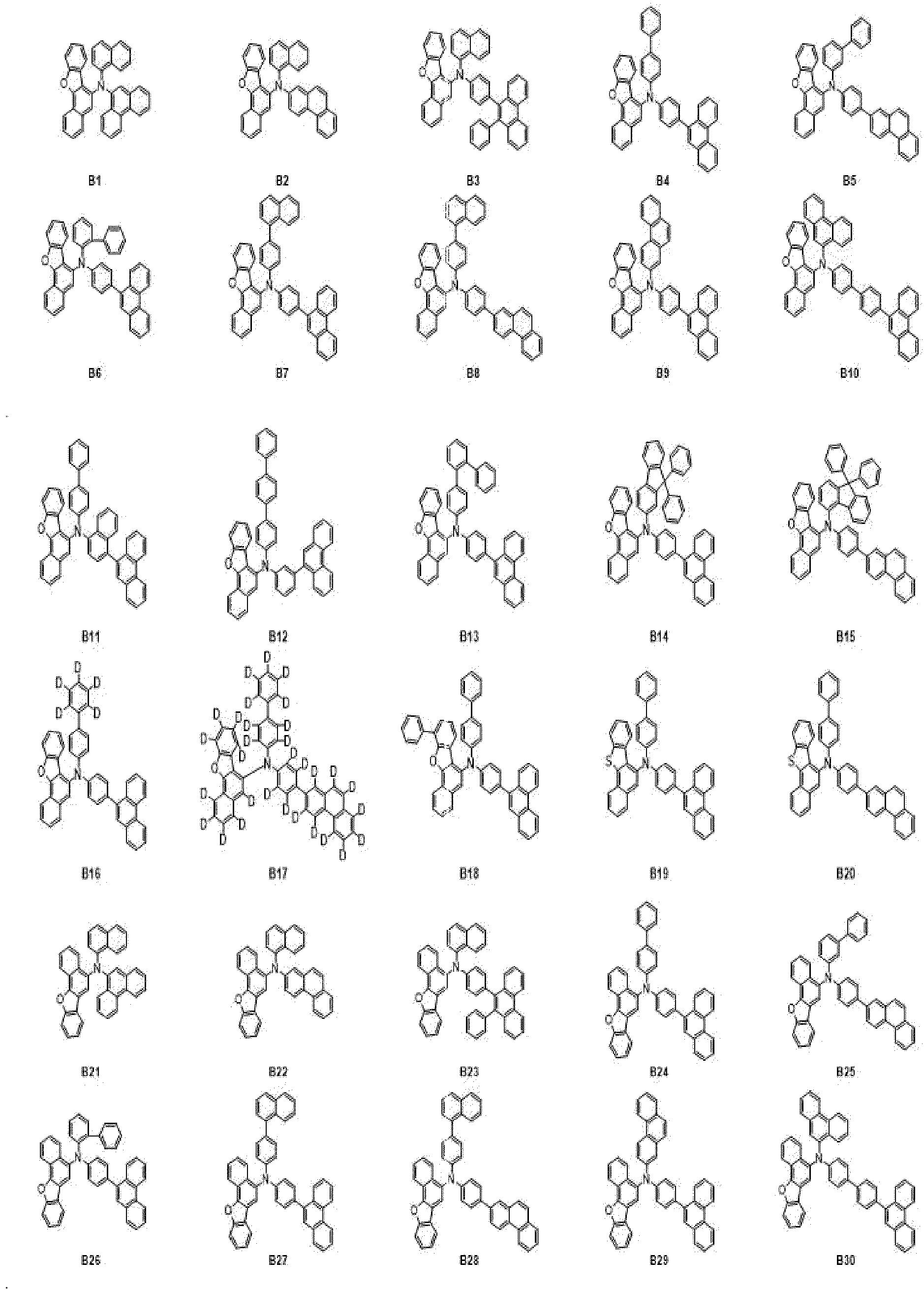

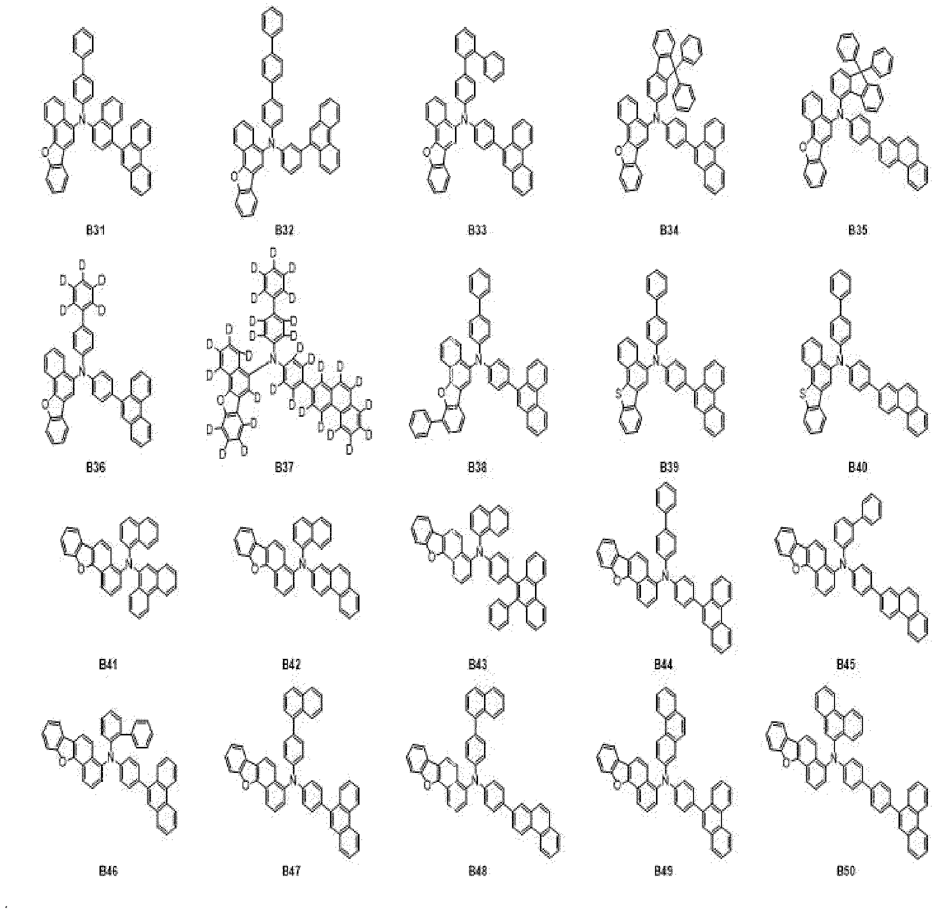

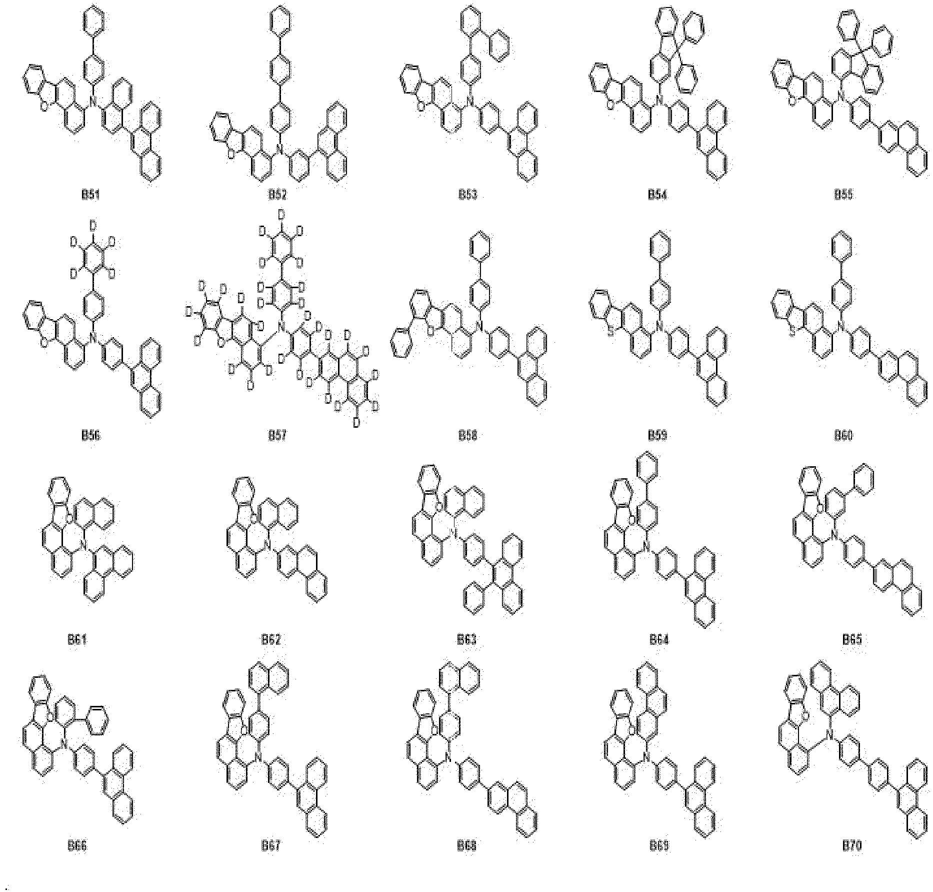

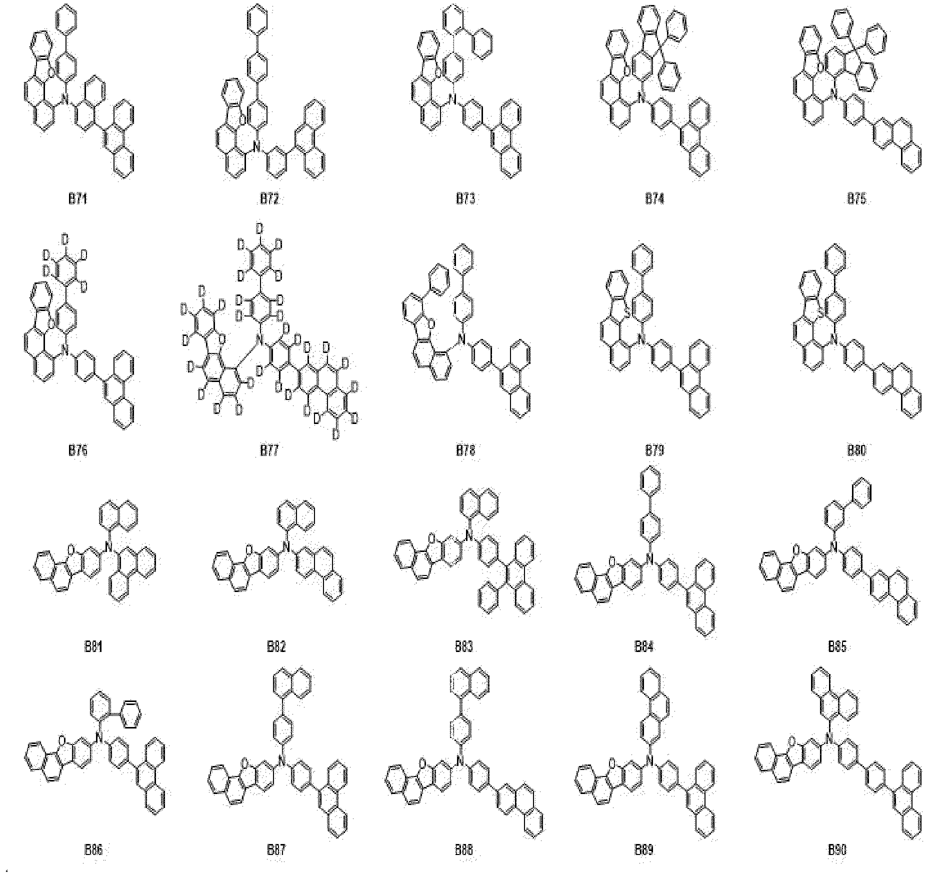

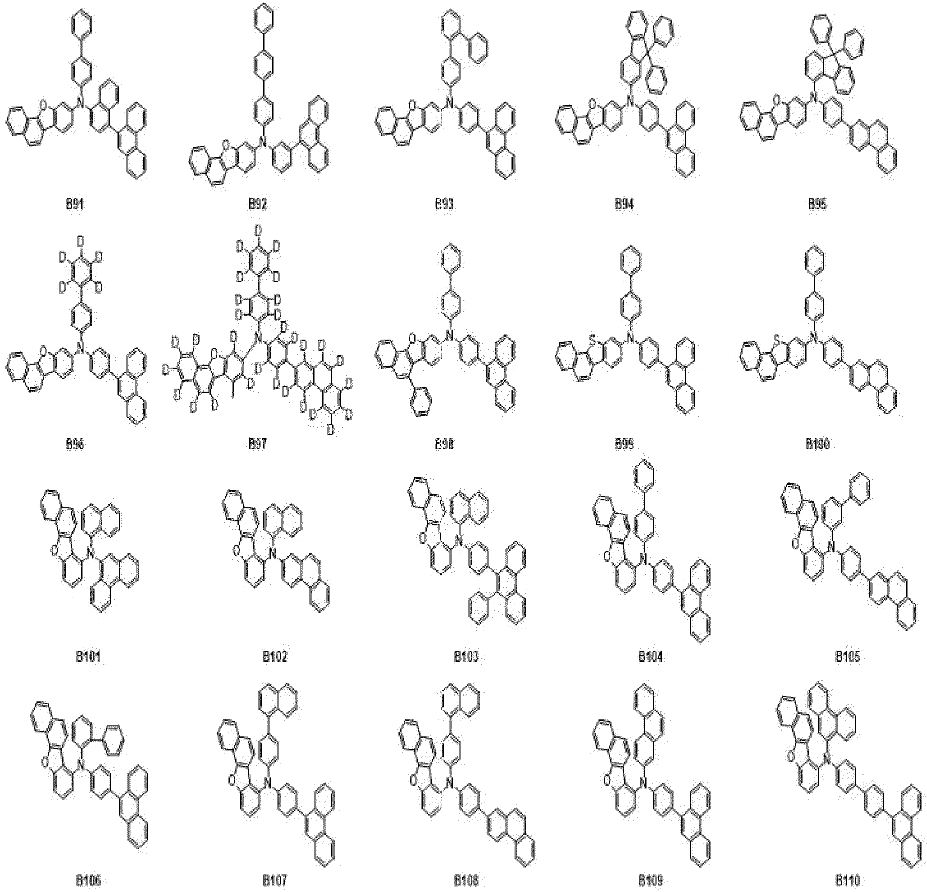

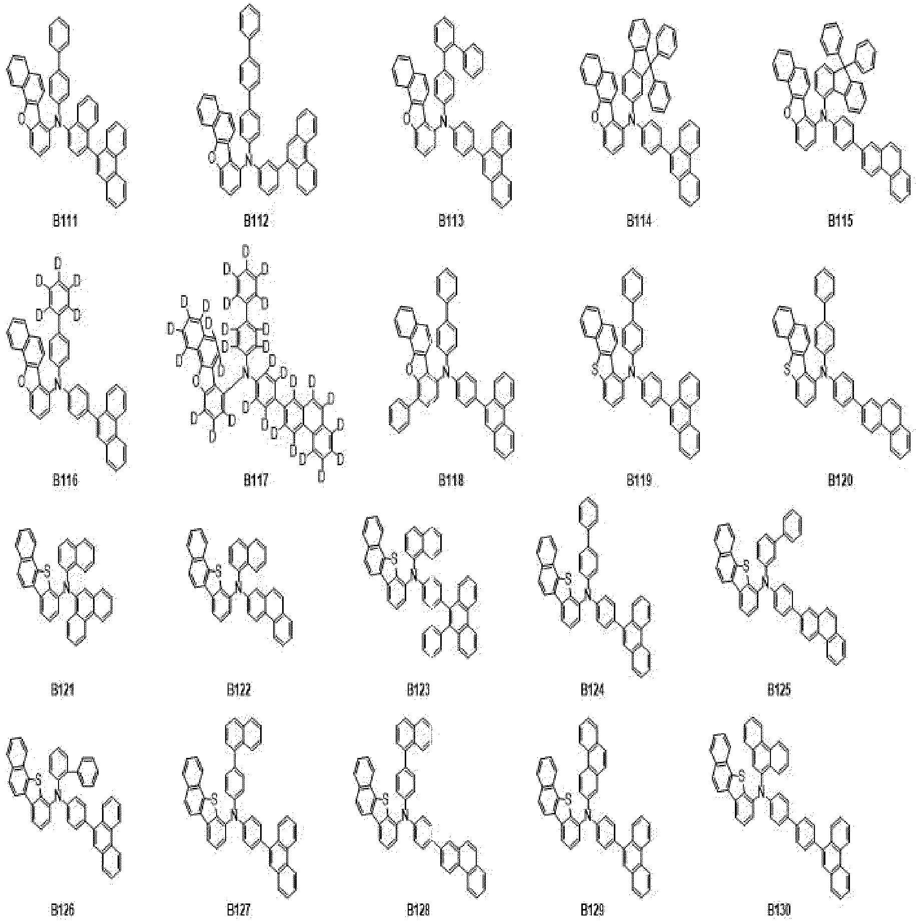

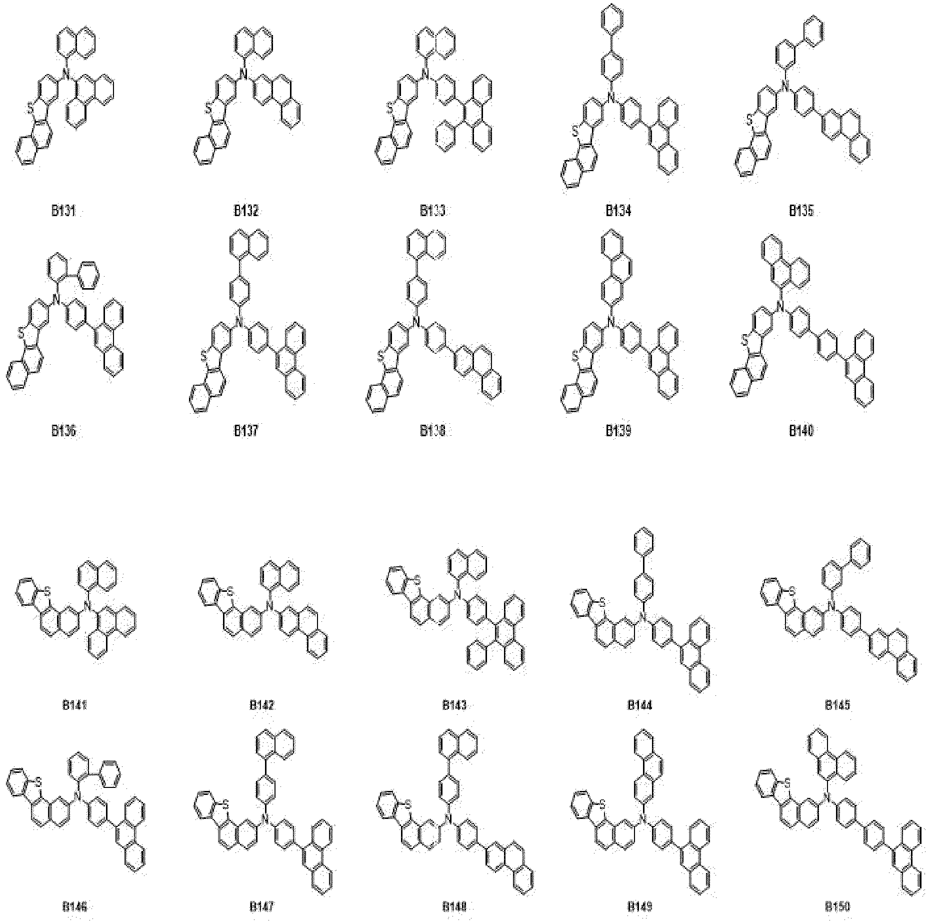

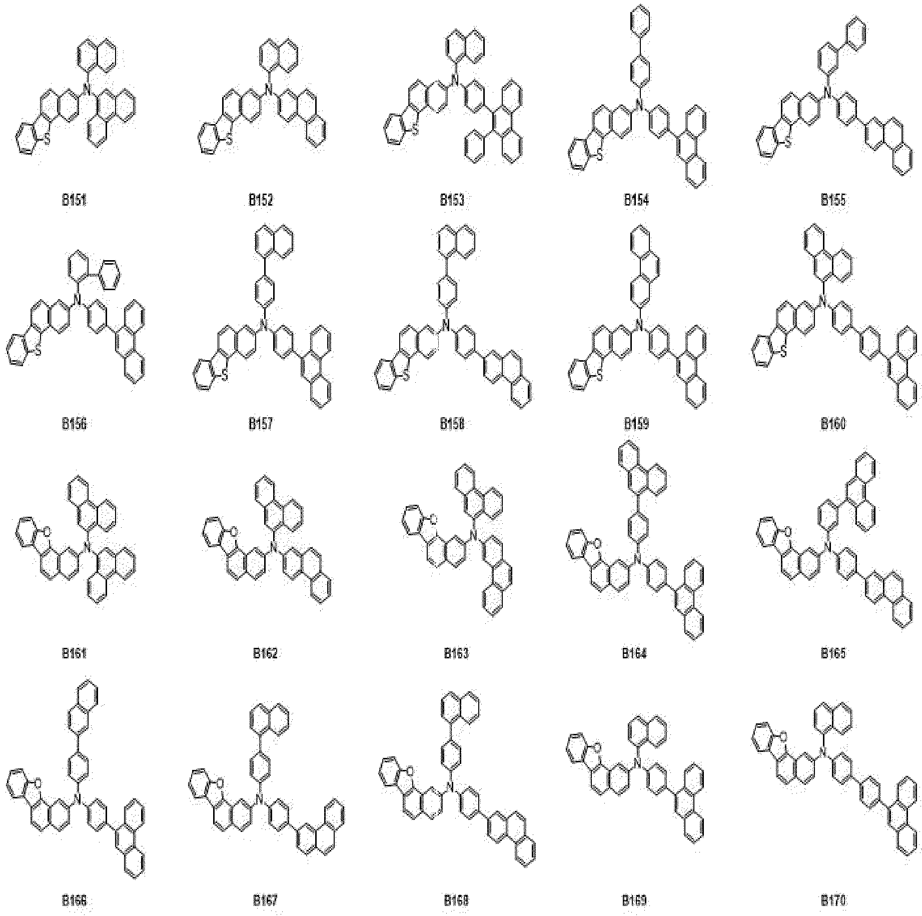

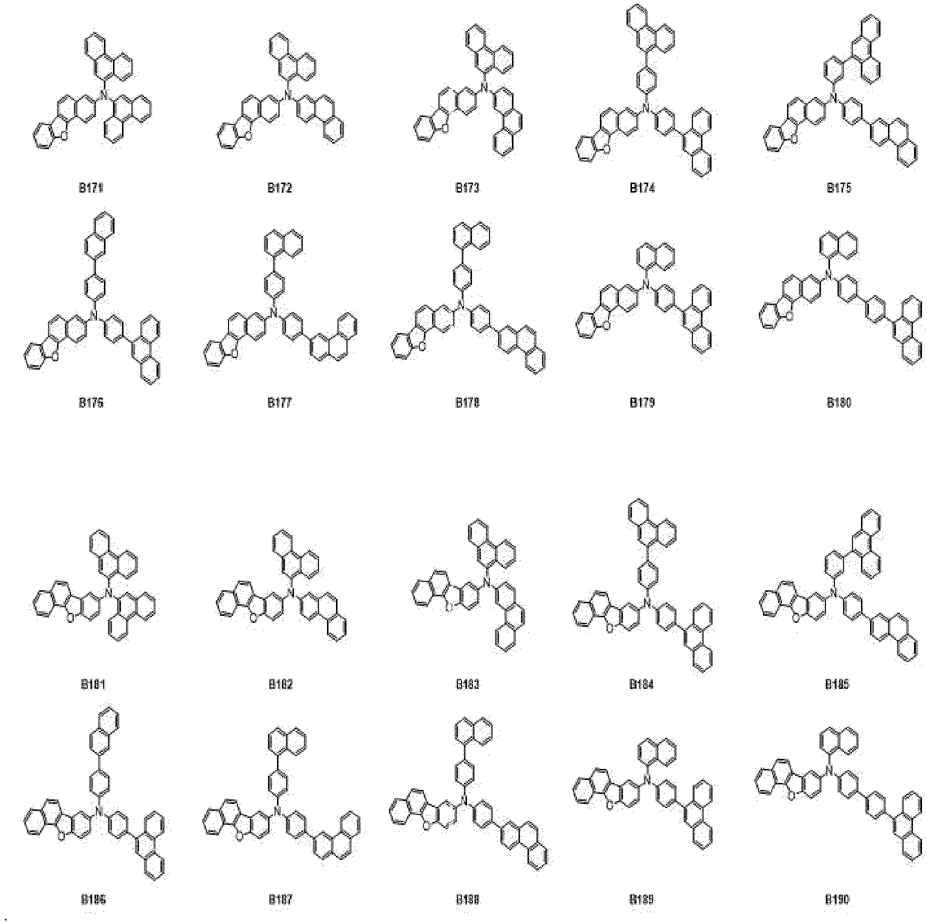

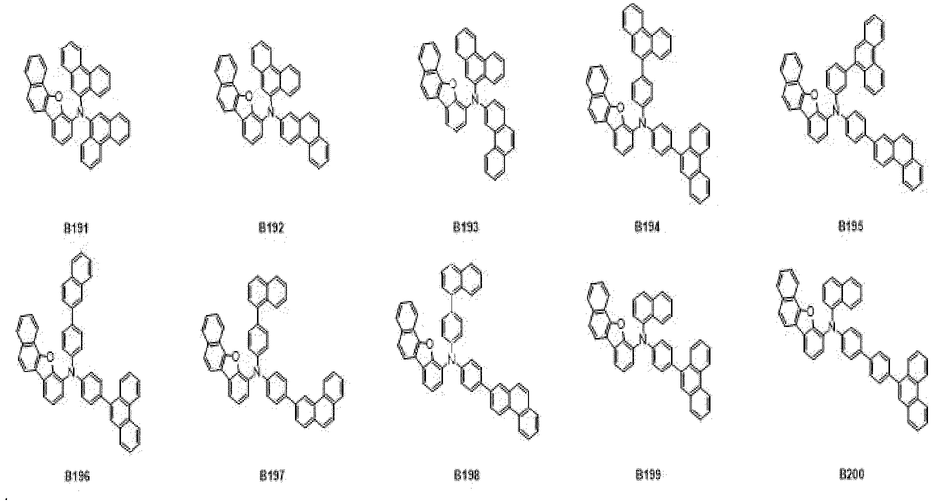

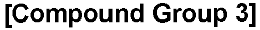

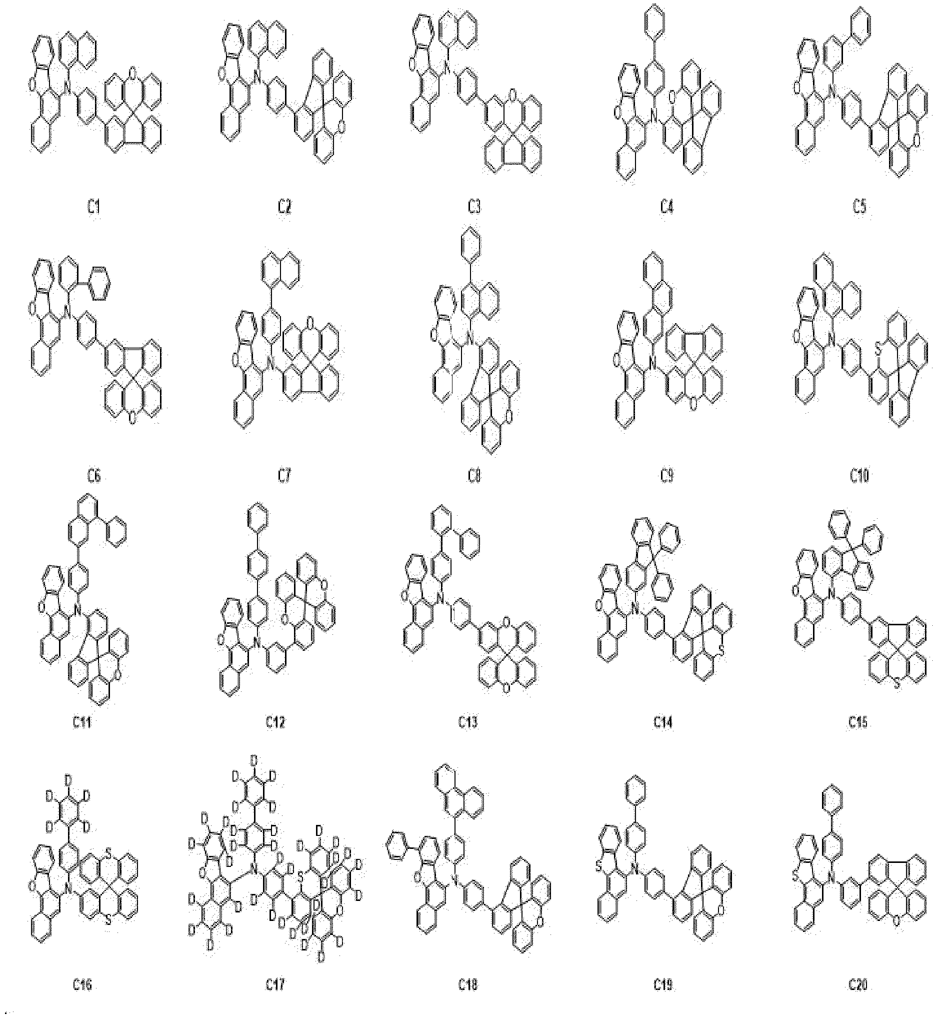

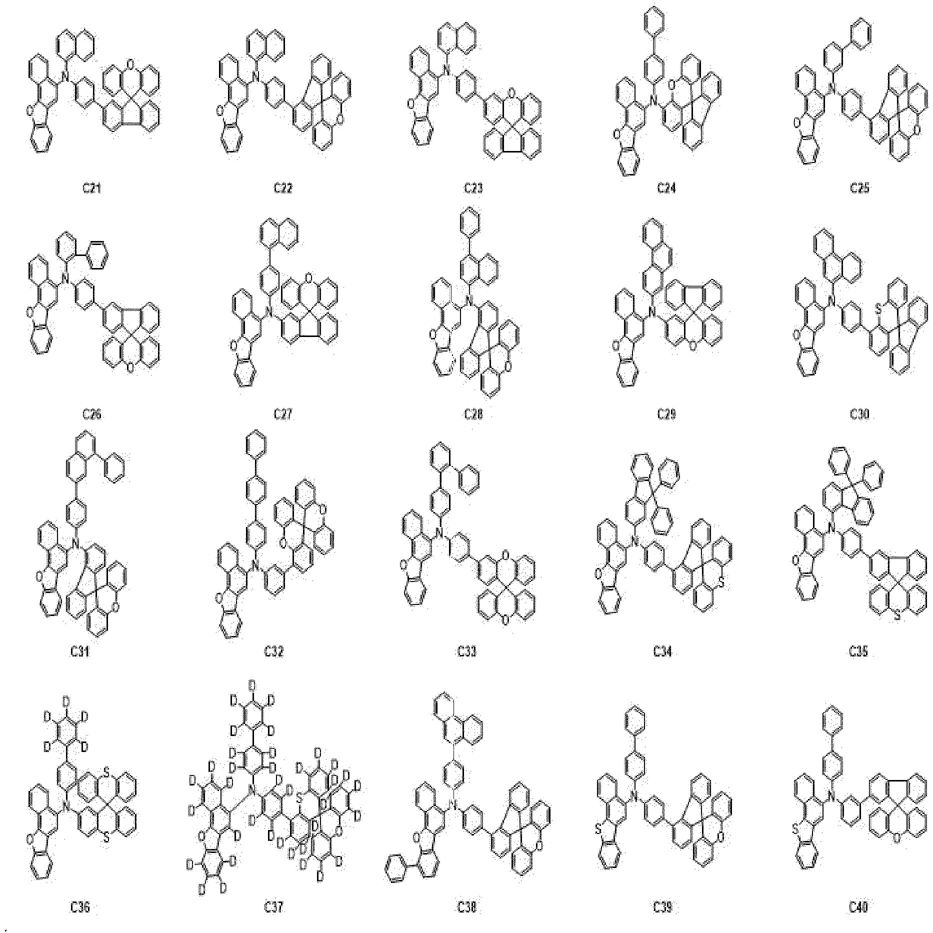

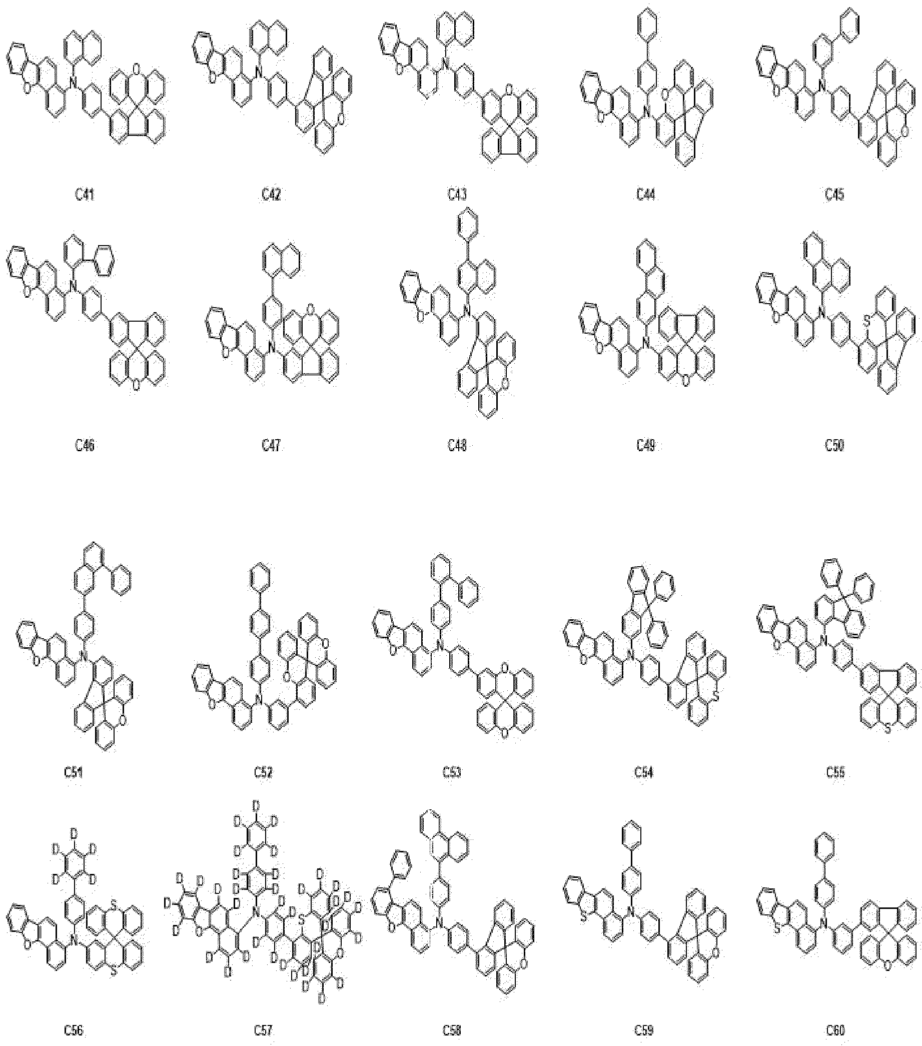

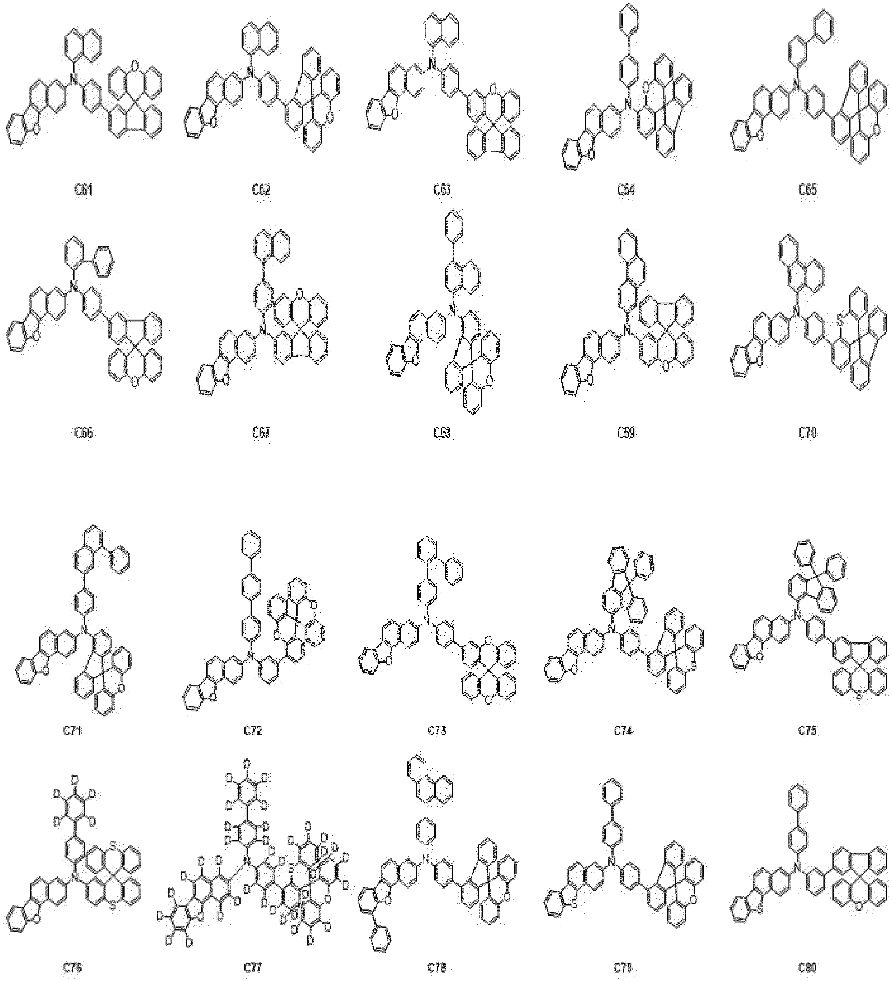

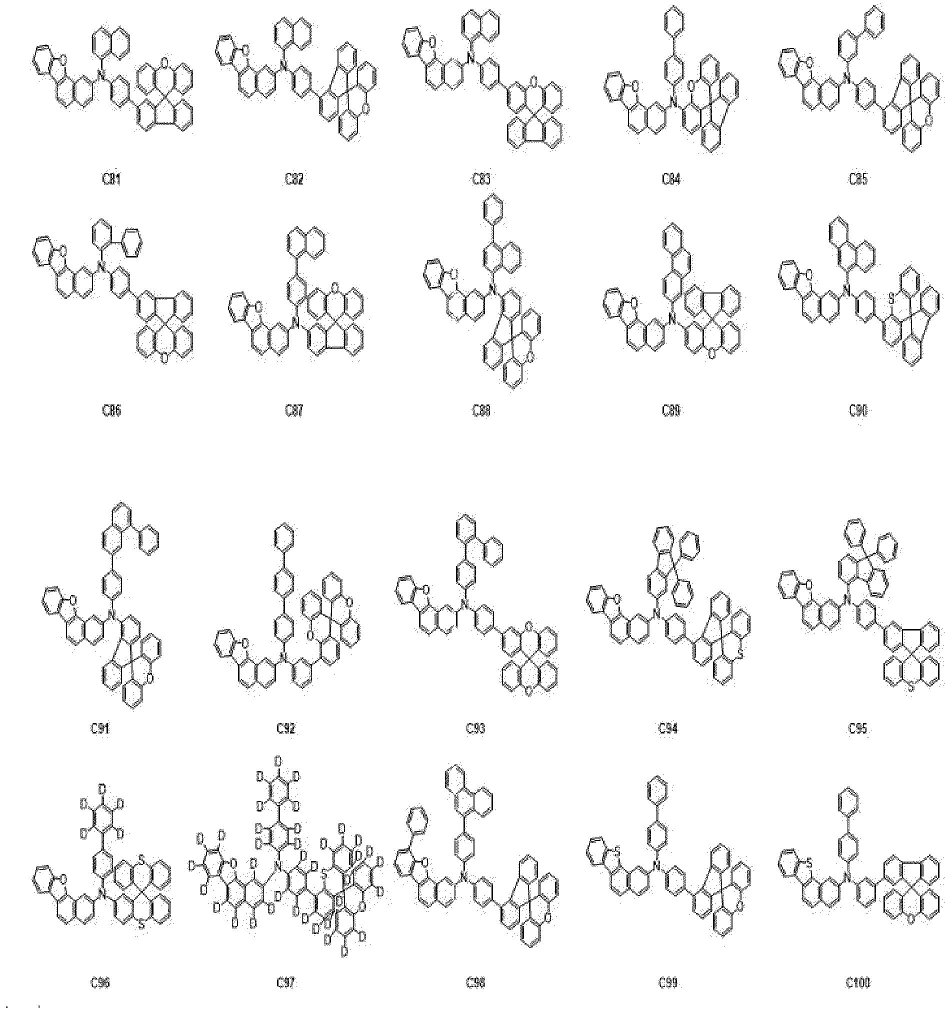

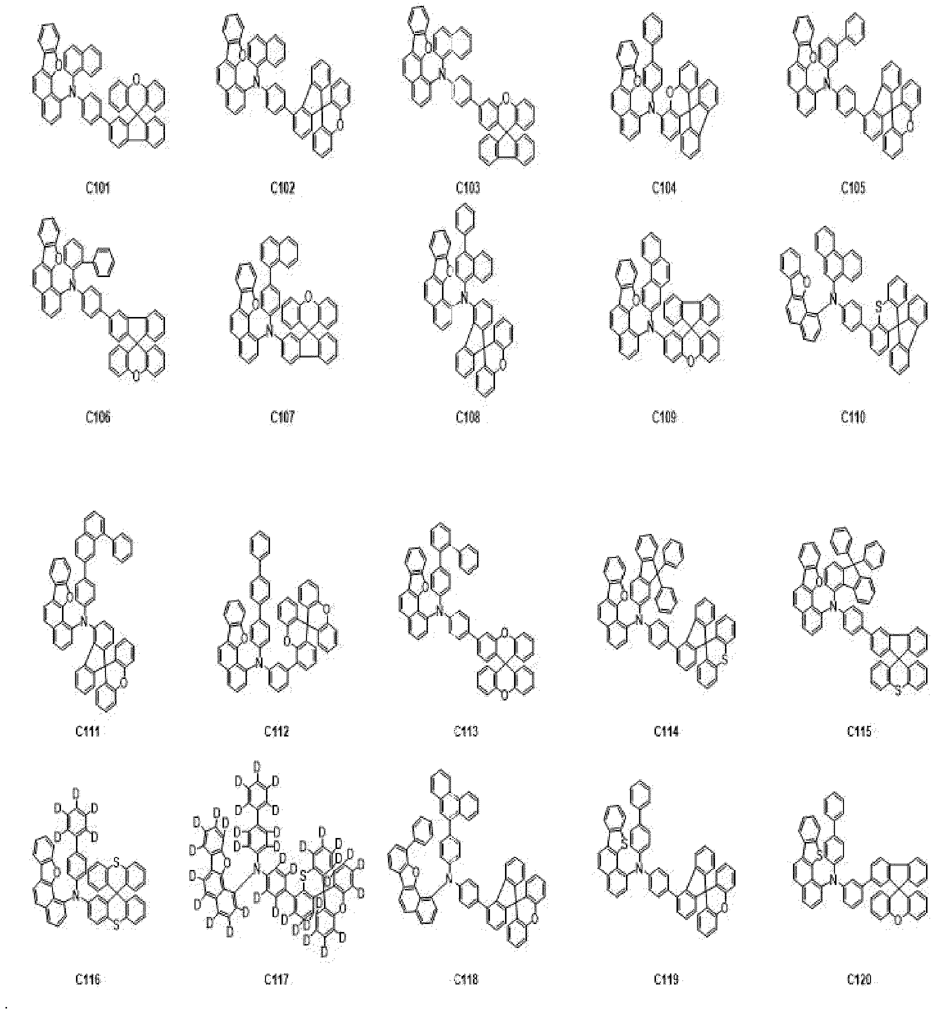

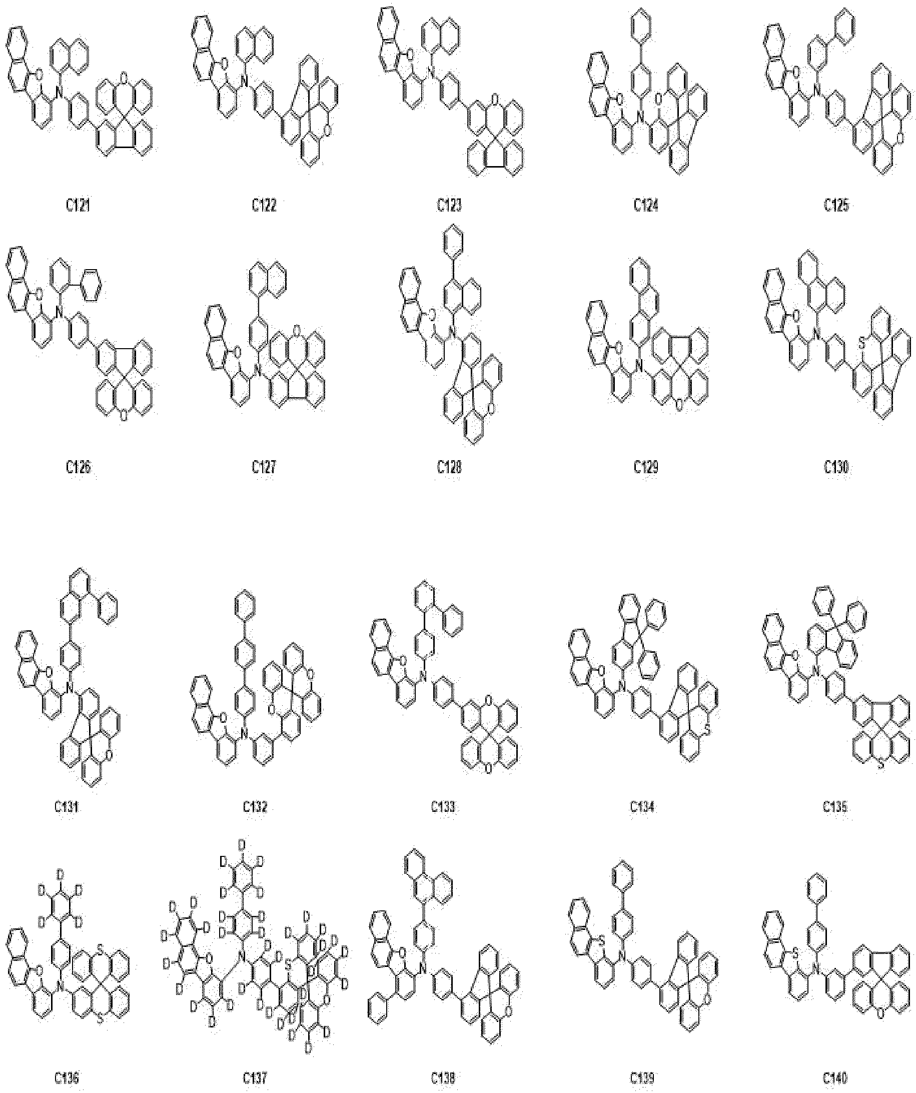

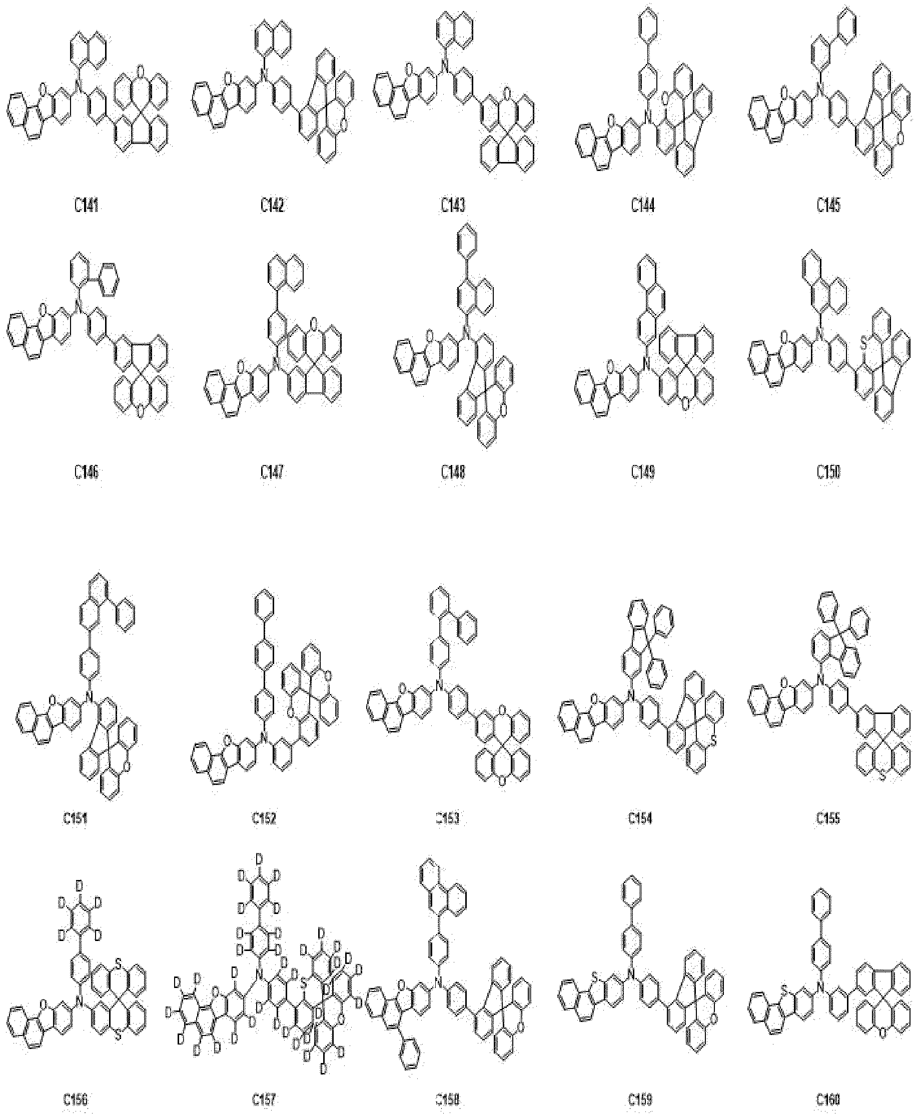

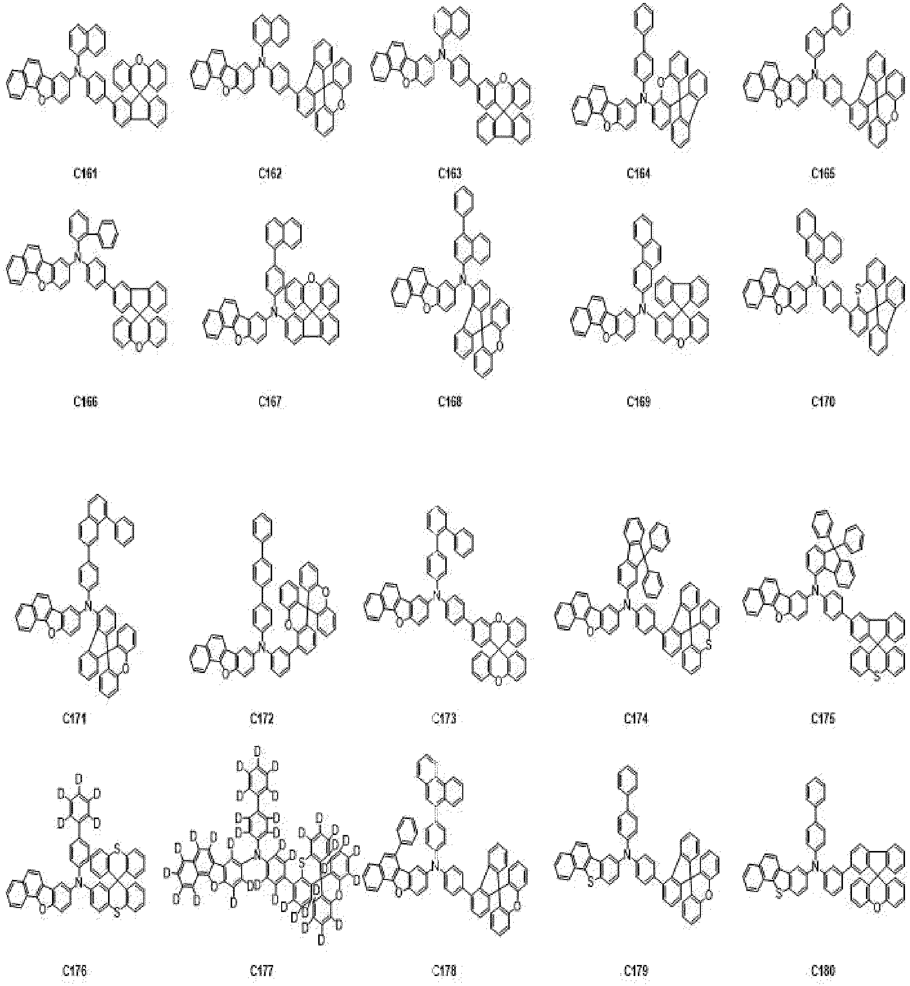

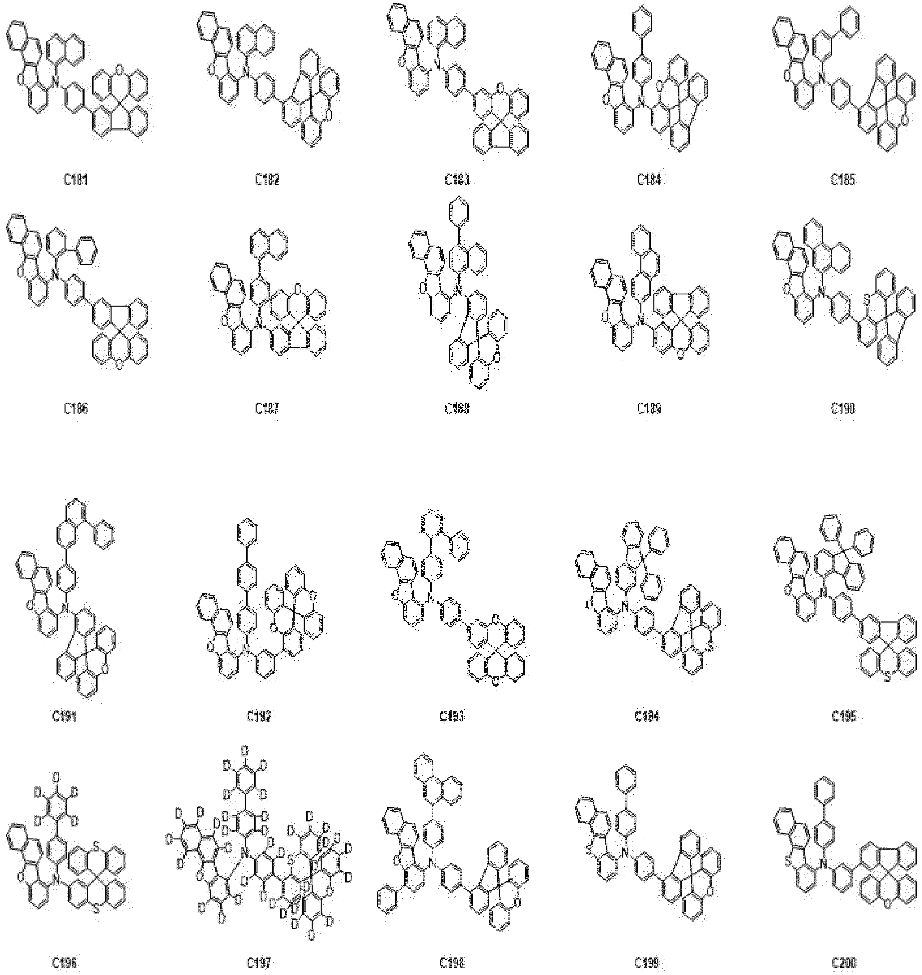

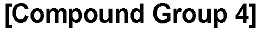

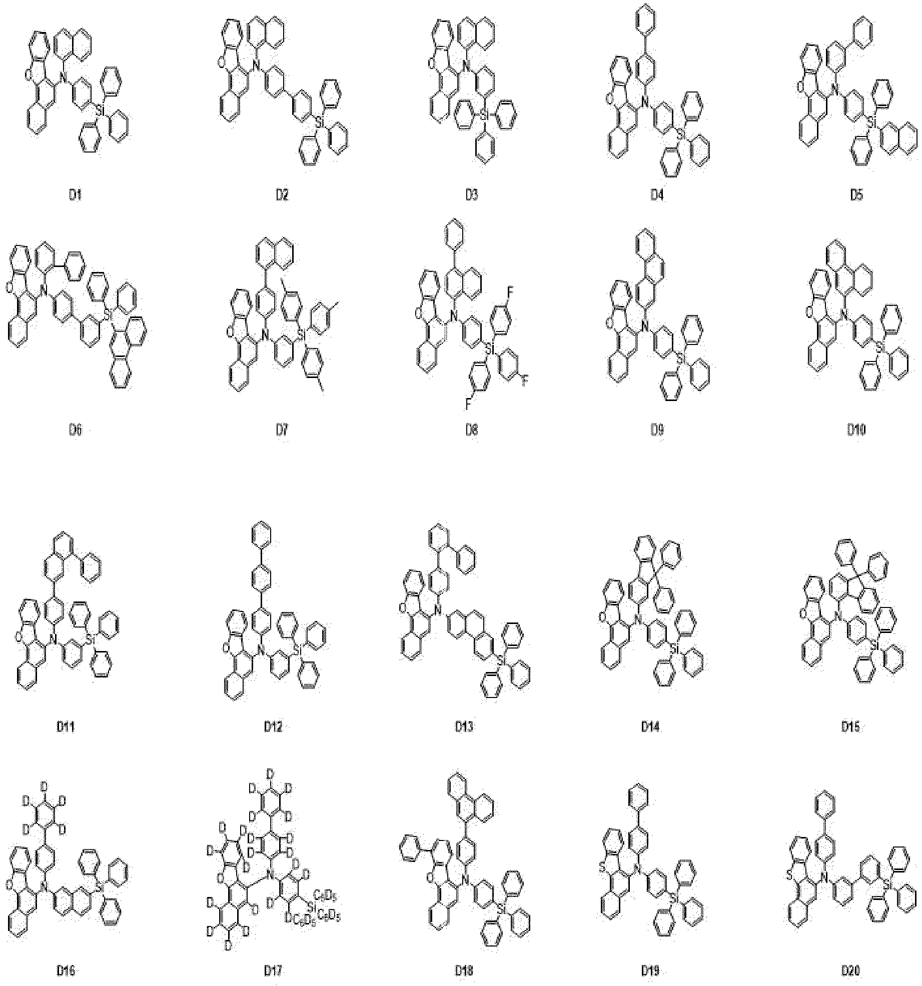

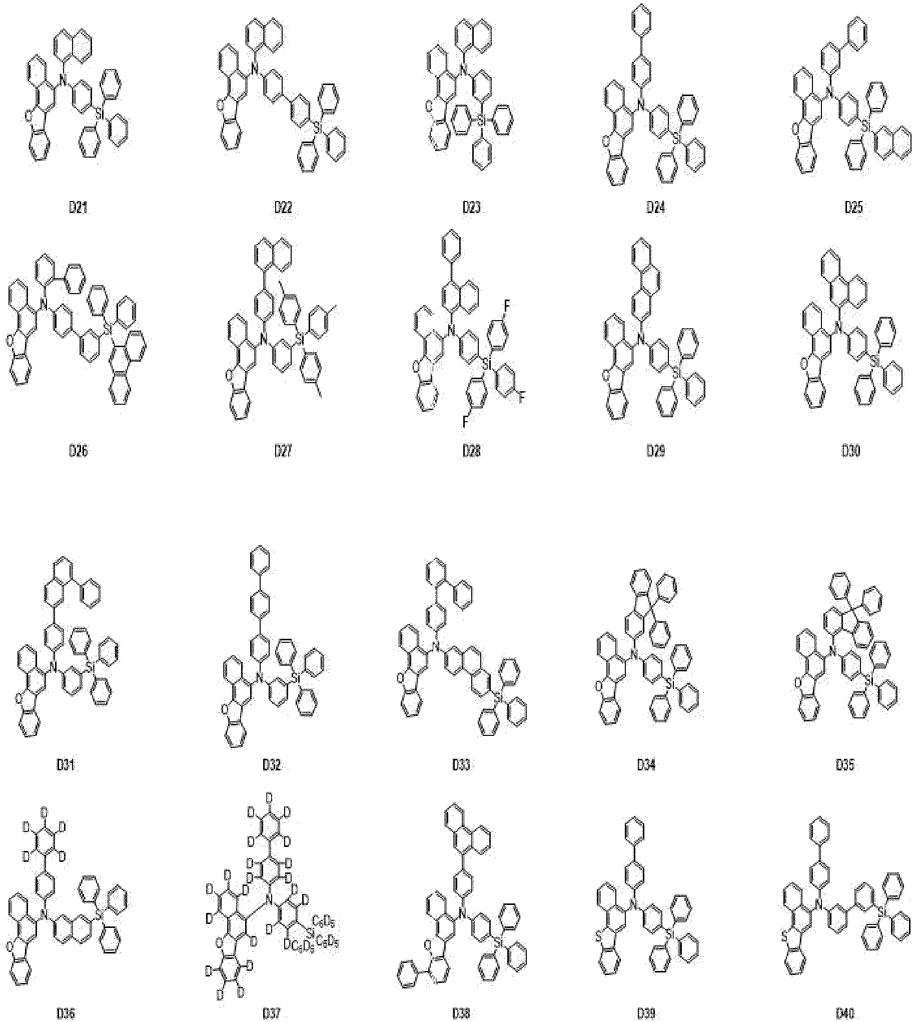

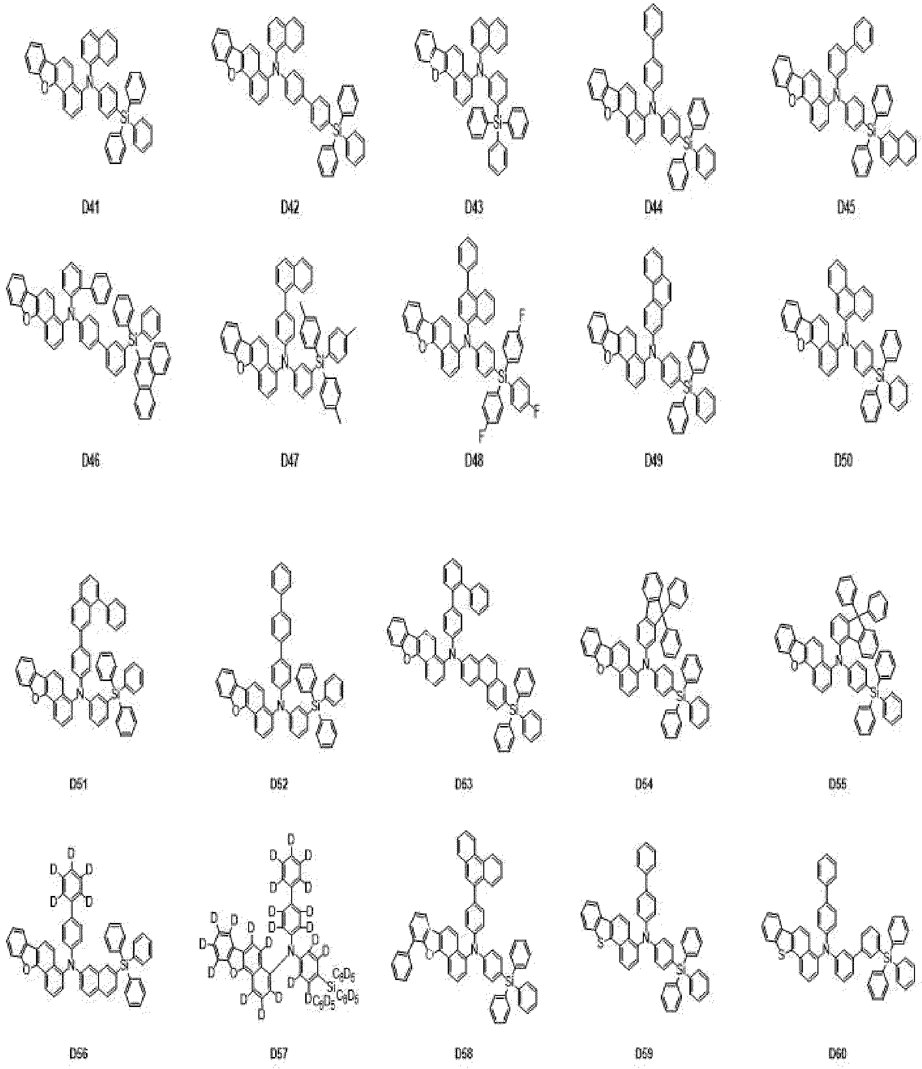

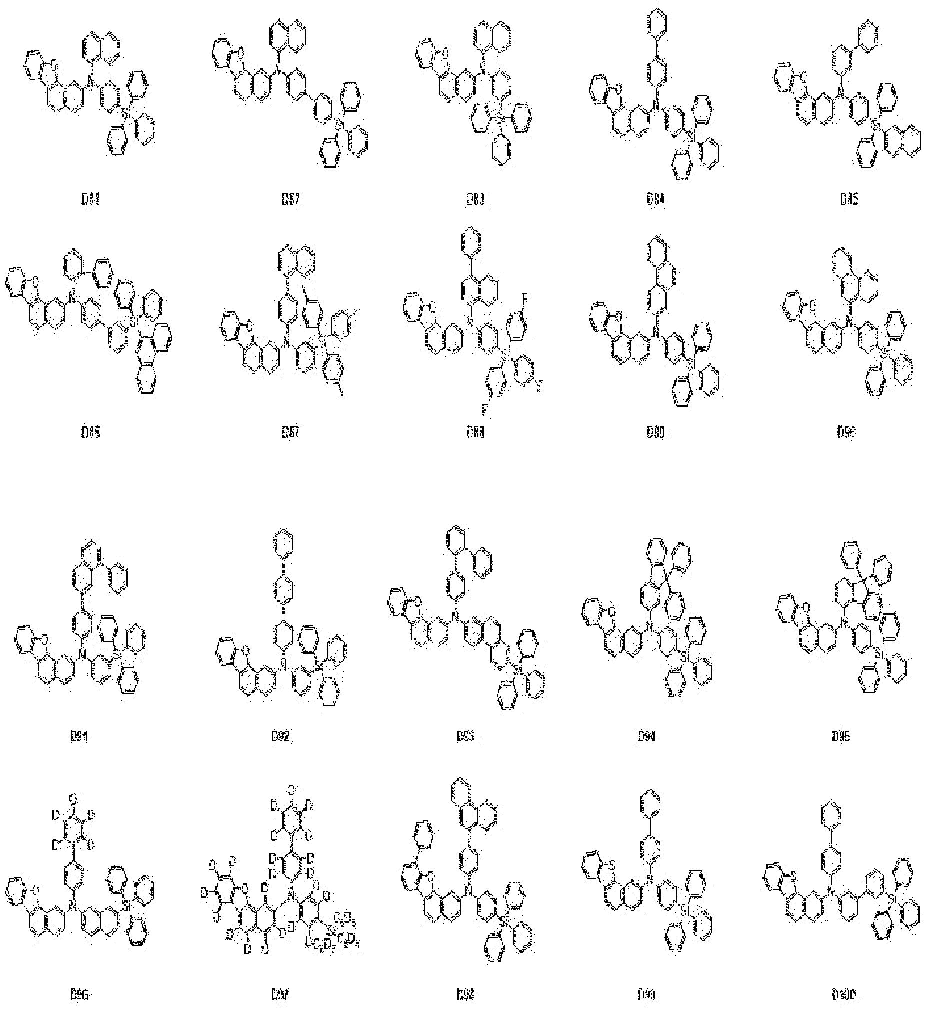

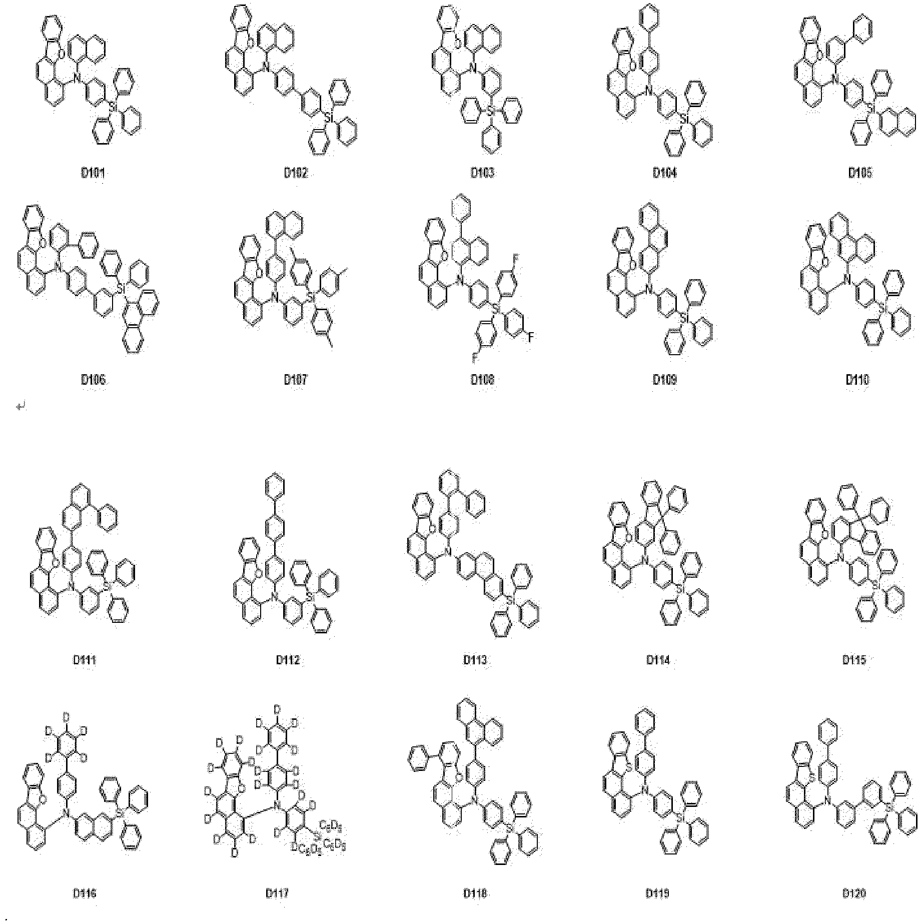

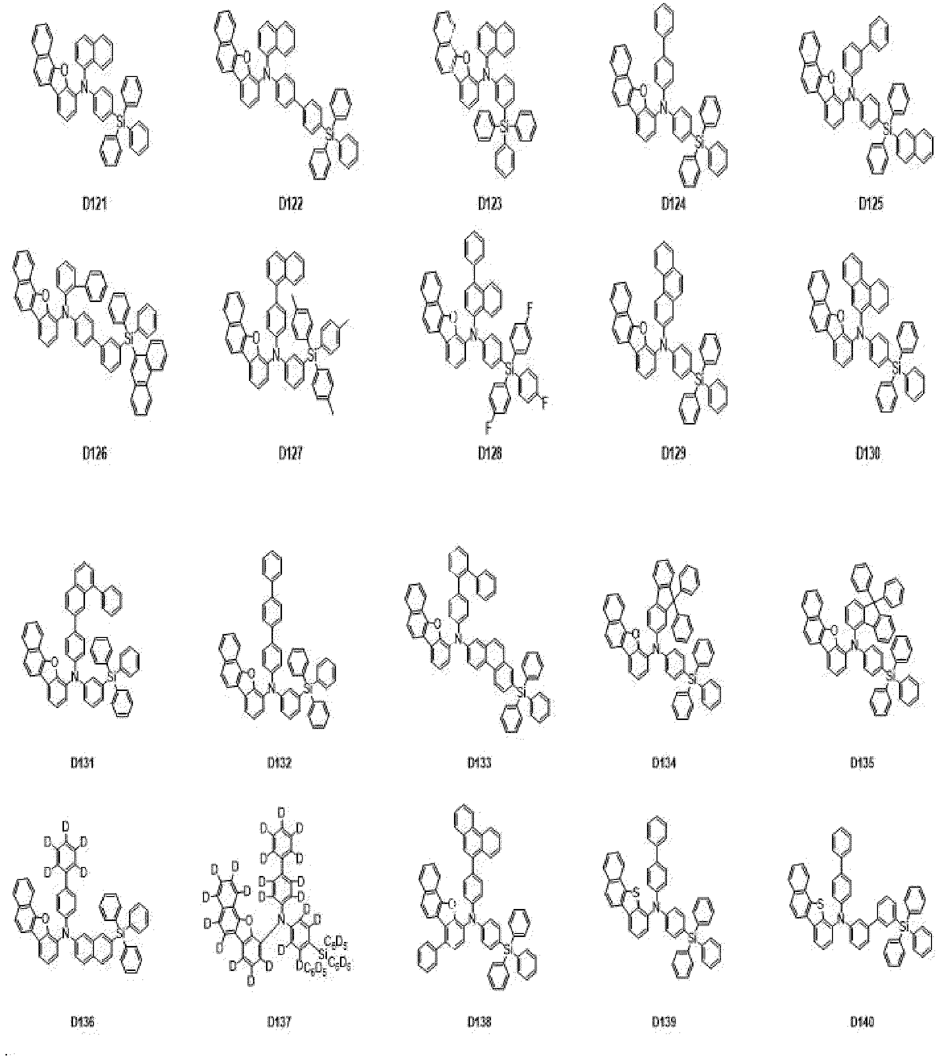

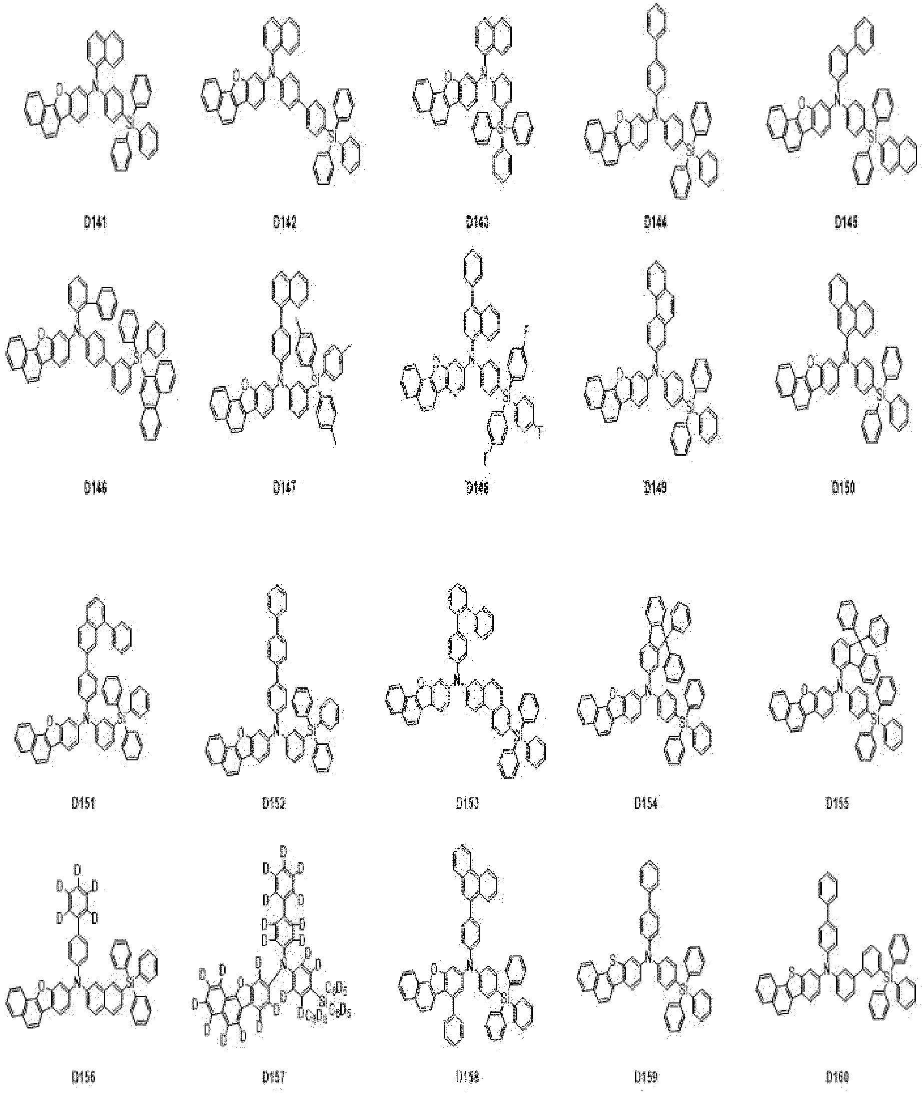

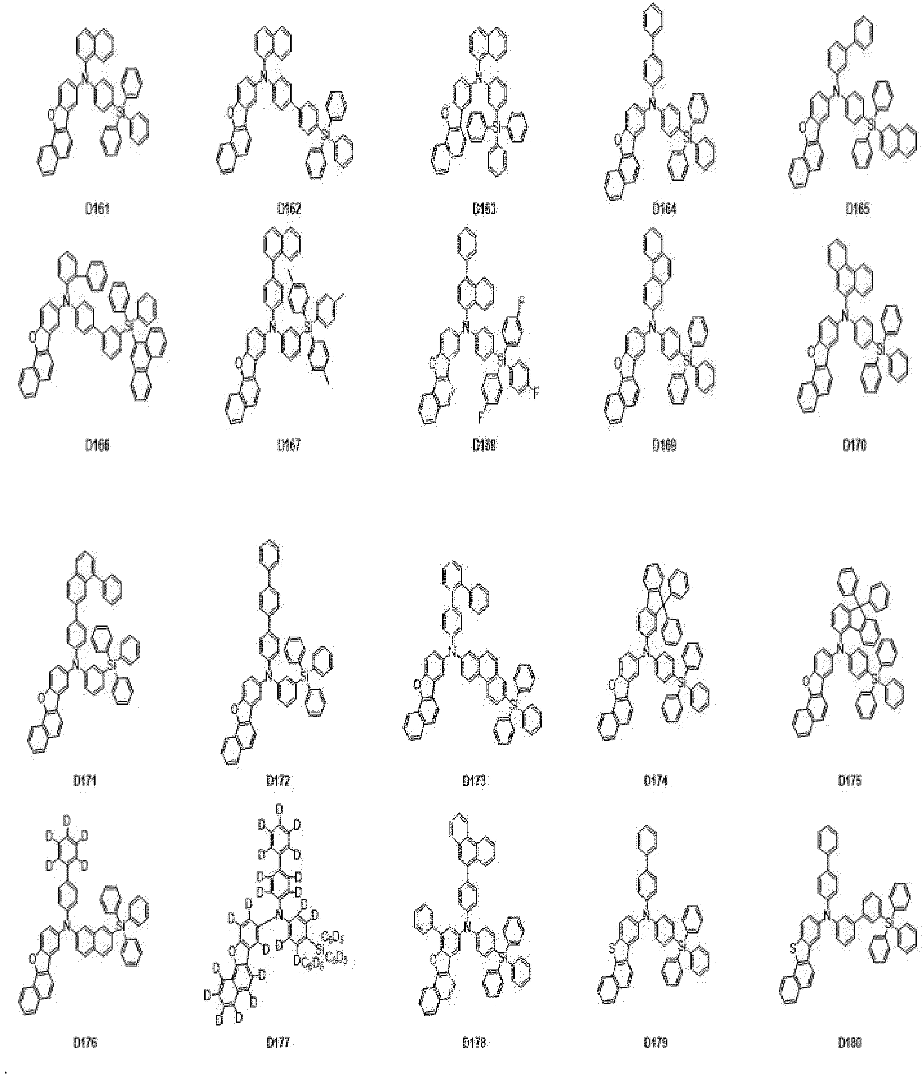

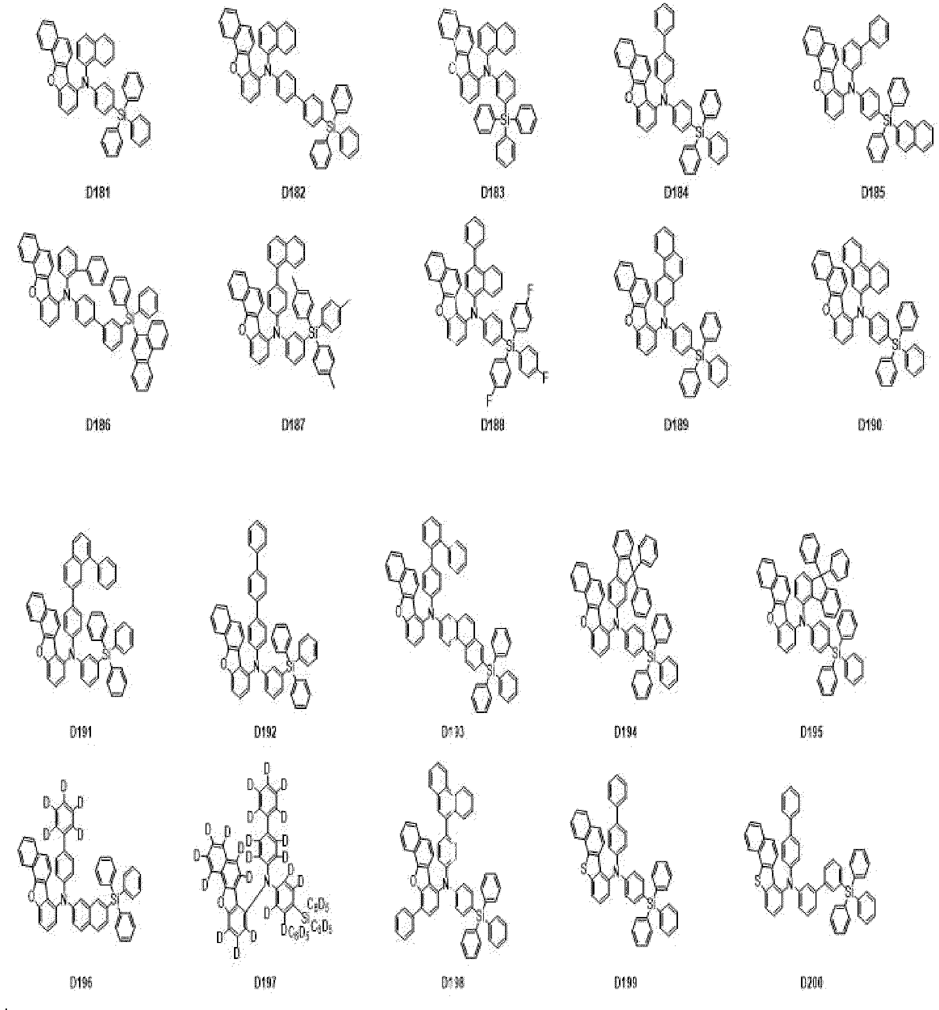

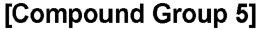

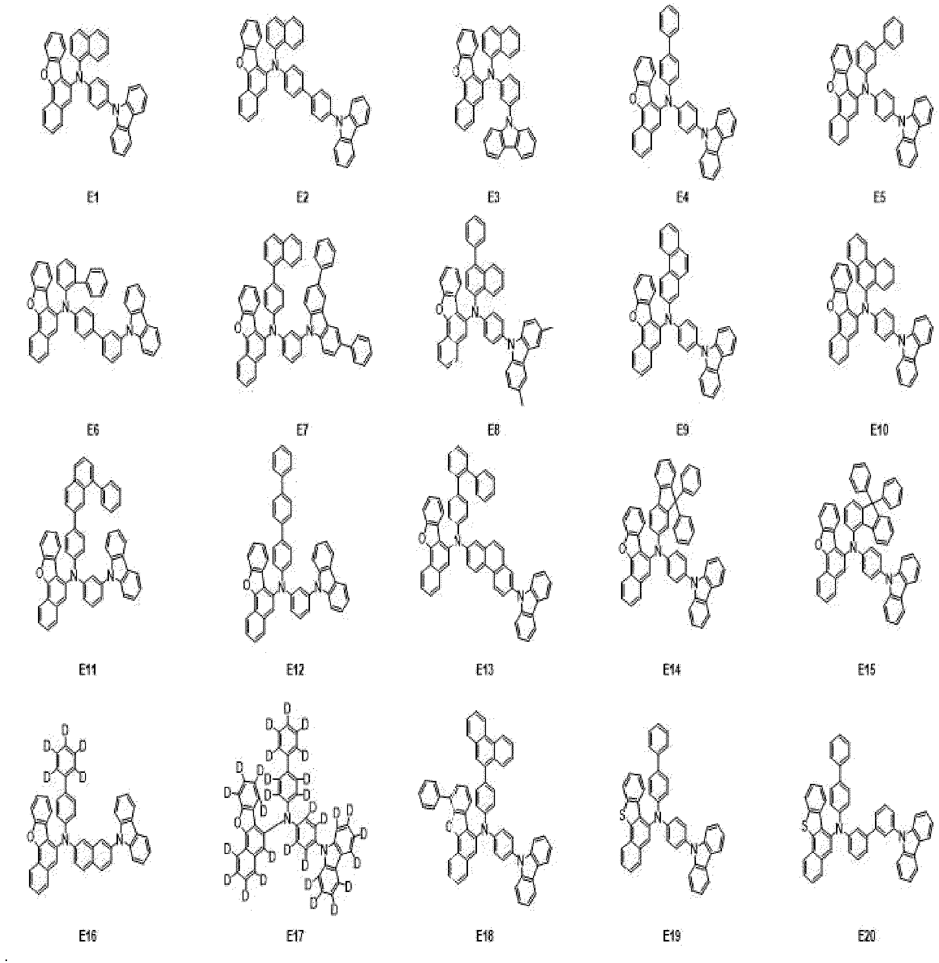

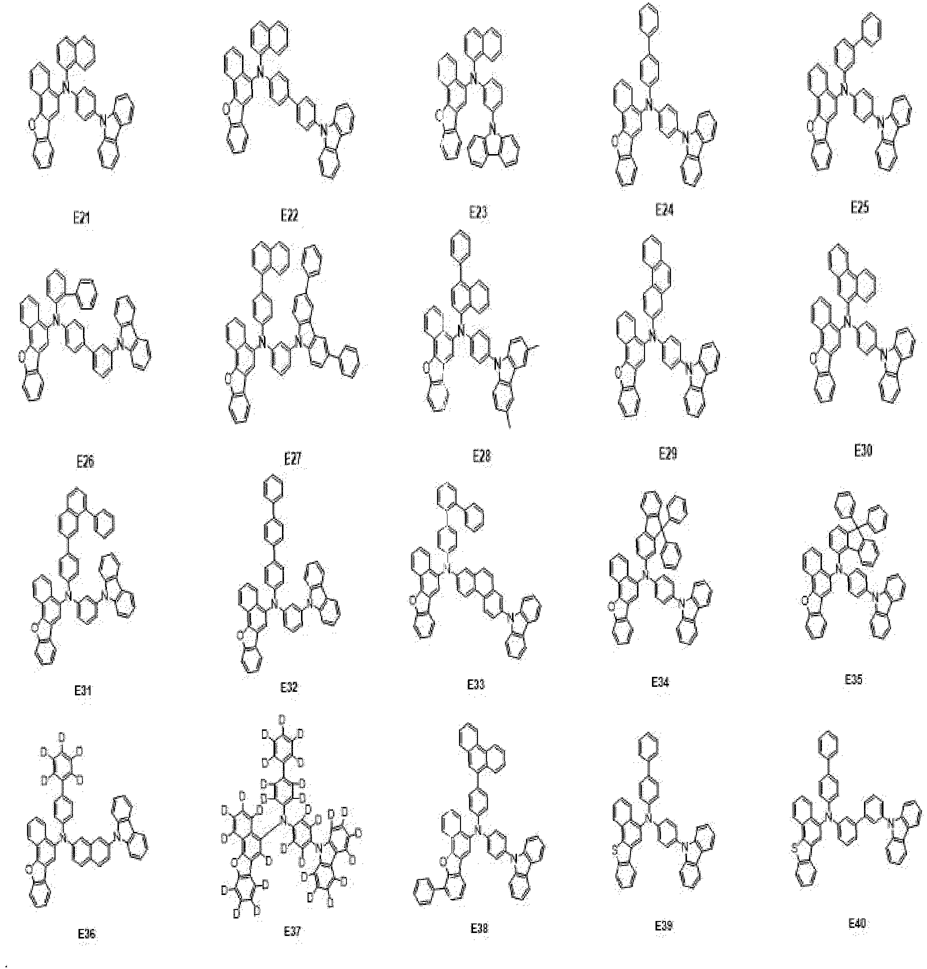

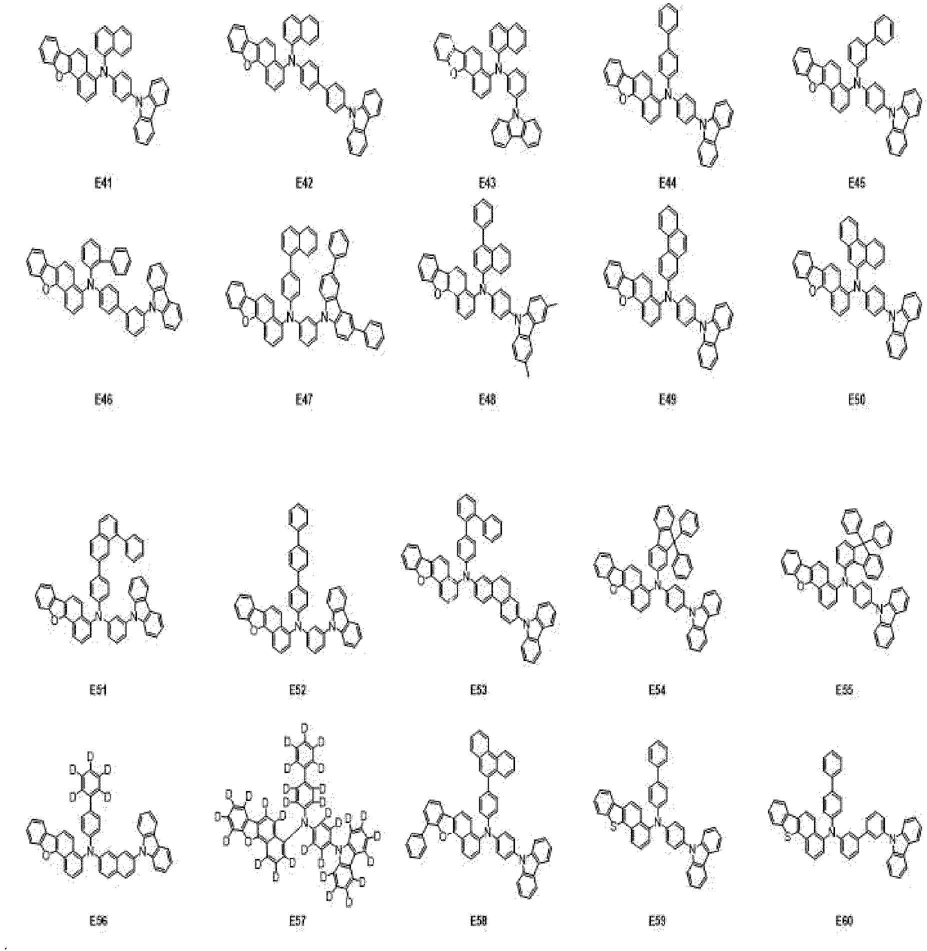

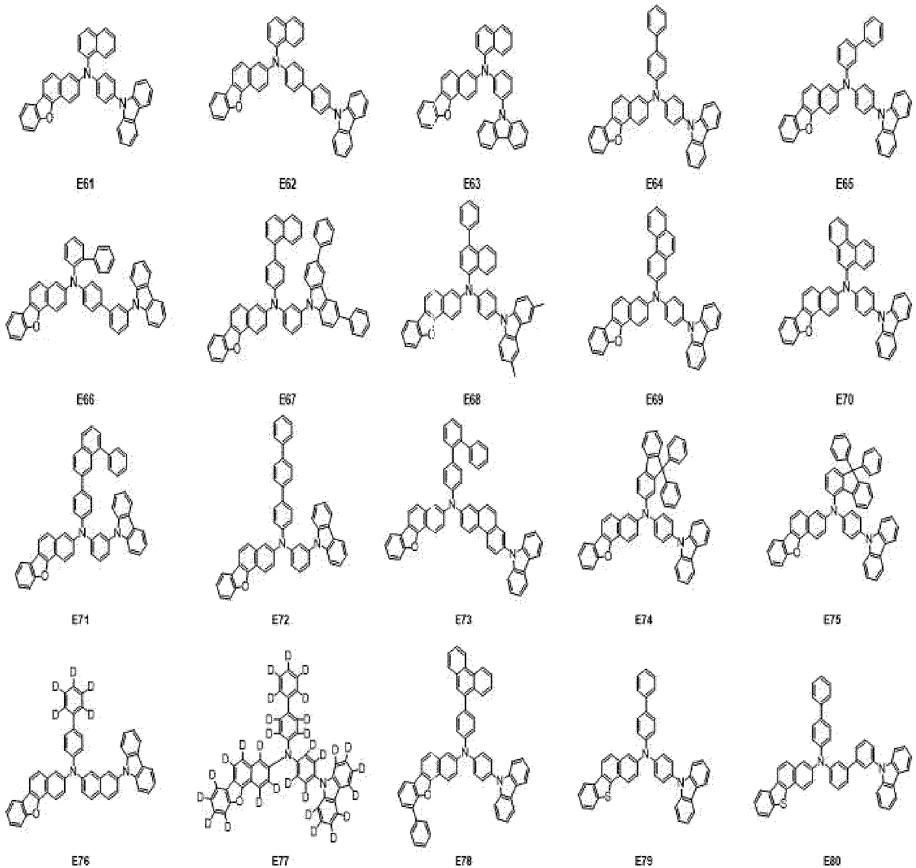

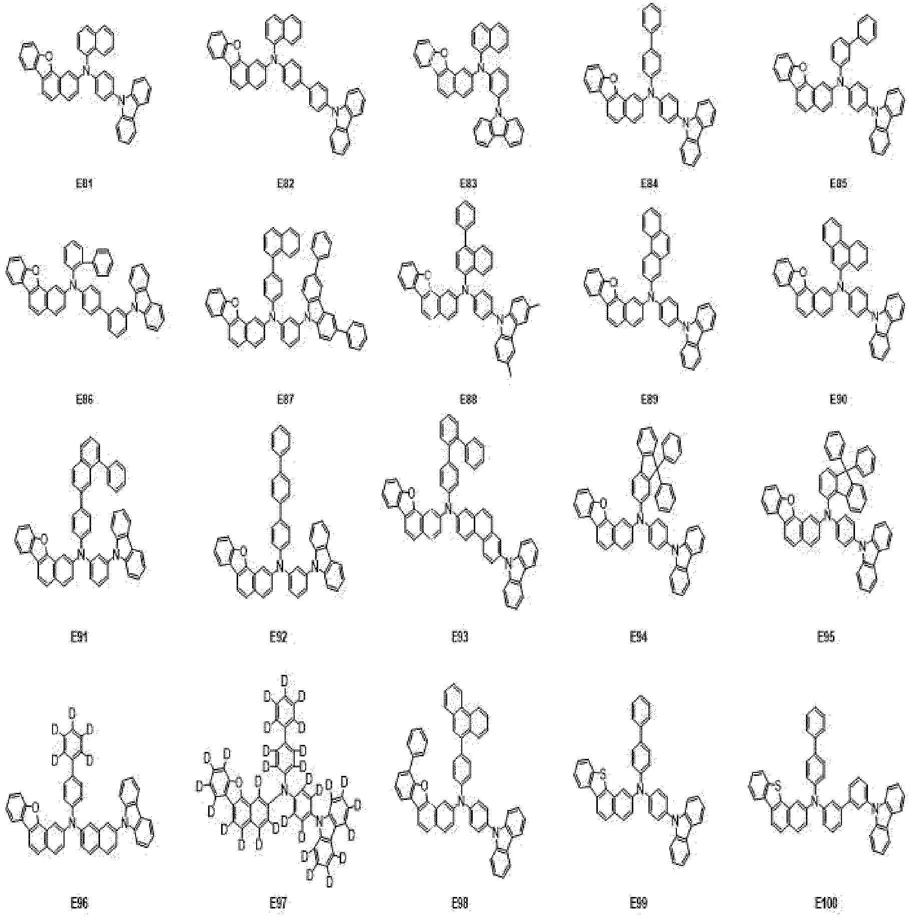

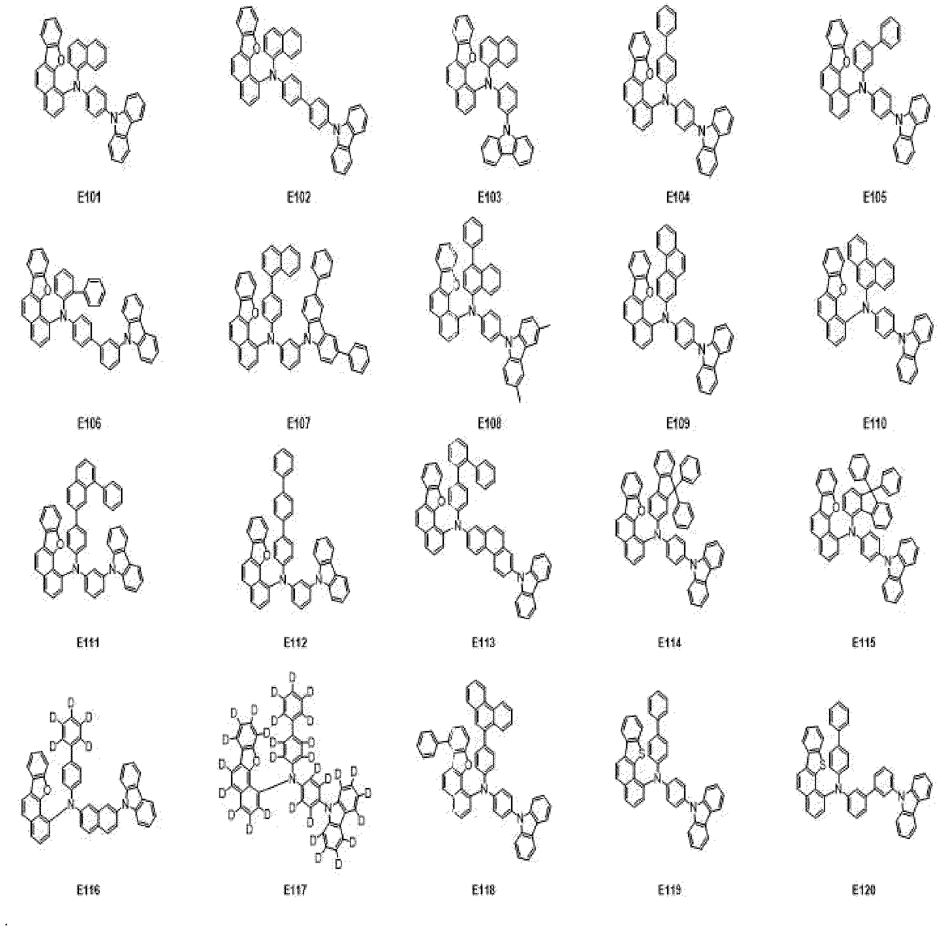

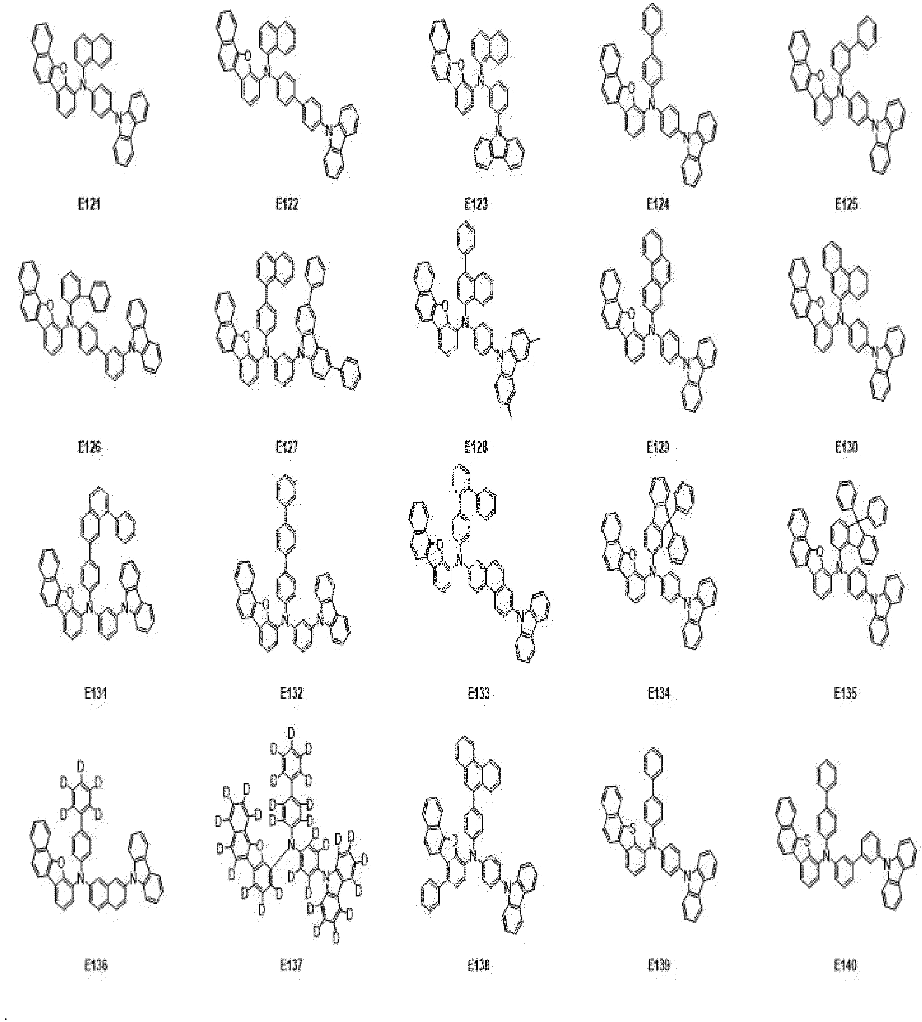

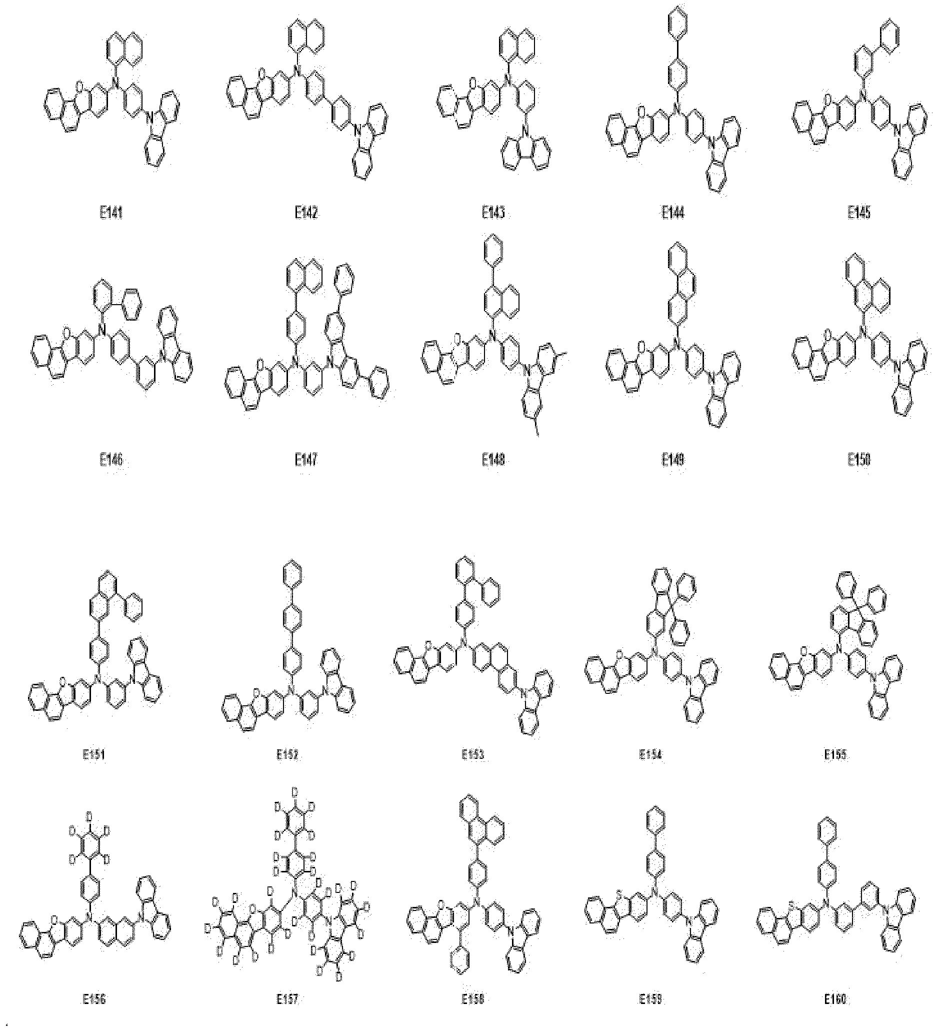

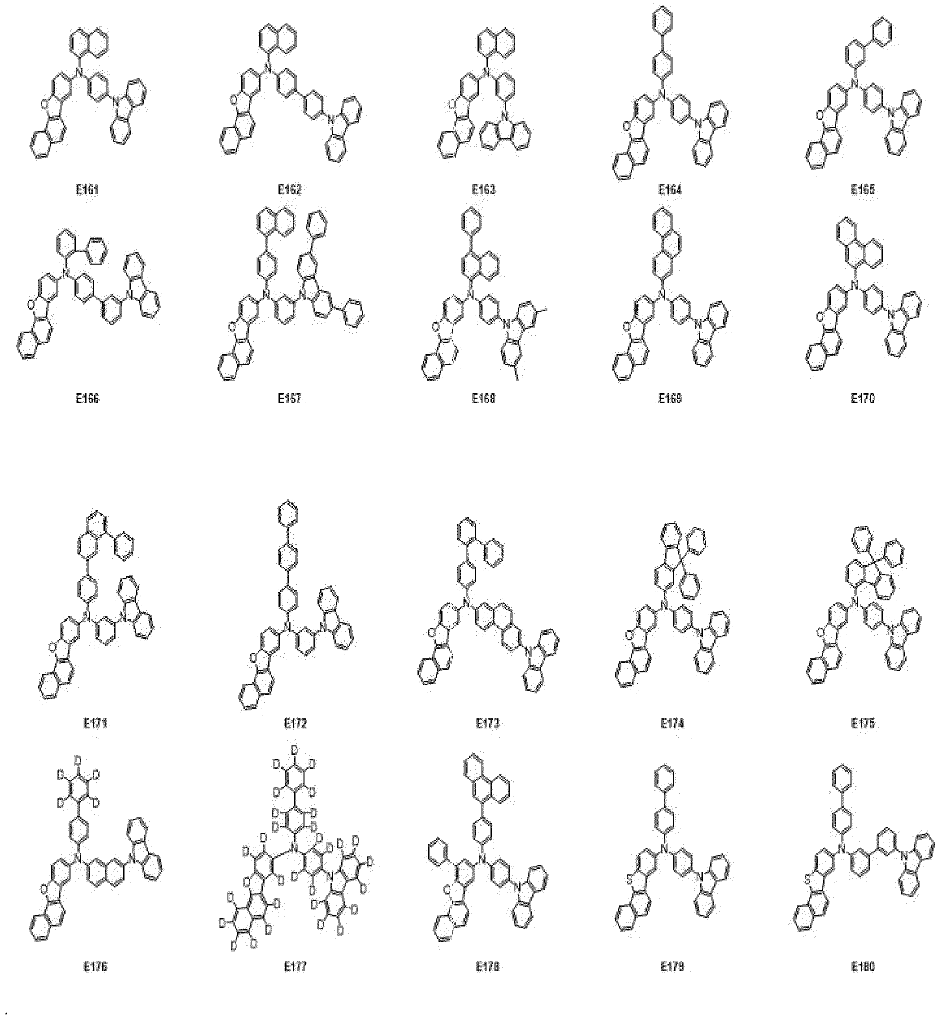

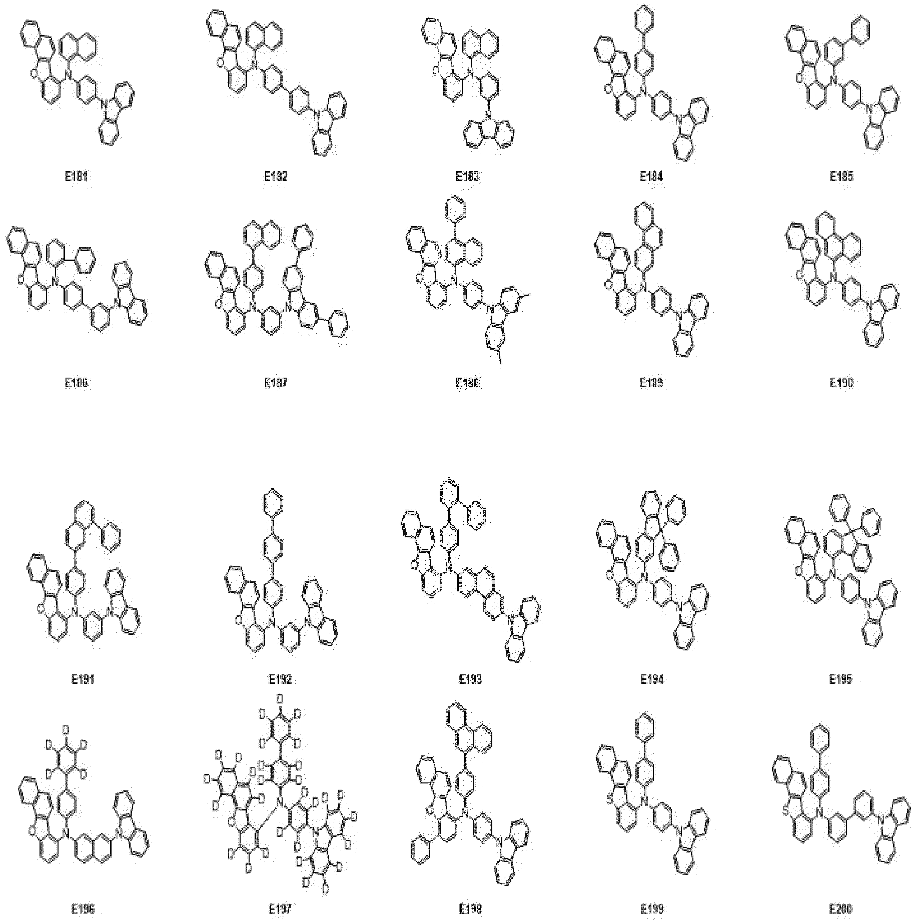

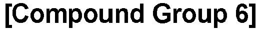

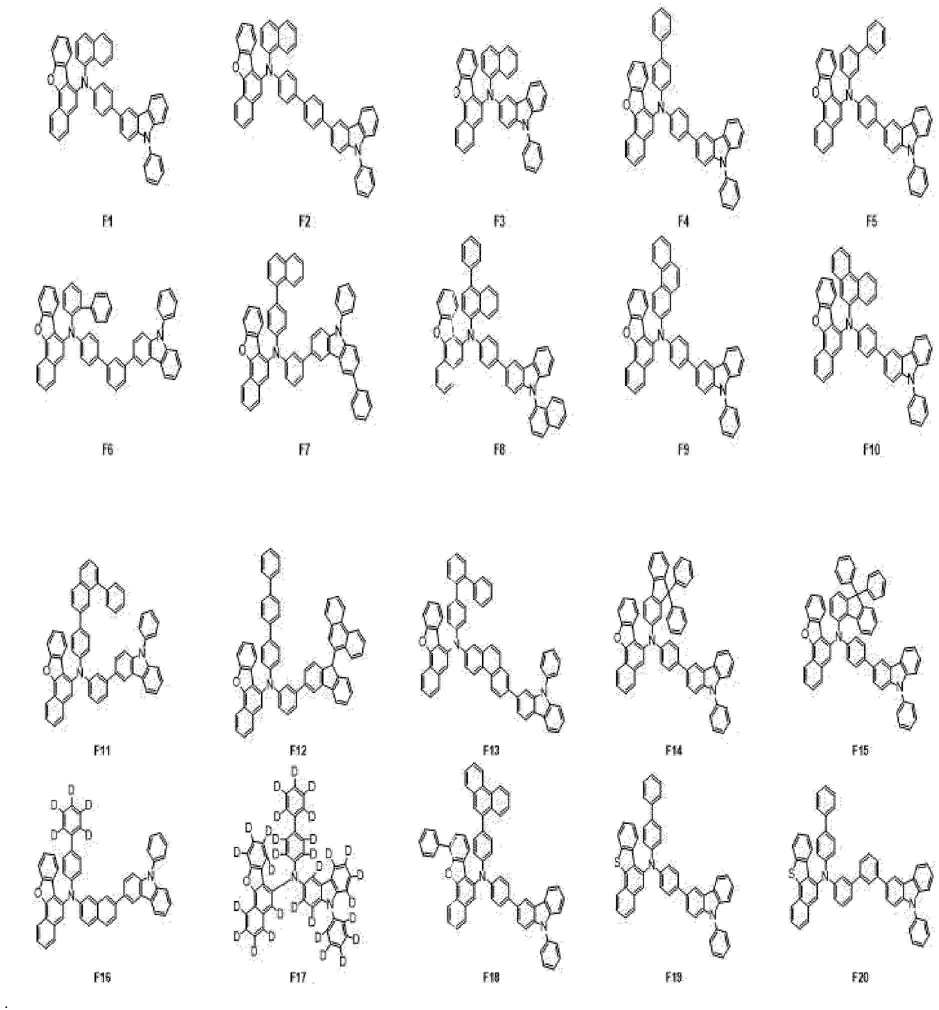

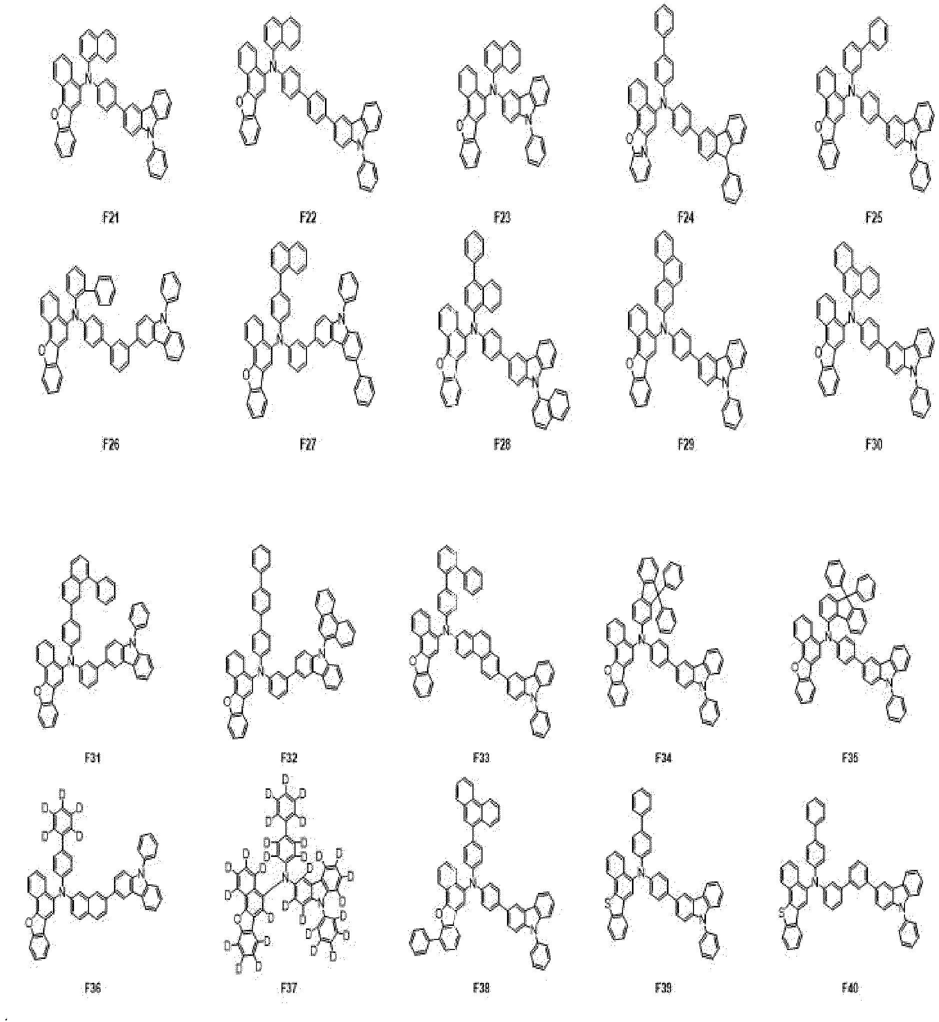

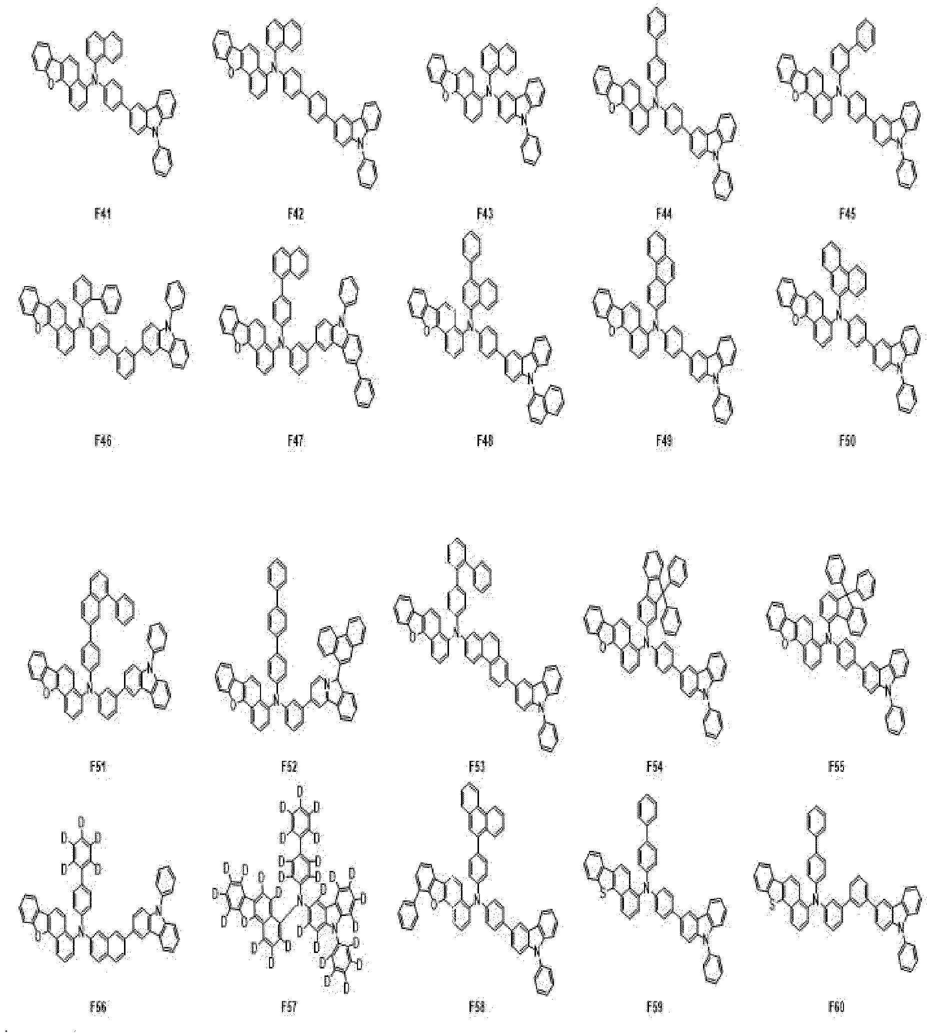

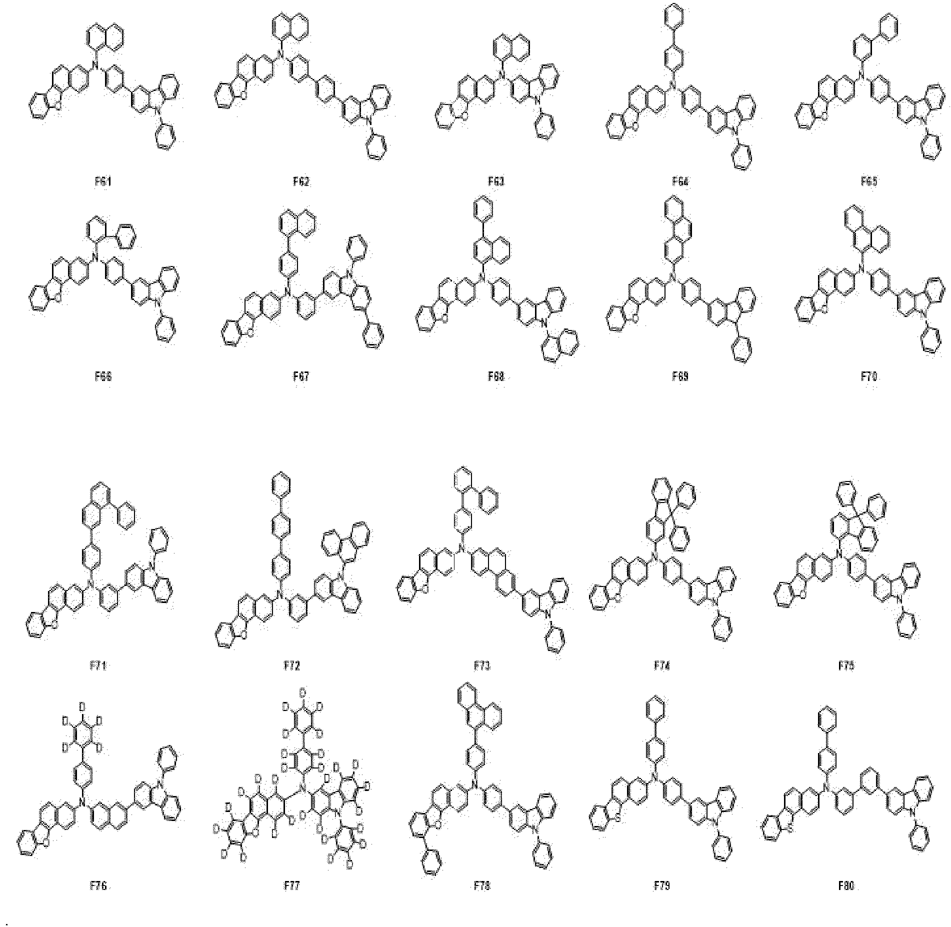

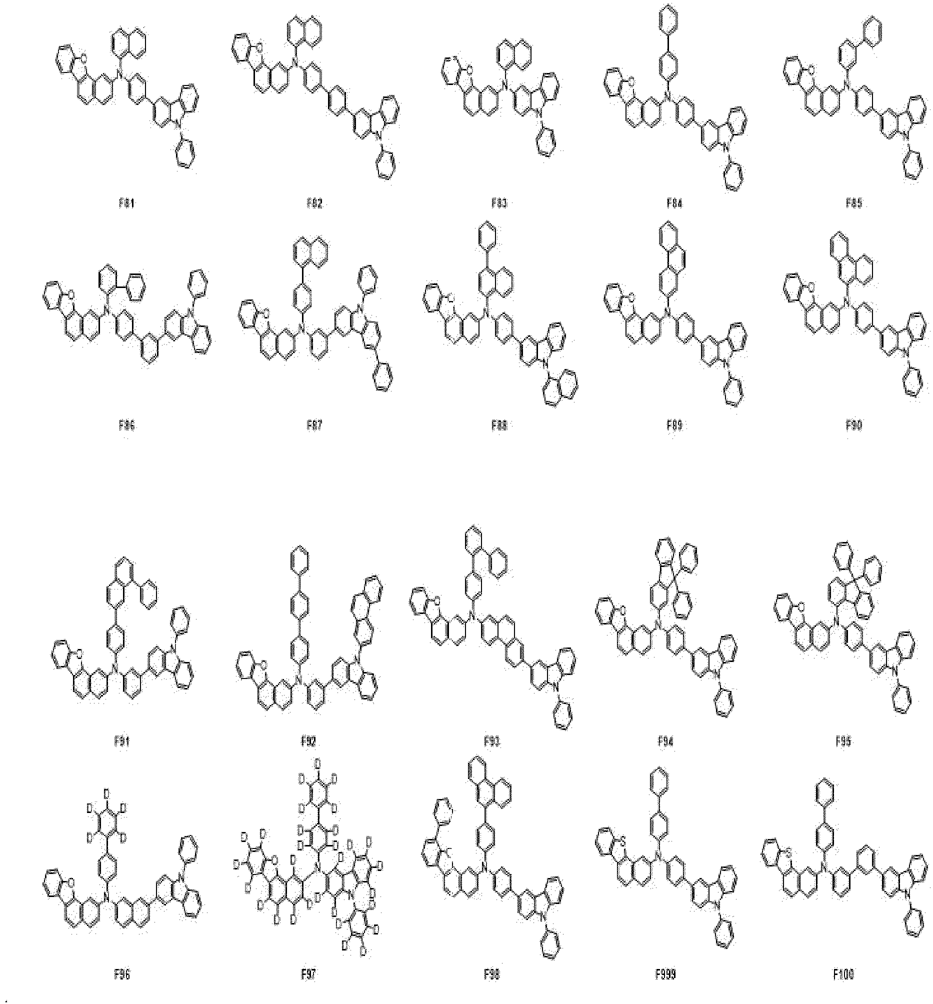

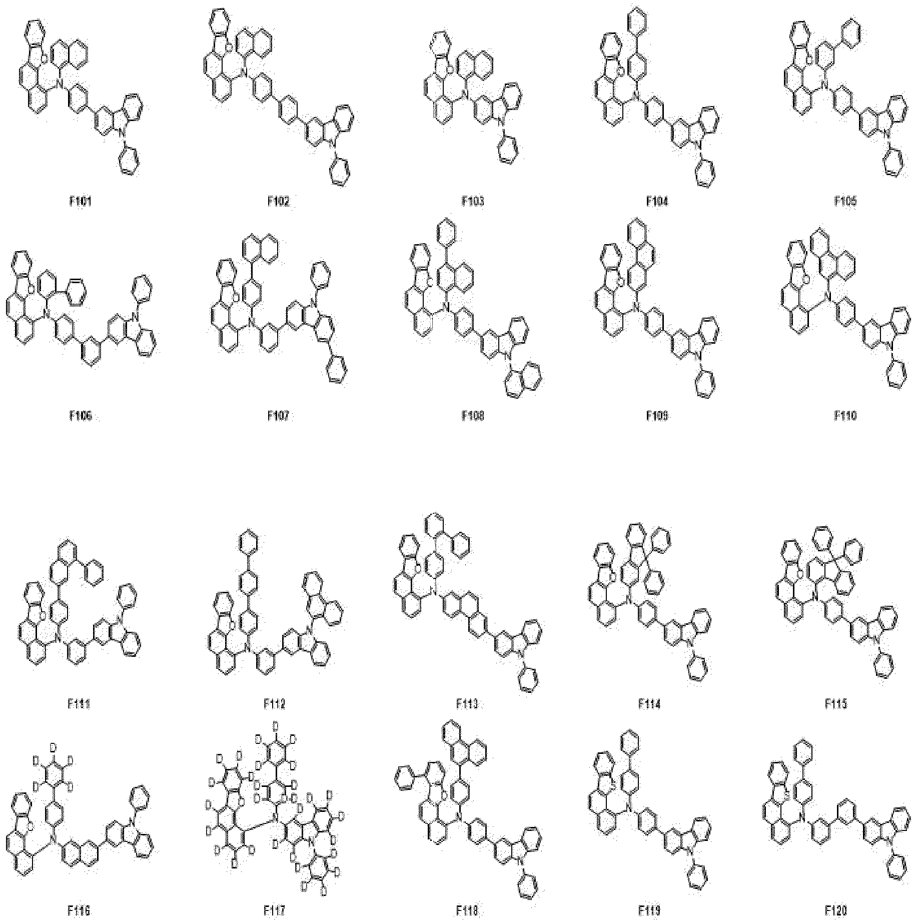

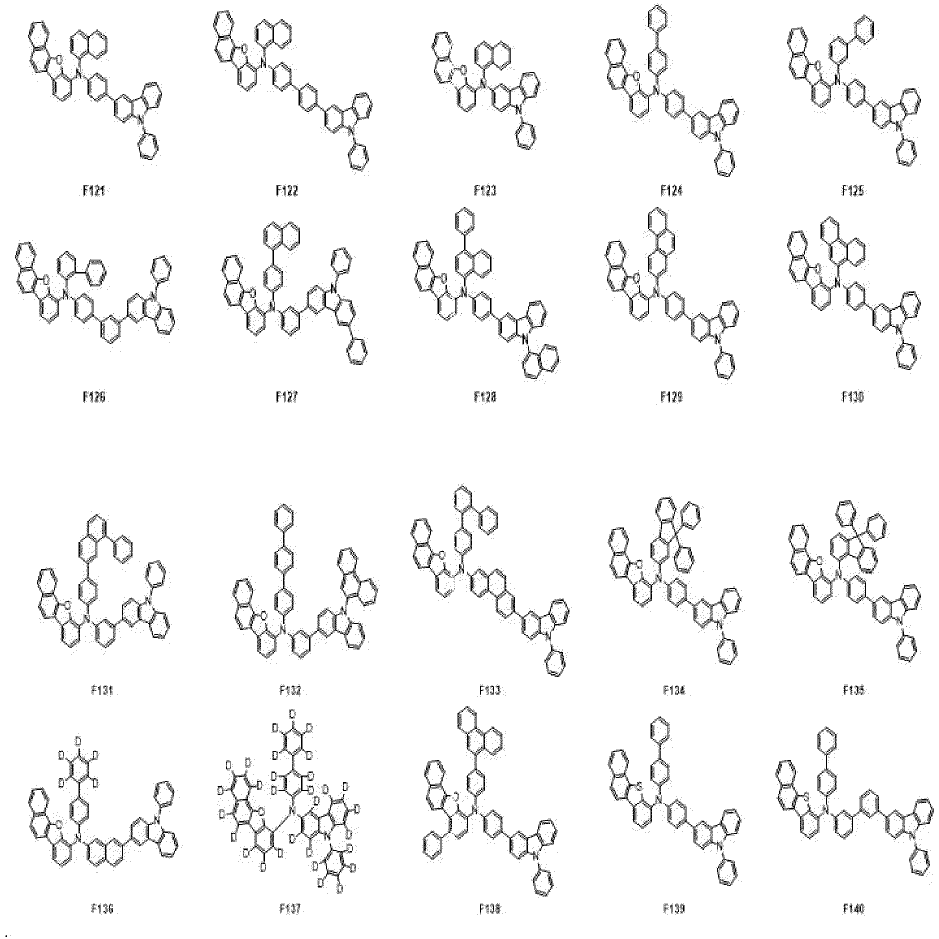

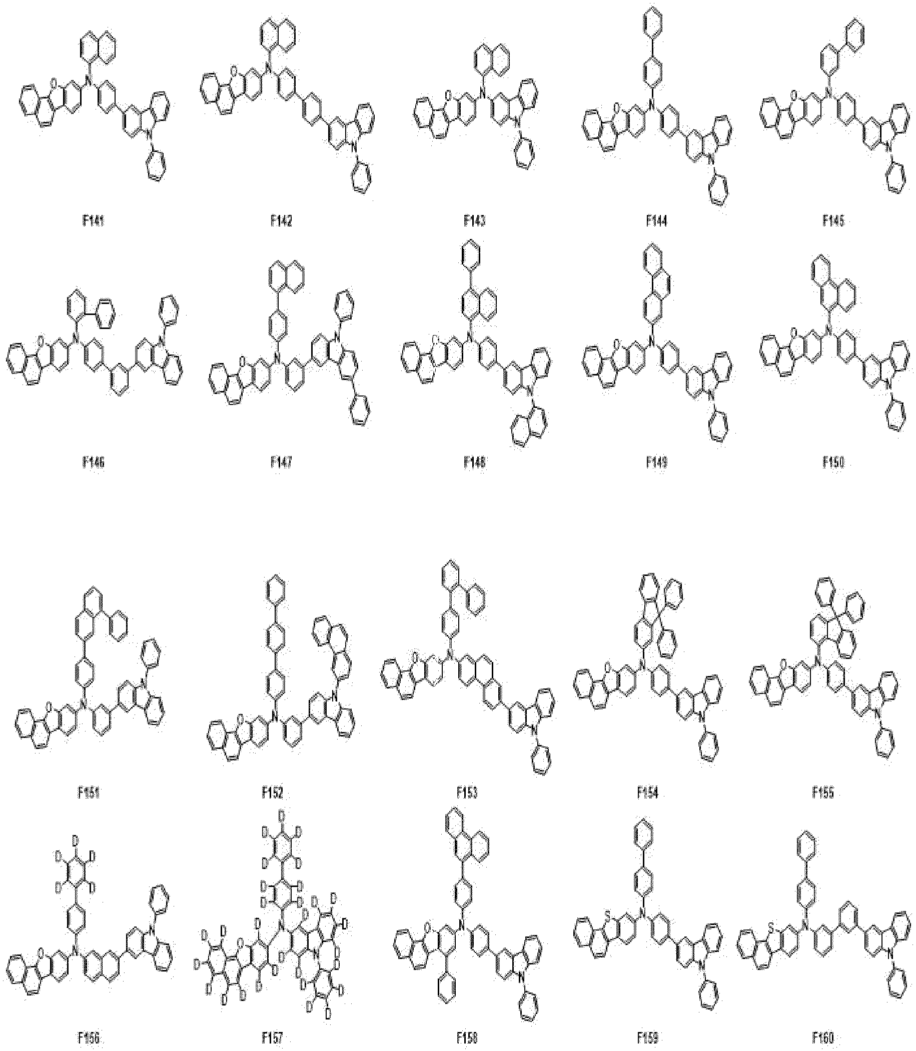

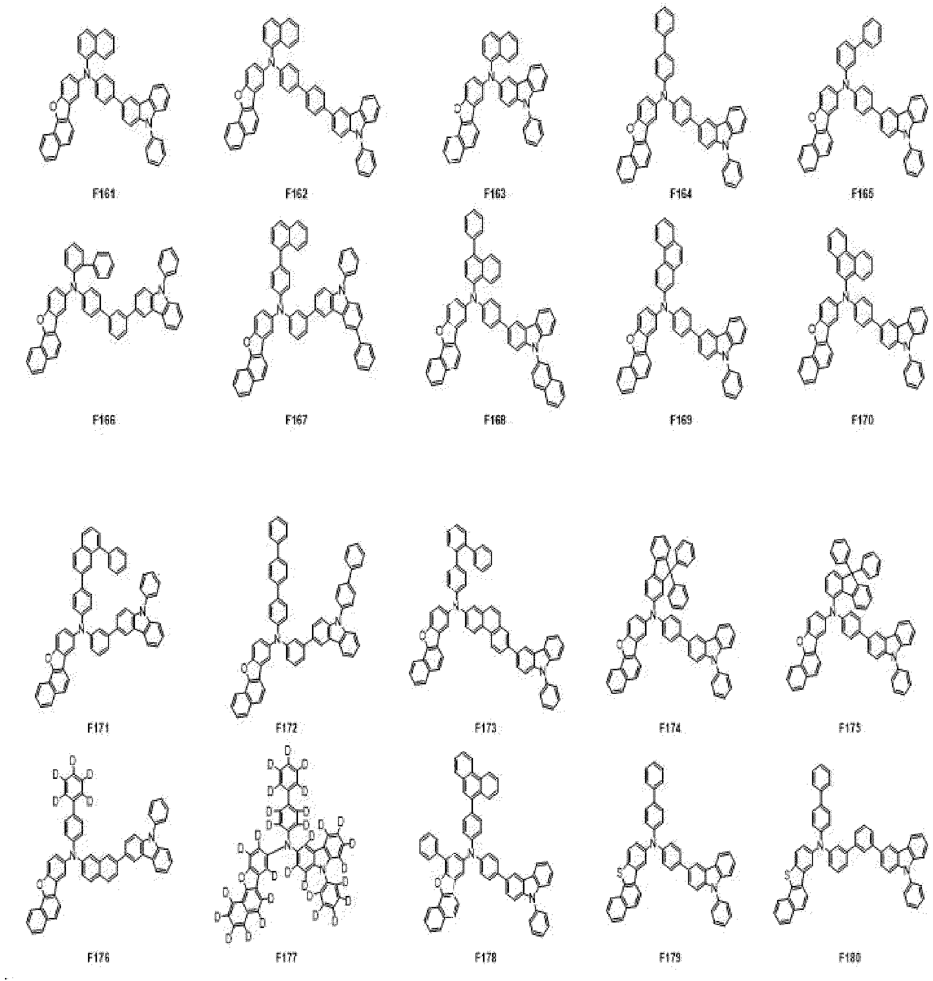

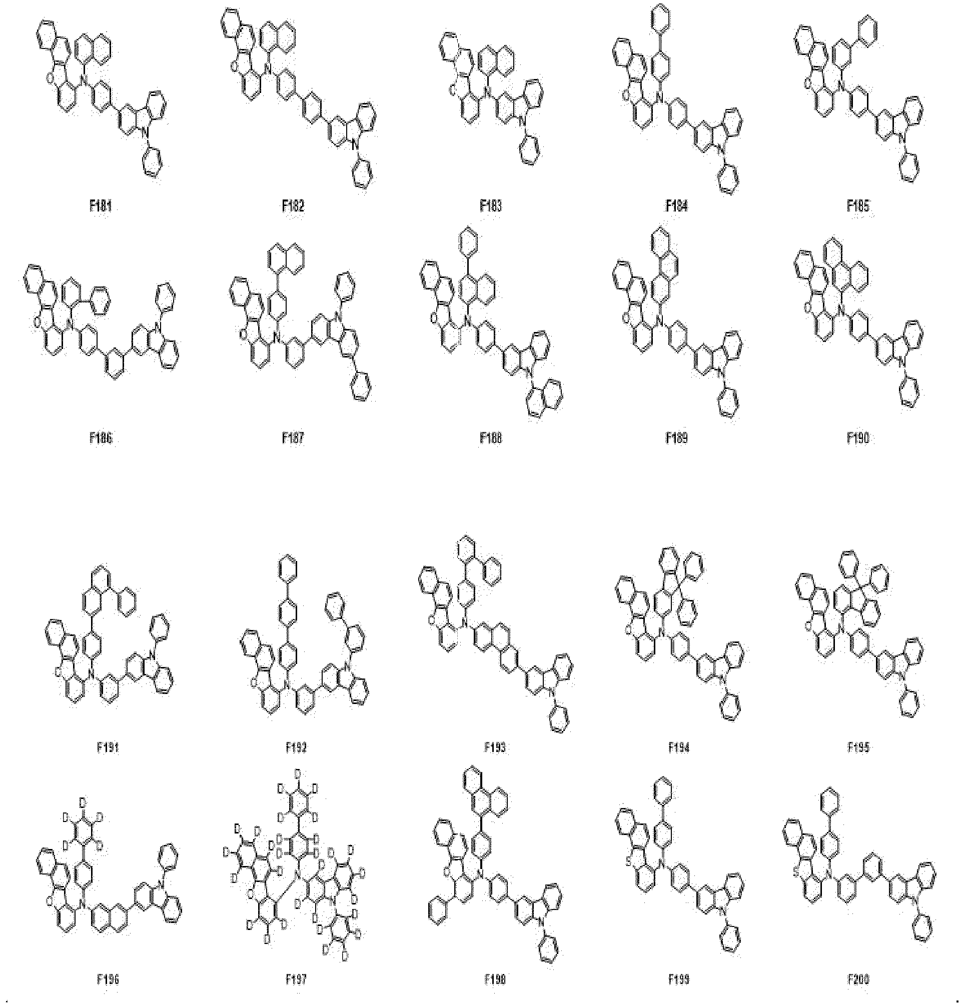

Referring to FIG. 1 to FIG. 3, the organic electroluminescence device according to an embodiment of the present disclosure will be explained.

As described above, the hole transport region HTR comprises the polycyclic compound according to an embodiment of the present disclosure. For example, the hole transport region HTR comprises the polycyclic compound represented by Formula 1.

When the hole transport region HTR has a multilayer structure having a plurality of layers, any one layer among the plurality of layers may comprise the polycyclic compound represented by Formula 1. For example, the hole transport region HTR may comprise a hole injection layer HIL disposed on the first electrode EL1 and a hole transport layer HTL disposed on the hole injection layer HIL, and the hole transport layer HTL may comprise the polycyclic compound represented by Formula 1. However, embodiments of the present disclosure are not limited thereto, and for example, the hole injection layer HIL may comprise the polycyclic compound represented by Formula 1.

The hole transport region HTR may comprise at least one structure of the polycyclic compound represented by Formula 1. For example, the hole transport region HTR may comprise at least one selected among the compounds represented in the Compound Group 1 to Compound Group 6 above.

The hole transport region HTR may be formed using any suitable method (such as a vacuum deposition method, a spin coating method, a cast method, a Langmuir-Blodgett (LB) method, an inkjet printing method, a laser printing method, and/or a laser induced thermal imaging (LITI) method).

However, the hole transport region may further comprise the materials below in each layer.

The hole injection layer HIL may comprise, for example, a phthalocyanine compound (such as copper phthalocyanine), N,N'-diphenyl-N,N'-bis-[4-(phenyl-m-tolyl-amino)-phenyl]-phenyl-4,4'-diamine (DNTPD), 4,4',4"-[tris(3-methylphenyl)phenylamino] triphenylamine (m-MTDATA), 4,4',4"-tris(N,N-diphenylamino)triphenylamine (TDATA), 4,4',4"-tris{N,-2-naphthyl)-N- phenylamino}-triphenylamine (2-TNATA), poly(3,4-ethylene dioxythiophene)/poly(4-styrene sulfonate) (PEDOT/PSS), polyaniline/dodecylbenzene sulfonic acid (PANI/DBSA), polyaniline/camphor sulfonic acid (PANI/CSA), polyaniline/poly(4-styrene sulfonate) (PANI/PSS), N,N'-di(1-naphthalene-1-yl)-N,N'-diphenyl-benzidine (NPD), triphenylamine-containing polyether ketone (TPAPEK), 4-isopropyl-4'-methyldiphenyliodonium [tetrakis(pentafluorophenyl)borate], and dipyrazino[2,3-f:2',3'-h]quinoxaline-2,3,6,7,10,11-hexacarbonitrile (HAT-CN).

The hole transport layer HTL may comprise any suitable hole transport material available in the art, for example, carbazole derivatives (such as N-phenyl carbazole and/or polyvinyl carbazole), fluorene-based derivatives, N,N'-bis(3-methylphenyl)-N,N'-diphenyl-[1,1-biphenyl]-4,4'-diamine (TPD), triphenylamine-based derivatives (such as 4,4',4"-tris(N-carbazolyl)triphenylamine (TCTA)), N,N'-di(1-naphthalene-1-yl)-N,N'-diphenyl-benzidine (NPD), 4,4'-cyclohexylidene bis[N,N-bis(4-methylphenyl)benzenamine (TAPC), 4,4'-bis[N,N'-(3-tolyl)amino]-3,3'-dimethylbiphenyl (HMTPD), 1,3-bis(N-carbazolyl)benzene (mCP), etc.

The electron blocking layer EBL may comprise, for example, carbazole derivatives (such as N-phenyl carbazole and/or polyvinyl carbazole), fluorene-based derivatives, N,N'-bis(3-methylphenyl)-N,N'-diphenyl-[1,1-biphenyl]-4,4'-diamine (TPD), triphenylamine-based derivatives (such as 4,4',4"-tris(N-carbazolyl)triphenylamine (TCTA)), N,N'-di(1-naphthalene-1-yl)-N,N'-diphenyl-benzidine (NPD), 4,4'-cyclohexylidene bis[N,N-bis(4-methylphenyl)benzenamine (TAPC), 4,4'-bis[N,N'-(3-tolyl)amino]-3,3'-dimethylbiphenyl (HMTPD), mCP, etc.

The thickness of the hole transport region HTR may be about 50 Å to about 15,000 Å, for example, about 100 Å to about 5,000 Å. The thickness of the hole injection region HIL may be, for example, about 30 Å to about 1,000 Å, and the thickness of the hole transport layer HTL may be about 30 Å to about 1,000 Å. For example, the thickness of the electron blocking layer EBL may be about 10 Å to about 1,000 Å. When the thicknesses of the hole transport region HTR, the hole injection layer HIL, the hole transport layer HTL and the electron blocking layer EBL satisfy the above-described ranges, satisfactory hole transport properties may be achieved without substantial increase of a driving voltage.

The hole transport region HTR may further comprise a charge generating material in addition to the above-described materials to increase conductivity. The charge generating material may be dispersed substantially uniformly or non-uniformly in the hole transport region HTR. The charge generating material may be, for example, a p-dopant. The p-dopant may be one of quinone derivatives, metal oxides, or cyano group-containing compounds, without limitation. For example, non-limiting examples of the p-dopant may comprise quinone derivatives (such as tetracyanoquinodimethane (TCNQ) and/or 2,3,5,6-tetrafluoro-7,7',8,8'-tetracyanoquinodimethane (F4-TCNQ)), metal halides (such as MgF₂, Cul, and/or RbI), metal oxides (such as tungsten oxide and/or molybdenum oxide), without limitation.

As described above, the hole transport region HTR may further comprise at least one of a hole butter layer or an electron blocking layer EBL. The hole buffer layer may compensate for an optical resonance distance of light emitted from an emission layer EML, and may increase the light emission efficiency of the device. Materials that may be comprised in a hole transport region HTR may also be used as materials in a hole buffer layer. The electron blocking layer EBL may prevent or reduce electron injection from the electron transport region ETR to the hole transport region HTR.

The emission layer EML is provided on the hole transport region HTR. The emission layer EML may have a thickness of, for example, about 100 Å to about 1,000 Å or about 100 Å to about 600 Å. The emission layer EML may have a single layer formed using a single material, a single layer formed using a plurality of different materials, or a multilayer structure having a plurality of layers formed using a plurality of different materials.

As the material of the emission layer EML, any suitable luminescent materials may be used, for example, fluoranthene derivatives, pyrene derivatives, arylacetylene derivatives, anthracene derivatives, fluorene derivatives, perylene derivatives, and chrysene derivatives. In some embodiments, pyrene derivatives, perylene derivatives, and anthracene derivatives may be comprised. For example, as the host material of the emission layer EML, anthracene derivatives represented by Formula 10 below may be used.

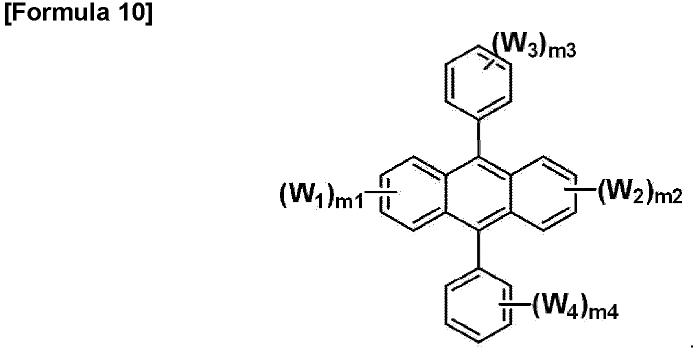

In Formula 10, W₁ to W₄ may each independently be a hydrogen atom, a deuterium atom, a halogen atom, a substituted or unsubstituted silyl group, a substituted or unsubstituted alkyl group of 1 to 20 carbon atoms, a substituted or unsubstituted aryl group of 6 to 30 carbon atoms for forming a ring, or a substituted or unsubstituted heteroaryl group of 2 to 30 carbon atoms for forming a ring, and/or may be combined with an adjacent group to form a ring. m1 and m2 may each independently be an integer of 0 to 4, and m3 and m4 may each independently be an integer of 0 to 5.

When m1 is 1, W₁ may not be a hydrogen atom; when m2 is 1, W₂ may not be a hydrogen atom; when m3 is 1, W₃ may not be a hydrogen atom; and when m4 is 1, W₄ may not be a hydrogen atom.

When m1 is 2 or more, a plurality of W₁ groups are the same or different; when m2 is 2 or more, a plurality of W₂ groups are the same or different; when m3 is 2 or more, a plurality of W₄ groups are the same or different; and when m4 is 2 or more, a plurality of W₄ groups are the same or different.

The compound represented by Formula 10 may comprise, for example, the compounds represented by the structures below. However, the compound represented by Formula 10 is not limited thereto:

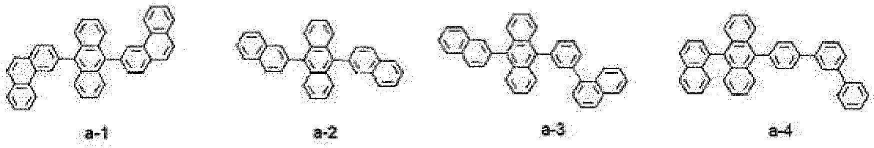

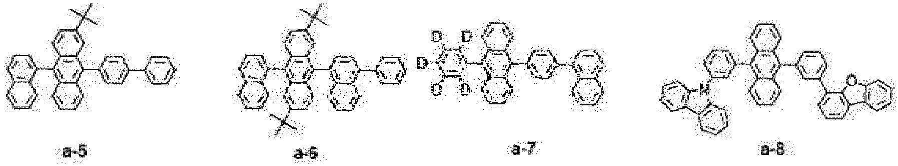

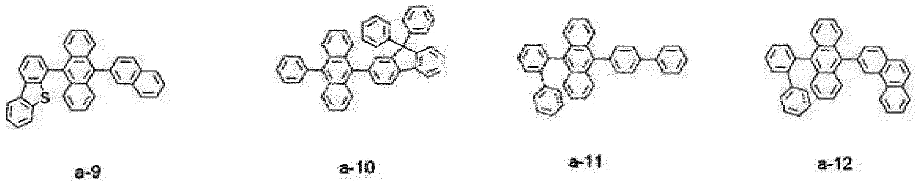

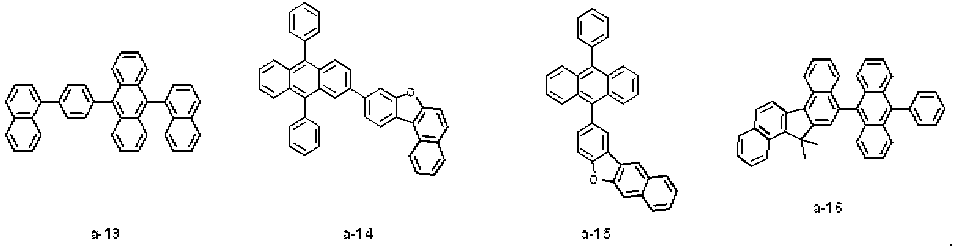

The emission layer EML may comprise a dopant, and the dopant may comprise any suitable dopant material. For example, the dopant may comprise at least one among styryl derivatives (for example, 1,4-bis[2-(3-N-ethylcarbazolyl)vinyl]benzene (BCzVB), 4-(di-p-tolylamino)-4"-[(di-p-tolylamino)styryl]stilbene (DPAVB), and N-(4-((E)-2-(6-((E)-4-(diphenylamino)styryl)naphthalen-2-yl)vinyl)phenyl)-N-phenylbenzenamine (N-BDAVBi)), perylene and derivatives thereof (for example, 2,5,8,11-tetra-t-butylperylene (TBP)), and pyrene and derivatives thereof (for example, 1,1-dipyrene, 1,4-dipyrenylbenzene, 1,4-bis(N,N-diphenylamino)pyrene), 1,6-bis(N,N-diphenylamino)pyrene), 2,5,8,11-tetra-t-butylperylene (TBP), and 1,3,5-tris(1-phenyl-1H-benz[d]imidazole-2-yl)benzene)), without limitation.

The emission layer EML may comprise a host material. For example, the emission layer EML may comprise as a host material, at least one among tris(8-hydroxyquinolino)aluminum (Alq₃), bis[2-(diphenylphosphino)phenyl]ether oxide (DPEPO), 4,4'-bis(N-carbazol-9-yl)biphenyl (CBP), 1,3-bis(carbazol-9-yl)benzene (mCP), 2,8-bis(diphenylphosphoryl)dibenzo[b,d]furan (PPF), 4,4',4"-tris(carbazol-9-yl)-triphenylamine (TCTA), poly(N-vinylcarbazole) (PVK), 9,10-di(naphthalene-2-yl)anthracene (ADN), 3-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH-2), hexaphenylcyclotrisiloxane (DPSiO₃), octaphenylcyclotetrasiloxane (DPSiO₄), 2,8-bis(diphenylphosphoryl)dibenzofuran (PPF), and 1,3,5-tris(1-phenyl-1H-benz[d]imidazol-2-yl)benzene (TPBi), without limitation.

When the emission layer EML is to emit red light, the emission layer EML may further comprise, for example, a fluorescence material comprising tris(dibenzoylmethanato)phenanthroline europium (PBD:Eu(DBM)s(Phen)) or perylene. When the emission layer EML is to emit red light, the dopant comprised in the emission layer EML may be selected from, for example, a metal complex or an organometallic complex (such as bis(1-phenylisoquinoline)acetylacetonate iridium (PIQIr(acac)), bis(1-phenylquinoline)acetylacetonate iridium (PQIr(acac)), tris(1-phenylquinoline)iridium (PQIr), and/or octaethylporphyrin platinum (PtOEP)), rubrene and derivatives thereof, and 4-dicyanomethylene-2-(p-dimethylaminostyryl)-6-methyl-4H-pyran (DCM) and derivatives thereof.

When the emission layer EML is to emit green light, the emission layer EML may further comprise, for example, a fluorescence material comprising tris(8-hydroxyquinolino)aluminum (Alq₃). When the emission layer EML is to emit green light, the dopant comprised in the emission layer EML may be selected from, for example, a metal complex or an organometallic complex (such as fac-tris(2-phenylpyridine)iridium (Ir(ppy)₃)), and coumarin and derivatives thereof.

When the emission layer EML is to emit blue light, the emission layer EML may further comprise a fluorescence material comprising any one selected from the group consisting of spiro-DPVBi, spiro-6P, distyryl-benzene (DSB), distyryl-arylene (DSA), a polyfluorene (PFO)-based polymer, and a poly(p-phenylene vinylene (PPV)-based polymer. When the emission layer EML is to emit blue light, the dopant comprised in the emission layer EML may be selected from, for example, a metal complex or an organometallic complex (such as (4,6-F₂ppy)₂Irpic), and perylene and derivatives thereof.

The electron transport region ETR is provided on the emission layer EML. The electron transport region ETR may comprise at least one of a hole blocking layer HBL, an electron transport layer ETL, or an electron injection layer EIL, but embodiments of the present disclosure are not limited thereto.

The electron transport region ETR may have a single layer formed using a single material, a single layer formed using a plurality of different materials, or a multilayer structure having a plurality of layers formed using a plurality of different materials.

For example, the electron transport region ETR may have a single layer structure of an electron injection layer EIL or an electron transport layer ETL, or a single layer structure formed using an electron injection material and an electron transport material. In some embodiments, the electron transport region ETR may have a single layer structure having a plurality of different materials, or a structure stacked from the emission layer EML of electron transport layer ETL/electron injection layer EIL, or hole blocking layer HBL/electron transport layer ETL/electron injection layer EIL, without limitation. The thickness of the electron transport region ETR may be, for example, about 100 Å to about 1,500 Å.

The electron transport region ETR may be formed using any suitable method (such as a vacuum deposition method, a spin coating method, a cast method, a Langmuir-Blodgett (LB) method, an inkjet printing method, a laser printing method, and/or a laser induced thermal imaging (LITI) method).

When the electron transport region ETR comprises an electron transport layer ETL, the electron transport region ETR may comprise an anthracene-based compound. The electron transport region may comprise, for example, tris(8-hydroxyquinolinato)aluminum (Alq3), 1,3,5-tri[(3-pyridyl)-phen-3-yl]benzene, 2,4,6-tris(3'-(pyridin-3-yl)biphenyl-3-yl)-1,3,5-triazine, bis[2-(diphenylphosphino)phenyl]ether oxide (DPEPO), 2-(4-(N-phenylbenzimidazolyl-1-ylphenyl)-9,10-dinaphthylanthracene, 1,3,5-tri(1-phenyl-1H-benz[d]imidazol-2-yl)phenyl (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminum (BAlq), beryllium bis(benzoquinolin-10-olate (Bebq₂), 9,10-di(naphthalene-2-yl)anthracene (ADN), or mixtures thereof, without limitation. The thickness of the electron transport layer ETL may be about 100 Å to about 1,000 Å and may be, for example, about 150 Å to about 500 Å. When the thickness of the electron transport layer ETL satisfies the above-described range, satisfactory electron transport properties may be obtained without substantial increase of a driving voltage.

When the electron transport region ETR comprises the electron injection layer EIL, the electron transport region ETR may comprise, a metal halide (such as LiF, NaCl, CsF, RbCl, and/or RbI), a lanthanide metal (such as Yb), a metal oxide (such as Li₂O and/or BaO), or lithium quinolate (LiQ). However, an embodiment of the present disclosure is not limited thereto. In some embodiments, the electron injection layer EIL may be formed using a mixture material of an electron transport material and an insulating organo metal salt. The organo metal salt may be a material having an energy band gap of about 4 eV or more. The organo metal salt may comprise, for example, metal acetates, metal benzoates, metal acetoacetates, metal acetylacetonates, and/or metal stearates. The thickness of the electron injection layer EIL may be about 1 Å to about 100 Å, and about 3 Å to about 90 Å. When the thickness of the electron injection layer EIL satisfies the above described range, satisfactory electron injection properties may be obtained without inducing a substantial increase in driving voltage.

The electron transport region ETR may comprise a hole blocking layer HBL as described above. The hole blocking layer HBL may comprise, for example, at least one of 2,9-dimethyl-4,7-diphenyl-1,10-phenanthroline (BCP), bis[2-(diphenylphosphino)phenyl]ether oxide (DPEPO), or 4,7-diphenyl-1,10-phenanthroline (Bphen). However, embodiments of the present disclosure are not limited thereto.

The second electrode EL2 is provided on the electron transport region ETR. The second electrode EL2 may be a common electrode or a cathode. The second electrode EL2 may be a transmissive electrode, a transflective electrode, or a reflective electrode. When the second electrode EL2 is a transmissive electrode, the second electrode EL2 may comprise a transparent metal oxide, for example, ITO, IZO, ZnO, ITZO, etc.

When the second electrode EL2 is a transflective electrode or a reflective electrode, the second electrode EL2 may comprise Ag, Mg, Cu, Al, Pt, Pd, Au, Ni, Nd, Ir, Cr, Li, Ca, LiF/Ca, LiF/Al, Mo, Ti, compounds thereof, or mixtures thereof (for example, a mixture of Ag and Mg). The second electrode EL2 may have a multilayered structure comprising a reflective layer or a transflective layer formed using the above-described materials and a transparent conductive layer formed using ITO, IZO, ZnO, ITZO, etc.

In some embodiments, the second electrode EL2 may be connected with an auxiliary electrode. When the second electrode EL2 is connected with the auxiliary electrode, the resistance of the second electrode EL2 may decrease.

Referring to FIG. 4, on the second electrode EL2 of the organic electroluminescence device 10 of an embodiment, a capping layer (CPL) may be further disposed. The capping layer CPL may have a single layer or multilayer.

In an embodiment, the capping layer CPL may be an organic layer or an inorganic layer. For example, when the capping layer CPL comprises an inorganic material, the inorganic material may comprise an alkali metal compound (such as LiF), and/or an alkaline earth compound (such as MgF₂, SiON, SiNₓ, SiO_{y}, etc.)

For example, when the capping layer (CPL) comprises an organic material, the organic material may comprise α-NPD, NPB, TPD, m-MTDATA, Alq₃, CuPc, N4,N4,N4',N4'-tetra(biphenyl-4-yl) biphenyl-4,4'-diamine (TPD15), 4,4',4"-tris(carbazol-9-yl) triphenylamine (TCTA), etc., or may comprise an epoxy resin, or acrylate such as methacrylate. However, an embodiment of the present disclosure is not limited thereto, and the capping layer CPL may comprise an amine compound. For example, the capping layer CPL may comprise any one among Compounds P1 to P5.

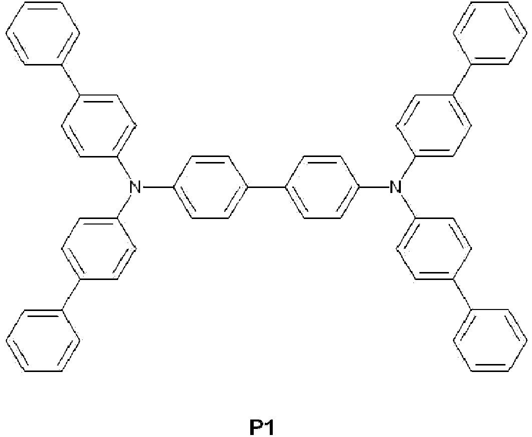

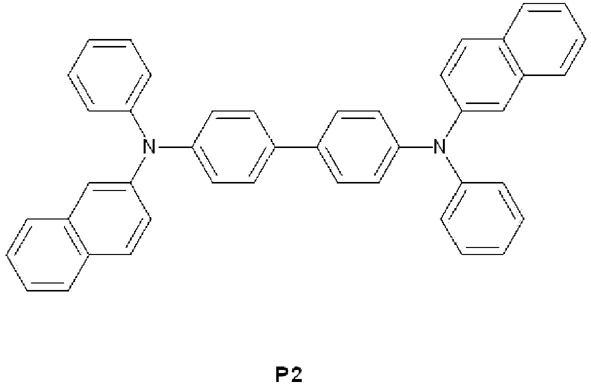

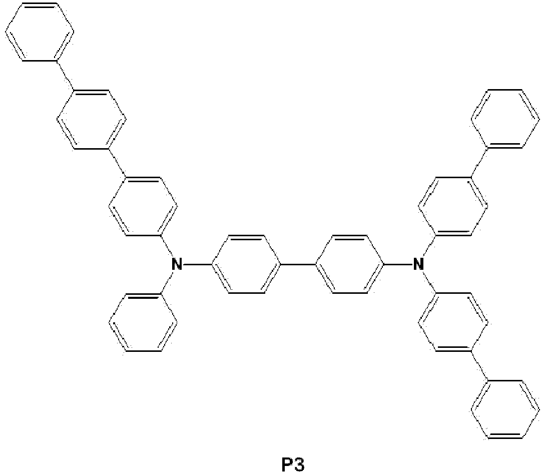

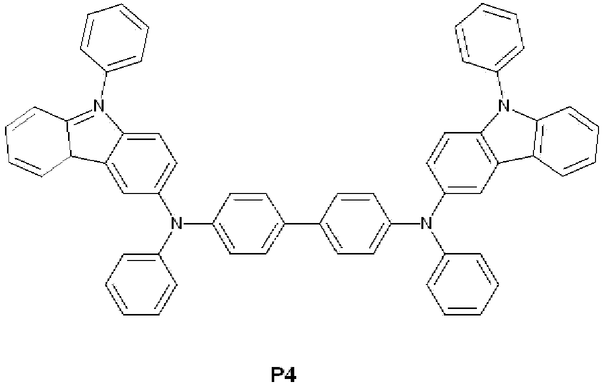

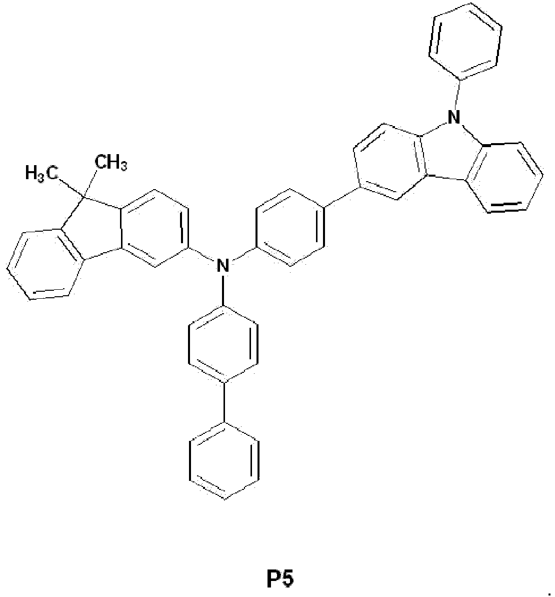

The refractive index of the capping layer CPL may be 1.6 or more. For example, the refractive index of the capping layer CPL may be 1.6 or more with respect to light having a wavelength of about 550 nm to about 660 nm.

In the organic electroluminescence device 10, according to the application of voltages to the first electrode EL1 and the second electrode EL2, respectively, holes injected from the first electrode EL1 move through the hole transport region HTR to the emission layer EML, and electrons injected from the second electrode EL2 move through the electron transport region ETR to the emission layer EML. The electrons and holes may recombine in the emission layer EML to produce excitons, and light may be emitted via transition of the excitons from an excited state to the ground state.

When the organic electroluminescence device 10 is a top emission type, the first electrode EL1 may be a reflective electrode, and the second electrode EL2 may be a transmissive or a transflective electrode. When the organic electroluminescence device 10 is a bottom emission type, the first electrode EL1 may be a transmissive or transflective electrode, and the second electrode EL2 may be a reflective electrode.

The organic electroluminescence device 10 according to an embodiment of the present disclosure comprises the polycyclic compound represented by Formula 1, and accordingly, high efficiency and/or long lifespan may be achieved. In addition, a decreased driving voltage may be achieved.

Hereinafter, the present disclosure will be explained referring to embodiments and comparative embodiments. The following example embodiments are only illustrations to assist the understanding of the present disclosure, and the scope of the present disclosure is not limited thereto.

### [Synthetic Examples]

The polycyclic compound according to an embodiment of the present disclosure may be synthesized, for example, as follows. However, the synthetic method of the polycyclic compound according to an embodiment of the present disclosure is not limited thereto.

### 1. Synthesis of Compound A1

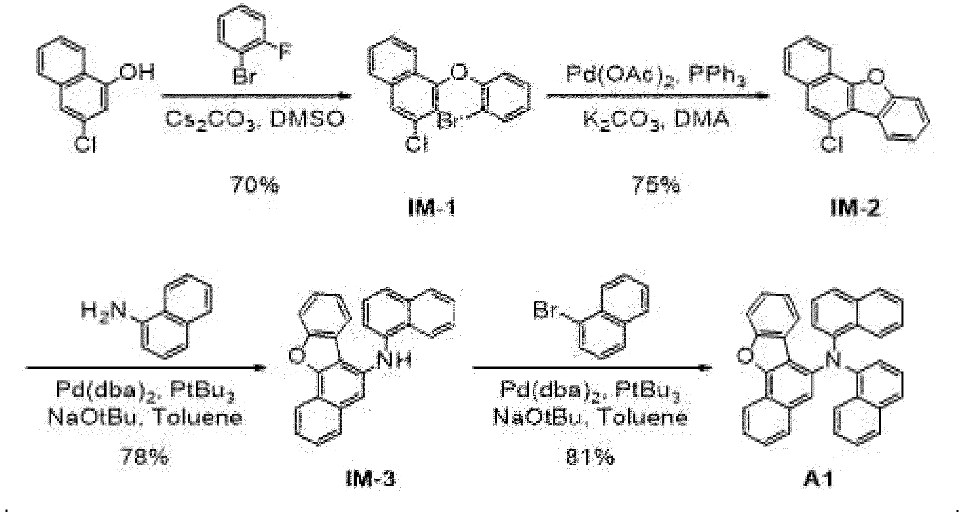

### (Synthesis of Intermediate IM-1)

Under an Ar atmosphere, to a 500 mL, three neck flask, 25.00 g (140.0 mmol) of 3-chloronaphthalen-1-ol, 29.39 g (1.2 eq, 168.0 mmol) of 1-bromo-2-fluorobenzene, 91.20 g (2.0 eq, 280.0 mmol) of Cs₂CO₃, and 280 mL of DMSO were added in order, followed by heating and stirring at about 110 °C. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-1 (32.68 g, yield 70%).

By FAB-MS measurement, mass number m/z = 333 was observed as the molecular ion peak, and Intermediate IM-1 was identified.

### (Synthesis of Intermediate IM-2)

Under an Ar atmosphere, to a 500 mL, three neck flask, 25.00 g (74.9 mmol) of IM-1, 0.84 g (0.05 eq, 3.7 mmol) of Pd(OAc)₂, 15.54 g (1.5 eq, 112.4 mmol) of K₂CO₃, 1.97 g (0.10 eq, 7.5 mmol) of PPh₃, and 300 mL of DMA were added in order, followed by heating to about 140 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-2 (14.20 g, yield 75%).

By FAB-MS measurement, mass number m/z = 252 was observed as the molecular ion peak, and Intermediate IM-2 was identified.

### (Synthesis of Intermediate IM-3)

Under an Ar atmosphere, to a 500 mL, three neck flask, 12.00 g (47.5 mmol) of IM-2, 0.82 g (0.03 eq, 1.4 mmol) of Pd(dba)₂, 4.56 g (2.0 eq, 47.5 mmol) of NaOtBu, 237 mL of toluene, 7.48 g (1.1 eq, 45.2 mmol) of naphthalene-1-amine, and 0.96 g (0.1 eq, 4.7 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-3 (13.31 g, yield 78%).

By FAB-MS measurement, mass number m/z = 359 was observed as the molecular ion peak, and Intermediate IM-3 was identified.

### (Synthesis of Compound A1)

Under an Ar atmosphere, to a 300 mL, three neck flask, 10.00 g (27.8 mmol) of IM-3, 0.48 g (0.03 eq, 0.8 mmol) of Pd(dba)₂, 5.35 g (2.0 eq, 55.6 mmol) of NaOtBu, 139 mL of toluene, 6.34 g (1.1 eq, 30.6 mmol) of 1-bromonaphthalene, and 0.56 g (0.1 eq, 2.8 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound A1 (10.94 g, yield 81%) as a solid.

By FAB-MS measurement, mass number m/z = 485 was observed as the molecular ion peak, and Compound A1 was identified.

### 2. Synthesis of Compound A19

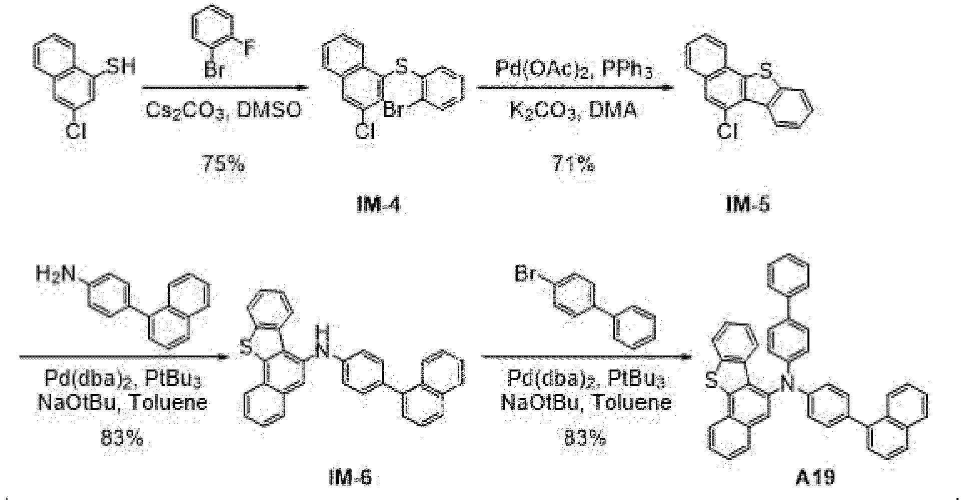

### (Synthesis of Intermediate IM-4)

Under an Ar atmosphere, to a 500 mL, three neck flask, 25.00 g (128.4 mmol) of 3-chloronaphthalen-1-thiol, 26,97 g (1.2 eq, 154.1 mmol) of 1-bromo-2-fluorobenzene, 83.68 g (2.0 eq, 256.8 mmol) of Cs₂CO₃, and 257 mL of DMSO were added in order, followed by heating and stirring at about 110 °C. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-4 (33.68 g, yield 75%).

By FAB-MS measurement, mass number m/z = 349 was observed as the molecular ion peak, and Intermediate IM-4 was identified.

### (Synthesis of Intermediate IM-5)

Under an Ar atmosphere, to a 500 mL, three neck flask, 25.00 g (71.5 mmol) of IM-4, 0.80 g (0.05 eq, 3.6 mmol) of Pd(OAc)₂, 14.52 g (1.5 eq, 107.2 mmol) of K₂CO₃, 1.88 g (0.10 eq, 7.1 mmol) of PPh₃, and 286 mL of DMA were added in order, followed by heating to about 140 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-5 (13.64 g, yield 71%).

By FAB-MS measurement, mass number m/z = 268 was observed as the molecular ion peak, and Intermediate IM-5 was identified.

### (Synthesis of Intermediate IM-6)

Under an Ar atmosphere, to a 500 mL, three neck flask, 12.00 g (44.7 mmol) of IM-6, 0.77 g (0.03 eq, 1.3 mmol) of Pd(dba)₂, 4.29 g (2.0 eq, 44.7 mmol) of NaOtBu, 224 mL of toluene, 10.77 g (1.1 eq, 49.1 mmol) of 4-(naphthalene-1-yl)aniline, and 0.90 g (0.1 eq, 4.5 mmol) of tBu₃P were added in order, followed by heating, refluxing and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-6 (16.74 g, yield 83%).

By FAB-MS measurement, mass number m/z = 451 was observed as the molecular ion peak, and Intermediate IM-6 was identified.

### (Synthesis of Compound A19)

Under an Ar atmosphere, to a 300 mL, three neck flask, 10.00 g (22.1 mmol) of IM-6, 0.38 g (0.03 eq, 0.7 mmol) of Pd(dba)₂, 4.26 g (2.0 eq, 44.3 mmol) of NaOtBu, 111 mL of toluene, 5.68 g (1.1 eq, 24.4 mmol) of 4-bromobiphenyl, and 0.45 g (0.1 eq, 2.2 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound A19 (11.10 g, yield 83%) as a solid.

By FAB-MS measurement, mass number m/z = 603 was observed as the molecular ion peak, and Compound A19 was identified.

### 3. Synthesis of Compound A65

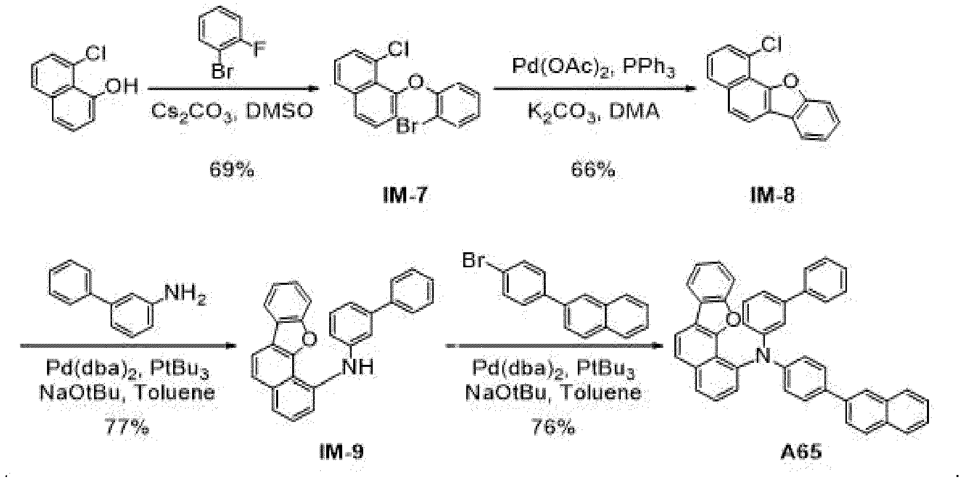

### (Synthesis of Intermediate IM-7)

Under an Ar atmosphere, to a 500 mL, three neck flask, 25.00 g (140.0 mmol) of 8-chloronaphthalen-1-ol, 29.39 g (1.2 eq, 168.0 mmol) of 1-bromo-2-fluorobenzene, 91.20 g (2.0 eq, 280.0 mmol) of Cs₂CO₃, and 280 mL of DMSO were added in order, followed by heating and stirring at about 110 °C. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-7 (32.22 g, yield 69%).

By FAB-MS measurement, mass number m/z = 333 was observed as the molecular ion peak, and Intermediate IM-7 was identified.

### (Synthesis of Intermediate IM-8)

Under an Ar atmosphere, to a 500 mL, three neck flask, 25.00 g (74.9 mmol) of IM-7, 0.84 g (0.05 eq, 3.7 mmol) of Pd(OAc)₂, 15.54 g (1.5 eq, 112.4 mmol) of K₂CO₃, 1.97 g (0.10 eq, 7.5 mmol) of PPh₃, and 300 mL of DMA were added in order, followed by heating to about 140 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-8 (12.50 g, yield 66%).

By FAB-MS measurement, mass number m/z = 252 was observed as the molecular ion peak, and Intermediate IM-8 was identified.

### (Synthesis of Intermediate IM-9)

Under an Ar atmosphere, to a 300 mL, three neck flask, 10.00 g (39.6 mmol) of IM-8, 0.68 g (0.03 eq, 1.2 mmol) of Pd(dba)₂, 3.80 g (1.0 eq, 39.6 mmol) of NaOtBu, 198 mL of toluene, 7.37 g (1.1 eq, 43.5 mmol) of [1,1'-biphenyl]-3-amine, and 0.80 g (0.1 eq, 4.0 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-9 (11.75 g, yield 77%).

By FAB-MS measurement, mass number m/z = 385 was observed as the molecular ion peak, and Intermediate IM-9 was identified.

### (Synthesis of Compound A65)

Under an Ar atmosphere, to a 300 mL, three neck flask, 10.00 g (25.9 mmol) of IM-9, 0.45 g (0.03 eq, 0.8 mmol) of Pd(dba)₂, 4.99 g (2.0 eq, 51.9 mmol) of NaOtBu, 130 mL of toluene, 8.08 g (1.1 eq, 28.5 mmol) of 2-(4-bromophenyl)naphthalene, and 0.52 g (0.1 eq, 2.6 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound A65 (11.59 g, yield 76%) as a solid.

By FAB-MS measurement, mass number m/z = 587 was observed as the molecular ion peak, and Compound A65 was identified.

### 4. Synthesis of Compound B49

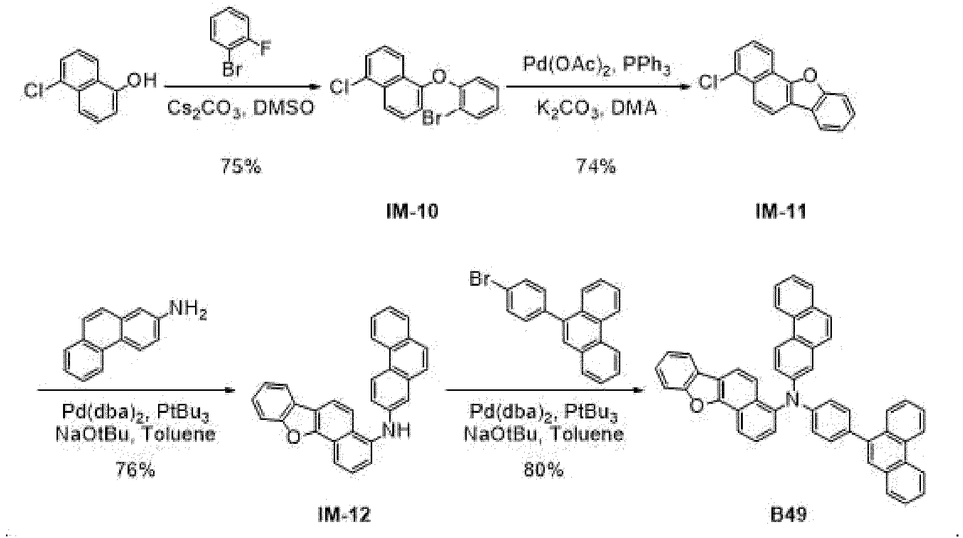

### (Synthesis of Intermediate IM-10)

Under an Ar atmosphere, to a 500 mL, three neck flask, 25.00 g (140.0 mmol) of 5-chloronaphthalen-1-ol, 29.39 g (1.2 eq, 168.0 mmol) of 1-bromo-2-fluorobenzene, 91.20 g (2.0 eq, 280.0 mmol) of Cs₂CO₃, and 280 mL of DMSO were added in order, followed by heating and stirring at about 110 °C. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-10 (35.02 g, yield 75%).

By FAB-MS measurement, mass number m/z = 333 was observed as the molecular ion peak, and Intermediate IM-10 was identified.

### (Synthesis of Intermediate IM-11)

Under an Ar atmosphere, to a 500 mL, three neck flask, 25.00 g (74.9 mmol) of IM-10, 0.84 g (0.05 eq, 3.7 mmol) of Pd(OAc)₂, 15.54 g (1.5 eq, 112.4 mmol) of K₂CO₃, 1.97 g (0.10 eq, 7.5 mmol) of PPh₃, and 300 mL of DMA were added in order, followed by heating to about 140 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-11 (14.01 g, yield 74%).

By FAB-MS measurement, mass number m/z = 252 was observed as the molecular ion peak, and Intermediate IM-11 was identified.

### (Synthesis of Intermediate IM-12)

Under an Ar atmosphere, to a 500 mL, three neck flask, 12.00 g (47.5 mmol) of IM-11, 0.82 g (0.03 eq, 1.4 mmol) of Pd(dba)₂, 4.57 g (1.0 eq, 47.5 mmol) of NaOtBu, 237 mL of toluene, 10.09 g (1.1 eq, 52.2 mmol) of phenanthren-2-amine, and 0.96 g (0.1 eq, 4.0 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-12 (14.78 g, yield 76%).

By FAB-MS measurement, mass number m/z = 409 was observed as the molecular ion peak, and Intermediate IM-12 was identified.

### (Synthesis of Compound B49)

Under an Ar atmosphere, to a 300 mL, three neck flask, 10.00 g (24.4 mmol) of IM-12, 0.42 g (0.03 eq, 0.7 mmol) of Pd(dba)₂, 4.69 g (2.0 eq, 48.8 mmol) of NaOtBu, 122 mL of toluene, 8.95 g (1.1 eq, 26.9 mmol) of 9-(4-bromophenyl)naphthalene, and 0.49 g (0.1 eq, 2.4 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound B49 (12.93 g, yield 80%) as a solid.

By FAB-MS measurement, mass number m/z = 661 was observed as the molecular ion peak, and Compound B49 was identified.

### 5. Synthesis of Compound B94

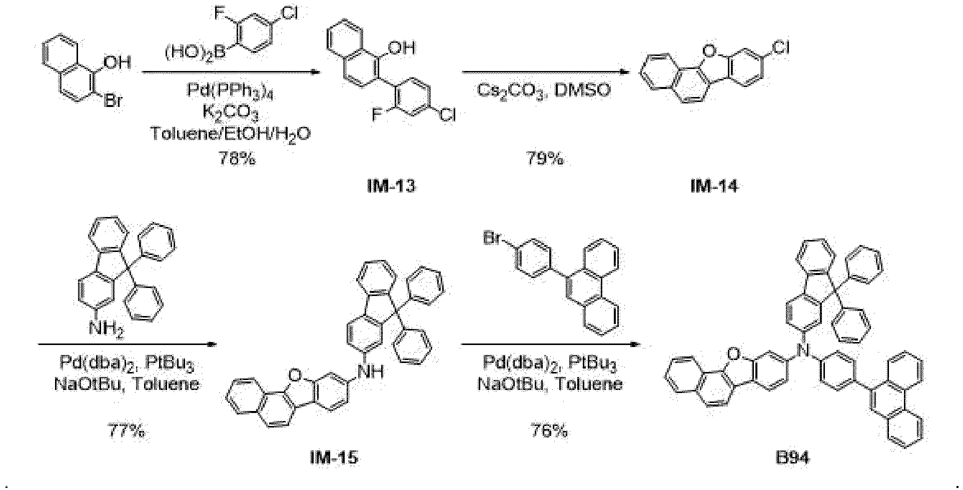

### (Synthesis of Intermediate IM-13)

Under an Ar atmosphere, to a 2,000 mL, three neck flask, 30.00 g (134.5 mmol) of 2-bromo-1-naphthol, 25.79 g (1.1 eq, 147.9 mmol) of (4-chloro-2-fluorophenyl)boronic acid, 55.76 g (3.0 eq, 403.5 mmol) of K₂CO₃, 7.77 g (0.05 eq, 6.7 mmol) of Pd(PPh₃)₄, and 941 mL of a mixture solution of toluene/EtOH/H₂O (4/2/1) were added in order, followed by heating and stirring at about 80 °C. After cooling to room temperature, the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-13 (28.61 g, yield 78%).

By FAB-MS measurement, mass number m/z = 272 was observed as the molecular ion peak, and Intermediate IM-13 was identified.

### (Synthesis of Intermediate IM-14)

Under an Ar atmosphere, to a 300 mL, three neck flask, 25.00 g (91.7 mmol) of IM-13, 59.74 g (2.0 eq, 183.4 mmol) of Cs₂CO₃, and 184 mL of DMSO were added in order, followed by heating to about 110 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-14 (18.30 g, yield 79%).

By FAB-MS measurement, mass number m/z = 252 was observed as the molecular ion peak, and Intermediate IM-14 was identified.

### (Synthesis of Intermediate IM-15)

Under an Ar atmosphere, to a 500 mL, three neck flask, 12.00 g (47.5 mmol) of IM-14, 0.82 g (0.03 eq, 1.4 mmol) of Pd(dba)₂, 4.57 g (1.0 eq, 47.5 mmol) of NaOtBu, 237 mL of toluene, 17.42 g (1.1 eq, 52.2 mmol) of 9,9-diphenyl-9H-fluoren-2-amine, and 0.96 g (0.1 eq, 4.0 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-15 (20.10 g, yield 77%).

By FAB-MS measurement, mass number m/z = 549 was observed as the molecular ion peak, and Intermediate IM-15 was identified.

### (Synthesis of Compound B94)

Under an Ar atmosphere, to a 300 mL, three neck flask, 10.00 g (18.2 mmol) of IM-15, 0.31 g (0.03 eq, 0.5 mmol) of Pd(dba)₂, 3.50 g (2.0 eq, 36.4 mmol) of NaOtBu, 91 mL of toluene, 6.67 g (1.1 eq, 20.0 mmol) of 9-(4-bromophenyl)phenanthrene, and 0.37 g (0.1 eq, 1.8 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound B94 (11.09 g, yield 76%) as a solid.

By FAB-MS measurement, mass number m/z = 801 was observed as the molecular ion peak, and Compound B94 was identified.

### 6. Synthesis of Compound C67

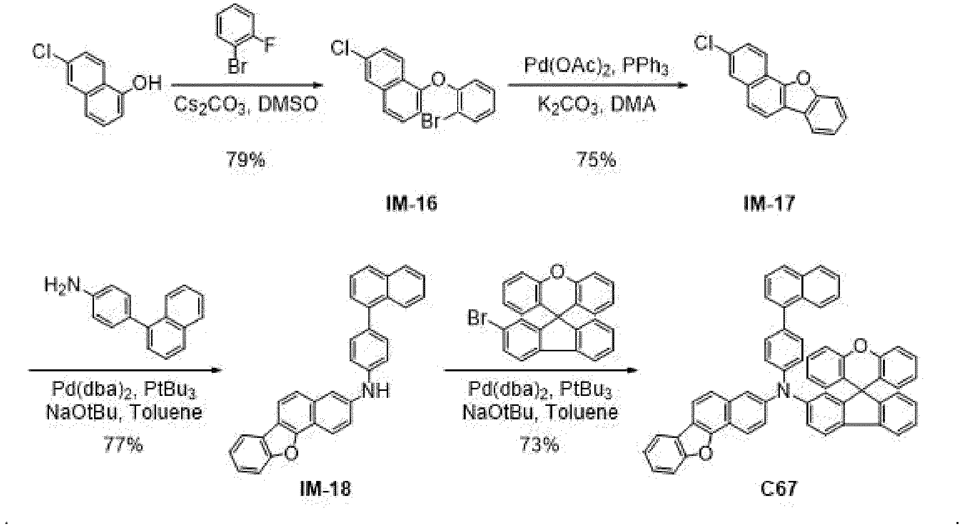

### (Synthesis of Intermediate IM-16)

Under an Ar atmosphere, to a 500 mL, three neck flask, 25.00 g (140.0 mmol) of 6-chloronaphthalen-1-ol, 29.39 g (1.2 eq, 168.0 mmol) of 1-bromo-2-fluorobenzene, 91.20 g (2.0 eq, 280.0 mmol) of Cs₂CO₃, and 280 mL of DMSO were added in order, followed by heating and stirring at about 110 °C. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-16 (36.89 g, yield 79%).

By FAB-MS measurement, mass number m/z = 333 was observed as the molecular ion peak, and Intermediate IM-16 was identified.

### (Synthesis of Intermediate IM-17)

Under an Ar atmosphere, to a 500 mL, three neck flask, 25.00 g (74.9 mmol) of IM-16, 0.84 g (0.05 eq, 3.7 mmol) of Pd(OAc)₂, 15.54 g (1.5 eq, 112.4 mmol) of K₂CO₃, 1.97 g (0.10 eq, 7.5 mmol) of PPh₃, and 300 mL of DMA were added in order, followed by heating to about 140 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-17 (14.20 g, yield 75%).

By FAB-MS measurement, mass number m/z = 252 was observed as the molecular ion peak, and Intermediate IM-17 was identified.

### (Synthesis of Intermediate IM-18)

Under an Ar atmosphere, to a 500 mL, three neck flask, 12.00 g (47.5 mmol) of IM-17, 0.82 g (0.03 eq, 1.4 mmol) of Pd(dba)₂, 4.57 g (1.0 eq, 47.5 mmol) of NaOtBu, 237 mL of toluene, 11.45 g (1.1 eq, 52.2 mmol) of 4-(naphthalene-1-yl)aniline, and 0.96 g (0.1 eq, 4.0 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-18 (15.93 g, yield 77%).

By FAB-MS measurement, mass number m/z = 435 was observed as the molecular ion peak, and Intermediate IM-18 was identified.

### (Synthesis of Compound C67)

Under an Ar atmosphere, to a 300 mL, three neck flask, 10.00 g (23.0 mmol) of IM-18, 0.40 g (0.03 eq, 0.7 mmol) of Pd(dba)₂, 4.41 g (2.0 eq, 45.9 mmol) of NaOtBu, 115 mL of toluene, 10.39 g (1.1 eq, 25.3 mmol) of 2-bromo-spiro[fluorene-9,9'-xanthene], and 0.47 g (0.1 eq, 2.3 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound C67 (12.84 g, yield 73%) as a solid.

By FAB-MS measurement, mass number m/z = 765 was observed as the molecular ion peak, and Compound C67 was identified.

### 7. Synthesis of Compound C124

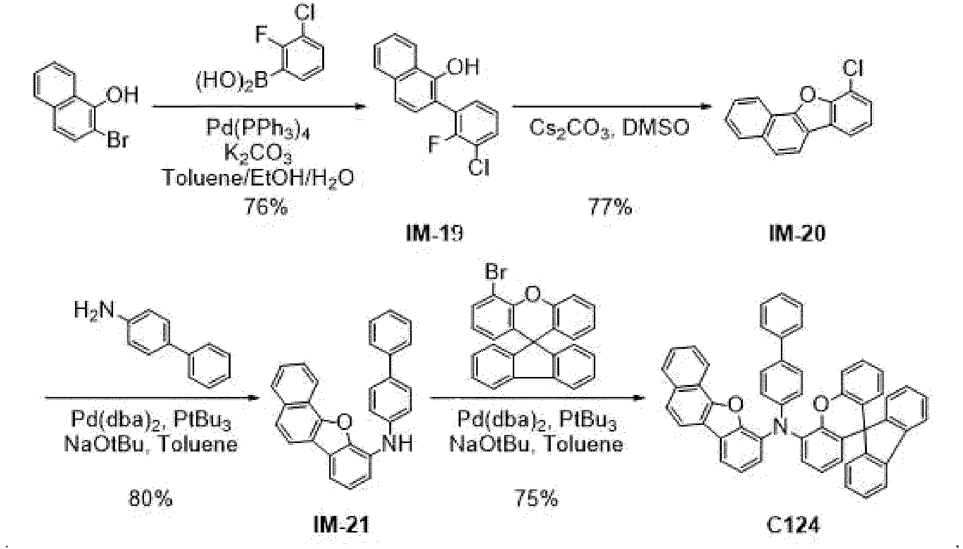

### (Synthesis of Intermediate IM-19)

Under an Ar atmosphere, to a 2,000 mL, three neck flask, 30.00 g (134.5 mmol) of 2-bromo-1-naphthol, 25.79 g (1.1 eq, 147.9 mmol) of (3-chloro-2-fluorophenyl)boronic acid, 55.76 g (3.0 eq, 403.5 mmol) of K₂CO₃, 7.77 g (0.05 eq, 6.7 mmol) of Pd(PPh₃)₄, and 941 mL of a mixture solution of toluene/EtOH/H₂O (4/2/1) were added in order, followed by heating and stirring at about 80 °C. After cooling to room temperature, the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-19 (27.87 g, yield 76%).

By FAB-MS measurement, mass number m/z = 272 was observed as the molecular ion peak, and Intermediate IM-19 was identified.

### (Synthesis of Intermediate IM-20)

Under an Ar atmosphere, to a 300 mL, three neck flask, 25.00 g (91.7 mmol) of IM-19, 59.74 g (2.0 eq, 183.4 mmol) of Cs₂CO₃, and 184 mL of DMSO were added in order, followed by heating to about 110 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-20 (17.84 g, yield 77%).

By FAB-MS measurement, mass number m/z = 252 was observed as the molecular ion peak, and Intermediate IM-20 was identified.

### (Synthesis of Intermediate IM-21)

Under an Ar atmosphere, to a 500 mL, three neck flask, 12.00 g (47.5 mmol) of IM-20, 0.82 g (0.03 eq, 1.4 mmol) of Pd(dba)₂, 4.57 g (1.0 eq, 47.5 mmol) of NaOtBu, 237 mL of toluene, 8.84 g (1.1 eq, 52.2 mmol) of 4-aminobiphenyl, and 0.96 g (0.1 eq, 4.0 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-21 (14.64 g, yield 80%).

By FAB-MS measurement, mass number m/z = 385 was observed as the molecular ion peak, and Intermediate IM-21 was identified.

### (Synthesis of Compound C124)

Under an Ar atmosphere, to a 300 mL, three neck flask, 10.00 g (25.9 mmol) of IM-21, 0.45 g (0.03 eq, 0.8 mmol) of Pd(dba)₂, 4.99 g (2.0 eq, 51.9 mmol) of NaOtBu, 130 mL of toluene, 11.74 g (1.1 eq, 28.5 mmol) of 4'-bromospiro[fluorene-9,9'-xanthene], and 0.52 g (0.1 eq, 2.6 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound C124 (13.93 g, yield 75%) as a solid.

By FAB-MS measurement, mass number m/z = 715 was observed as the molecular ion peak, and Compound C124 was identified.

### 8. Synthesis of Compound C153

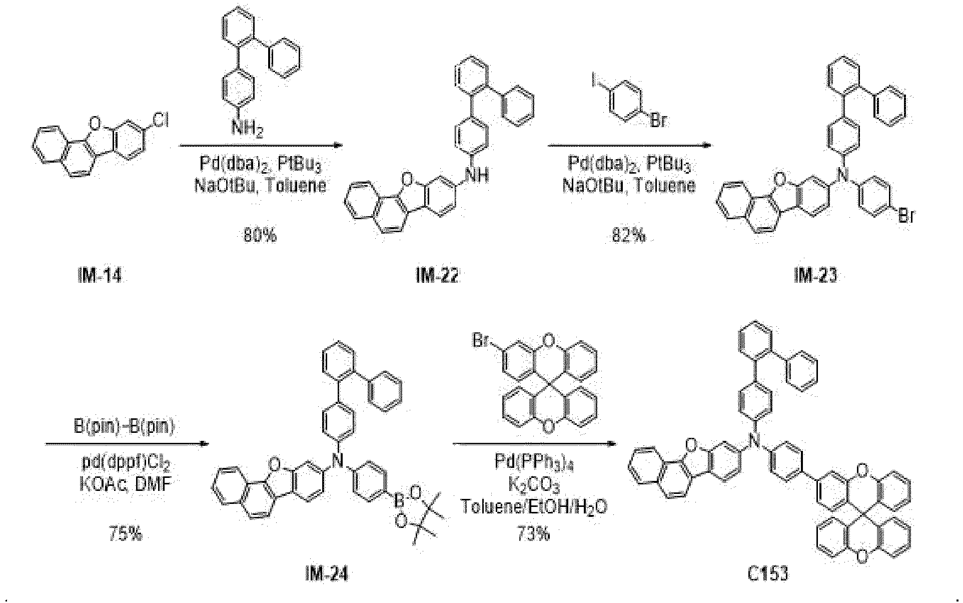

### (Synthesis of Intermediate IM-22)

Under an Ar atmosphere, to a 500 mL, three neck flask, 12.00 g (47.5 mmol) of IM-14, 0.82 g (0.03 eq, 1.4 mmol) of Pd(dba)₂, 4.57 g (1.0 eq, 47.5 mmol) of NaOtBu, 237 mL of toluene, 12.81 g (1.1 eq, 52.2 mmol) of [1,1':2',1"-terphenyl]-4-amine, and 0.96 g (0.1 eq, 4.0 mmol) of tBu₃P were added in order, followed by heating, refluxing, and stirring. After cooling to room temperature, water was added to the reaction solution, and an organic layer was separated. Toluene was added to an aqueous layer, and further organic layers were extracted. The organic layers were collected, washed with a saturated saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-22 (17.53 g, yield 80%).

By FAB-MS measurement, mass number m/z = 461 was observed as the molecular ion peak, and Intermediate IM-22 was identified.

### (Synthesis of Intermediate IM-23)

Under an Ar atmosphere, to a 500 mL, three neck flask, 15.00 g (32.5 mmol) of IM-22, 0.56 g (0.03 eq, 1.0 mmol) of Pd(dba)₂, 6.25 g (1.0 eq, 65.0 mmol) of NaOtBu, 162 mL of toluene, 10.11 g (1.1 eq, 35.7 mmol) of 1-bromo-4-iodobenzene, and 0.66 g (0.1 eq, 3.2 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-23 (16.43 g, yield 82%).

By FAB-MS measurement, mass number m/z = 616 was observed as the molecular ion peak, and Intermediate IM-23 was identified.

### (Synthesis of Intermediate IM-24)

Under an Ar atmosphere, to a 300 mL, three neck flask, 15.00 g (24.3 mmol) of IM-23, 1.99 g (0.10 eq, 2.4 mmol) of Pd(dppf)Cl₂, 4.77 g (2.0 eq, 48.7 mmol) of KOAc, 122 mL of DMF, and 7.41 g (1.2 eq, 29.2 mmol) of bis(pinacolato)diboron were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-24 (12.11 g, yield 75%).

By FAB-MS measurement, mass number m/z = 663 was observed as the molecular ion peak, and Intermediate IM-24 was identified.

### (Synthesis of Compound C153)

Under an Ar atmosphere, to a 300 mL, three neck flask, 10.00 g (31.8 mmol) of IM-24, 7.08 g (1.1 eq, 16.6 mmol) of 3-bromo-9,9'-spirobi[xanthene], 6.25 g (3.0 eq, 45.2 mmol) of K₂CO₃, 0.87 g (0.05 eq, 0.8 mmol) of Pd(PPh₃)₄, and 105 mL of a mixture solution of toluene/EtOH/H₂O (4/2/1) were added in order, followed by heating to about 80 °C and stirring. After cooling to room temperature, the reaction solution was extracted with toluene. An aqueous layer was removed, and an organic layer was washed with a saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound C153 (9.72 g, yield 73%) as a solid.

By FAB-MS measurement, mass number m/z = 884 was observed as the molecular ion peak, and Compound C153 was identified.

### 9. Synthesis of Compound D32

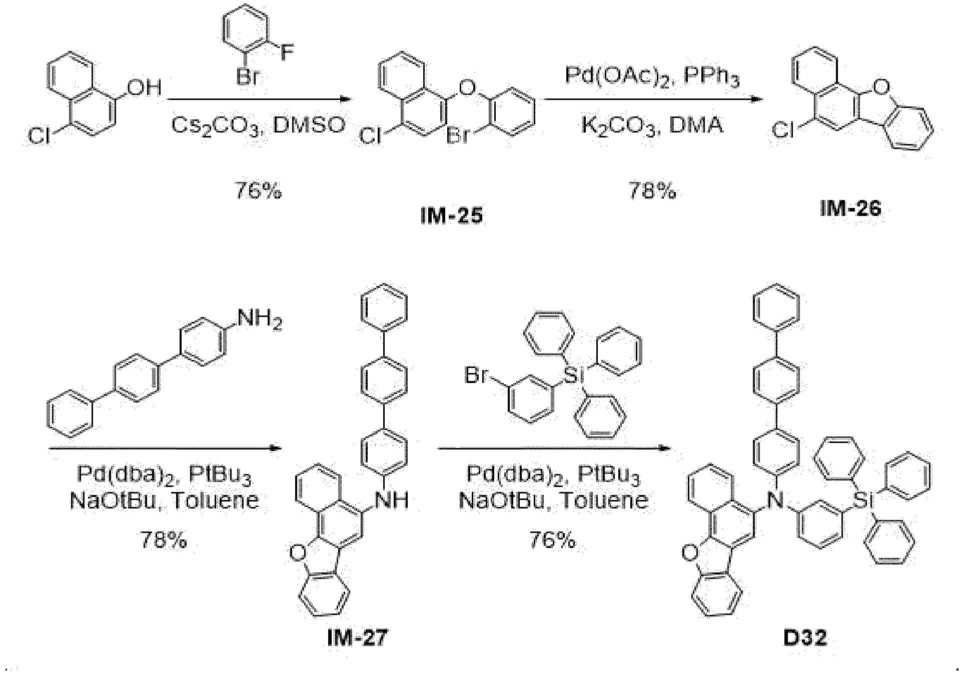

### (Synthesis of Intermediate IM-25)

Under an Ar atmosphere, to a 500 mL, three neck flask, 25.00 g (140.0 mmol) of 4-chloronaphthalen-1-ol, 29.39 g (1.2 eq, 168.0 mmol) of 1-bromo-2-fluorobenzene, 91.20 g (2.0 eq, 280.0 mmol) of Cs₂CO₃, and 280 mL of DMSO were added in order, followed by heating and stirring at about 110 °C. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-25 (35.49 g, yield 76%).

By FAB-MS measurement, mass number m/z = 333 was observed as the molecular ion peak, and Intermediate IM-25 was identified.

### (Synthesis of Intermediate IM-26)

Under an Ar atmosphere, to a 500 mL, three neck flask, 25.00 g (74.9 mmol) of IM-25, 0.84 g (0.05 eq, 3.7 mmol) of Pd(OAc)₂, 15.54 g (1.5 eq, 112.4 mmol) of K₂CO₃, 1.97 g (0.10 eq, 7.5 mmol) of PPh₃, and 300 mL of DMA were added in order, followed by heating to about 140 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-26 (14.77 g, yield 78%).

By FAB-MS measurement, mass number m/z = 252 was observed as the molecular ion peak, and Intermediate IM-26 was identified.

### (Synthesis of Intermediate IM-27)

Under an Ar atmosphere, to a 500 mL, three neck flask, 12.00 g (47.5 mmol) of IM-26, 0.82 g (0.03 eq, 1.4 mmol) of Pd(dba)₂, 4.57 g (1.0 eq, 47.5 mmol) of NaOtBu, 237 mL of toluene, 12.81 g (1.1 eq, 52.2 mmol) of [1,1':4',1"-terphenyl]-4-amine, and 0.96 g (0.1 eq, 4.0 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-27 (17.10 g, yield 78%).

By FAB-MS measurement, mass number m/z = 461 was observed as the molecular ion peak, and Intermediate IM-27 was identified.

### (Synthesis of Compound D32)

Under an Ar atmosphere, to a 300 mL, three neck flask, 10.00 g (21.7 mmol) of IM-27, 0.37 g (0.03 eq, 0.6 mmol) of Pd(dba)₂, 4.16 g (2.0 eq, 43.3 mmol) of NaOtBu, 108 mL of toluene, 9.90 g (1.1 eq, 23.8 mmol) of 3-bromotetraphenylsilane, and 0.44 g (0.1 eq, 2.2 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound D32 (13.11 g, yield 76%) as a solid.

By FAB-MS measurement, mass number m/z = 796 was observed as the molecular ion peak, and Compound D32 was identified.

### 10. Synthesis of Compound D162

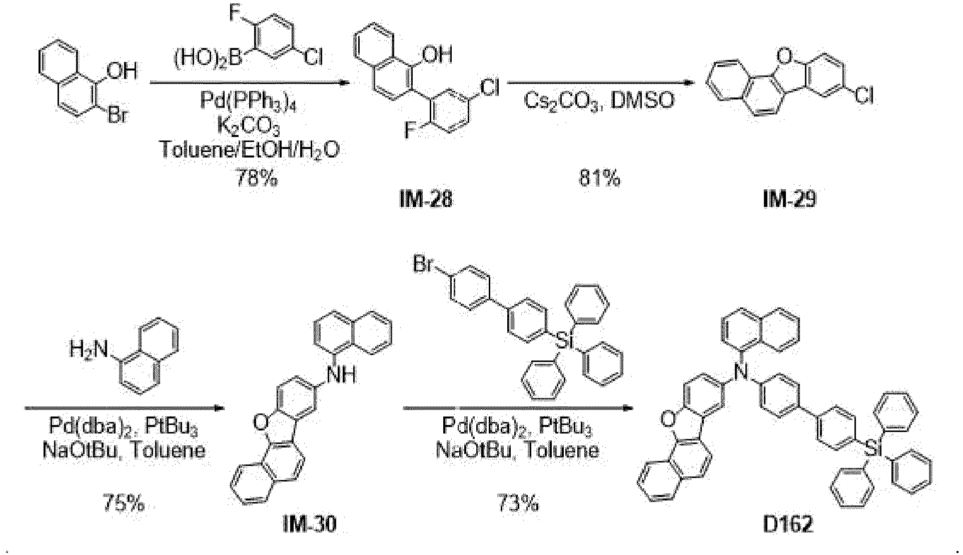

### (Synthesis of Intermediate IM-28)

Under an Ar atmosphere, to a 2,000 mL, three neck flask, 30.00 g (134.5 mmol) of 2-bromo-1-naphthol, 25.79 g (1.1 eq, 147.9 mmol) of (5-chloro-2-fluorophenyl)boronic acid, 55.76 g (3.0 eq, 403.5 mmol) of K₂CO₃, 7.77 g (0.05 eq, 6.7 mmol) of Pd(PPh₃)₄, and 941 mL of a mixture solution of toluene/EtOH/H₂O (4/2/1) were added in order, followed by heating and stirring at about 80 °C. After cooling to room temperature, the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-28 (13.83 g, yield 78%).

By FAB-MS measurement, mass number m/z = 272 was observed as the molecular ion peak, and Intermediate IM-28 was identified.

### (Synthesis of Intermediate IM-29)

Under an Ar atmosphere, to a 300 mL, three neck flask, 25.00 g (91.7 mmol) of IM-28, 59.74 g (2.0 eq, 183.4 mmol) of Cs₂CO₃, and 184 mL of DMSO were added in order, followed by heating to about 110 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-29 (18.76 g, yield 81%).

By FAB-MS measurement, mass number m/z = 252 was observed as the molecular ion peak, and Intermediate IM-29 was identified.

### (Synthesis of Intermediate IM-30)

Under an Ar atmosphere, to a 500 mL, three neck flask, 12.00 g (47.5 mmol) of IM-29, 0.82 g (0.03 eq, 1.4 mmol) of Pd(dba)₂, 4.57 g (1.0 eq, 47.5 mmol) of NaOtBu, 237 mL of toluene, 7.48 g (1.1 eq, 52.2 mmol) of naphthalene-1-amine, and 0.96 g (0.1 eq, 4.0 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-30 (12.80 g, yield 75%).

By FAB-MS measurement, mass number m/z = 359 was observed as the molecular ion peak, and Intermediate IM-30 was identified.

### (Synthesis of Compound D162)

Under an Ar atmosphere, to a 300 mL, three neck flask, 10.00 g (27.8 mmol) of IM-30, 0.48 g (0.03 eq, 0.8 mmol) of Pd(dba)₂, 5.35 g (2.0 eq, 55.6 mmol) of NaOtBu, 139 mL of toluene, 15.04 g (1.1 eq, 30.6 mmol) of [4'-bromo-(1,1'-biphenyl)-4-yl]triphenylsilane, and 0.56 g (0.1 eq, 2.8 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound D162 (15.64 g, yield 73%) as a solid.

By FAB-MS measurement, mass number m/z = 770 was observed as the molecular ion peak, and Compound D162 was identified.

### 11. Synthesis of Compound E87

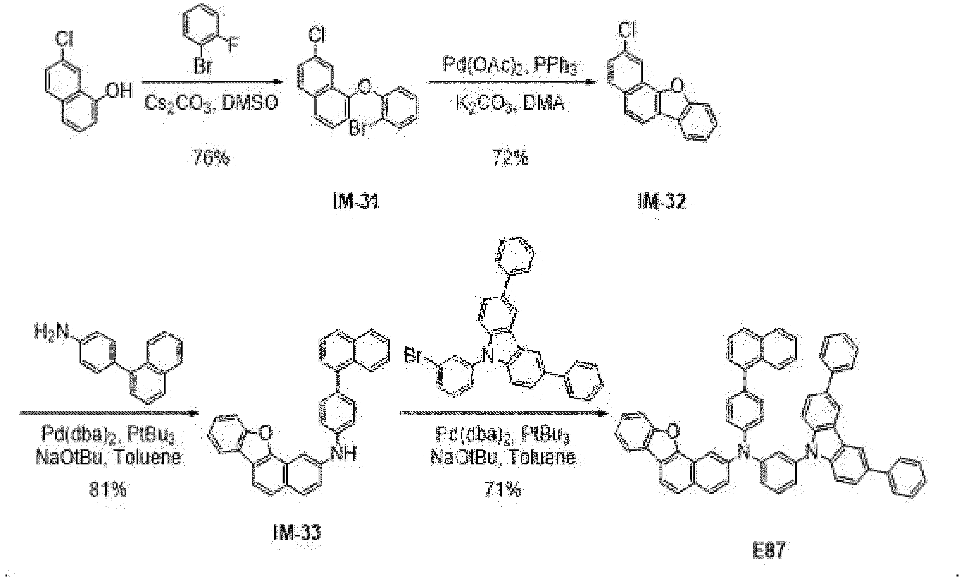

### (Synthesis of Intermediate IM-31)

Under an Ar atmosphere, to a 500 mL, three neck flask, 25.00 g (140.0 mmol) of 7-chloronaphthalen-1-ol, 29.39 g (1.2 eq, 168.0 mmol) of 1-bromo-2-fluorobenzene, 91.20 g (2.0 eq, 280.0 mmol) of Cs₂CO₃, and 280 mL of DMSO were added in order, followed by heating and stirring at about 110 °C. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-31 (35.49 g, yield 76%).

By FAB-MS measurement, mass number m/z = 333 was observed as the molecular ion peak, and Intermediate IM-31 was identified.

### (Synthesis of Intermediate IM-32)

Under an Ar atmosphere, to a 500 mL, three neck flask, 25.00 g (74.9 mmol) of IM-31, 0.84 g (0.05 eq, 3.7 mmol) of Pd(OAc)₂, 15.54 g (1.5 eq, 112.4 mmol) of K₂CO₃, 1.97 g (0.10 eq, 7.5 mmol) of PPh₃, and 300 mL of DMA were added in order, followed by heating to about 140 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-32 (13.63 g, yield 72%).

By FAB-MS measurement, mass number m/z = 252 was observed as the molecular ion peak, and Intermediate IM-32 was identified.

### (Synthesis of Intermediate IM-33)

Under an Ar atmosphere, to a 500 mL, three neck flask, 12.00 g (47.5 mmol) of IM-32, 0.82 g (0.03 eq, 1.4 mmol) of Pd(dba)₂, 4.57 g (1.0 eq, 47.5 mmol) of NaOtBu, 237 mL of toluene, 11.45 g (1.1 eq, 52.2 mmol) of 4-(naphthalen-1-yl)aniline, and 0.96 g (0.1 eq, 4.0 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-33 (16.75 g, yield 81%).

By FAB-MS measurement, mass number m/z = 435 was observed as the molecular ion peak, and Intermediate IM-33 was identified.

### (Synthesis of Compound E87)

Under an Ar atmosphere, to a 300 mL, three neck flask, 10.00 g (23.0 mmol) of IM-33, 0.40 g (0.03 eq, 0.7 mmol) of Pd(dba)₂, 4.41 g (2.0 eq, 45.9 mmol) of NaOtBu, 115 mL of toluene, 11.98 g (1.1 eq, 25.3 mmol) of 9-(3-bromotetraphenyl)-3,6-diphenyl-9H-carbazole, and 0.46 g (0.1 eq, 2.3 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound E87 (13.51 g, yield 71%) as a solid.

By FAB-MS measurement, mass number m/z = 829 was observed as the molecular ion peak, and Compound E87 was identified.

### 12. Synthesis of Compound E190

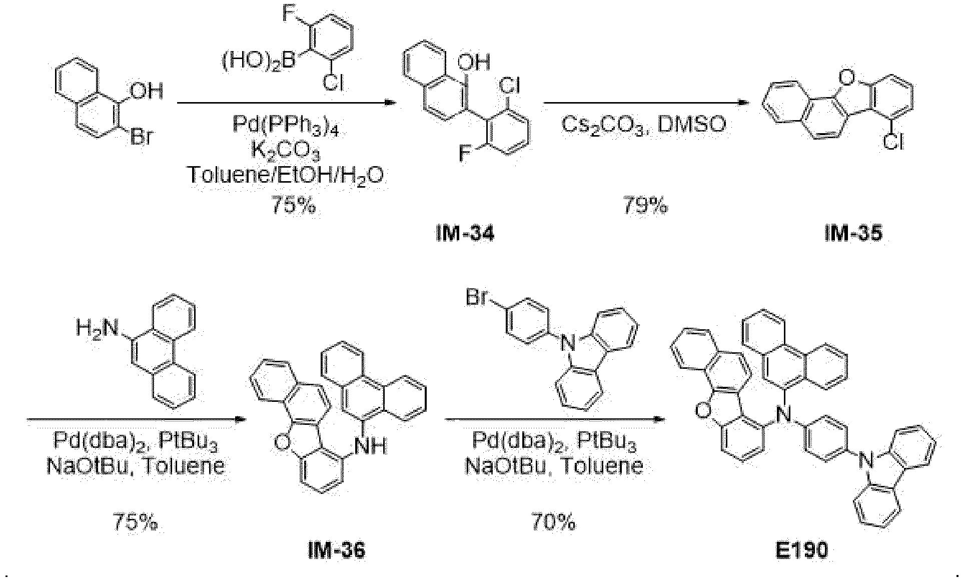

### (Synthesis of Intermediate IM-34)

Under an Ar atmosphere, to a 2,000 mL, three neck flask, 30.00 g (134.5 mmol) of 2-bromo-1-naphthol, 25.79 g (1.1 eq, 147.9 mmol) of (2-chloro-6-fluorophenyl)boronic acid, 55.76 g (3.0 eq, 403.5 mmol) of K₂CO₃, 7.77 g (0.05 eq, 6.7 mmol) of Pd(PPh₃)₄, and 941 mL of a mixture solution of toluene/EtOH/H₂O (4/2/1) were added in order, followed by heating and stirring at about 80 °C. After cooling to room temperature, the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-34 (27.51 g, yield 75%).

By FAB-MS measurement, mass number m/z = 272 was observed as the molecular ion peak, and Intermediate IM-34 was identified.

### (Synthesis of Intermediate IM-35)

Under an Ar atmosphere, to a 300 mL, three neck flask, 25.00 g (91.7 mmol) of IM-34, 59.74 g (2.0 eq, 183.4 mmol) of Cs₂CO₃, and 184 mL of DMSO were added in order, followed by heating to about 110 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-35 (18.30 g, yield 79%).

By FAB-MS measurement, mass number m/z = 252 was observed as the molecular ion peak, and Intermediate IM-35 was identified.

### (Synthesis of Intermediate IM-36)

Under an Ar atmosphere, to a 500 mL, three neck flask, 12.00 g (47.5 mmol) of IM-35, 0.82 g (0.03 eq, 1.4 mmol) of Pd(dba)₂, 4.57 g (1.0 eq, 47.5 mmol) of NaOtBu, 237 mL of toluene, 10.09 g (1.1 eq, 52.2 mmol) of 9-aminophenanthrene, and 0.96 g (0.1 eq, 4.0 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-36 (14.58 g, yield 75%).

By FAB-MS measurement, mass number m/z = 650 was observed as the molecular ion peak, and Intermediate IM-36 was identified.

### (Synthesis of Compound E190)

Under an Ar atmosphere, to a 300 mL, three neck flask, 10.00 g (24.4 mmol) of IM-36, 0.42 g (0.03 eq, 0.7 mmol) of Pd(dba)₂, 4.69 g (2.0 eq, 48.8 mmol) of NaOtBu, 122 mL of toluene, 8.66 g (1.1 eq, 26.9 mmol) of 9-(4-bromophenyl)-9H-carbazole, and 0.49 g (0.1 eq, 2.4 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound E190 (11.12 g, yield 70%) as a solid.

By FAB-MS measurement, mass number m/z = 650 was observed as the molecular ion peak, and Compound E190 was identified.

### 13. Synthesis of Compound F15

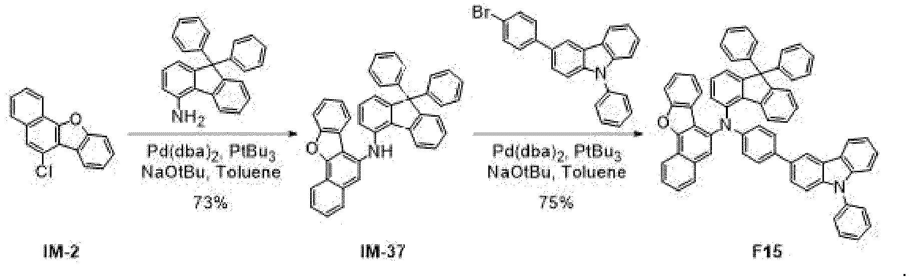

### (Synthesis of Intermediate IM-37)

Under an Ar atmosphere, to a 500 mL, three neck flask, 12.00 g (47.5 mmol) of IM-2, 0.82 g (0.03 eq, 1.4 mmol) of Pd(dba)₂, 4.57 g (1.0 eq, 47.5 mmol) of NaOtBu, 237 mL of toluene, 17.42 g (1.1 eq, 52.2 mmol) of 9,9-diphenyl-9H-fluoren-4-amine, and 0.96 g (0.1 eq, 4.0 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-37 (19.05 g, yield 73%).

By FAB-MS measurement, mass number m/z = 549 was observed as the molecular ion peak, and Intermediate IM-37 was identified.

### (Synthesis of Compound F15)

Under an Ar atmosphere, to a 300 mL, three neck flask, 10.00 g (18.2 mmol) of IM-37, 0.31 g (0.03 eq, 0.5 mmol) of Pd(dba)₂, 3.50 g (2.0 eq, 36.4 mmol) of NaOtBu, 91 mL of toluene, 7.97 g (1.1 eq, 20.0 mmol) of 3-(4-bromophenyl)-9-phenyl-9H-carbazole, and 0.37 g (0.1 eq, 1.8 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound F15 (11.83 g, yield 75%) as a solid.

By FAB-MS measurement, mass number m/z = 867 was observed as the molecular ion peak, and Compound F15 was identified.

### 14. Synthesis of Compound F146

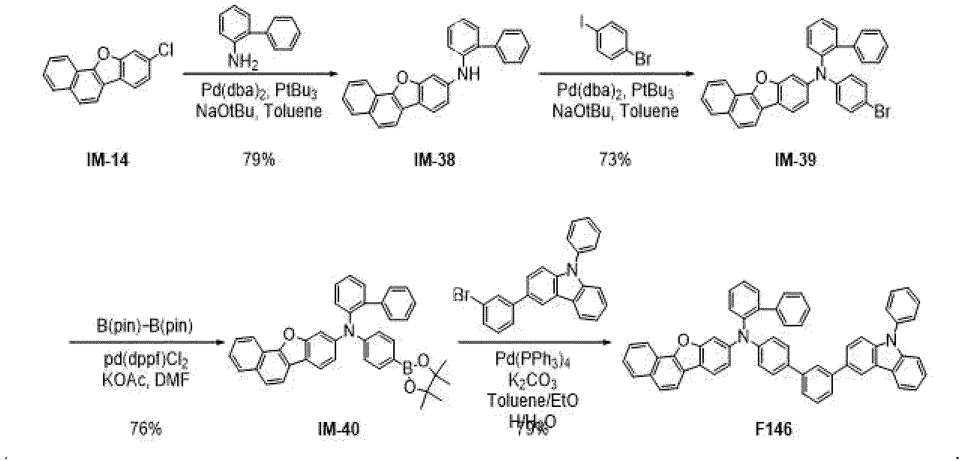

### (Synthesis of Intermediate IM-38)

Under an Ar atmosphere, to a 500 mL, three neck flask, 12.00 g (47.5 mmol) of IM-14, 0.82 g (0.03 eq, 1.4 mmol) of Pd(dba)₂, 4.57 g (1.0 eq, 47.5 mmol) of NaOtBu, 237 mL of toluene, 8.84 g (1.1 eq, 52.2 mmol) of [1,1'-biphenyl]-2-amine, and 0.96 g (0.1 eq, 4.0 mmol) of tBu₃P were added in order, followed by heating, refluxing, and stirring. After cooling to room temperature, water was added to the reaction solution, and an organic layer was separated. Toluene was added to an aqueous layer, and further organic layers were extracted. The organic layers were collected, washed with a saturated saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-38 (14.46 g, yield 79%).

By FAB-MS measurement, mass number m/z = 385 was observed as the molecular ion peak, and Intermediate IM-38 was identified.

### (Synthesis of Intermediate IM-39)

Under an Ar atmosphere, to a 500 mL, three neck flask, 14.00 g (36.3 mmol) of IM-38, 0.63 g (0.03 eq, 1.1 mmol) of Pd(dba)₂, 6.98 g (1.0 eq, 72.6 mmol) of NaOtBu, 182 mL of toluene, 11.30 g (1.1 eq, 40.0 mmol) of 1-bromo-4-iodobenzene, and 0.73 g (0.1 eq, 3.6 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-39 (14.33 g, yield 73%).

By FAB-MS measurement, mass number m/z = 540 was observed as the molecular ion peak, and Intermediate IM-39 was identified.

### (Synthesis of Intermediate IM-40)

Under an Ar atmosphere, to a 300 mL, three neck flask, 13.00 g (24.1 mmol) of IM-39, 1.96 g (0.10 eq, 2.4 mmol) of Pd(dppf)Cl₂, 4.72 g (2.0 eq, 48.1 mmol) of KOAc, 120 mL of DMF, and 7.33 g (1.2 eq, 28.9 mmol) of bis(pinacolato)diboron were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-40 (10.74 g, yield 76%).

By FAB-MS measurement, mass number m/z = 587 was observed as the molecular ion peak, and Intermediate IM-40 was identified.

### (Synthesis of Compound F146)

Under an Ar atmosphere, to a 300 mL, three neck flask, 10.00 g (17.0 mmol) of IM-40, 7.46 g (1.1 eq, 18.7 mmol) of 3-(3-bromophenyl)-9-phenyl-9H-carbazole, 7.05 g (3.0 eq, 51.1 mmol) of K₂CO₃, 0.98 g (0.05 eq, 0.9 mmol) of Pd(PPh₃)₄, and 119 mL of a mixture solution of toluene/EtOH/H₂O (4/2/1) were added in order, followed by heating to about 80 °C and stirring. After cooling to room temperature, the reaction solution was extracted with toluene. An aqueous layer was removed, and an organic layer was washed with a saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound F146 (10.47 g, yield 79%) as a solid.

By FAB-MS measurement, mass number m/z = 778 was observed as the molecular ion peak, and Compound F146 was identified.

### 15. Synthesis of Compound F198

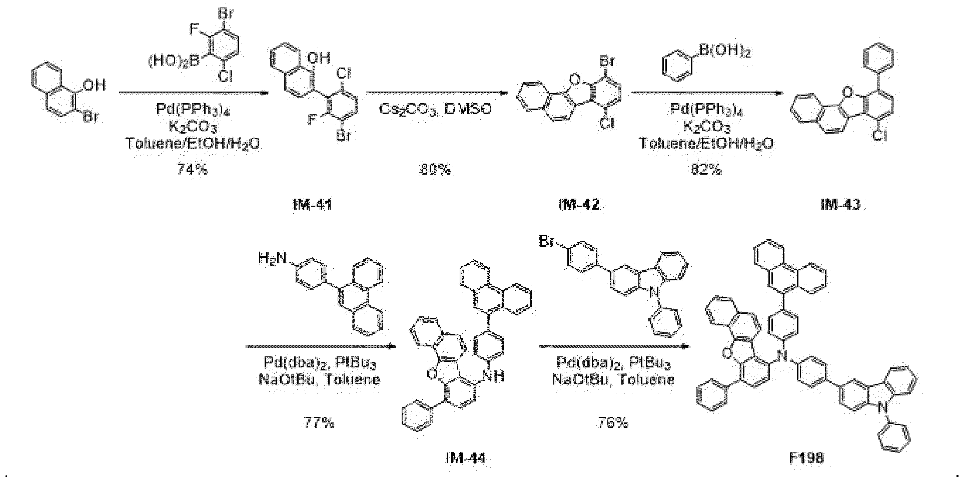

### (Synthesis of Intermediate IM-41)

Under an Ar atmosphere, to a 2,000 mL, three neck flask, 30.00 g (134.5 mmol) of 2-bromo-1-naphthol, 37.47 g (1.1 eq, 147.9 mmol) of (3-bromo-6-chloro-2-fluorophenyl)boronic acid, 55.76 g (3.0 eq, 403.5 mmol) of K₂CO₃, 7.77 g (0.05 eq, 6.7 mmol) of Pd(PPh₃)₄, and 941 mL of a mixture solution of toluene/EtOH/H₂O (4/2/1) were added in order, followed by heating and stirring at about 80 °C. After cooling to room temperature, the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-41 (35.00 g, yield 74%).

By FAB-MS measurement, mass number m/z = 351 was observed as the molecular ion peak, and Intermediate IM-41 was identified.

### (Synthesis of Intermediate IM-42)

Under an Ar atmosphere, to a 300 mL, three neck flask, 30.00 g (85.3 mmol) of IM-41, 55.60 g (2.0 eq, 170.6 mmol) of Cs₂CO₃, and 170 mL of DMSO were added in order, followed by heating to about 110 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-42 (22.63 g, yield 80%).

By FAB-MS measurement, mass number m/z = 331 was observed as the molecular ion peak, and Intermediate IM-42 was identified.

### (Synthesis of Intermediate IM-43)

Under an Ar atmosphere, to a 1,000 mL, three neck flask, 20.00 g (60.3 mmol) of IM-42, 8.09 g (1.1 eq, 66.3 mmol) of phenylboronic acid, 25.01 g (3.0 eq, 180.9 mmol) of K₂CO₃, 3.48 g (0.05 eq, 3.0 mmol) of Pd(PPh₃)₄, and 422 mL of a mixture solution of toluene/EtOH/H₂O (4/2/1) were added in order, followed by heating and stirring at about 80 °C. After cooling to room temperature, the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-43 (16.26 g, yield 82%).

By FAB-MS measurement, mass number m/z = 328 was observed as the molecular ion peak, and Intermediate IM-43 was identified.

### (Synthesis of Intermediate IM-44)

Under an Ar atmosphere, to a 500 mL, three neck flask, 12.00 g (36.5 mmol) of IM-43, 0.63 g (0.03 eq, 1.1 mmol) of Pd(dba)₂, 3.51 g (1.0 eq, 36.5 mmol) of NaOtBu, 182 mL of toluene, 10.81 g (1.1 eq, 40.1 mmol) of 4-(phenanthren-9-yl)aniline, and 0.74 g (0.1 eq, 3.6 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-44 (15.78 g, yield 77%).

By FAB-MS measurement, mass number m/z = 561 was observed as the molecular ion peak, and Intermediate IM-44 was identified.

### (Synthesis of Compound F198)

Under an Ar atmosphere, to a 300 mL, three neck flask, 10.00 g (17.8 mmol) of IM-44, 0.31 g (0.03 eq, 0.5 mmol) of Pd(dba)₂, 3.42 g (2.0 eq, 35.6 mmol) of NaOtBu, 89 mL of toluene, 7.80 g (1.1 eq, 19.6 mmol) of 3-(4-bromophenyl)-9-phenyl-9H-carbazole, and 0.36 g (0.1 eq, 1.8 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound F198 (11.89 g, yield 76%) as a solid.

By FAB-MS measurement, mass number m/z = 879 was observed as the molecular ion peak, and Compound F198 was identified.

### 16. Synthesis of Compound A127

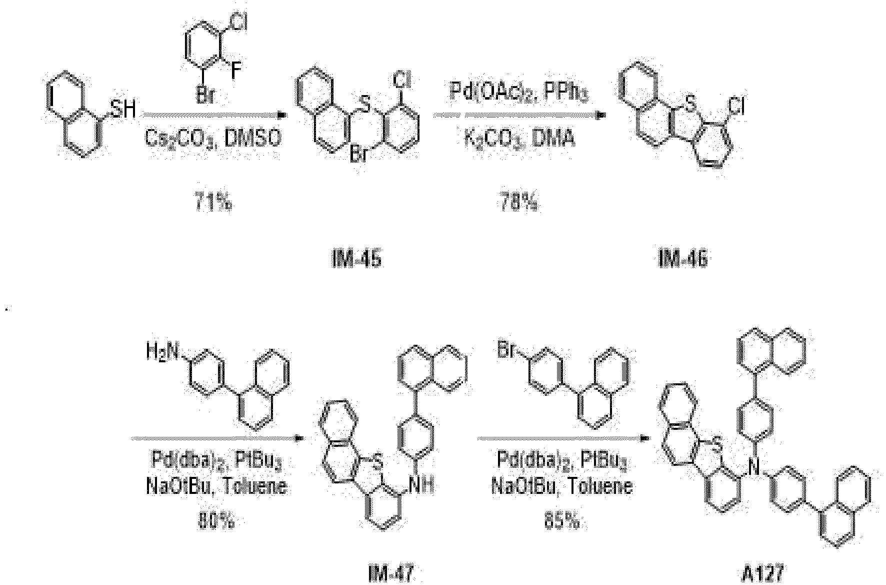

### (Synthesis of Intermediate IM-45)

Under an Ar atmosphere, to a 500 mL, three neck flask, 25.00 g (156.0 mmol) of naphthalene-1-thiol, 39.21 g (1.2 eq, 187.2 mmol) of 1-bromo-3-chloro-2-fluorobenzene, 101.67 g (2.0 eq, 312.1 mmol) of Cs₂CO₃, and 312 mL of DMSO were added in order, followed by heating and stirring at about 110 °C. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-45 (38.74 g, yield 71%).

By FAB-MS measurement, mass number m/z = 349 was observed as the molecular ion peak, and Intermediate IM-45 was identified.

### (Synthesis of Intermediate IM-46)

Under an Ar atmosphere, to a 500 mL, three neck flask, 25.00 g (71.5 mmol) of IM-45, 0.80 g (0.05 eq, 3.6 mmol) of Pd(OAc)₂, 14.52 g (1.5 eq, 107.2 mmol) of K₂CO₃, 1.88 g (0.10 eq, 7.1 mmol) of PPh₃, and 286 mL of DMA were added in order, followed by heating to about 140 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-46 (14.99 g, yield 71%).

By FAB-MS measurement, mass number m/z = 268 was observed as the molecular ion peak, and Intermediate IM-46 was identified.

### (Synthesis of Intermediate IM-47)

Under an Ar atmosphere, to a 500 mL, three neck flask, 12.00 g (44.7 mmol) of IM-46, 0.77 g (0.03 eq, 1.3 mmol) of Pd(dba)₂, 4.29 g (2.0 eq, 44.7 mmol) of NaOtBu, 224 mL of toluene, 10.77 g (1.1 eq, 49.1 mmol) of 4-(naphthalen-1-yl)aniline, and 0.90 g (0.1 eq, 4.5 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-47 (16.13 g, yield 80%).

By FAB-MS measurement, mass number m/z = 451 was observed as the molecular ion peak, and Intermediate IM-47 was identified.

### (Synthesis of Compound A127)

Under an Ar atmosphere, to a 300 mL, three neck flask, 10.00 g (22.1 mmol) of IM-47, 0.38 g (0.03 eq, 0.7 mmol) of Pd(dba)₂, 4.26 g (2.0 eq, 44.3 mmol) of NaOtBu, 111 mL of toluene, 6.90 g (1.1 eq, 24.4 mmol) of 1-(4-bromophenyl)naphthalene, and 0.45 g (0.1 eq, 2.2 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound A127 (12.31 g, yield 85%) as a solid.

By FAB-MS measurement, mass number m/z = 653 was observed as the molecular ion peak, and Compound A127 was identified.

### 17. Synthesis of Compound B138

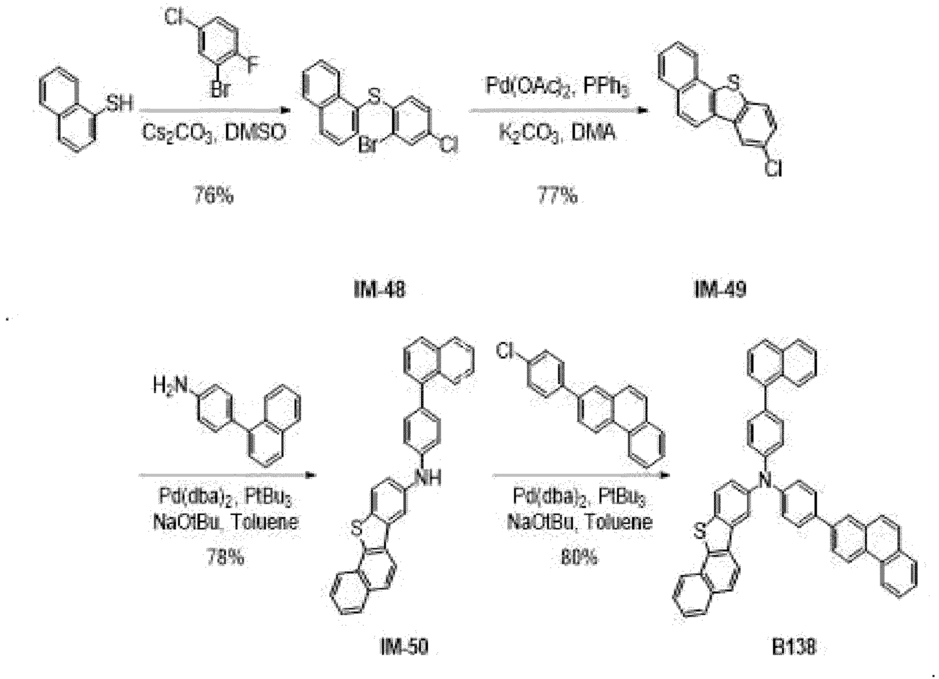

### (Synthesis of Intermediate IM-48)

Under an Ar atmosphere, to a 500 mL, three neck flask, 25.00 g (156.0 mmol) of naphthalen-1-thiol, 39.21 g (1.2 eq, 187.2 mmol) of 1-bromo-3-chlorofluorobenzene, 101.67 g (2.0 eq, 312.1 mmol) of Cs₂CO₃, and 312 mL of DMSO were added in order, followed by heating and stirring at about 110 °C. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-48 (41.46 g, yield 76%).

By FAB-MS measurement, mass number m/z = 349 was observed as the molecular ion peak, and Intermediate IM-48 was identified.

### (Synthesis of Intermediate IM-49)

Under an Ar atmosphere, to a 500 mL, three neck flask, 25.00 g (71.5 mmol) of IM-48, 0.80 g (0.05 eq, 3.6 mmol) of Pd(OAc)₂, 14.52 g (1.5 eq, 107.2 mmol) of K₂CO₃, 1.88 g (0.10 eq, 7.1 mmol) of PPh₃, and 286 mL of DMA were added in order, followed by heating to about 140 °C and stirring. After cooling to room temperature, water was added to the reaction solution, and the reaction solution was extracted with toluene. An aqueous layer was removed, and an organic layer was washed with a saturated saline solution and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-49 (12.68 g, yield 77%).

By FAB-MS measurement, mass number m/z = 268 was observed as the molecular ion peak, and Intermediate IM-49 was identified.

### (Synthesis of Intermediate IM-50)

Under an Ar atmosphere, to a 500 mL, three neck flask, 12.00 g (44.7 mmol) of IM-49, 0.77 g (0.03 eq, 1.3 mmol) of Pd(dba)₂, 4.29 g (2.0 eq, 44.7 mmol) of NaOtBu, 224 mL of toluene, 10.77 g (1.1 eq, 49.1 mmol) of 4-(naphthalen-1-yl)aniline, and 0.90 g (0.1 eq, 4.5 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Intermediate IM-50 (16.08 g, yield 78%).

By FAB-MS measurement, mass number m/z = 451 was observed as the molecular ion peak, and Intermediate IM-50 was identified.

### (Synthesis of Compound B138)

Under an Ar atmosphere, to a 300 mL, three neck flask, 10.00 g (22.1 mmol) of IM-50, 0.38 g (0.03 eq, 0.7 mmol) of Pd(dba)₂, 4.26 g (2.0 eq, 44.3 mmol) of NaOtBu, 111 mL of toluene, 6.88 g (1.1 eq, 24.4 mmol) of 2-(4-chlorophenyl)phenanthrene, and 0.45 g (0.1 eq, 2.2 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound B138 (12.20 g, yield 80%) as a solid.

By FAB-MS measurement, mass number m/z = 703 was observed as the molecular ion peak, and Compound B138 was identified.

### 18. Synthesis of Compound A178

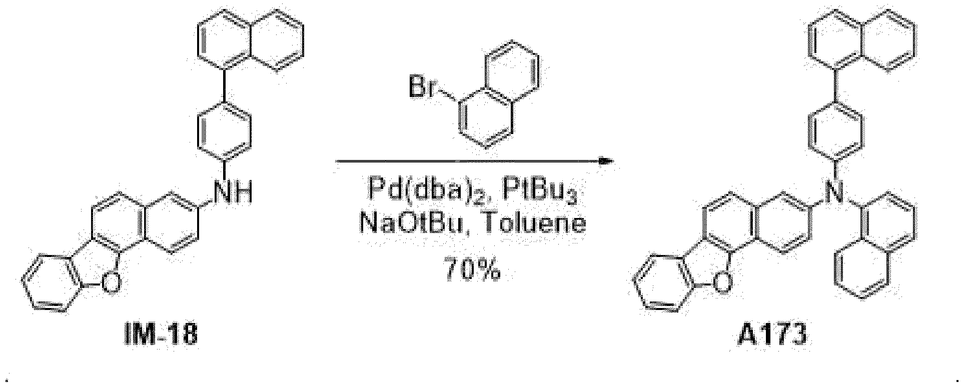

Under an Ar atmosphere, to a 300 mL, three neck flask, 10.00 g (23.0 mmol) of IM-18, 0.40 g (0.03 eq, 0.7 mmol) of Pd(dba)₂, 4.41 g (2.0 eq, 45.9 mmol) of NaOtBu, 115 mL of toluene, 5.23 g (1.1 eq, 25.3 mmol) of 1-bromonaphthalene, and 0.46 g (0.1 eq, 2.3 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound A173 (9.03 g, yield 70%) as a solid.

By FAB-MS measurement, mass number m/z = 561 was observed as the molecular ion peak, and Compound A173 was identified.

### 19. Synthesis of Compound A195

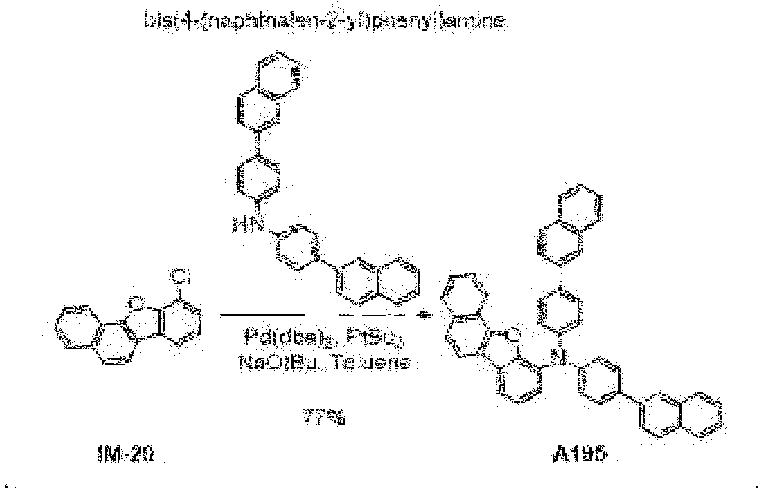

Under an Ar atmosphere, to a 300 mL, three neck flask, 10.00 g (39.6 mmol) of IM-20, 0.68 g (0.03 eq, 1.2 mmol) of Pd(dba)₂, 7.61 g (2.0 eq, 79.1 mmol) of NaOtBu, 198 mL of toluene, 18.35 g (1.1 eq, 43.5 mmol) of bis[4-(naphthalen-2-yl)phenyl]amine, and 0.80 g (0.1 eq, 4.0 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound A195 (19.43 g, yield 77%) as a solid.

By FAB-MS measurement, mass number m/z = 637 was observed as the molecular ion peak, and Compound A195 was identified.

### 20. Synthesis of Compound B189

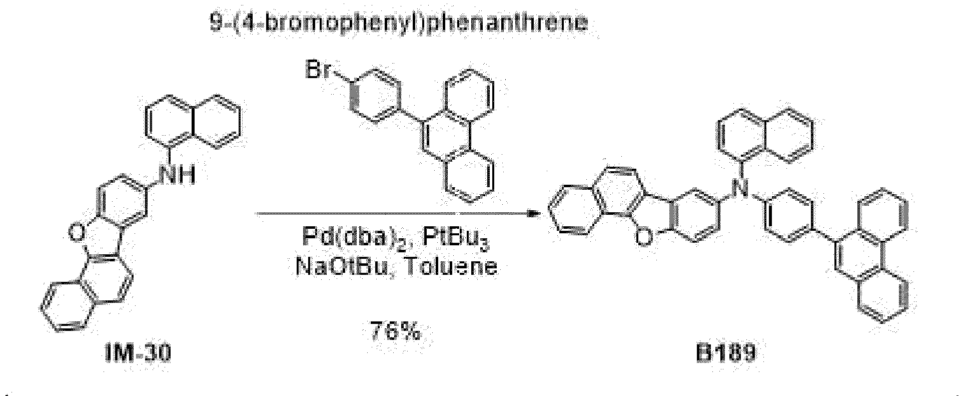

Under an Ar atmosphere, to a 300 mL, three neck flask, 10.00 g (27.8 mmol) of IM-30, 0.48 g (0.03 eq, 0.8 mmol) of Pd(dba)₂, 5.35 g (2.0 eq, 55.6 mmol) of NaOtBu, 139 mL of toluene, 10.20 g (1.1 eq, 30.6 mmol) of 9-(4-bromophenyl)phenanthrene, and 0.56 g (0.1 eq, 2.8 mmol) of tBu₃P were added in order, followed by heating, refluxing, and stirring. After cooling to room temperature, water was added to the reaction solution, and an organic layer was separated. Toluene was added to the aqueous layer, and further organic layers were extracted. The organic layers were collected, washed with a saline solution, and dried with MgSO₄. MgSO₄ was separated by filtering, an organic layer was concentrated, and the crude product thus obtained was separated by silica gel column chromatography (a mixture solvent of hexane and toluene was used as an eluent) to obtain Compound B189 (12.93 g, yield 76%) as a solid.

By FAB-MS measurement, mass number m/z = 611 was observed as the molecular ion peak, and Compound B189 was identified.

### (Device manufacturing examples)

Organic electroluminescence devices were manufactured using Example Compounds, Reference Compounds and Comparative Compounds as materials for a hole transport region:

### [Example Compounds]

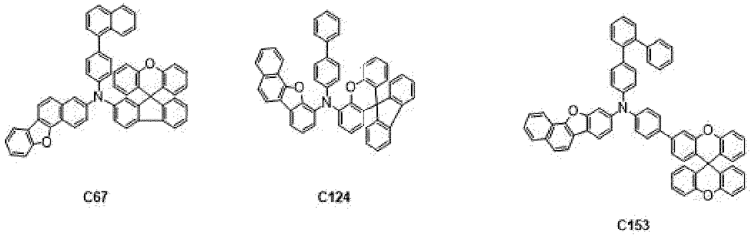

### [Reference Compounds]

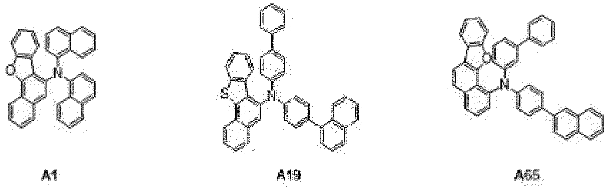

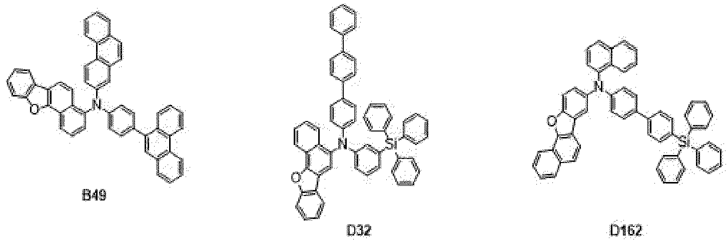

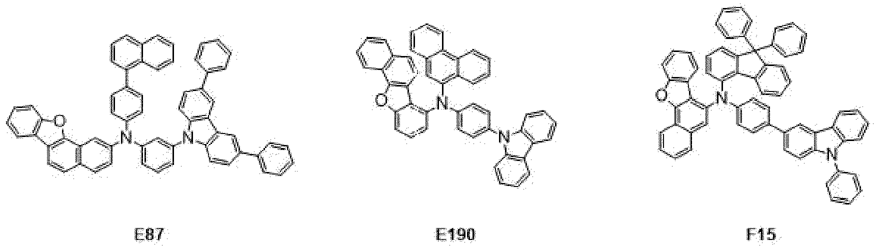

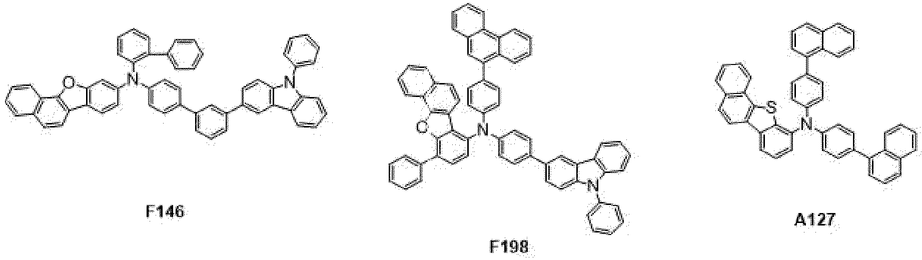

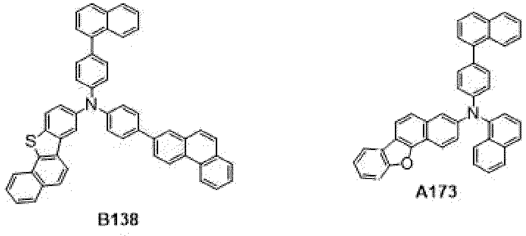

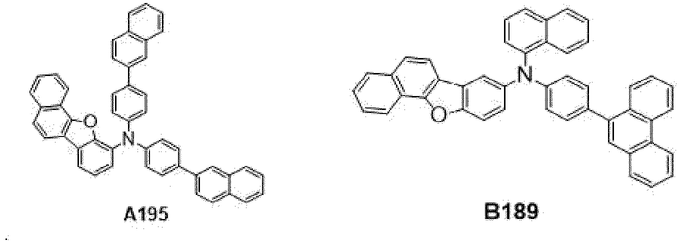

### [Comparative Compounds]

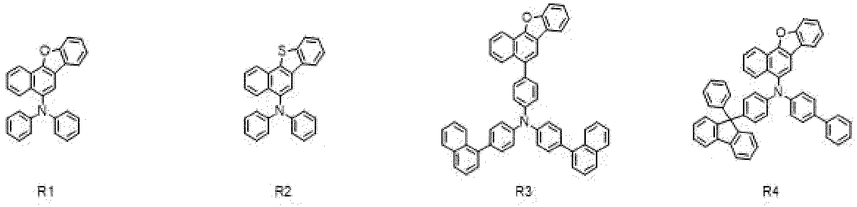

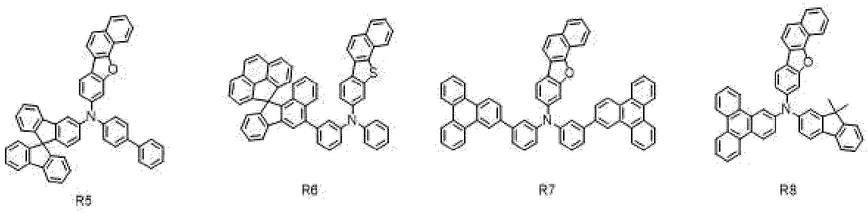

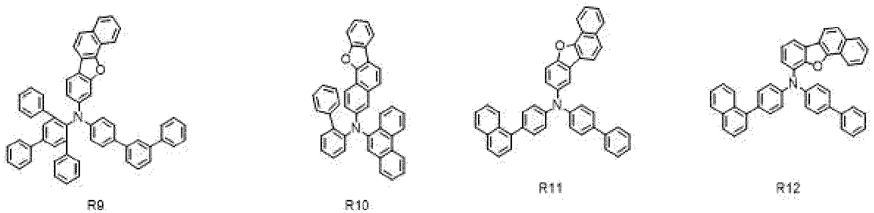

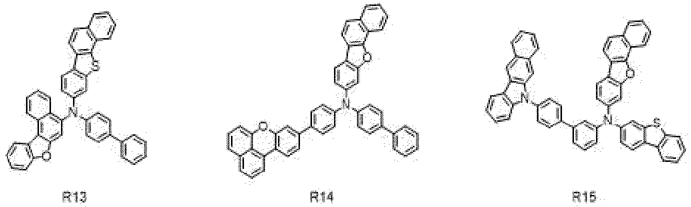

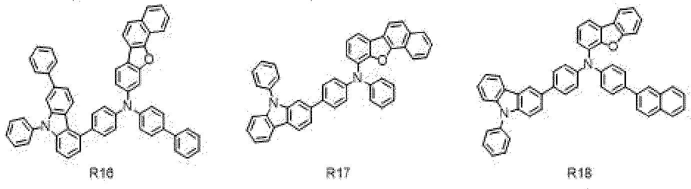

Each of the organic electroluminescence devices of the Examples and Comparative Examples was manufactured using the method below. ITO with a thickness of about 150 nm was patterned on a glass substrate, washed with ultra-pure water, and treated with UV-ozone for about 10 minutes to form a first electrode. Then, 1-TNATA was deposited to a thickness of about 60 nm, and a hole transport layer with a thickness of about 30 nm was formed using each of the Example Compounds or the Comparative Compounds. Then, an emission layer with a thickness of about 25 nm was formed using ADN doped with 3% TBP, and on the emission layer, a layer with a thickness of about 25 nm was formed using Alq₃, and a layer with a thickness of about 1 nm was formed using LiF to form an electron transport region. After that, a second electrode with a thickness of about 100 nm was formed using aluminum (Al). Then, Compound P4 was deposited to a thickness of about 70 nm to form a capping layer. Each layer was formed by a vacuum deposition method.

The emission efficiencies of the organic electroluminescence devices according to Examples 1 to 20 and Comparative Examples 1 to 18 are shown in Table 1. The efficiency was measured at a current density of about 10 mA/cm², and the half life LT₅₀ is the time elapsed for the luminance to fall from an initial value of about 1,000 cd/m² to half.

**[Table 1]**

| | Hole transport layer | Voltage (V) | Efficiency (cd/A) | Life (LT₅₀ (h)) |
|---|---|---|---|---|
| Example 1 (Reference) | Reference Compound A1 | 5.6 | 7.7 | 2100 |
| Example 2 (Reference) | Reference Compound A19 | 5.5 | 7.6 | 2150 |
| Example 3 (Reference) | Reference Compound A65 | 5.5 | 7.5 | 2150 |
| Example 4 (Reference) | Reference Compound B49 | 5.5 | 7.6 | 2100 |
| Example 5 (Reference) | Reference Compound B94 | 5.4 | 7.7 | 2050 |
| Example 6 | Example Compound C67 | 5.5 | 7.6 | 2200 |
| Example 7 | Example Compound C124 | 5.6 | 7.8 | 2000 |
| Example 8 | Example Compound C153 | 5.5 | 7.8 | 2050 |
| Example 9 (Reference) | Reference Compound D32 | 5.6 | 7.5 | 2100 |
| Example 10 (Reference) | Reference Compound D162 | 5.6 | 7.8 | 2050 |
| Example 11 (Reference) | Reference Compound E87 | 5.4 | 7.6 | 2100 |
| Example 12 | Reference | 5.4 | 7.9 | 1950 |
| (Reference) | Compound E190 | | | |
| Example 13 (Reference) | Reference Compound F15 | 5.4 | 7.7 | 2150 |
| Example 14 (Reference) | Reference Compound F146 | 5.5 | 7.9 | 2000 |
| Example 15 (Reference) | Reference Compound F198 | 5.5 | 7.8 | 2050 |
| Example 16 (Reference) | Reference Compound A127 | 5.4 | 7.9 | 2000 |
| Example 17 (Reference) | Reference Compound B138 | 5.5 | 7.8 | 2100 |
| Example 18 (Reference) | Reference Compound A173 | 5.5 | 7.5 | 2150 |
| Example 19 (Reference) | Reference Compound A195 | 5.6 | 7.8 | 2100 |
| Example 20 (Reference) | Reference Compound B189 | 5.5 | 7.6 | 2050 |
| Comparative Example 1 | Comparative Compound R1 | 6.4 | 5.7 | 1500 |
| Comparative Example 2 | Comparative Compound R2 | 6.5 | 5.8 | 1450 |
| Comparative Example 3 | Comparative Compound R3 | 6.3 | 6.0 | 1650 |
| Comparative Example 4 | Comparative Compound R4 | 6.3 | 6.2 | 1550 |
| Comparative Example 5 | Comparative Compound R5 | 6.5 | 6.4 | 1600 |
| Comparative Example 6 | Comparative Compound R6 | 6.4 | 6.0 | 1550 |
| Comparative Example 7 | Comparative Compound R7 | 6.5 | 6.2 | 1550 |
| Comparative Example 8 | Comparative Compound R8 | 6.3 | 6.1 | 1450 |
| Comparative Example 9 | Comparative Compound R9 | 6.5 | 6.4 | 1500 |
| Comparative Example 10 | Comparative Compound R10 | 6.5 | 6.6 | 1550 |
| Comparative Example 11 | Comparative Compound R11 | 6.5 | 6.7 | 1700 |
| Comparative Example 12 | Comparative Compound R12 | 6.5 | 6.8 | 1650 |
| Comparative Example 13 | Comparative Compound R13 | 6.5 | 6.5 | 1650 |
| Comparative Example 14 | Comparative Compound R14 | 6.5 | 6.1 | 1550 |
| Comparative Example 15 | Comparative Compound R15 | 6.5 | 6.6 | 1650 |
| Comparative Example 16 | Comparative Compound R16 | 6.5 | 6.5 | 1650 |
| Comparative Example 17 | Comparative Compound R17 | 6.5 | 6.8 | 1600 |
| Comparative Example 18 | Comparative Compound R18 | 6.5 | 6.8 | 1700 |

Referring to Table 1, it could be confirmed that each of Examples 1 to 20 exhibited a lower voltage, a longer life and a higher efficiency than each of Comparative Examples 1 to 18.

The polycyclic compound according to the present disclosure has a core structure comprising benzonaphthofuran or benzonaphthothiophene combined with an amine group having a particular substituent, and without being bound by the correctness of any theory or explanation, this structure supports a decreased driving voltage, and increased lifespan and efficiency in an organic electroluminescence device. It is thought that the heteroatom (e.g., O or S) comprised in the core structure of benzonaphthofuran or benzonaphthothiophene improves hole transport capacity, such that the recombination probability of holes and electrons in an emission layer is improved, and the emission efficiency is improved.

Examples 1 to 4, 6, 9, 11, 13 and 18 each include a compound in which the amine group is bound to the naphthalene ring in the core structure of benzonaphthofuran or benzonaphthothiophene, and exhibit improved device life. Without being bound by the correctness of any theory or explanation, it is believed that because π electrons around the amine were widely expanded in the naphthalene ring, stability of radical states was enhanced.

Examples 5, 7, 8, 10, 12, 14 to 17, 19 and 20 each include a compound in which the amine group is bound to the benzene ring in the core structure of benzonaphthofuran or benzonaphthothiophene, and exhibit improved emission efficiency. Without being bound by the correctness of any theory or explanation, it is believed that because the heteroatom included in the benzonaphthofuran or benzonaphthothiophene skeleton and the nitrogen atom of an amine group are substituted on the same ring, hole transport effects due to the heteroatom were enhanced.

Comparative Examples 1 and 2 each showed reduced emission efficiency and lifespan when compared with the Examples. Comparative Examples 1 and 2 are compounds in which an amine group is combined with the core structure of benzonaphthofuran or benzonaphthothiophene, but an unsubstituted phenyl group is combined with a nitrogen atom (e.g., the amine group includes only unsubstituted phenyl groups). Without being bound by the correctness of any theory or explanation, it is believed that the glass transition temperature (Tg) of the material was insufficient such that the charge tolerance of the unsubstituted phenyl group was low, and the materials were deteriorated during continuous driving.

Comparative Example 3 showed reduced device efficiency and life when compared with the Examples. Comparative Example 3 is a compound in which benzonaphthofuran and an amine group are combined via a linker (e.g., an intervening phenyl ring), such that the planarity of a molecule was increased, and decomposition occurred during deposition for forming a hole transport layer.

Comparative Example 4 showed reduced device efficiency and life when compared with the Examples. In the compound of Comparative Example 4, a phenyl substituent on the amine is substituted with a fluorenyl group, and without being bound by the correctness of any theory or explanation, it is believed that instability of the sp³ carbon in the fluorenyl group resulted in decomposition under a radical state and high temperature conditions.

In contrast, the polycyclic compounds of Examples 6 to 8 comprising a spirocyclic structure with a heteroatom showed improved stability, and provided excellent device properties. In Example 5, because a nitrogen atom was combined on (e.g., directly bonded to) a fluorene ring, the stability of a material was improved due to multi-resonance effects. Accordingly, improved emission efficiency and life were exhibited compared with Comparative Example 4.

Comparative Examples 5 and 6 showed particularly reduced emission efficiencies when compared with Examples 6 to 8. Without being bound by the correctness of any theory or explanation, it is believed that because a heteroatom was not included in the spirocycle of the compounds of Comparative Examples 5 and 6, hole transport properties were degraded, and the recombination probability of holes and electrons in an emission layer was decreased.

Comparative Examples 7 and 8 are amine compounds including a triphenylene substituent, and without being bound by the correctness of any theory or explanation, it is believed that because the planarity of the molecule was increased, the molecule was decomposed during hole transport layer formation, and device efficiency and lifespan were both (e.g., simultaneously) degraded.

Comparative Example 9 is an amine compound including a (2,4,6-triphenyl)phenyl group. Without being bound by the correctness of any theory or explanation, it is thought that because the volume around the nitrogen atom is excessively large, the molecule was decomposed during hole transport layer formation, and device efficiency and lifespan were both degraded.

In addition, though an amine group having a phenanthrene group may be combined with the core structure of benzonaphthofuran similar to the present disclosure, the effect on device efficiency and lifespan may depend on the type (e.g., position) of substituent. For example, if the amine group is at position 2 of Chemical Formula 1 or at position 3 of Chemical Formula 1 as in Comparative Example 10, device efficiency and lifespan are both degraded. Without being bound by the correctness of any theory or explanation, it is thought that in this case, the planarity of the molecule as a whole was increased, intermolecular stacking was enhanced, and hole transport properties were deteriorated.

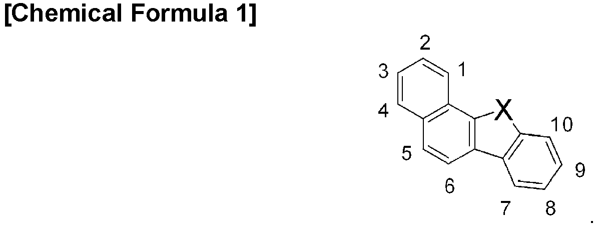

In contrast, as confirmed in Examples 6 and 11, when Ar₁ and Ar₂ are substituents including a heteroatom, hole transport properties are improved due to the influence of the heteroatom in Ar₁ and Ar₂, and excellent device properties may be shown observed regardless of the position at which the amine group is bound to the core structure. In addition, as shown in Example 18, when Ar₁ and Ar₂ are substituents having polycyclic aromatic rings, interactions between the polycyclic aromatic rings of Ar₁ and Ar₂ and the emission layer host material may be enhanced, hole transport properties may be improved, and excellent device properties may be shown.

Comparative Examples 11 and 12 have a structure in which an amine group having a naphthyl substituent is combined with a benzonaphthofuran core structure, similar to the polycyclic compound of the present disclosure. However, compared with the polycyclic compound of the present disclosure, the position of the amine group is different. In the compounds of Comparative Examples 11 and 12, the oxygen atom and the nitrogen atom are ortho- or para- to each other on the benzene ring, which induces electronic instability, and device efficiency and lifespan were both degraded when compared with the Examples.

By comparison to Example 9, however, when the oxygen atom and the nitrogen atom in the benzonaphthofuran ring are para to each other on the naphthalene ring, electrons may be expanded (e.g., due to resonance) so that the molecular structure may be stabilized, and excellent device properties may be shown.

Further, as confirmed in Examples 7 and 10, when the amine has a substituent having a heteroatom, the electronic instability issue may be solved due to the effects of the heteroatom, and excellent device properties may be shown regardless of the combination position of the amine group. For example, it could be confirmed that if an amine group does not have a substituent including a heteroatom, the bonding position of the nitrogen atom with benzonaphthofuran or benzonaphthothiophene significantly influences device properties, and only a case where the amine group is combined at a specific position showed improved efficiency and life.

In addition, as confirmed in Examples 16 and 17, in a compound having a benzonaphthothiophene core, even when a sulfur atom and a nitrogen atom are combined at ortho or para position, excellent device properties are shown. Compared with an oxygen atom, a sulfur atom has smaller electronegativity (e.g., is less electronegative), and electrons may be more delocalized around the ring. Accordingly, even when the nitrogen atom is bound ortho or para to the sulfur atom, the electronic instability issue may be solved, and efficiency and lifespan may be improved.

Comparative Examples 13 and 15 each include a dibenzoheterole substituent on the amine, but carrier balance is collapsed, and device efficiency and lifespan were both degraded compared with the Examples. Comparative Example 14 comprises a benzoxanthene substituent on the amine, but the heat resistance of the benzoxanthene skeleton is insufficient, and device efficiency and lifespan were both degraded when compared with the Examples.

Comparative Examples 16 and 17 are amine compounds having a carbazole group substituents, but the binding positions of the carbazole groups are different from the materials of the Examples, and the device efficiency and lifespan were both degraded when compared with the Examples. In contrast, in the polycyclic compound according to the present disclosure, the amine moiety is bound to position 3 or position 9 of the carbazole ring, which are the most electron-rich positions, such that hole transport properties were improved, and emission efficiency was improved.

Comparative Example 18 is an amine compound having a dibenzofuran substituent, and thermal and charge tolerance were degraded, and device efficiency and lifespan were both degraded compared with the Examples.

The polycyclic compound according to an embodiment of the present disclosure may be comprised in a hole transport region to contribute to the decrease of the driving voltage and the increase of the efficiency and lifespan of an organic electroluminescence device.

The organic electroluminescence device according to an embodiment of the present disclosure may have excellent efficiency.

The polycyclic compound according to an embodiment of the present disclosure may be used as a material for the hole transport region of an organic electroluminescence device, and the efficiency of the organic electroluminescence device may be improved by the compound.

As used herein, the terms "substantially," "about," and similar terms are used as terms of approximation and not as terms of degree, and are intended to account for the inherent deviations in measured or calculated values that would be recognized by those of ordinary skill in the art.

Any numerical range recited herein is intended to comprise all sub-ranges of the same numerical precision subsumed within the recited range. For example, a range of "1.0 to 10.0" is intended to comprise all subranges between (and comprising) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to comprise all lower numerical limitations subsumed therein and any minimum numerical limitation recited in this specification is intended to comprise all higher numerical limitations subsumed therein. Accordingly, Applicant reserves the right to amend this specification, comprising the claims, to expressly recite any sub-range subsumed within the ranges expressly recited herein.

Although example embodiments of the present disclosure have been described, it is understood that the present disclosure is not limited to these example embodiments, and that various changes and modifications can be made by one of ordinary skill in the art that would be within the scope of the present disclosure, as set forth in the following claims.

## Claims

1. A polycyclic compound represented by Formula 1: 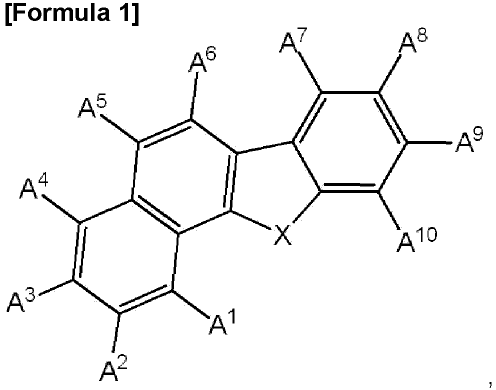 wherein in Formula 1,
X is O or S,
one of A¹ to A¹⁰ is a substituted amine group, wherein at least one substituent of the substituted amine group comprises a cyclic group comprising between 6 and 30 ring forming carbon atoms or a polycyclic group comprising between 6 and 30 ring forming carbon atoms;
and the remainder are each independently a hydrogen atom, a deuterium atom, a halogen atom, a substituted or unsubstituted alkyl group comprising 1 to 20 carbon atoms, or a substituted or unsubstituted aryl group comprising 6 to 30 ring forming carbon atoms;
wherein the substituted amine group is represented by Formula 2-2: 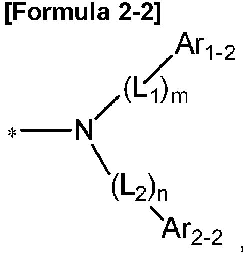 wherein in Formula 2-2,
L₁ and L₂ are each independently a bond, a substituted or unsubstituted arylene group comprising 6 to 30 ring forming carbon atoms, or a substituted or unsubstituted heteroarylene group comprising 2 to 30 ring forming carbon atoms,
"m" and "n" are each independently an integer of 0 to 2, and
Ar₁₋₂ and Ar₂₋₂ are each independently a substituted or unsubstituted aryl group comprising 10 to 40 ring forming carbon atoms or are represented by Formula 3-3 or Formula 3-4, where at least one among Ar₁₋₂ and Ar₂₋₂ is represented by Formula 3-3: 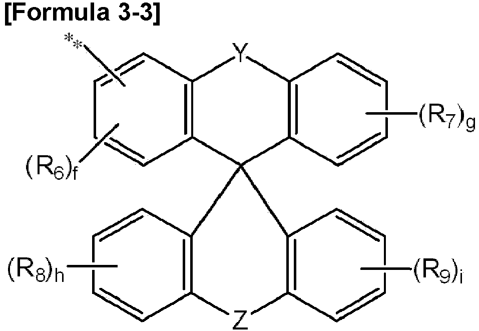 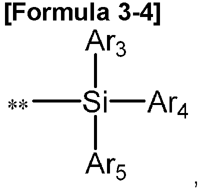 and wherein in Formula 3-3 and Formula 3-4,
Y and Z are each independently a bond, O, or S, and at least one of Y and Z is O or S,
R₆ to R₉ are each independently a hydrogen atom, a deuterium atom, a halogen atom, a substituted or unsubstituted alkyl group comprising 1 to 20 carbon atoms, a substituted or unsubstituted aryl group comprising 6 to 30 ring forming carbon atoms, or a substituted or unsubstituted heteroaryl group comprising 2 to 30 ring forming carbon atoms,
"f" is an integer of 0 to 3,
"g" to "i" are each independently an integer of 0 to 4, and
Ar₃ to Ar₅ are each independently a substituted or unsubstituted aryl group comprising 6 to 30 ring forming carbon atoms.

2. The polycyclic compound of claim 1, wherein Formula 2-2 is represented by Formula 5-1: 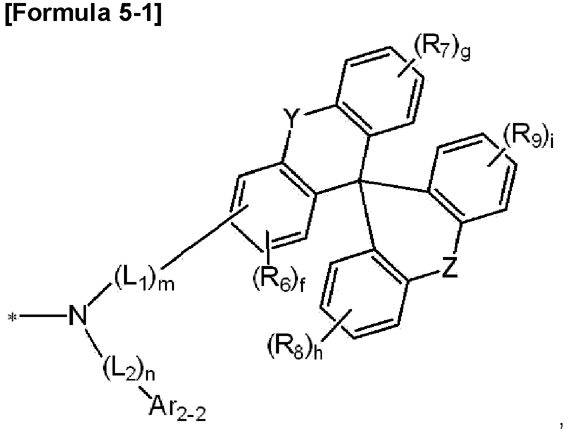 and wherein in Formula 5-1,
Ar₂₋₂ is a substituted or unsubstituted aryl group comprising 10 to 40 ring forming carbon atoms,
L₁, L₂, "m" and "n" are each independently the same as defined in claim 1, and
Y, Z, R₆ to R₉, "f" to "i", and Ar₃ to Ar₅ are each independently the same as defined in claim 1.

3. The polycyclic compound of any preceding claim, wherein any one of A¹ to A⁶ in Formula 1 is the substituted amine group, or wherein any one of A⁷ or A¹⁰ in Formula 1 is the substituted amine group.

4. The polycyclic compound of any one of claims 1 to 3, wherein L₁ and L₂ are each independently a bond, a substituted or unsubstituted phenylene group, or a substituted or unsubstituted naphthyl group.

5. The polycyclic compound of any one of claims 1 to 4, wherein:
L₂ is a bond or a substituted or unsubstituted phenyl group,
"n" is 1,
Ar₂₋₂ is a substituted or unsubstituted phenyl group, a substituted or unsubstituted biphenyl group, a substituted or unsubstituted terphenyl group, a substituted or unsubstituted phenanthrene group, a substituted or unsubstituted naphthyl group, or a substituted or unsubstituted fluorenyl group, and L₂ and Ar₂₋₂ are not both phenyl groups.

6. The polycyclic compound of any one of claims 1 to 5, wherein the polycyclic compound represented by Formula 1 is at least one selected from compounds represented in Compound Group 3:

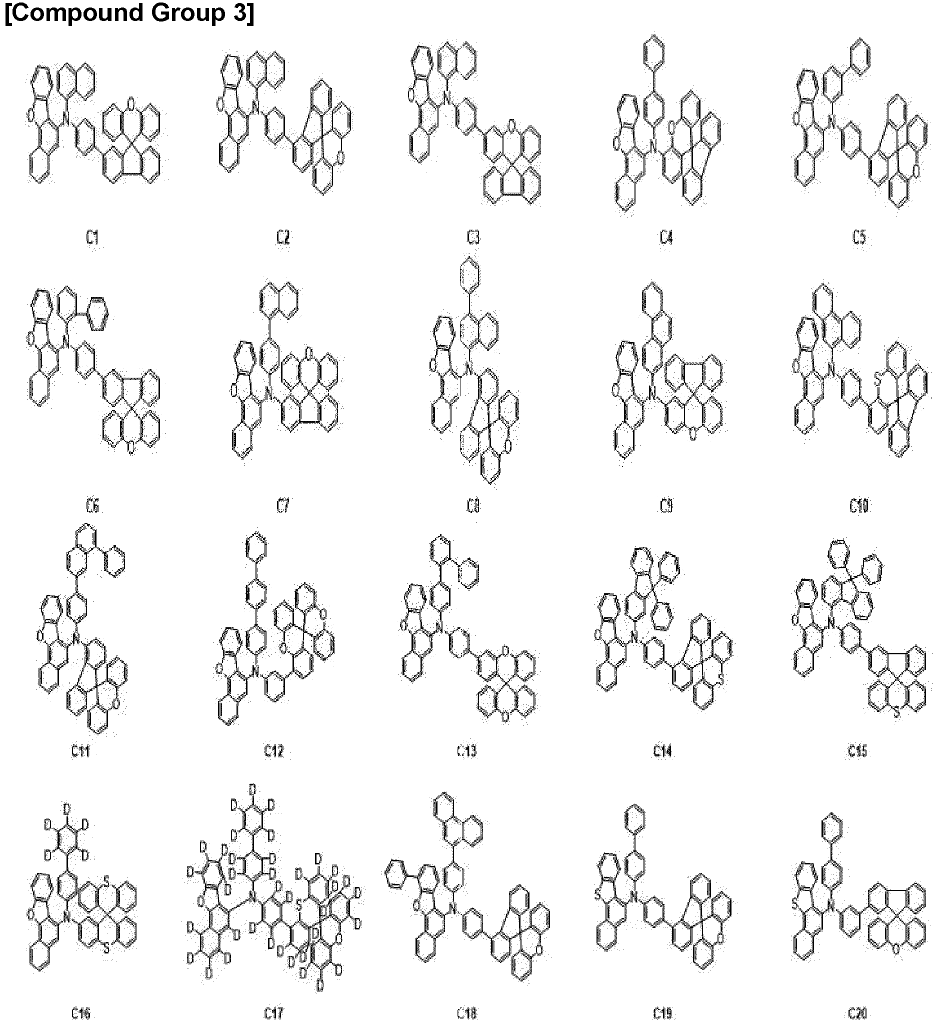

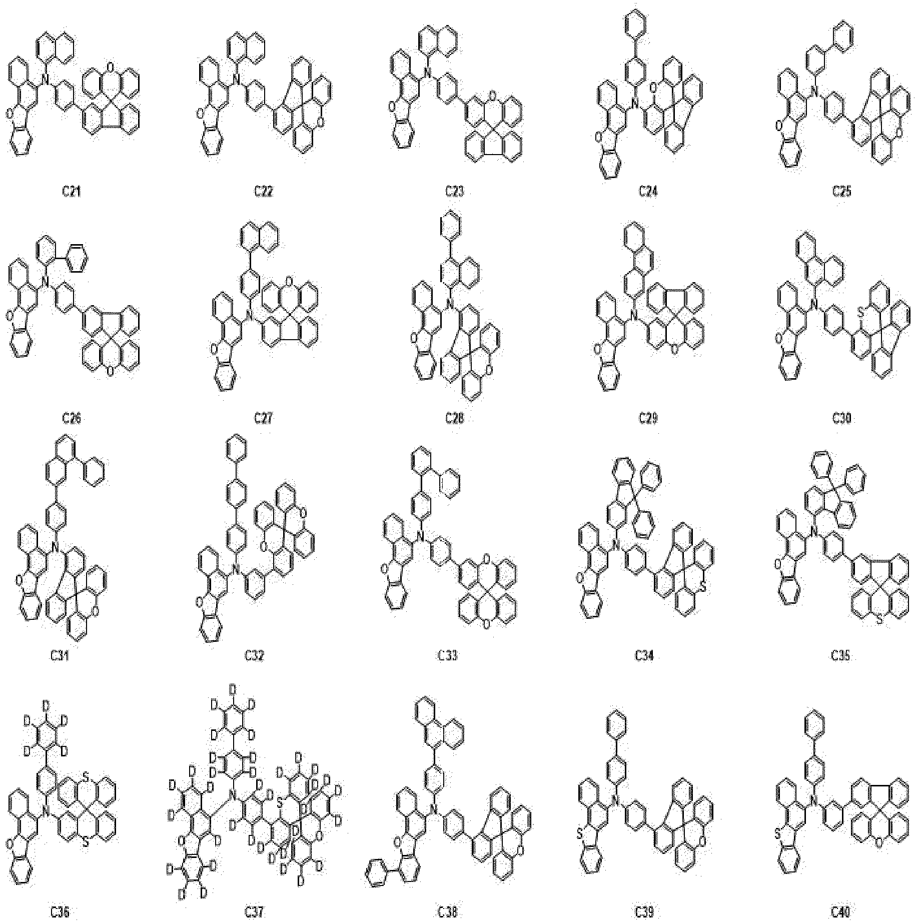

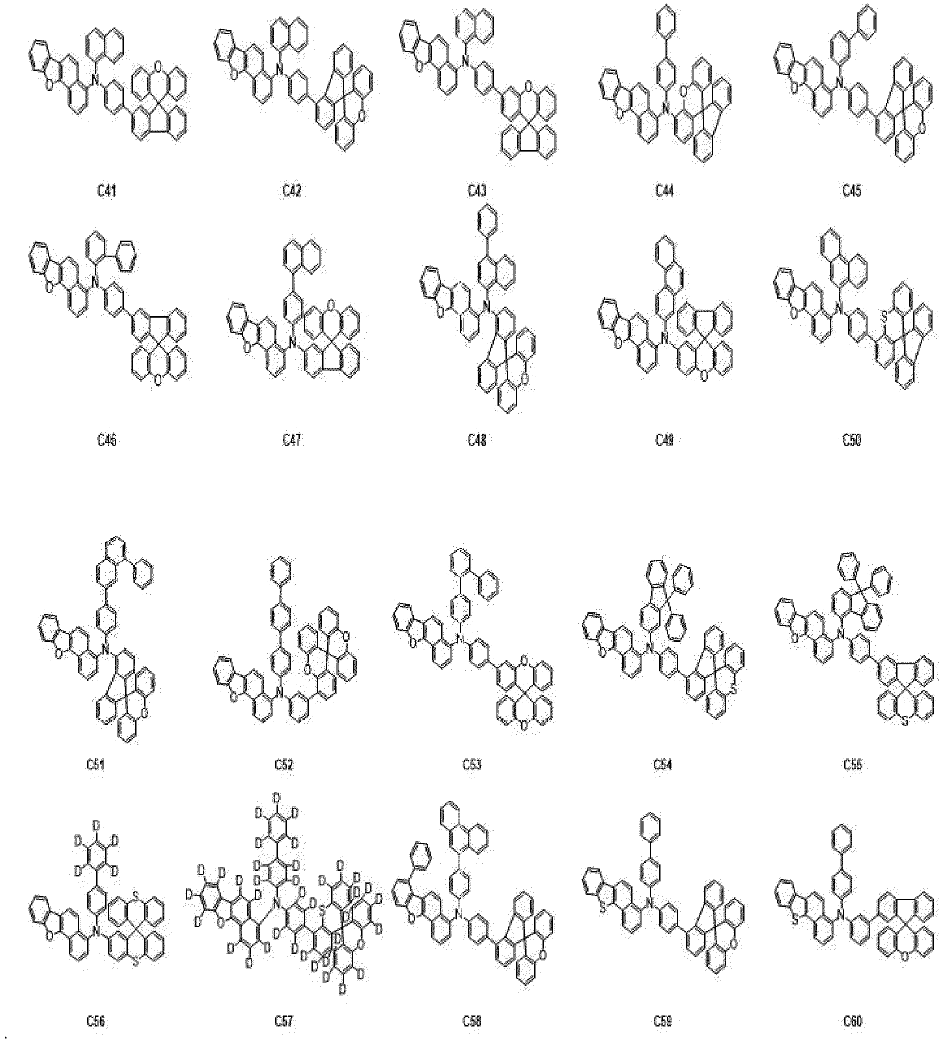

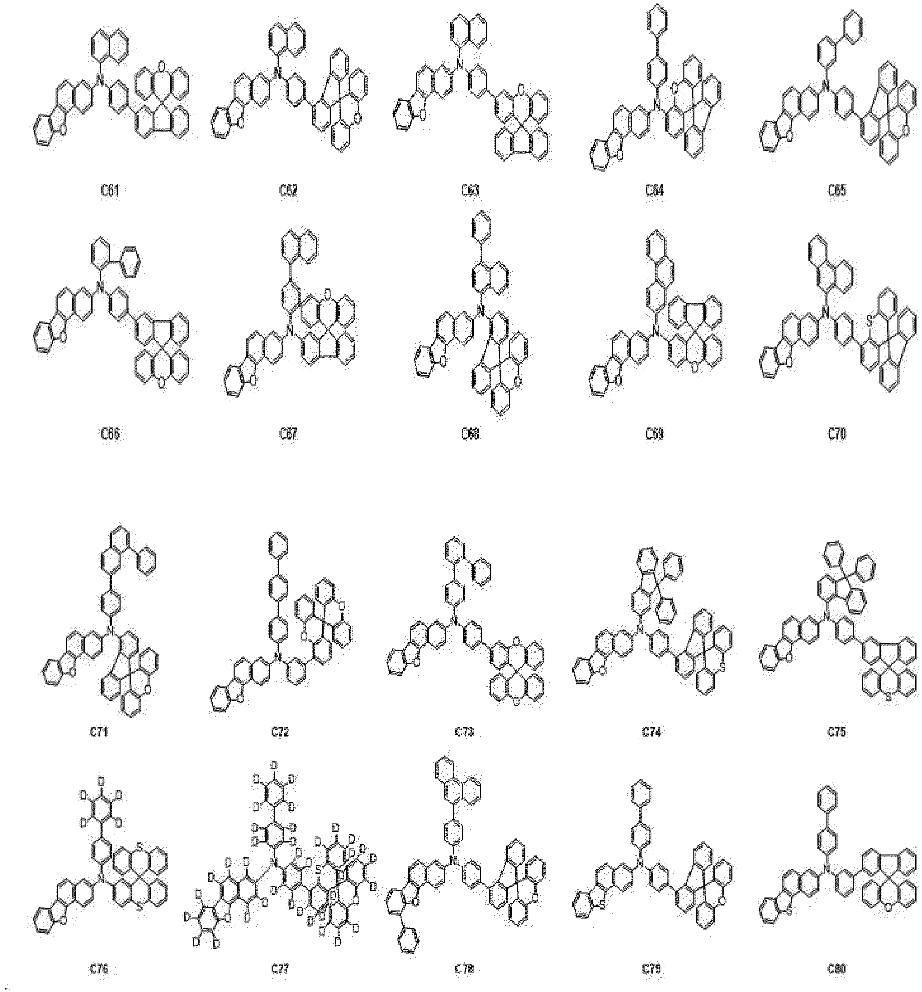

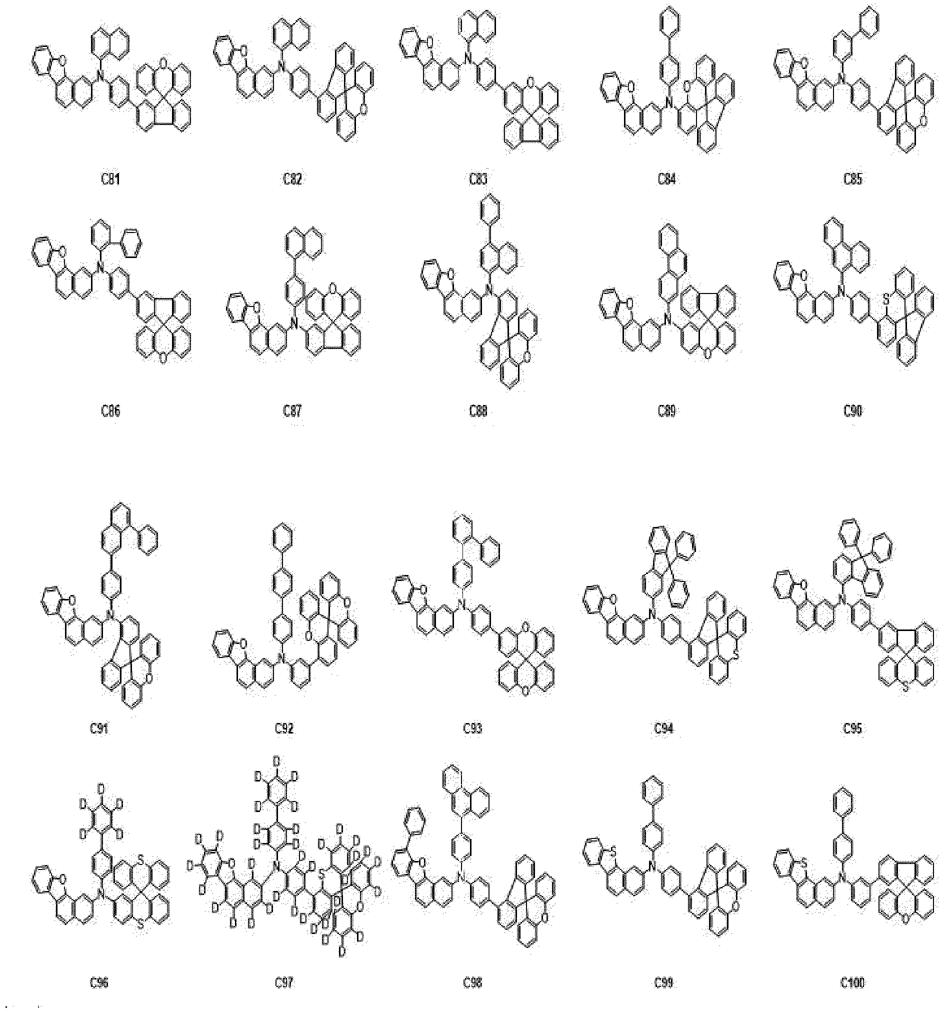

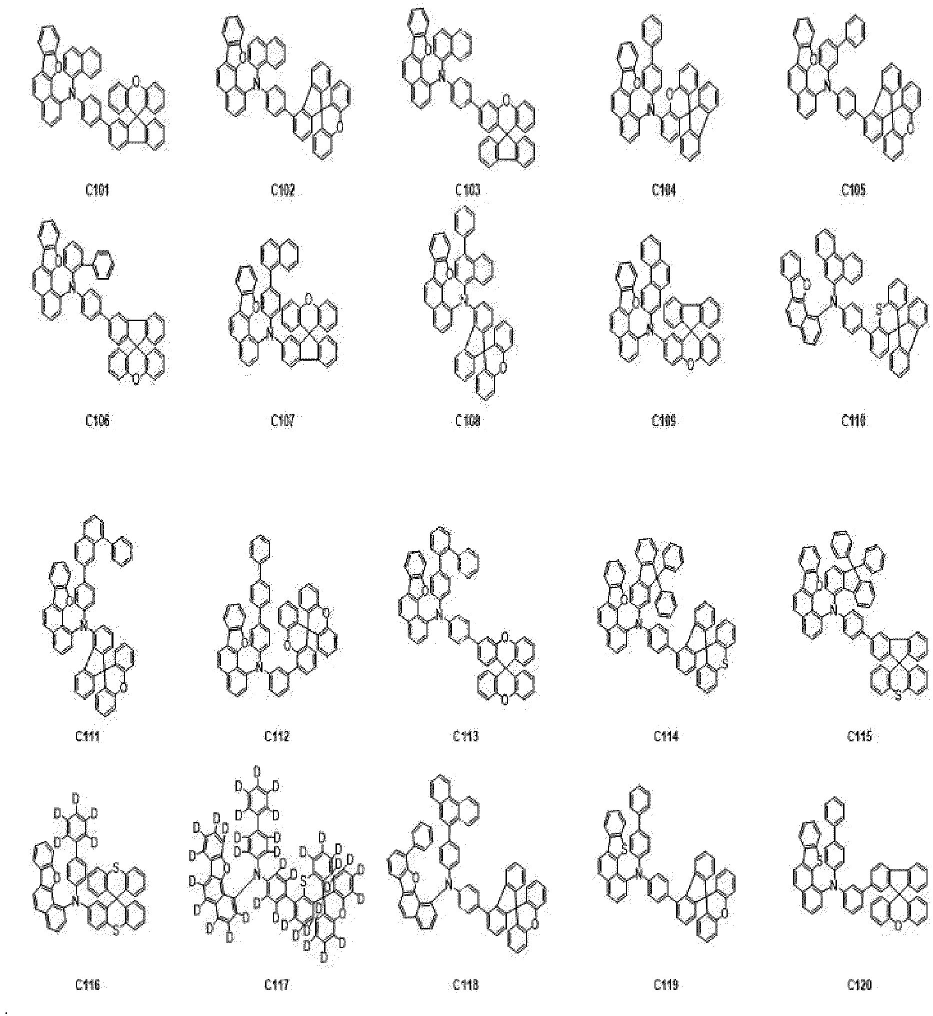

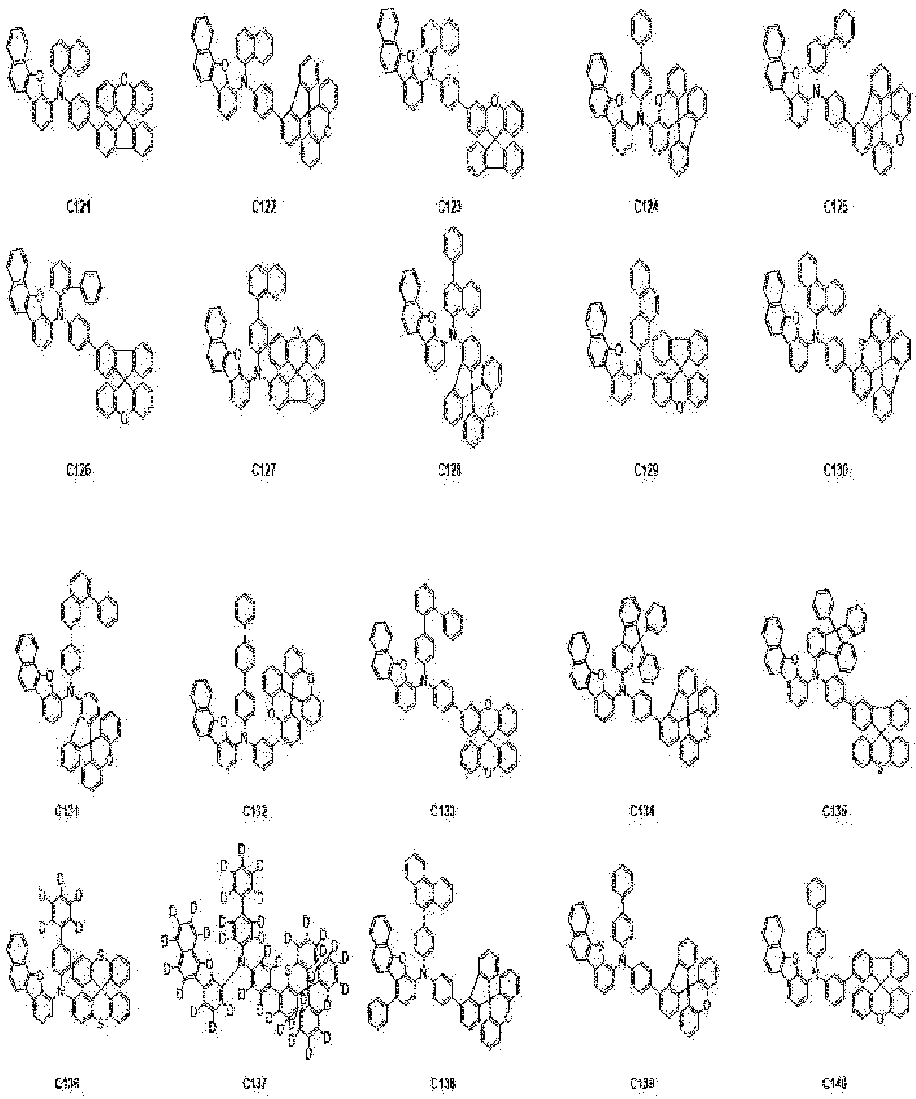

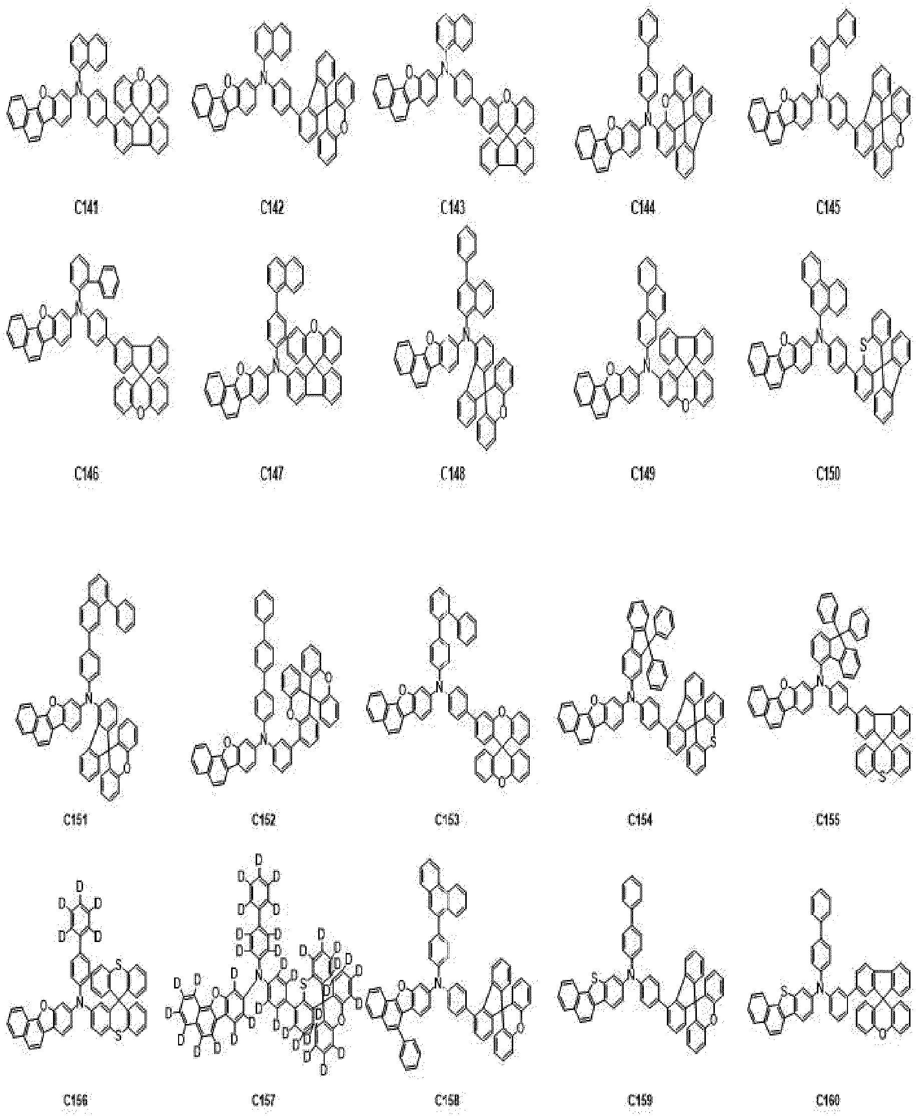

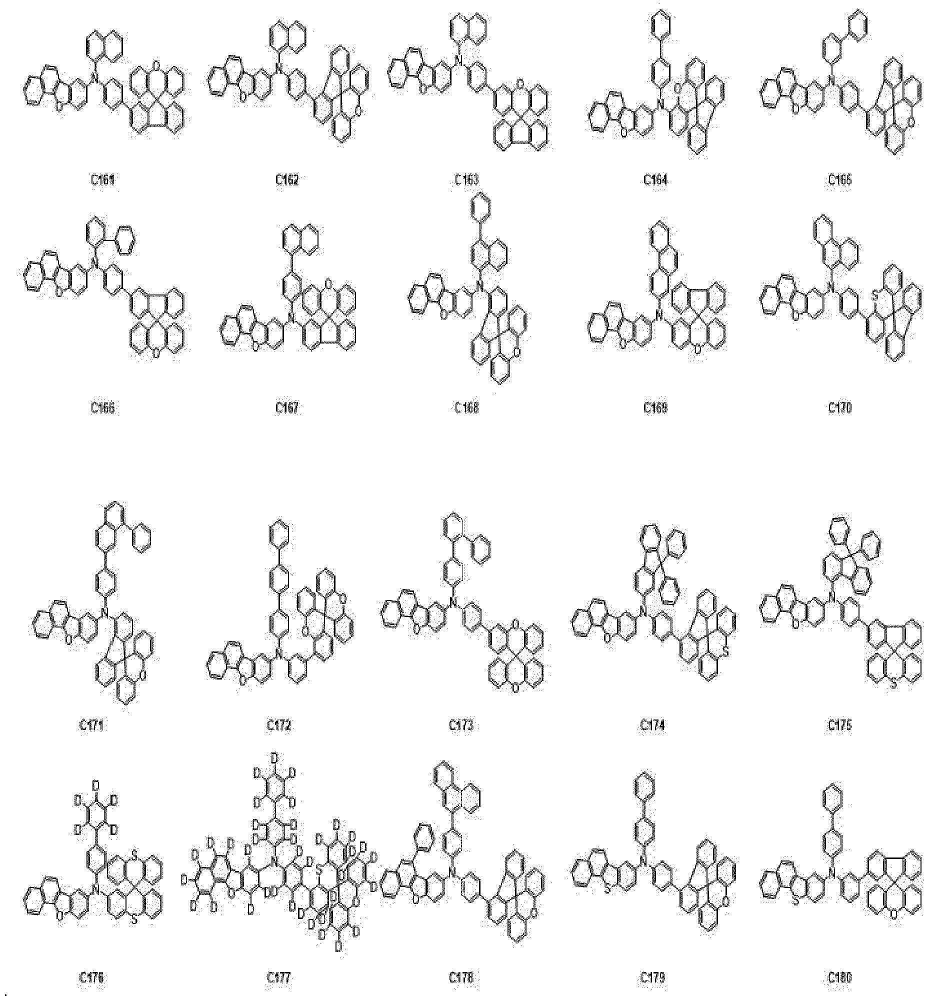

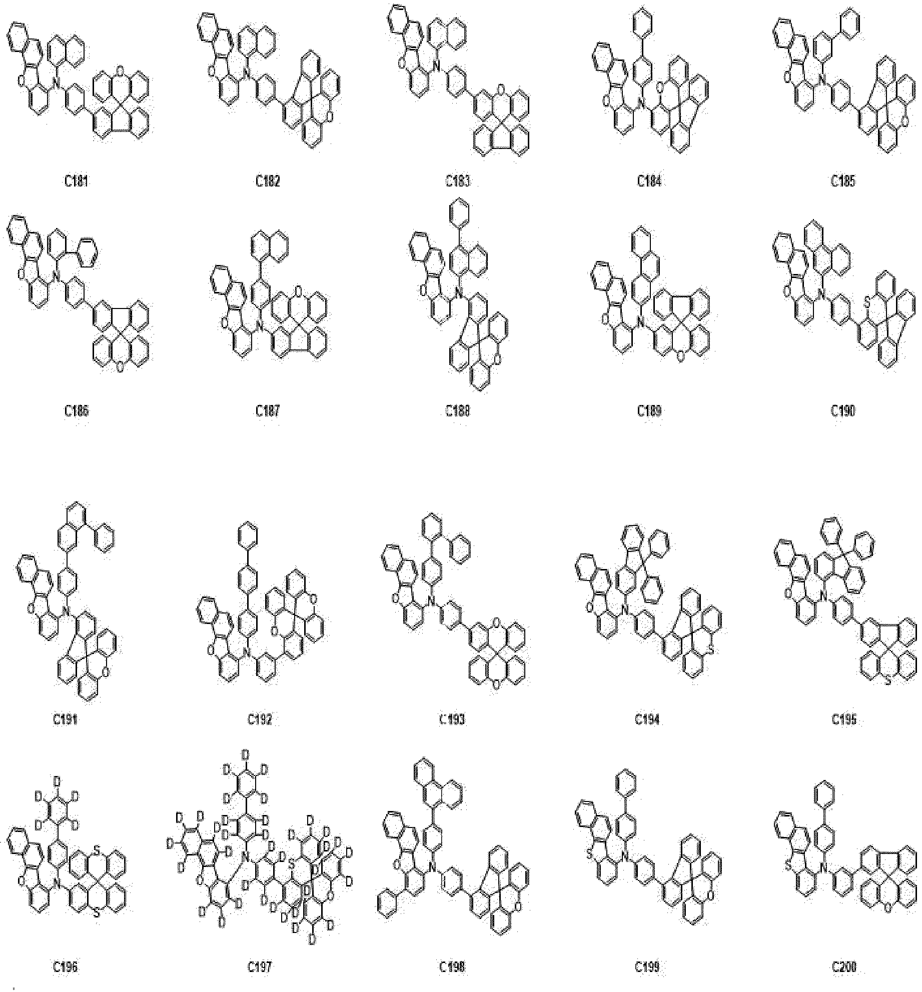

7. An organic electroluminescence device, comprising:
a first electrode;
a hole transport region on the first electrode;
an emission layer on the hole transport region;
an electron transport region on the emission layer; and
a second electrode on the electron transport region,
wherein the hole transport region comprises the polycyclic compound of any one of claims 1 to 6.

8. The organic electroluminescence device according claim 7, wherein the hole transport region comprises:
a hole injection layer on the first electrode; and
a hole transport layer on the hole injection layer, and
the hole transport layer comprises the polycyclic compound of any one of claims 1 to 6.

## Patentansprüche

1. Polycyclische Verbindung, dargestellt durch Formel 1: 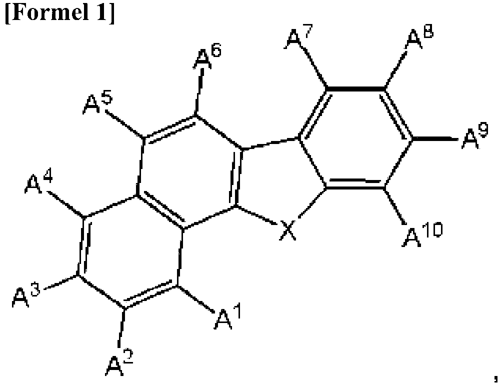 wobei in Formel 1
X O oder S ist,
eines von A¹ bis A¹⁰ eine substituierte Amingruppe ist, wobei mindestens ein Substituent der substituierten Amingruppe eine cyclische Gruppe, umfassend zwischen 6 und 30 ringbildende Kohlenstoffatome, oder eine polycyclische Gruppe, umfassend zwischen 6 und 30 ringbildende Kohlenstoffatome, umfasst;
und der Rest jeweils unabhängig ein Wasserstoffatom, ein Deuteriumatom, ein Halogenatom, eine substituierte oder unsubstituierte Alkylgruppe, umfassend 1 bis 20 Kohlenstoffatome, oder eine substituierte oder unsubstituierte Arylgruppe, umfassend 6 bis 30 ringbildende Kohlenstoffatome, ist;
wobei die substituierte Amingruppe dargestellt ist durch Formel 2-2: 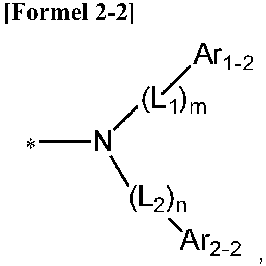 wobei in Formel 2-2
L₁ und L₂ jeweils unabhängig eine Bindung, eine substituierte oder unsubstituierte Arylengruppe, umfassend 6 bis 30 ringbildende Kohlenstoffatome, oder eine substituierte oder unsubstituierte Heteroarylengruppe, umfassend 2 bis 30 ringbildende Kohlenstoffatome, ist,
"m" und "n" jeweils unabhängig eine ganze Zahl von 0 bis 2 sind, und
Ar₁₋₂ und Ar₂₋₂ jeweils unabhängig eine substituierte oder unsubstituierte Arylgruppe sind, umfassend 10 bis 40 ringbildende Kohlenstoffatome, oder durch Formel 3-3 oder Formel 3-4 dargestellt sind, wobei mindestens eines von Ar₁₋₂ und Ar₂₋₂ dargestellt ist durch Formel 3-3: 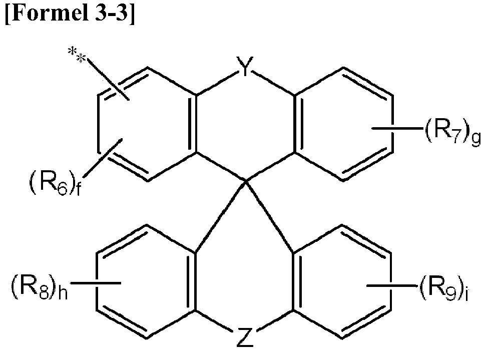 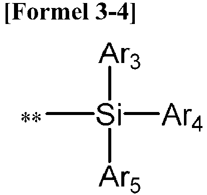 und wobei in Formel 3-3 und Formel 3-4
Y und Z jeweils unabhängig eine Bindung, O oder S sind, und mindestens eines von Y und Z O oder S ist,
R₆ bis R₉ jeweils unabhängig ein Wasserstoffatom, ein Deuteriumatom, ein Halogenatom, eine substituierte oder unsubstituierte Alkylgruppe, umfassend 1 bis 20 Kohlenstoffatome, eine substituierte oder unsubstituierte Arylgruppe, umfassend 6 bis 30 ringbildende Kohlenstoffatome, oder eine substituierte oder unsubstituierte Heteroarylgruppe, umfassend 2 bis 30 ringbildende Kohlenstoffatome, sind,
"f" eine ganze Zahl von 0 bis 3 ist,
"g" bis "i" jeweils unabhängig eine ganze Zahl von 0 bis 4 sind, und
Ar₃ bis Ar₅ jeweils unabhängig eine substituierte oder unsubstituierte Arylgruppe sind, umfassend 6 bis 30 ringbildende Kohlenstoffatome.

2. Polycyclische Verbindung nach Anspruch 1, wobei Formel 2-2 durch Formel 5-1 dargestellt ist: 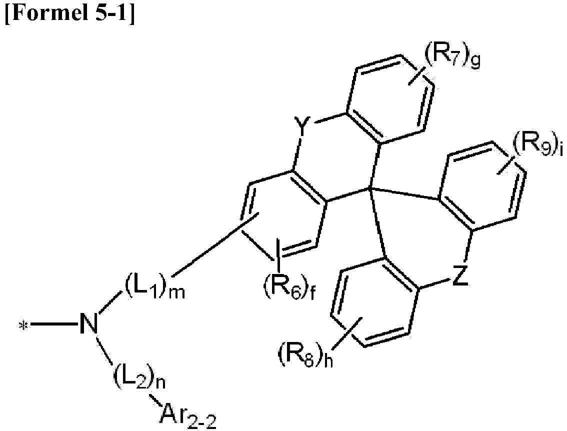 und wobei in Formel 5-1
Ar₂₋₂ eine substituierte oder unsubstituierte Arylgruppe ist, umfassend 10 bis 40 ringbildende Kohlenstoffatome,
L₁, L₂, "m" und "n" jeweils unabhängig die gleiche Bedeutung wie definiert in Anspruch 1 haben, und
Y, Z, R₆ bis R₉, "f" bis "i" und Ar₃ bis Ar₅ jeweils unabhängig die gleiche Bedeutung wie definiert in Anspruch 1 haben.

3. Polycyclische Verbindung nach einem der vorhergehenden Ansprüche, wobei ein beliebiges von A¹ bis A⁶ in Formel 1 die substituierte Amingruppe ist, oder wobei ein beliebiges von A⁷ oder A¹⁰ in Formel 1 die substituierte Amingruppe ist.

4. Polycyclische Verbindung nach einem der Ansprüche 1 bis 3, wobei L₁ und L₂ jeweils unabhängig eine Bindung, eine substituierte oder unsubstituierte Phenylengruppe oder eine substituierte oder unsubstituierte Naphthylgruppe sind.

5. Polycyclische Verbindung nach einem der Ansprüche 1 bis 4, wobei:
L₂ eine Bindung oder eine substituierte oder unsubstituierte Phenylgruppe ist,
"n" 1 ist,
Ar₂₋₂ eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Phenanthrengruppe, eine substituierte oder unsubstituierte Naphthylgruppe oder eine substituierte oder unsubstituierte Fluorenylgruppe ist, und L₂ und Ar₂₋₂ nicht beide Phenylgruppen sind.

6. Polycyclische Verbindung nach einem der Ansprüche 1 bis 5, wobei die durch Formel 1 dargestellte polycyclische Verbindung mindestens eine ist, die ausgewählt ist aus Verbindungen, die in Verbindungsgruppe 3 dargestellt sind:

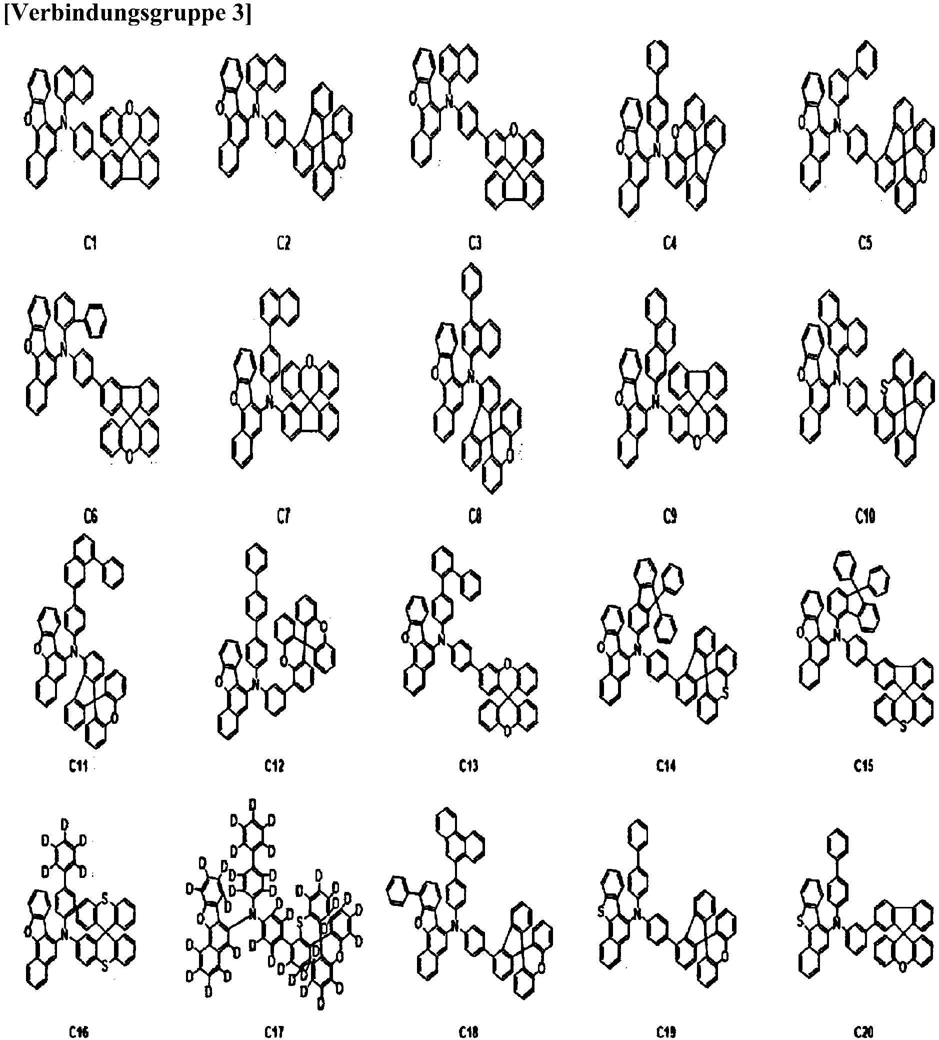

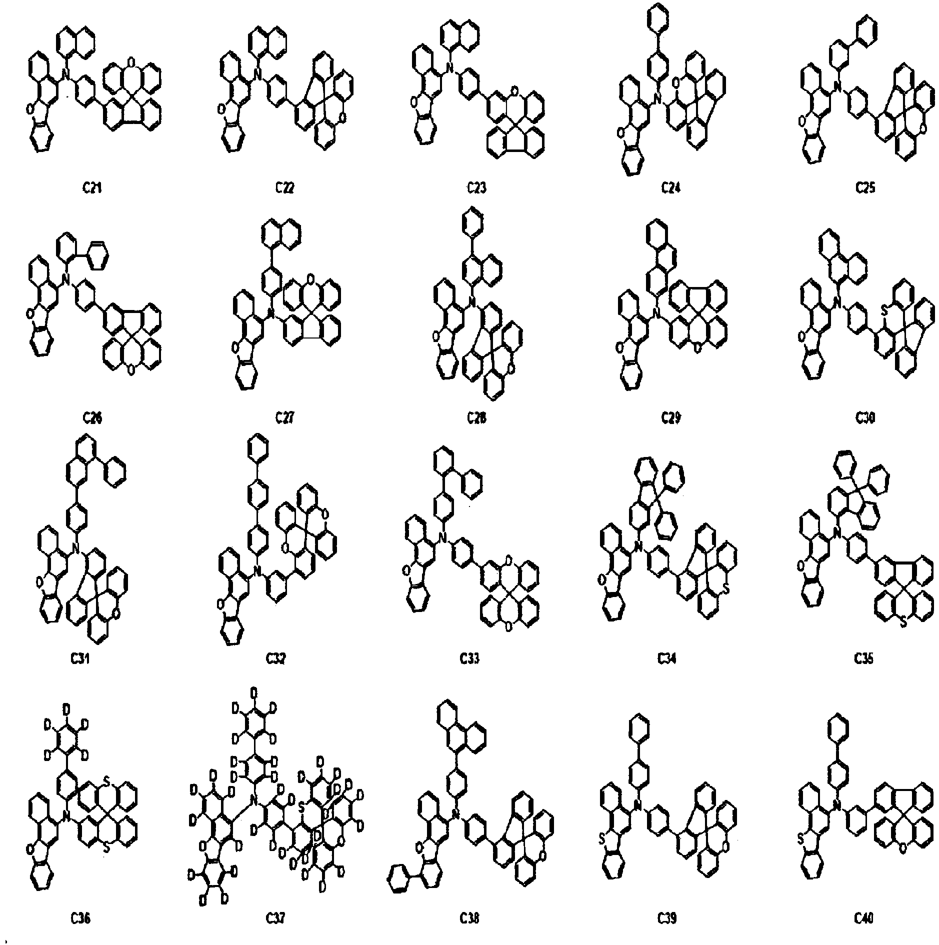

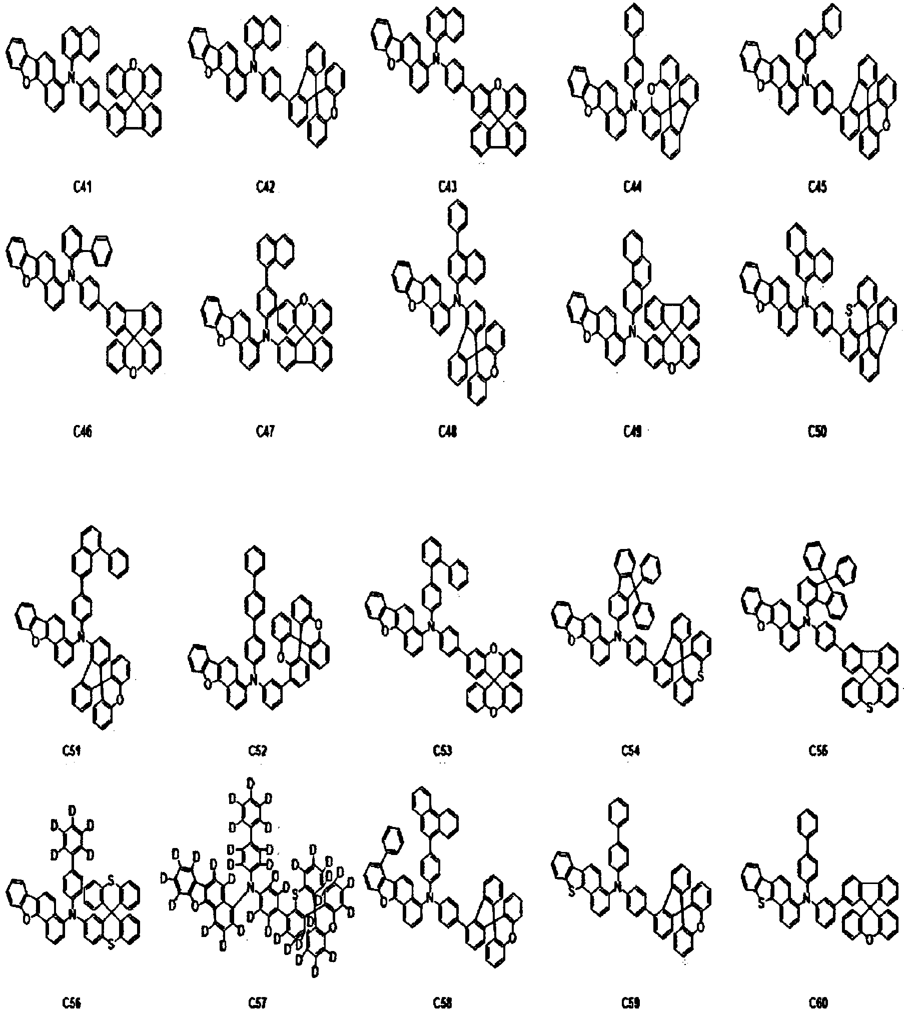

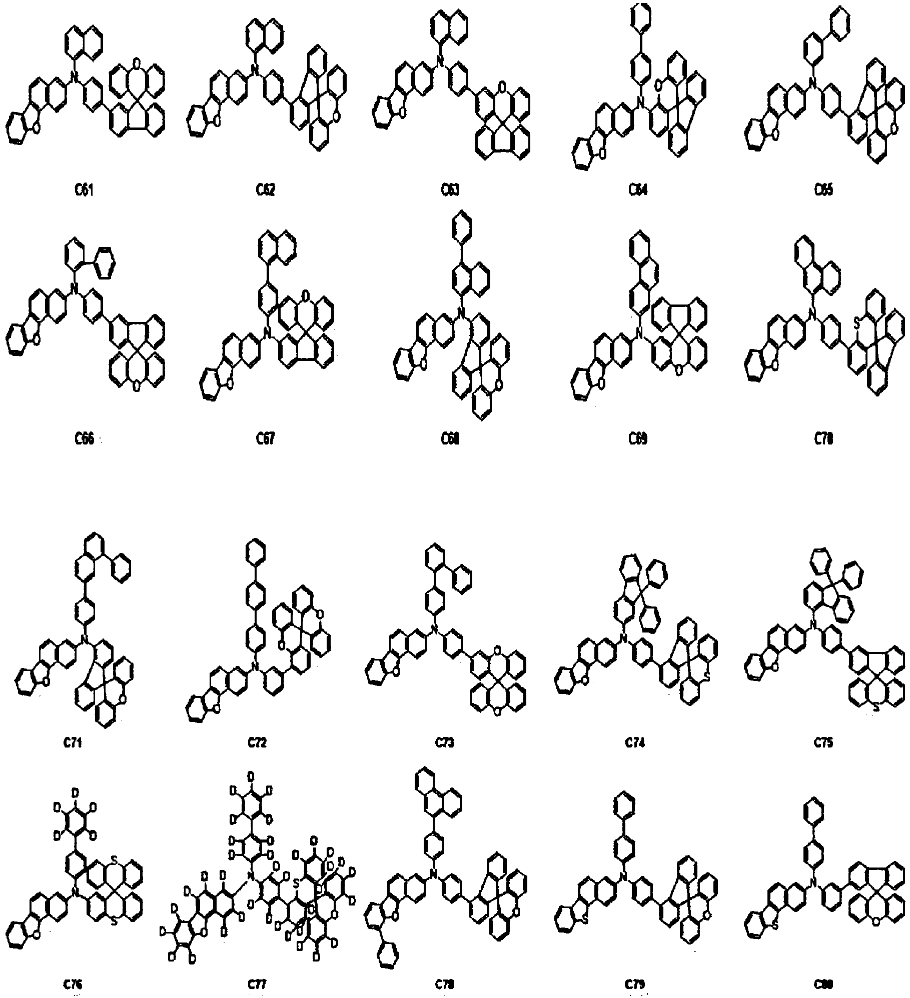

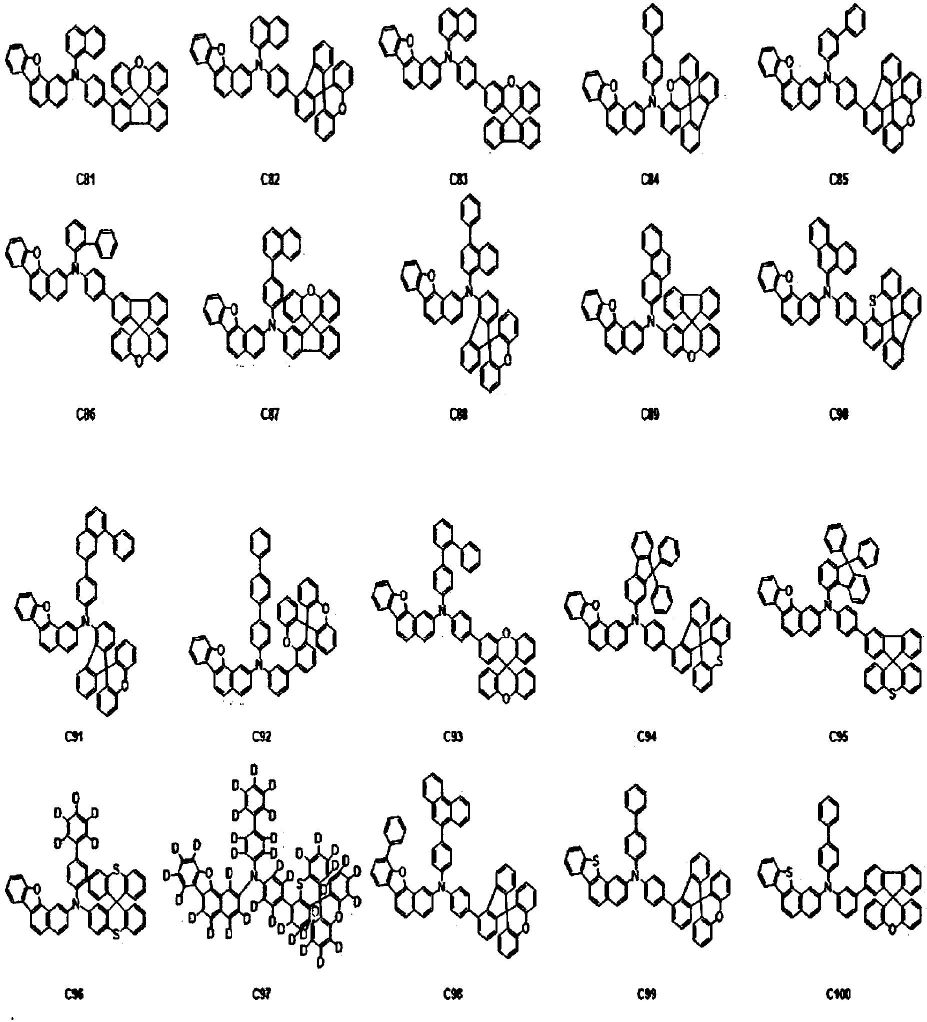

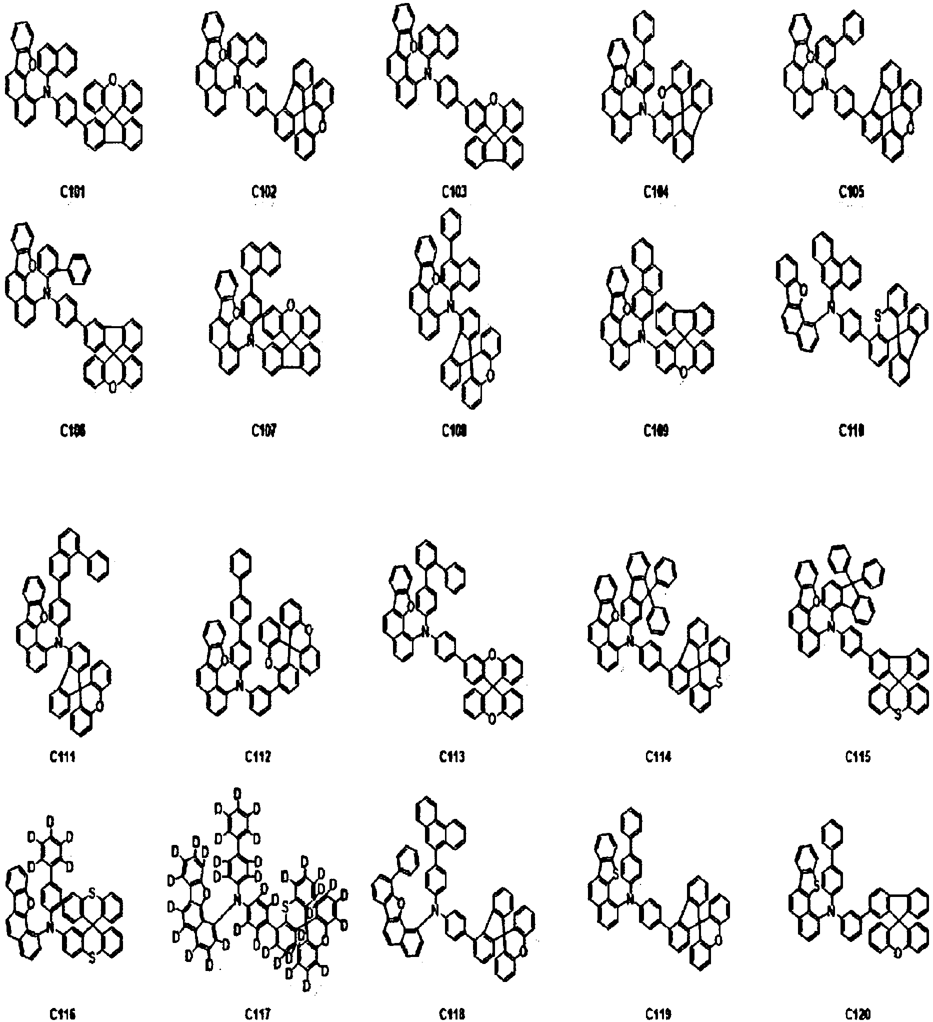

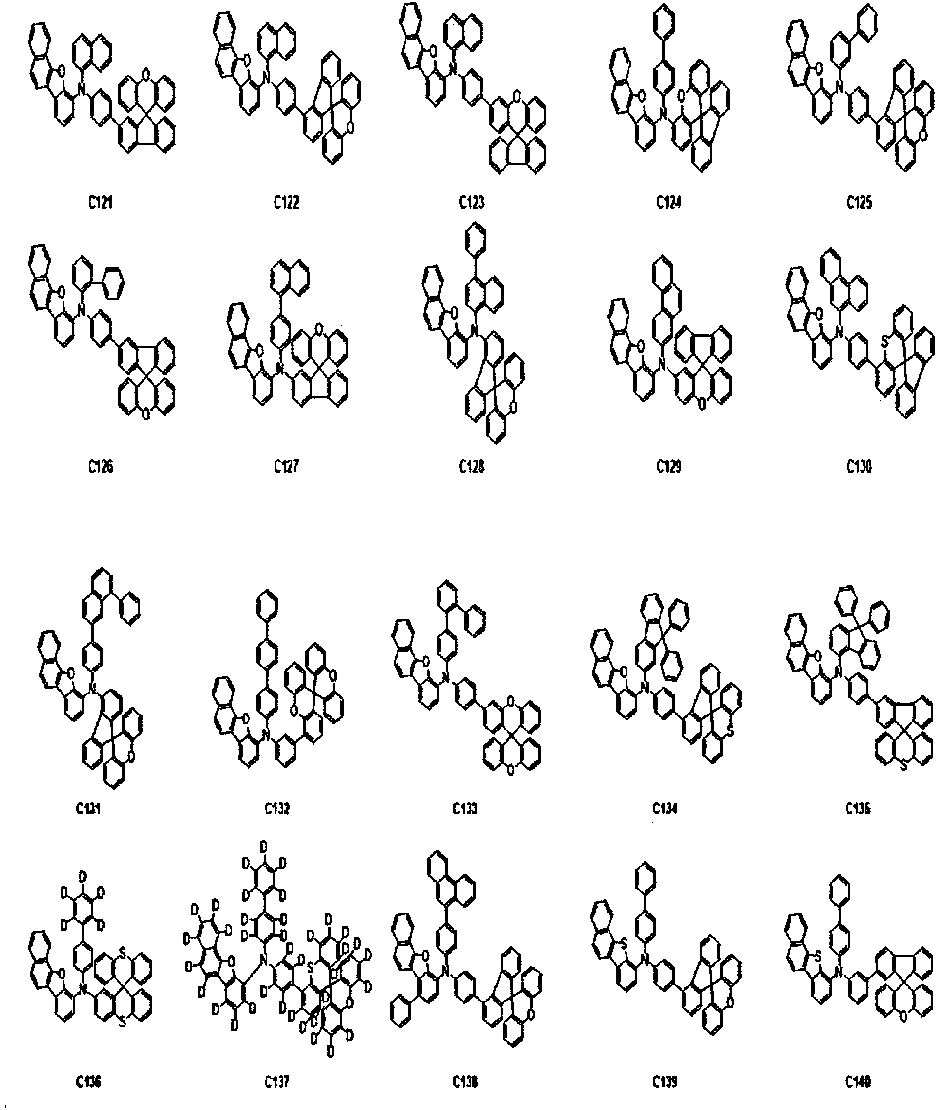

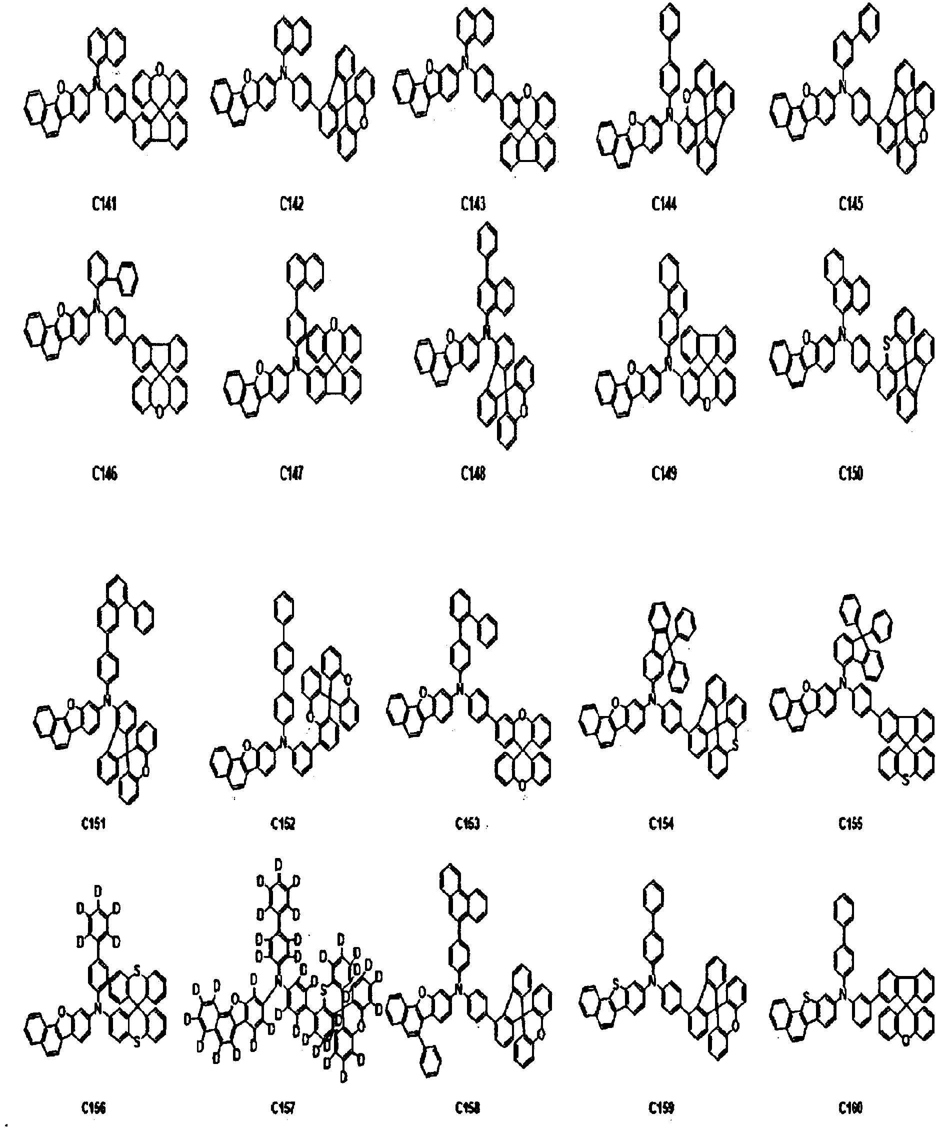

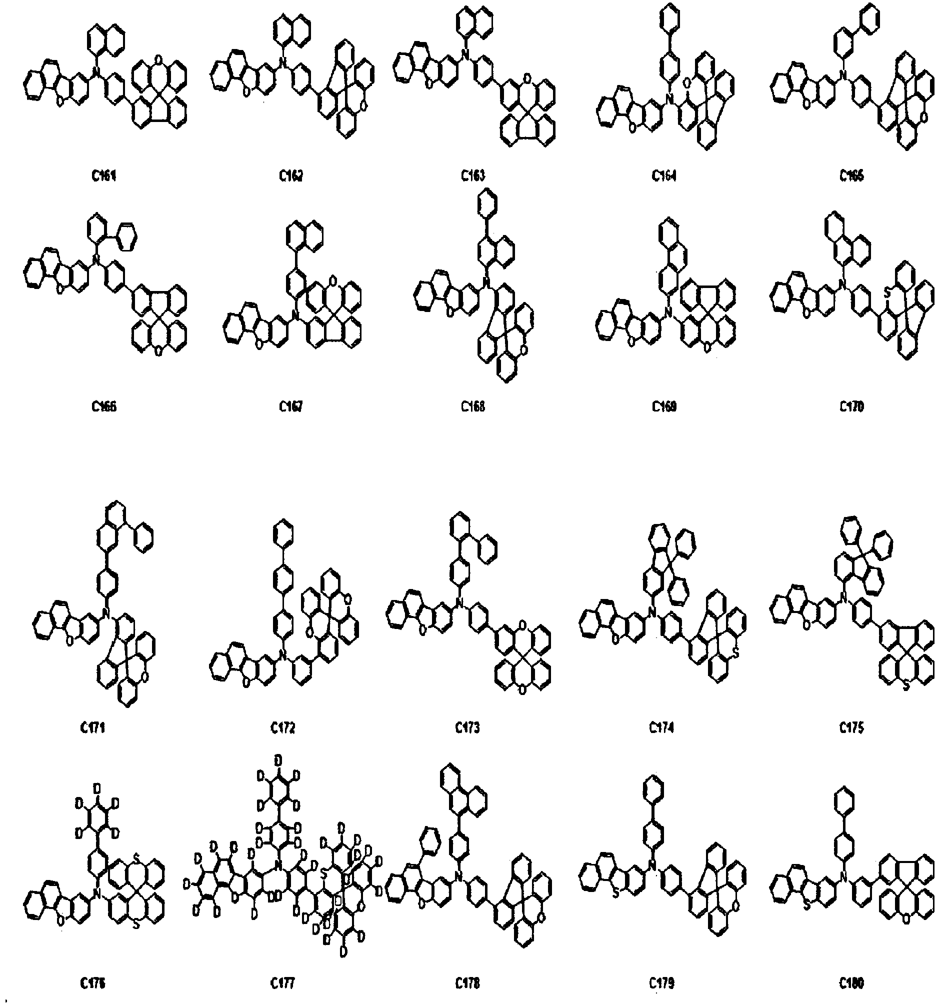

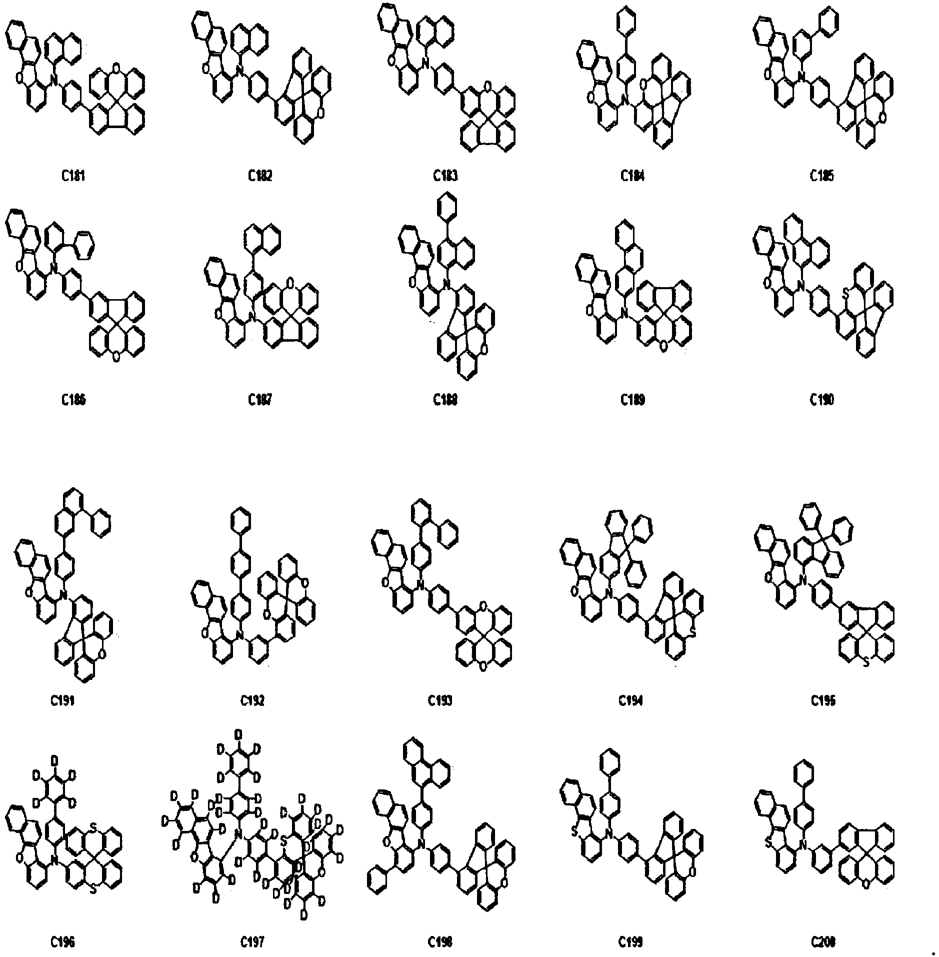

7. Organische Elektrolumineszenzvorrichtung, umfassend:
eine erste Elektrode;
einen Lochtransportbereich auf der ersten Elektrode;
eine Emissionsschicht auf dem Lochtransportbereich;
einen Elektronentransportbereich auf der Emissionsschicht; und
eine zweite Elektrode auf dem Elektronentransportbereich,
wobei der Lochtransportbereich die polycyclische Verbindung nach einem der Ansprüche 1 bis 6 umfasst.

8. Organische Elektrolumineszenzvorrichtung gemäß Anspruch 7, wobei der Lochtransportbereich Folgendes umfasst:
eine Lochinjektionsschicht auf der ersten Elektrode; und
eine Lochtransportschicht auf der Lochinjektionsschicht, und
wobei die Lochtransportschicht die polycyclische Verbindung nach einem der Ansprüche 1 bis 6 umfasst.

## Revendications

1. Composé polycyclique représenté par la Formule 1 : 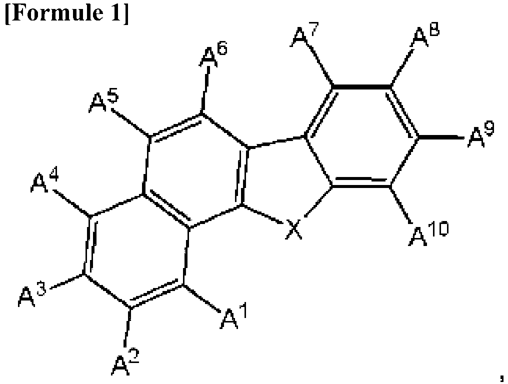 dans lequel dans la Formule 1,
X est O ou S,
l'un de A¹ à A¹⁰ est un groupe amine substitué, dans lequel au moins un substituant du groupe amine substitué comprend un groupe cyclique comprenant entre 6 et 30 atomes de carbone formant un cycle ou un groupe polycyclique comprenant entre 6 et 30 atomes de carbone formant un cycle ;
et les autres sont chacun indépendamment un atome d'hydrogène, un atome de deutérium, un atome d'halogène, un groupe alkyle substitué ou non substitué comprenant 1 à 20 atomes de carbone, ou un groupe aryle substitué ou non substitué comprenant 6 à 30 atomes de carbone formant un cycle ;
dans lequel le groupe amine substitué est représenté par la Formule 2-2 : 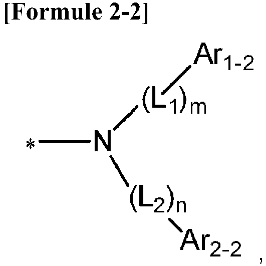 dans lequel dans la Formule 2-2,
Li et L₂ sont chacun indépendamment une liaison, un groupe arylène substitué ou non substitué comprenant 6 à 30 atomes de carbone formant un cycle, ou un groupe hétéroarylène substitué ou non substitué comprenant 2 à 30 atomes de carbone formant un cycle,
« m » et « n » sont chacun indépendamment un nombre entier de 0 à 2, et
Ar₁₋₂ et Ar₂₋₂ sont chacun indépendamment un groupe aryle substitué ou non substitué comprenant 10 à 40 atomes de carbone formant un cycle ou sont représentés par la Formule 3-3 ou la Formule 3-4, où au moins l'un parmi Ar₁₋₂ et Ar₂₋₂ est représenté par la Formule 3-3 : 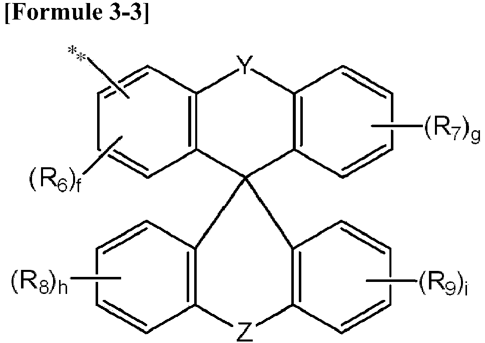 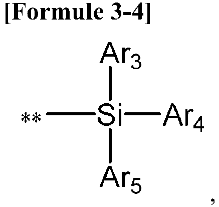 et dans lequel dans la Formule 3-3 et la Formule 3-4,
Y et Z sont chacun indépendamment une liaison, O ou S, et au moins d'un de Y et Z est O ou S,
R₆ à R₉ sont chacun indépendamment un atome d'hydrogène, un atome de deutérium, un atome d'halogène, un groupe alkyle substitué ou non substitué comprenant 1 à 20 atomes de carbone, un groupe aryle substitué ou non substitué comprenant 6 à 30 atomes de carbone formant un cycle, ou un groupe hétéroaryle substitué ou non substitué comprenant 2 à 30 atomes de carbone formant un cycle,
« f » est un nombre entier de 0 à 3,
« g » à « i » sont chacun indépendamment un nombre entier de 0 à 4, et
Ar₃ à Ar₅ sont chacun indépendamment un groupe aryle substitué ou non substitué comprenant 6 à 30 atomes de carbone formant un cycle.

2. Composé polycyclique de la revendication 1, dans lequel la Formule 2-2 est représentée par la Formule 5-1 : 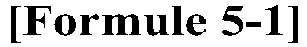 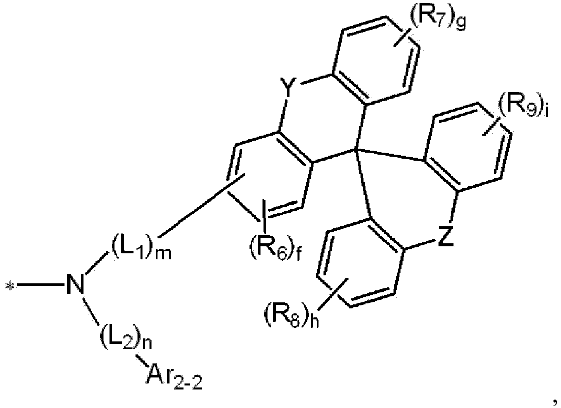 et dans lequel dans la Formule 5-1,
Ar₂₋₂ est un groupe aryle substitué ou non substitué comprenant 10 à 40 atomes de carbone formant un cycle,
L₁, L₂, « m » et « n » sont chacun indépendamment les mêmes que définis dans la revendication 1, et
Y, Z, R₆ à R₉, « f » à « i », et Ar₃ à Ar₅ sont chacun indépendamment les mêmes que définis dans la revendication 1.

3. Composé polycyclique de l'une quelconque des revendications précédentes, dans lequel l'un quelconque de A¹ à A⁶ dans la Formule 1 est le groupe amine substitué, ou dans lequel l'un quelconque de A⁷ ou A¹⁰ dans la Formule 1 est le groupe amine substitué.

4. Composé polycyclique de l'une quelconque des revendications 1 à 3, dans lequel L₁ et L₂ sont chacun indépendamment une liaison, un groupe phénylène substitué ou non substitué, ou un groupe naphthyle substitué ou non substitué.

5. Composé polycyclique de l'une quelconque des revendications 1 à 4, dans lequel :
L₂ est une liaison ou un groupe phényle substitué ou non substitué,
« n » est 1,
Ar₂₋₂ est un groupe phényle substitué ou non substitué, un groupe biphényle substitué ou non substitué, un groupe terphényle substitué ou non substitué, un groupe phénanthrène substitué ou non substitué, un groupe naphtyle substitué ou non substitué, un groupe fluorényle substitué ou non substitué, et L₂ et Ar₂₋₂ ne sont pas tous deux des groupes phényle.

6. Composé polycyclique de l'une quelconque des revendications 1 à 5, ledit composé polycyclique représenté par la Formule1 étant au moins l'un choisi parmi les composés représentés dans le Groupe 3 de composés :

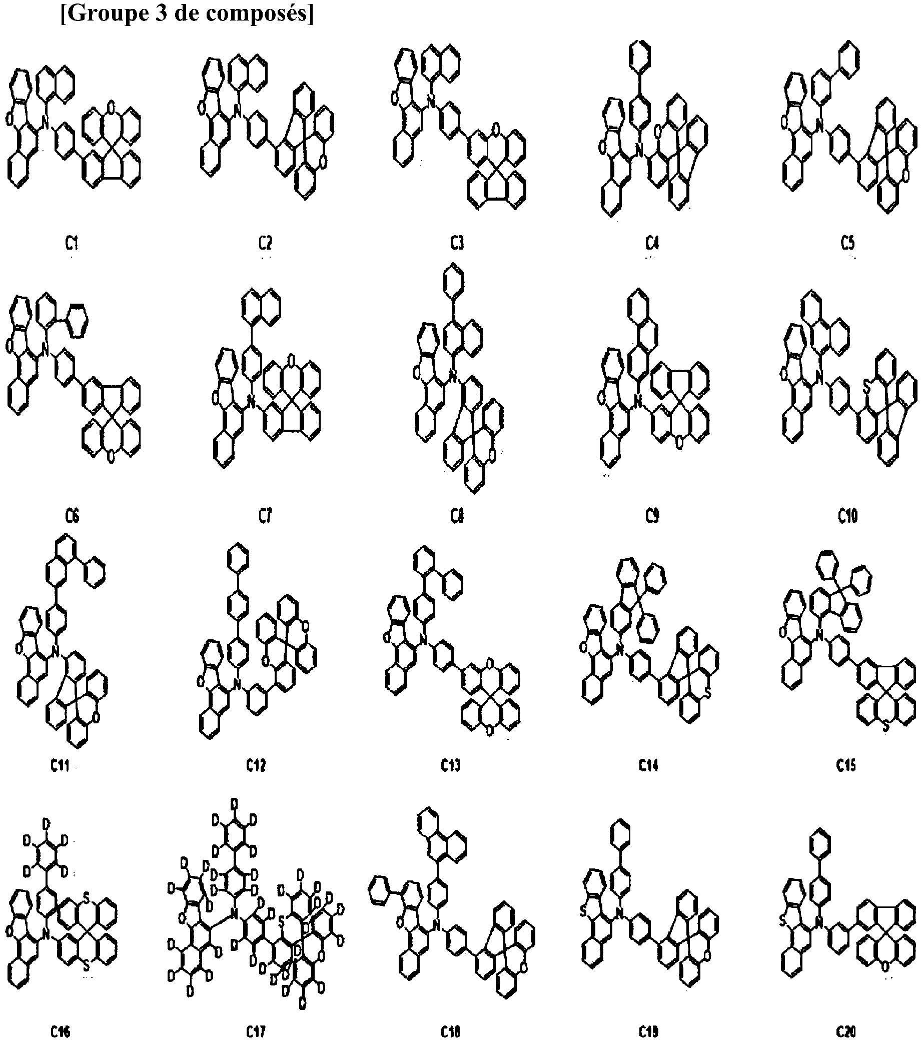

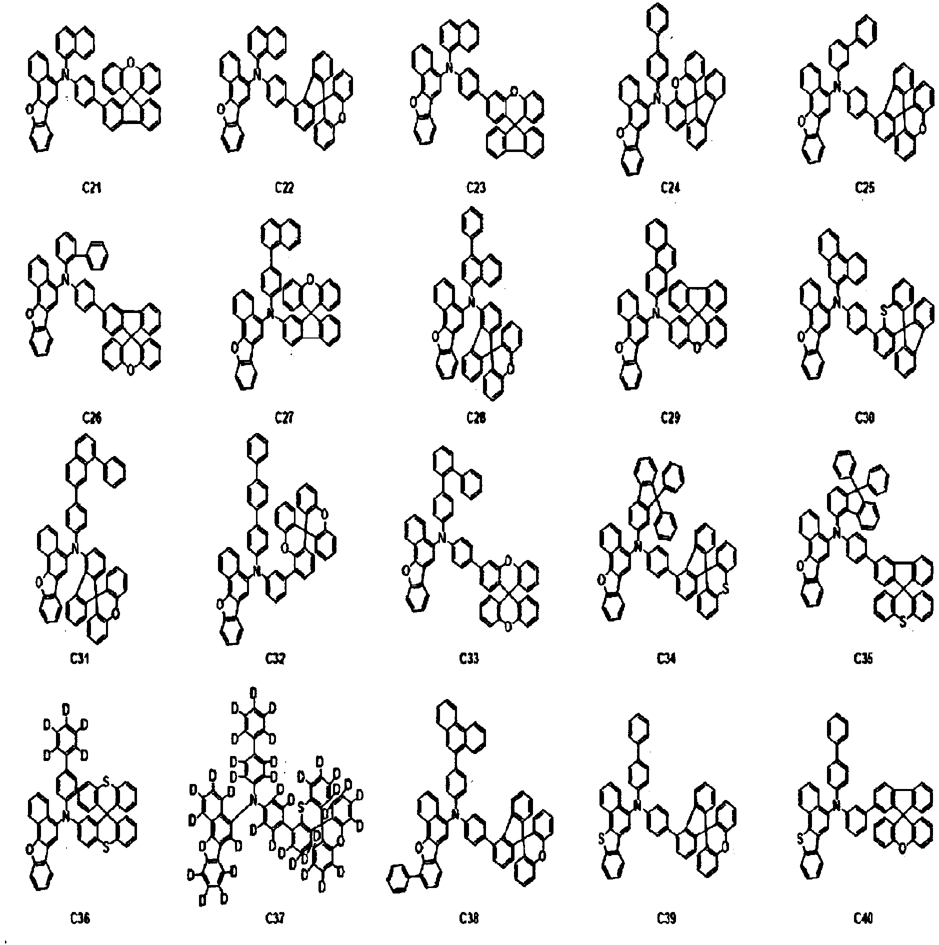

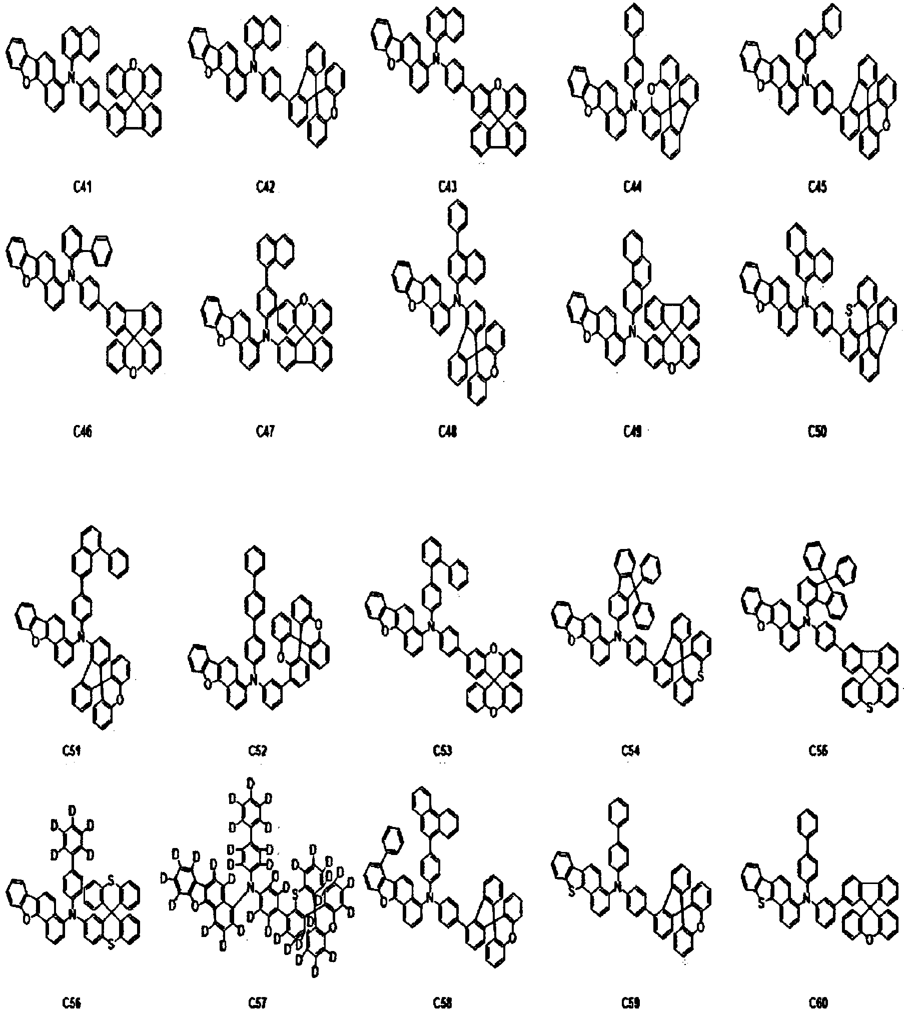

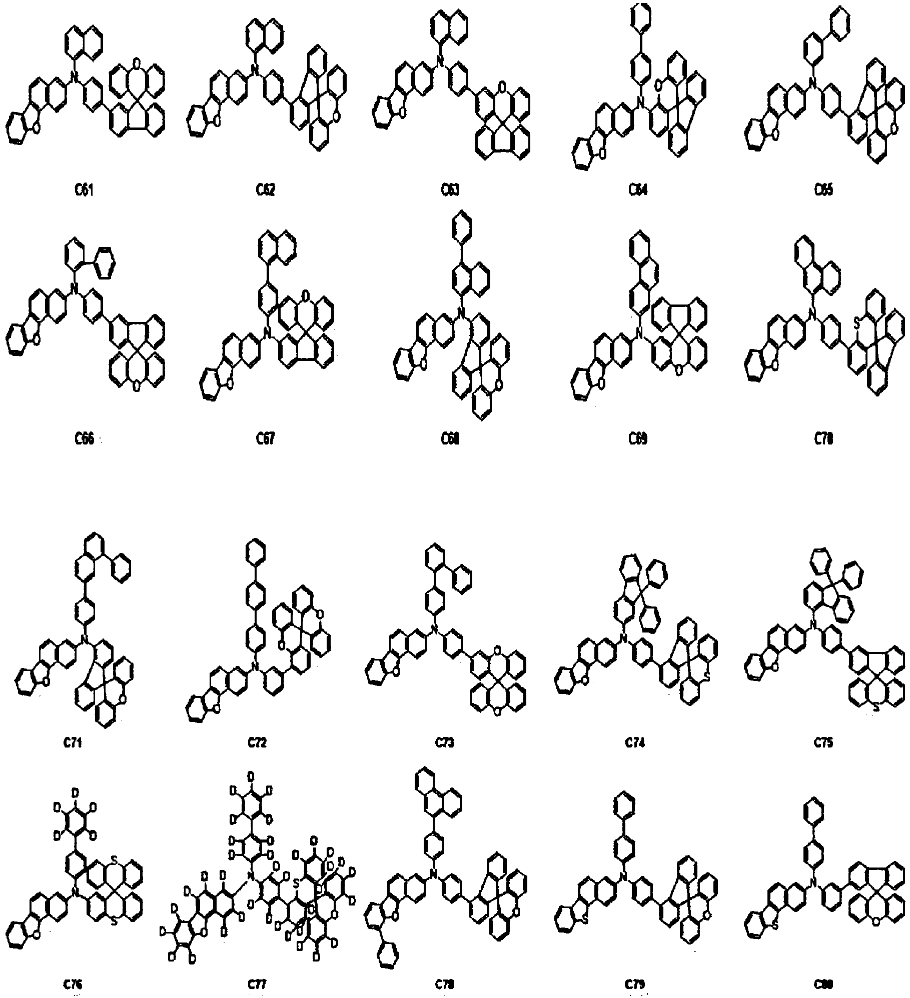

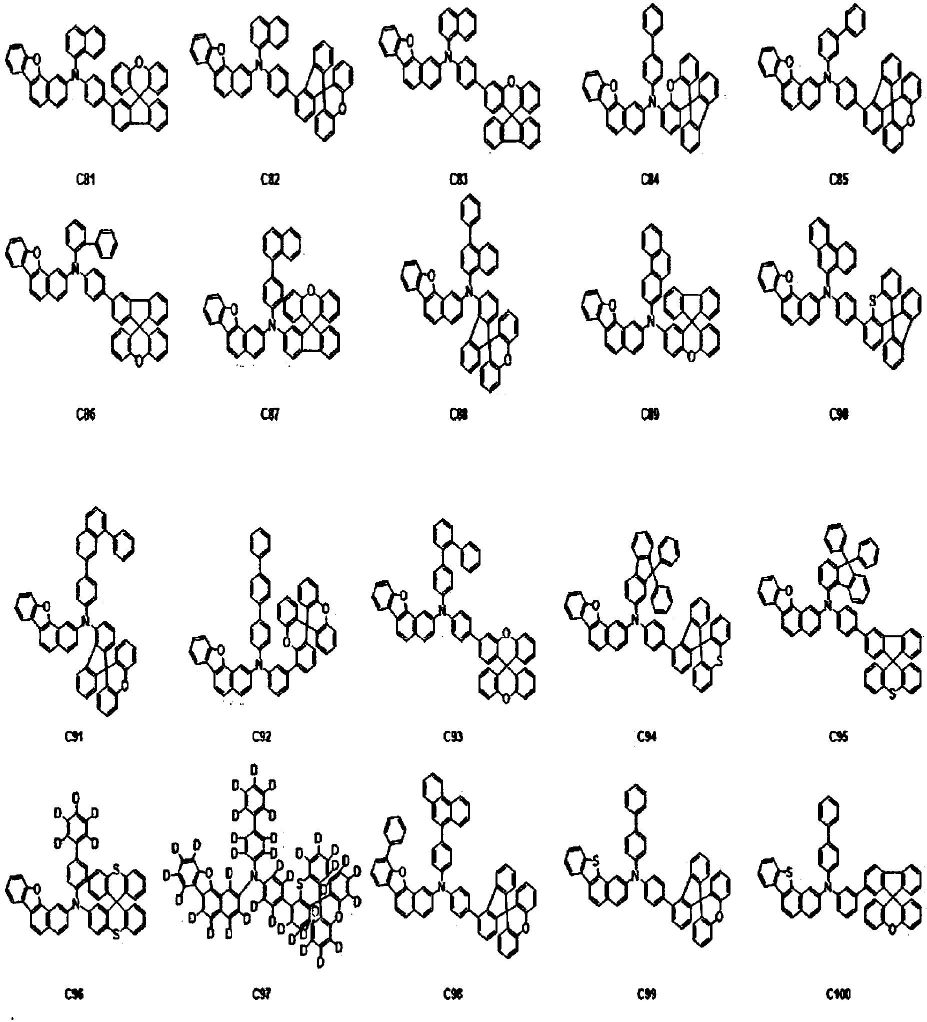

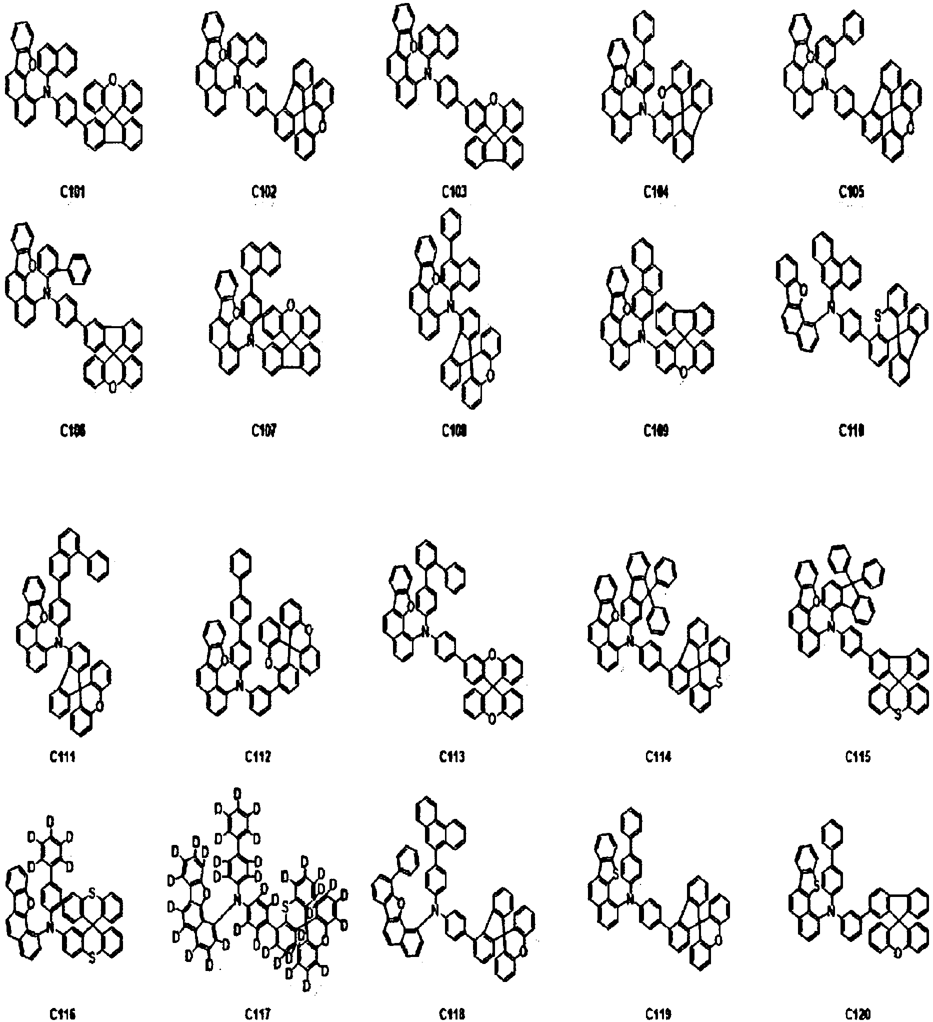

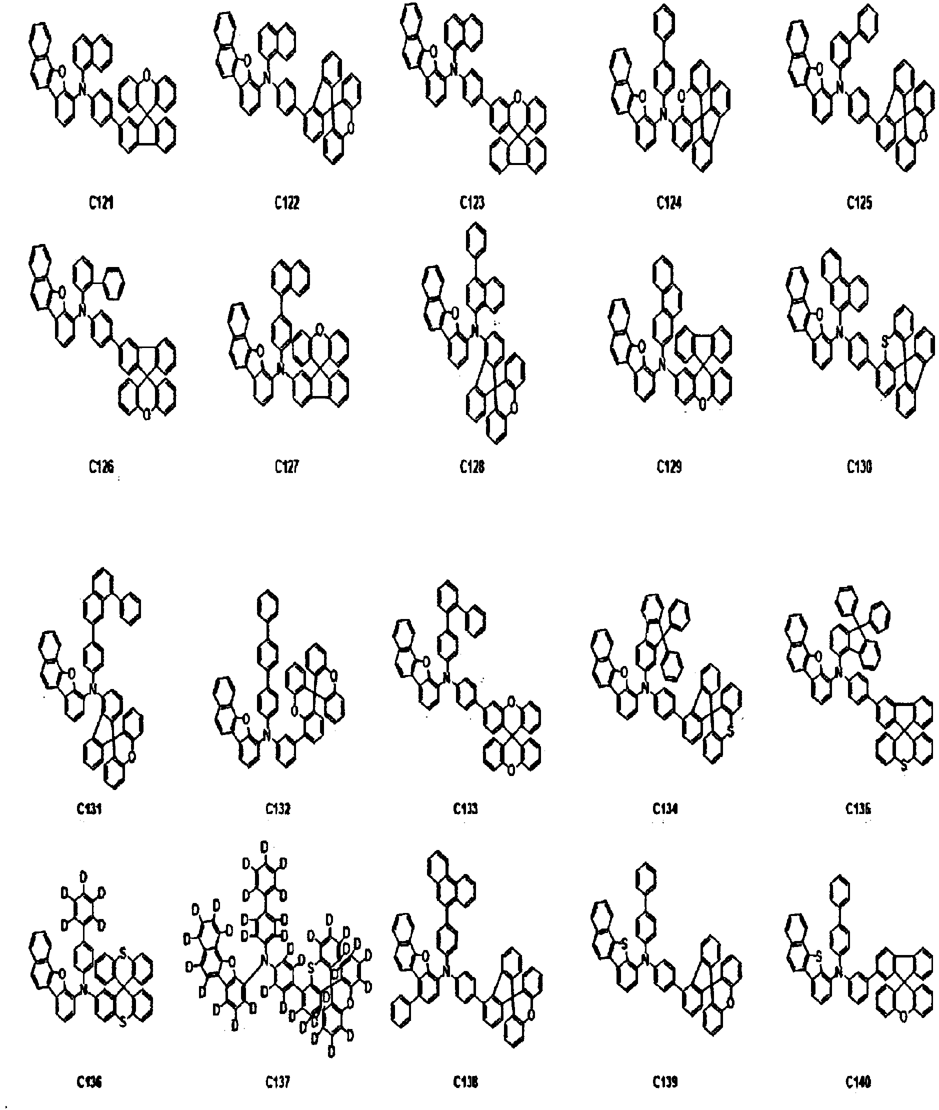

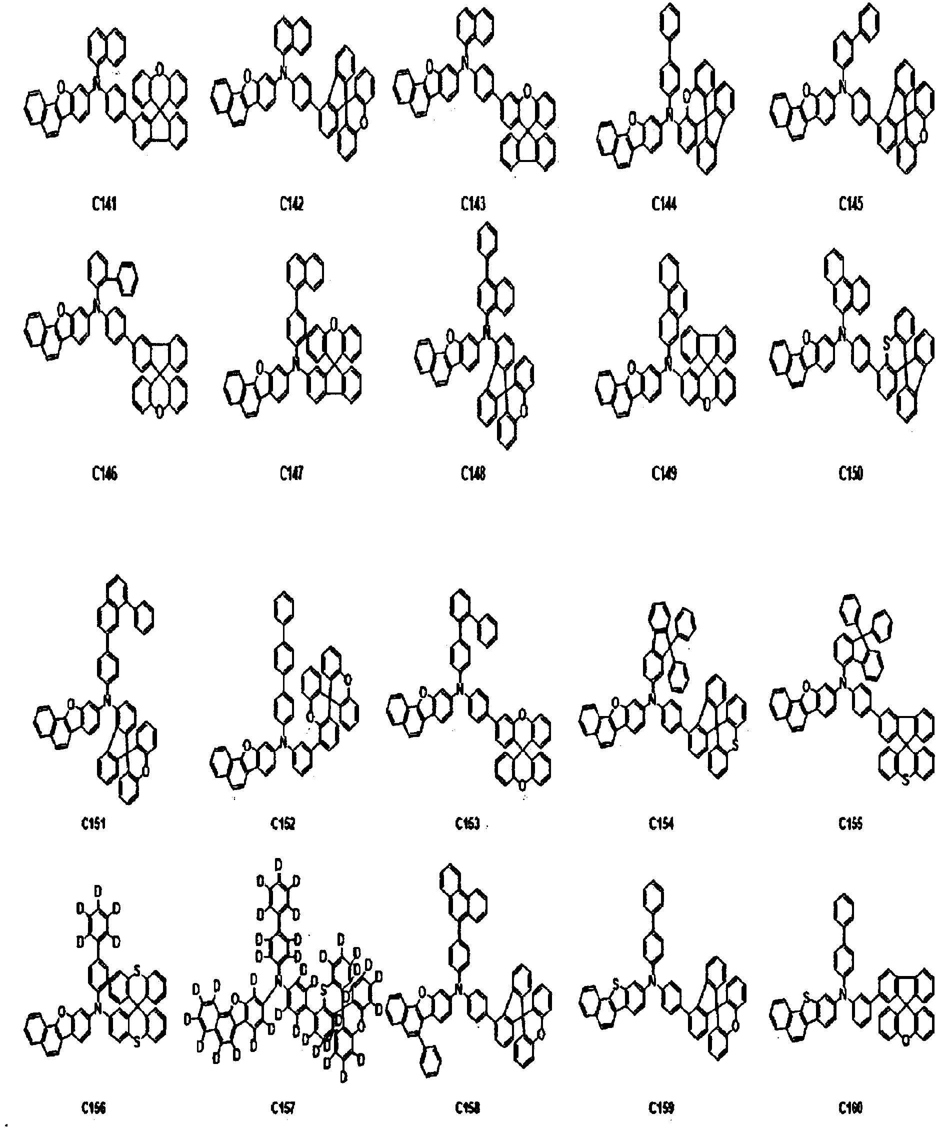

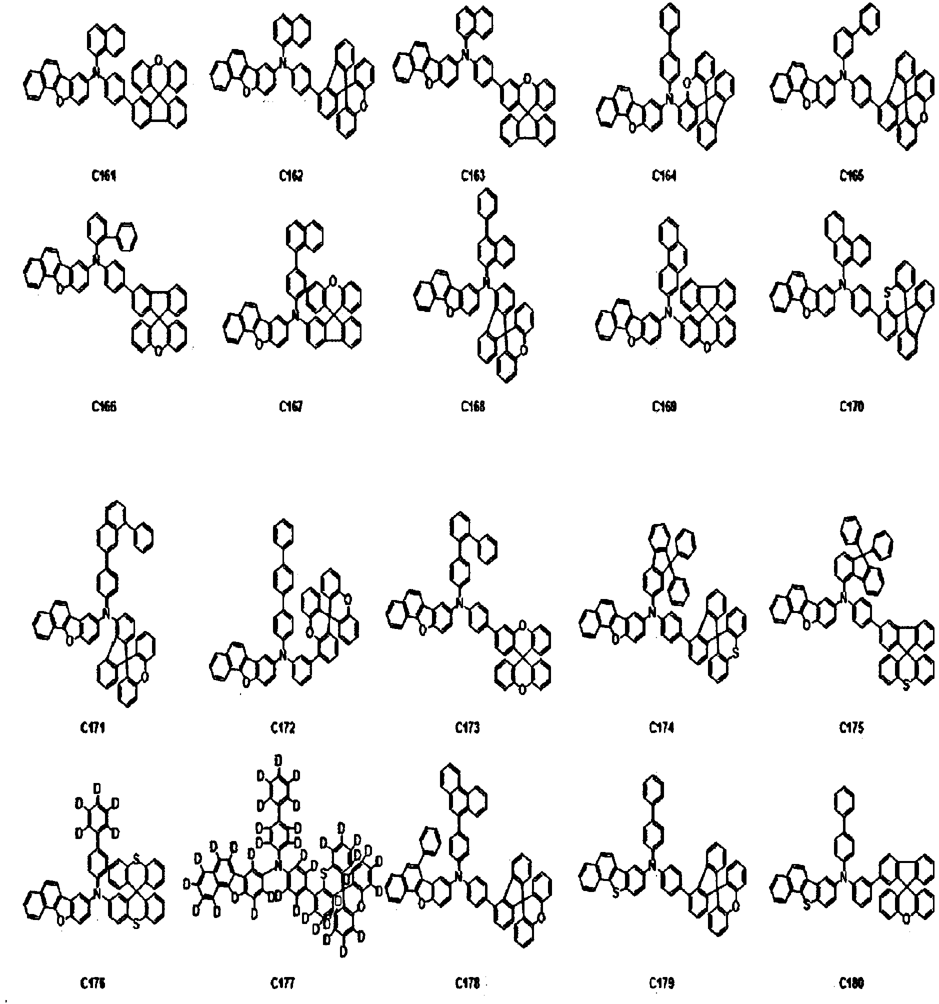

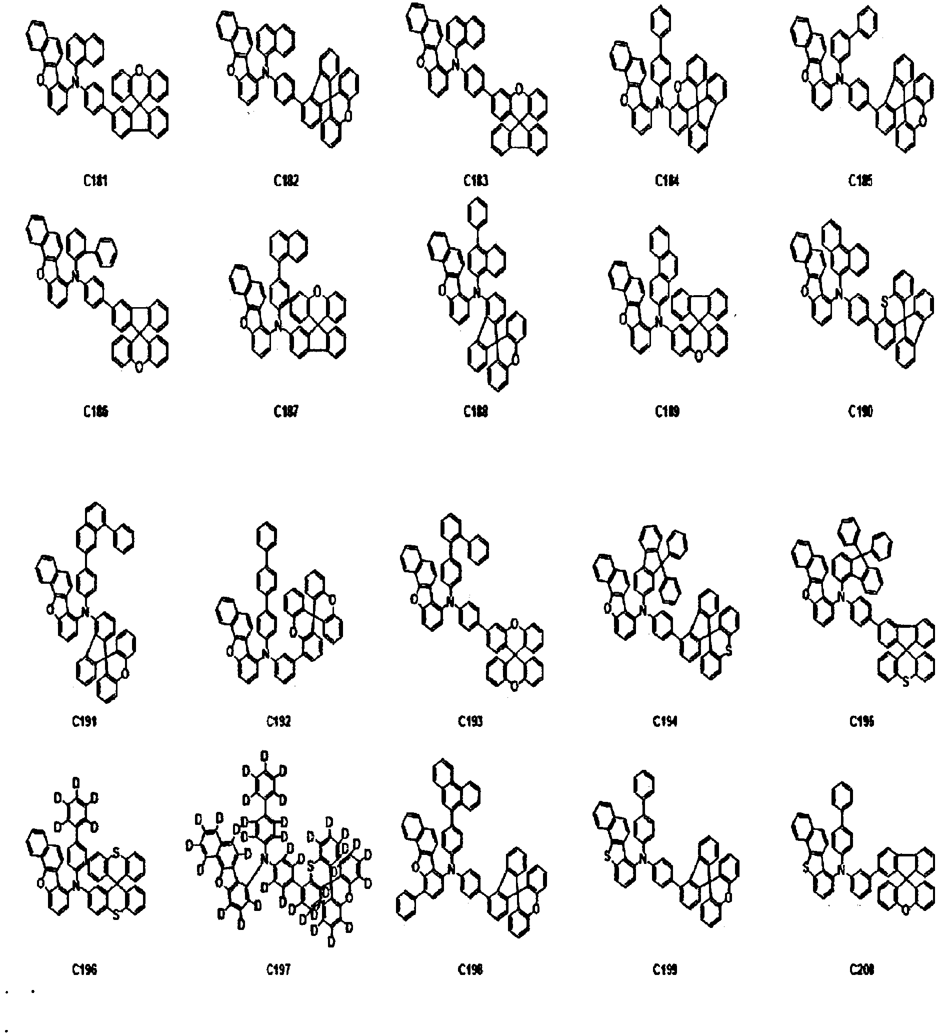

7. Dispositif électroluminescent organique, comprenant :
une première électrode ;
une région de transport de trous sur la première électrode ;
une couche d'émission sur la région de transport de trous ;
une région de transport d'électrons sur la couche d'émission ; et
une seconde électrode sur la région de transport d'électrons,
dans lequel la région de transport de trous comprend le composé polycyclique de l'une quelconque des revendications 1 à 6.

8. Dispositif électroluminescent organique selon la revendication 7, dans lequel la région de transport de trous comprend :
une couche d'injection de trous sur la première électrode ; et
une couche de transport de trous sur la couche d'injection de trous, et
la couche de transport de trous comprend le composé polycyclique de l'une quelconque des revendications 1 à 6.
